(19) Europäisches Patentamt
European Patent Office
Office européen des brevets

(11) EP 2 067 791 A1

(12) EUROPEAN PATENT APPLICATION
published in accordance with Art. 153(4) EPC

(43) Date of publication:
**10.06.2009 Bulletin 2009/24**

(21) Application number: **07829644.9**

(22) Date of filing: **04.10.2007**

(51) Int Cl.:
***C07K 16/32*** (2006.01) ***A61K 39/395*** (2006.01)
***A61P 35/00*** (2006.01) ***C07K 16/18*** (2006.01)
***C12N 5/10*** (2006.01) ***C12N 15/02*** (2006.01)
***C12P 21/08*** (2006.01)

(86) International application number:
**PCT/JP2007/069908**

(87) International publication number:
**WO 2008/044754 (17.04.2008 Gazette 2008/16)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **06.10.2006 PCT/JP2006/320429**
**04.04.2007 AR P070101453**
**06.04.2007 JP 2007100876**

(71) Applicant: **Takeda Pharmaceutical Company Limited**
**Osaka- shi, Osaka 541-0045 (JP)**

(72) Inventors:
- **SATO, Shuji**
**South San Francisco, CA 94080-4804 (US)**
- **OSHIMA, Tsutomu**
**South San Francisco, CA 94080-4804 (US)**
- **KUROKAWA, Tomofumi**
**South San Francisco, CA 94080-4804 (US)**

(74) Representative: **Jones, Nicholas Andrew et al**
**Withers & Rogers LLP**
**Goldings House**
**2 Hays Lane**
**London**
**SE1 2HW (GB)**

(54) **PROPHYLACTIC OR THERAPEUTIC AGENT FOR CANCER**

(57) A human monoclonal antibody against a protein comprising the same or substantially the same amino acid sequence as the amino acid sequence represented by SEQ ID NO: 1 or SEQ ID NO: 3, its partial peptide, or a salt thereof, is useful as an agent for preventing/treating cancer, etc., an apoptosis inducer of cancer cells, a growth inhibitor of cancer cells, a cytotoxic agent against cancer cells through a host defense mechanism mediated by the Fc region of an antibody, and so on.

EP 2 067 791 A1

SEQUENCE LISTING

<110> Takeda Pharmaceutical Company Limited

<120> Preventing and treating agent for cancer

<130> PCT07-0057

<150> PCT/JP2006/320429
<151> 2006-10-06
<150> JP2007-100876
<151> 2007-04-06

<160> 317

<170> PatentIn version 3.3

<210> 1
<211> 479
<212> PRT
<213> Homo sapiens

<400> 1

```
Met Ala Arg Ala Ala Ala Leu Leu Pro Ser Arg Ser Pro Pro Thr Pro
1               5                   10                  15
Leu Leu Trp Pro Leu Leu Leu Leu Leu Leu Leu Glu Thr Gly Ala Gln
            20                  25                  30
Asp Val Arg Val Gln Val Leu Pro Glu Val Arg Gly Gln Leu Gly Gly
        35                  40                  45
Thr Val Glu Leu Pro Cys His Leu Leu Pro Pro Val Pro Gly Leu Tyr
    50                  55                  60
Ile Ser Leu Val Thr Trp Gln Arg Pro Asp Ala Pro Ala Asn His Gln
65                  70                  75                  80
Asn Val Ala Ala Phe His Pro Lys Met Gly Pro Ser Phe Pro Ser Pro
                85                  90                  95
Lys Pro Gly Ser Glu Arg Leu Ser Phe Val Ser Ala Lys Gln Ser Thr
            100                 105                 110
Gly Gln Asp Thr Glu Ala Glu Leu Gln Asp Ala Thr Leu Ala Leu His
        115                 120                 125
Gly Leu Thr Val Glu Asp Glu Gly Asn Tyr Thr Cys Glu Phe Ala Thr
    130                 135                 140
Phe Pro Lys Gly Ser Val Arg Gly Met Thr Trp Leu Arg Val Ile Ala
145                 150                 155                 160
Lys Pro Lys Asn Gln Ala Glu Ala Gln Lys Val Thr Phe Ser Gln Asp
                165                 170                 175
Pro Thr Thr Val Ala Leu Cys Ile Ser Lys Glu Gly Arg Pro Pro Ala
            180                 185                 190
Arg Ile Ser Trp Leu Ser Ser Leu Asp Trp Glu Ala Lys Glu Thr Gln
        195                 200                 205
Val Ser Gly Thr Leu Ala Gly Thr Val Thr Val Thr Ser Arg Phe Thr
    210                 215                 220
Leu Val Pro Ser Gly Arg Ala Asp Gly Val Thr Val Thr Cys Lys Val
225                 230                 235                 240
Glu His Glu Ser Phe Glu Glu Pro Ala Leu Ile Pro Val Thr Leu Ser
                245                 250                 255
Val Arg Tyr Pro Pro Glu Val Ser Ile Ser Gly Tyr Asp Asp Asn Trp
            260                 265                 270
```

Tyr Leu Gly Arg Thr Asp Ala Thr Leu Ser Cys Asp Val Arg Ser Asn
275 280 285
Pro Glu Pro Thr Gly Tyr Asp Trp Ser Thr Thr Ser Gly Thr Phe Pro
290 295 300
Thr Ser Ala Val Ala Gln Gly Ser Gln Leu Val Ile His Ala Val Asp
305 310 315 320
Ser Leu Phe Asn Thr Thr Phe Val Cys Thr Val Thr Asn Ala Val Gly
325 330 335
Met Gly Arg Ala Glu Gln Val Ile Phe Val Arg Glu Thr Pro Arg Ala
340 345 350
Ser Pro Arg Asp Val Gly Pro Leu Val Trp Gly Ala Val Gly Gly Thr
355 360 365
Leu Leu Val Leu Leu Leu Leu Ala Gly Gly Ser Leu Ala Phe Ile Leu
370 375 380
Leu Arg Val Arg Arg Arg Arg Lys Ser Pro Gly Gly Ala Gly Gly Gly
385 390 395 400
Ala Ser Gly Asp Gly Gly Phe Tyr Asp Pro Lys Ala Gln Val Leu Gly
405 410 415
Asn Gly Asp Pro Val Phe Trp Thr Pro Val Val Pro Gly Pro Met Glu
420 425 430
Pro Asp Gly Lys Asp Glu Glu Glu Glu Glu Glu Glu Glu Lys Ala Glu
435 440 445
Lys Gly Leu Met Leu Pro Pro Pro Pro Ala Leu Glu Asp Asp Met Glu
450 455 460
Ser Gln Leu Asp Gly Ser Leu Ile Ser Arg Arg Ala Val Tyr Val
465 470 475

<210> 2
<211> 1437
<212> DNA
<213> Homo sapiens

<400> 2
atggcccggg ccgctgccct cctgccgtcg agatcgccgc cgacgccgct gctgtggccg 60
ctgctgctgc tgctgctcct ggaaaccgga gcccaggatg tgcgagttca agtgctaccc 120
gaggtgcgag gccagctcgg gggcaccgtg gagctgccgt gccacctgct gccacctgtt 180
cctggactgt acatctccct ggtgacctgg cagcgcccag atgcacctgc gaaccaccag 240
aatgtggccg ccttccaccc taagatgggt cccagcttcc ccagcccgaa gcctggcagc 300
gagcggctgt ccttcgtctc tgccaagcag agcactgggc aagacacaga ggcagagctc 360
caggacgcca cgctggccct ccacgggctc acggtggagg acgagggcaa ctacacttgc 420
gagtttgcca ccttccccaa ggggtccgtc cgagggatga cctggctcag agtcatagcc 480
aagcccaaga accaagctga ggcccagaag gtcacgttca gccaggaccc tacgacagtg 540
gccctctgca tctccaaaga gggccgccca cctgcccgga tctcctggct ctcatccctg 600
gactgggaag ccaaagagac tcaggtgtca gggaccctgg ccggaactgt cactgtcacc 660
agccgcttca ccttggtgcc ctcgggccga gcagatggtg tcacggtcac ctgcaaagtg 720
gagcatgaga gcttcgagga accagccctg atacctgtga ccctctctgt acgctaccct 780
cctgaagtgt ccatctccgg ctatgatgac aactggtacc tcggccgtac tgatgccacc 840
ctgagctgtg acgtccgcag caacccagag cccacgggct atgactggag cacgacctca 900
ggcaccttcc cgacctccgc agtggcccag ggctcccagc tggtcatcca cgcagtggac 960
agtctgttca ataccacctt cgtctgcaca gtcaccaatg ccgtgggcat gggccgcgct 1020
gagcaggtca tctttgtccg agaaaccccc agggcctcgc cccgagatgt gggcccgctg 1080
gtgtgggggg ccgtgggggg gacactgctg gtgctgctgc ttctggctgg ggggtccttg 1140
gccttcatcc tgctgagggt gaggaggagg aggaagagcc ctggaggagc aggaggagga 1200
gccagtggcg acgggggatt ctacgatccg aaagctcagg tgttgggaaa tggggacccc 1260
gtcttctgga caccagtagt ccctggtccc atggaaccag atggcaagga tgaggaggag 1320
gaggaggagg aagagaaggc agagaaaggc ctcatgttgc ctccaccccc agcactcgag 1380
gatgacatgg agtcccagct ggacggctcc ctcatctcac ggcgggcagt ttatgtg 1437

<210> 3
<211> 538
<212> PRT
<213> Homo sapiens

<400> 3
Met Ala Arg Ala Ala Ala Leu Leu Pro Ser Arg Ser Pro Pro Thr Pro
1               5                   10                  15
Leu Leu Trp Pro Leu Leu Leu Leu Leu Leu Leu Glu Thr Gly Ala Gln
            20                  25                  30
Asp Val Arg Val Gln Val Leu Pro Glu Val Arg Gly Gln Leu Gly Gly
        35                  40                  45
Thr Val Glu Leu Pro Cys His Leu Leu Pro Pro Val Pro Gly Leu Tyr
    50                  55                  60
Ile Ser Leu Val Thr Trp Gln Arg Pro Asp Ala Pro Ala Asn His Gln
65                  70                  75                  80
Asn Val Ala Ala Phe His Pro Lys Met Gly Pro Ser Phe Pro Ser Pro
                85                  90                  95
Lys Pro Gly Ser Glu Arg Leu Ser Phe Val Ser Ala Lys Gln Ser Thr
            100                 105                 110
Gly Gln Asp Thr Glu Ala Glu Leu Gln Asp Ala Thr Leu Ala Leu His
        115                 120                 125
Gly Leu Thr Val Glu Asp Glu Gly Asn Tyr Thr Cys Glu Phe Ala Thr
    130                 135                 140
Phe Pro Lys Gly Ser Val Arg Gly Met Thr Trp Leu Arg Val Ile Ala
145                 150                 155                 160
Lys Pro Lys Asn Gln Ala Glu Ala Gln Lys Val Thr Phe Ser Gln Asp
                165                 170                 175
Pro Thr Thr Val Ala Leu Cys Ile Ser Lys Glu Gly Arg Pro Pro Ala
            180                 185                 190
Arg Ile Ser Trp Leu Ser Ser Leu Asp Trp Glu Ala Lys Glu Thr Gln
        195                 200                 205
Val Ser Gly Thr Leu Ala Gly Thr Val Thr Val Thr Ser Arg Phe Thr
    210                 215                 220
Leu Val Pro Ser Gly Arg Ala Asp Gly Val Thr Val Thr Cys Lys Val
225                 230                 235                 240
Glu His Glu Ser Phe Glu Glu Pro Ala Leu Ile Pro Val Thr Leu Ser
                245                 250                 255
Val Arg Tyr Pro Pro Glu Val Ser Ile Ser Gly Tyr Asp Asp Asn Trp
            260                 265                 270
Tyr Leu Gly Arg Thr Asp Ala Thr Leu Ser Cys Asp Val Arg Ser Asn
        275                 280                 285
Pro Glu Pro Thr Gly Tyr Asp Trp Ser Thr Thr Ser Gly Thr Phe Pro
    290                 295                 300
Thr Ser Ala Val Ala Gln Gly Ser Gln Leu Val Ile His Ala Val Asp
305                 310                 315                 320
Ser Leu Phe Asn Thr Thr Phe Val Cys Thr Val Thr Asn Ala Val Gly
                325                 330                 335
Met Gly Arg Ala Glu Gln Val Ile Phe Val Arg Glu Thr Pro Asn Thr
            340                 345                 350
Ala Gly Ala Gly Ala Thr Gly Gly Ile Ile Gly Gly Ile Ile Ala Ala
        355                 360                 365
Ile Ile Ala Thr Ala Val Ala Ala Thr Gly Ile Leu Ile Cys Arg Gln
    370                 375                 380
Gln Arg Lys Glu Gln Thr Leu Gln Gly Ala Glu Glu Asp Glu Asp Leu
385                 390                 395                 400

Glu Gly Pro Pro Ser Tyr Lys Pro Pro Thr Pro Lys Ala Lys Leu Glu
405 410 415
Ala Gln Glu Met Pro Ser Gln Leu Phe Thr Leu Gly Ala Ser Glu His
420 425 430
Ser Pro Leu Lys Thr Pro Tyr Phe Asp Ala Gly Ala Ser Cys Thr Glu
435 440 445
Gln Glu Met Pro Arg Tyr His Glu Leu Pro Thr Leu Glu Glu Arg Ser
450 455 460
Gly Pro Leu His Pro Gly Ala Thr Ser Leu Gly Ser Pro Ile Pro Val
465 470 475 480
Pro Pro Gly Pro Pro Ala Val Glu Asp Val Ser Leu Asp Leu Glu Asp
485 490 495
Glu Glu Gly Glu Glu Glu Glu Glu Tyr Leu Asp Lys Ile Asn Pro Ile
500 505 510
Tyr Asp Ala Leu Ser Tyr Ser Ser Pro Ser Asp Ser Tyr Gln Gly Lys
515 520 525
Gly Phe Val Met Ser Arg Ala Met Tyr Val
530 535

<210> 4
<211> 1614
<212> DNA
<213> Homo sapiens

<400> 4
atggcccggg ccgctgccct cctgccgtcg agatcgccgc cgacgccgct gctgtggccg 60
ctgctgctgc tgctgctcct ggaaaccgga gcccaggatg tgcgagttca agtgctaccc 120
gaggtgcgag gccagctcgg gggcaccgtg gagctgccgt gccacctgct gccacctgtt 180
cctggactgt acatttccct ggtgacctgg cagcgcccag atgcacctgc gaaccaccag 240
aatgtggccg ccttccaccc taagatgggt cccagcttcc ccagcccgaa gcctggcagc 300
gagcggctgt ccttcgtctc tgccaagcag agcactgggc aagacacaga ggcagagctc 360
caggacgcca cgctggccct ccacgggctc acggtggagg acgagggcaa ctacacttgc 420
gagtttgcca ccttccccaa ggggtccgtc cgagggatga cctggctcag agtcatagcc 480
aagcccaaga accaagctga ggcccagaag gtcacgttca gccaggaccc tacgacagtg 540
gccctctgca tctccaaaga gggccgccca cctgcccgga tctcctggct ctcatccctg 600
gactgggaag ccaaagagac tcaggtgtca gggaccctgg ccggaactgt cactgtcacc 660
agccgcttca ccttggtgcc ctcgggccga gcagatggtg tcacggtcac ctgcaaagtg 720
gagcatgaga gcttcgagga accagccctg atacctgtga ccctctctgt acgctaccct 780
cctgaagtgt ccatctccgg ctatgatgac aactggtacc tcggccgtac tgatgccacc 840
ctgagctgtg acgtccgcag caacccagag cccacgggct atgactggag cacgacctca 900
ggcaccttcc cgacctccgc agtggcccag ggctcccagc tggtcatcca cgcagtggac 960
agtctgttca ataccacctt cgtctgcaca gtcaccaatg ccgtgggcat gggccgcgct 1020
gagcaggtca tctttgtccg agagaccccc aacacagcag gcgcaggggc cacaggcggc 1080
atcatcgggg gcatcatcgc cgccatcatt gctactgctg tggctgccac gggcatcctt 1140
atctgccggc agcagcggaa ggagcagacg ctgcaggggg cagaggagga cgaagacctg 1200
gagggacctc cctcctacaa gccaccgacc ccaaaagcga agctggaggc acaggagatg 1260
ccctcccagc tcttcactct gggggcctcg gagcacagcc cactcaagac cccctacttt 1320
gatgctggcg cctcatgcac tgagcaggaa atgcctcgat accatgagct gcccaccttg 1380
gaagaacggt caggaccctt gcaccctgga gccacaagcc tggggtcccc catcccggtg 1440
cctccagggc cacctgctgt ggaagacgtt tccctggatc tagaggatga ggaggggggag 1500
gaggaggaag agtatctgga caagatcaac cccatctatg atgctctgtc ctatagcagc 1560
ccctctgatt cctaccaggg caaaggcttt gtcatgtccc gggccatgta tgtg 1614

<210> 5
<211> 549
<212> PRT
<213> Homo sapiens

<400> 5

Met Ala Arg Thr Leu Arg Pro Ser Pro Leu Cys Pro Gly Gly Gly Lys
5 10 15
Ala Gln Leu Ser Ser Ala Ser Leu Leu Gly Ala Gly Leu Leu Leu Gln
20 25 30
Pro Pro Thr Pro Pro Pro Leu Leu Leu Leu Leu Phe Pro Leu Leu Leu
35 40 45
Phe Ser Arg Leu Cys Gly Ala Leu Ala Gly Pro Ile Ile Val Glu Pro
50 55 60
His Val Thr Ala Val Trp Gly Lys Asn Val Ser Leu Lys Cys Leu Ile
65 70 75 80
Glu Val Asn Glu Thr Ile Thr Gln Ile Ser Trp Glu Lys Ile His Gly
85 90 95
Lys Ser Ser Gln Thr Val Ala Val His His Pro Gln Tyr Gly Phe Ser
100 105 110
Val Gln Gly Glu Tyr Gln Gly Arg Val Leu Phe Lys Asn Tyr Ser Leu
115 120 125
Asn Asp Ala Thr Ile Thr Leu His Asn Ile Gly Phe Ser Asp Ser Gly
130 135 140
Lys Tyr Ile Cys Lys Ala Val Thr Phe Pro Leu Gly Asn Ala Gln Ser
145 150 155 160
Ser Thr Thr Val Thr Val Leu Val Glu Pro Thr Val Ser Leu Ile Lys
165 170 175
Gly Pro Asp Ser Leu Ile Asp Gly Gly Asn Glu Thr Val Ala Ala Ile
180 185 190
Cys Ile Ala Ala Thr Gly Lys Pro Val Ala His Ile Asp Trp Glu Gly
195 200 205
Asp Leu Gly Glu Met Glu Ser Thr Thr Thr Ser Phe Pro Asn Glu Thr
210 215 220
Ala Thr Ile Ile Ser Gln Tyr Lys Leu Phe Pro Thr Arg Phe Ala Arg
225 230 235 240
Gly Arg Arg Ile Thr Cys Val Val Lys His Pro Ala Leu Glu Lys Asp
245 250 255
Ile Arg Tyr Ser Phe Ile Leu Asp Ile Gln Tyr Ala Pro Glu Val Ser
260 265 270
Val Thr Gly Tyr Asp Gly Asn Trp Phe Val Gly Arg Lys Gly Val Asn
275 280 285
Leu Lys Cys Asn Ala Asp Ala Asn Pro Pro Pro Phe Lys Ser Val Trp
290 295 300
Ser Arg Leu Asp Gly Gln Trp Pro Asp Gly Leu Leu Ala Ser Asp Asn
305 310 315 320
Thr Leu His Phe Val His Pro Leu Thr Phe Asn Tyr Ser Gly Val Tyr
325 330 335
Ile Cys Lys Val Thr Asn Ser Leu Gly Gln Arg Ser Asp Gln Lys Val
340 345 350
Ile Tyr Ile Ser Asp Pro Pro Thr Thr Thr Leu Gln Pro Thr Ile
355 360 365
Gln Trp His Pro Ser Thr Ala Asp Ile Glu Asp Leu Ala Thr Glu Pro
370 375 380
Lys Lys Leu Pro Phe Pro Leu Ser Thr Leu Ala Thr Ile Lys Asp Asp
385 390 395 400
Thr Ile Ala Thr Ile Ile Ala Ser Val Val Gly Gly Ala Leu Phe Ile
405 410 415
Val Leu Val Ser Val Leu Ala Gly Ile Phe Cys Tyr Arg Arg Arg Arg
420 425 430
Thr Phe Arg Gly Asp Tyr Phe Ala Lys Asn Tyr Ile Pro Pro Ser Asp

```
            435                 440                 445
Met Gln Lys Glu Ser Gln Ile Asp Val Leu Gln Gln Asp Glu Leu Asp
        450                 455                 460
Ser Tyr Pro Asp Ser Val Lys Lys Glu Asn Lys Asn Pro Val Asn Asn
465                 470                 475                 480
Leu Ile Arg Lys Asp Tyr Leu Glu Glu Pro Glu Lys Thr Gln Trp Asn
                485                 490                 495
Asn Val Glu Asn Leu Asn Arg Phe Glu Arg Pro Met Asp Tyr Tyr Glu
            500                 505                 510
Asp Leu Lys Met Gly Met Lys Phe Val Ser Asp Glu His Tyr Asp Glu
        515                 520                 525
Asn Glu Asp Asp Leu Val Ser His Val Asp Gly Ser Val Ile Ser Arg
    530                 535                 540
Arg Glu Trp Tyr Val
545
```

<210> 6
<211> 1647
<212> DNA
<213> Homo sapiens

<400> 6

```
atggcgcgga ccctgcggcc gtccccgctg tgtcctggag gcggcaaagc acaactttcc    60
tccgcttctc tcctcggagc cgggctcctg ctgcagcccc cgacgccacc tccgctgctg   120
ctgctgctct tcccgctgct gctcttctcc aggctctgtg gtgccttagc tggaccaatt   180
attgtggagc cacatgtcac agcagtatgg ggaaagaatg tttcattaaa gtgtttaatt   240
gaagtaaatg aaaccataac acagatttca tgggagaaga tacatggcaa aagttcacag   300
actgttgcag ttcaccatcc ccaatatgga ttctctgttc aaggagaata tcagggaaga   360
gtcttgttta aaaattactc acttaatgat gcaacaatta ctctgcataa cataggattc   420
tctgattctg gaaaatacat ctgcaaagct gttacattcc cgcttggaaa tgcccagtcc   480
tctacaactg taactgtgtt agttgaaccc actgtgagcc tgataaaagg gccagattct   540
ttaattgatg gaggaaatga aacagtagca gccatttgca tcgcagccac tggaaaaccc   600
gttgcacata ttgactggga aggtgatctt ggtgaaatgg aatccactac aacttctttt   660
ccaaatgaaa cggcaacgat tatcagccag tacaagctat ttccaaccag atttgctaga   720
ggaaggcgaa ttacttgtgt tgtaaaacat ccagccttgg aaaaggacat ccgatactct   780
ttcatattag acatacagta tgctcctgaa gtttcggtaa caggatatga tggaaattgg   840
tttgtaggaa gaaaaggtgt taatctcaaa tgtaatgctg atgcaaatcc accacccttc   900
aaatctgtgt ggagcaggtt ggatggacaa tggcctgatg gtttattggc ttcagacaat   960
actcttcatt ttgtccatcc attgactttc aattattctg gtgtttatat ctgtaaagtg  1020
accaattccc ttggtcaaag aagtgaccaa aaagtcatct acatttcaga tcctcctact  1080
actaccaccc ttcagcctac aattcagtgg catccctcaa ctgctgacat cgaggatcta  1140
gcaacagaac ctaaaaaatt gcccttccca ttgtcaactt tggcaacaat taaggatgac  1200
acaattgcca cgatcattgc tagtgtagtg ggtggggctc tcttcatagt acttgtaagt  1260
gttttggctg gaatattctg ctataggaga agacggacgt ttcgtggaga ctactttgcc  1320
aagaactaca ttccaccatc agatatgcaa aaagaatcac aaatagatgt tcttcaacaa  1380
gatgagcttg attcttaccc agacagtgta aaaaaagaaa acaaaaatcc agtgaacaat  1440
ctaatacgta aagactattt agaagagcct gaaaaaactc agtggaacaa tgtagaaaat  1500
ctcaataggt ttgaaagacc aatggattat tatgaagatc taaaaatggg aatgaagttt  1560
gtcagtgatg aacattatga tgaaaacgaa gatgacttag tttcacatgt agatggttcc  1620
gtaatttcca ggagggagtg gtatgtt                                      1647
```

<210> 7
<211> 20
<212> DNA
<213> Artificial Sequence

<220>

<223> Antisense oligonucleotide 1

<400> 7
tcccaacacc tgagctttcg 20

<210> 8
<211> 20
<212> DNA
<213> Artificial Sequence

<220>
<223> Control oligonucleotide 1

<400> 8
gctttcgagt ccacaaccct 20

<210> 9
<211> 22
<212> DNA
<213> Artificial Sequence

<220>
<223> Primer

<400> 9
ggtcatcttt gtccgagaaa cc 22

<210> 10
<211> 22
<212> DNA
<213> Artificial Sequence

<220>
<223> Primer

<400> 10
tgagctttcg gatcgtagaa tc 22

<210> 11
<211> 18
<212> DNA
<213> Artificial Sequence

<220>
<223> TaqMan Probe 1

<400> 11
ccgagatgtg ggcccgct 18

<210> 12
<211> 22
<212> DNA
<213> Artificial Sequence

<220>
<223> Primer

<400> 12
cccactcaag accccctact tt 22

<210> 13
<211> 22
<212> DNA
<213> Artificial Sequence

<220>
<223> Primer

<400> 13
gctcatggta tcgaggcatt tc 22

<210> 14
<211> 23
<212> DNA
<213> Artificial Sequence

<220>
<223> TaqMan Probe 2

<400> 14
atgctggcgc ctcatgcact gag 23

<210> 15
<211> 25
<212> DNA
<213> Artificial Sequence

<220>
<223> Primer

<400> 15
aattgaattc atggcccggg ccgct 25

<210> 16
<211> 32
<212> DNA
<213> Artificial Sequence

<220>
<223> Primer

<400> 16
aattgatatc tcacacataa actgcccgcc gt 32

<210> 17
<211> 29
<212> DNA
<213> Artificial Sequence

<220>
<223> Primer

<400> 17
gatatctcac acatacatgg cccgggaca 29

<210> 18
<211> 34
<212> DNA
<213> Artificial Sequence

<220>
<223> Primer

<400> 18
attgatatct cacacataca tggcccggga catg 34

<210> 19
<211> 19
<212> RNA
<213> Artificial Sequence

<220>
<223> siRNA-1

<400> 19
acuacacuug cgaguuugc 19

<210> 20
<211> 19
<212> RNA
<213> Artificial Sequence

<220>
<223> siRNA-1

<400> 20
gcaaacucgc aaguguagu 19

<210> 21
<211> 19
<212> RNA
<213> Artificial Sequence

<220>
<223> siRNA-2

<400> 21
aaaguggagc augagagcu 19

<210> 22
<211> 19
<212> RNA
<213> Artificial Sequence

<220>
<223> siRNA-2

<400> 22
agcucucaug cuccacuuu 19

<210> 23
<211> 19
<212> RNA
<213> Artificial Sequence

<220>
<223> siRNA-3

<400> 23
ggucaucuuu guccgagaa 19

<210> 24
<211> 19
<212> RNA
<213> Artificial Sequence

<220>
<223> siRNA-3

<400> 24
uucucggaca aagaugacc 19

<210> 25
<211> 19
<212> RNA
<213> Artificial Sequence

<220>
<223> siRNA-4

<400> 25
gauucuacga uccgaaagc 19

<210> 26
<211> 19
<212> RNA
<213> Artificial Sequence

<220>
<223> siRNA-4

<400> 26
gcuuucggau cguagaauc 19

<210> 27
<211> 19
<212> RNA
<213> Artificial Sequence

<220>
<223> siRNA-5

<400> 27
ggaagagaag gcagagaaa 19

<210> 28
<211> 19

<212> RNA
<213> Artificial Sequence

<220>
<223> siRNA-5

<400> 28
uuucucugcc uucucuucc 19

<210> 29
<211> 25
<212> DNA
<213> Artificial Sequence

<220>
<223> Primer

<400> 29
aattgaattc atggcccggg ccgct 25

<210> 30
<211> 30
<212> DNA
<213> Artificial Sequence

<220>
<223> Primer

<400> 30
aattctcgag ggcccctgcg cctgctgtgt 30

<210> 31
<211> 371
<212> PRT
<213> Homo sapiens

<400> 31
```
Met Ala Arg Ala Ala Ala Leu Leu Pro Ser Arg Ser Pro Pro Thr Pro
                    5                   10                  15
Leu Leu Trp Pro Leu Leu Leu Leu Leu Leu Leu Glu Thr Gly Ala Gln
            20                  25                  30
Asp Val Arg Val Gln Val Leu Pro Glu Val Arg Gly Gln Leu Gly Gly
        35                  40                  45
Thr Val Glu Leu Pro Cys His Leu Leu Pro Pro Val Pro Gly Leu Tyr
    50                  55                  60
Ile Ser Leu Val Thr Trp Gln Arg Pro Asp Ala Pro Ala Asn His Gln
65                  70                  75                  80
Asn Val Ala Ala Phe His Pro Lys Met Gly Pro Ser Phe Pro Ser Pro
                85                  90                  95
Lys Pro Gly Ser Glu Arg Leu Ser Phe Val Ser Ala Lys Gln Ser Thr
            100                 105                 110
Gly Gln Asp Thr Glu Ala Glu Leu Gln Asp Ala Thr Leu Ala Leu His
        115                 120                 125
Gly Leu Thr Val Glu Asp Glu Gly Asn Tyr Thr Cys Glu Phe Ala Thr
    130                 135                 140
Phe Pro Lys Gly Ser Val Arg Gly Met Thr Trp Leu Arg Val Ile Ala
145                 150                 155                 160
```

Lys Pro Lys Asn Gln Ala Glu Ala Gln Lys Val Thr Phe Ser Gln Asp
165 170 175
Pro Thr Thr Val Ala Leu Cys Ile Ser Lys Glu Gly Arg Pro Pro Ala
180 185 190
Arg Ile Ser Trp Leu Ser Ser Leu Asp Trp Glu Ala Lys Glu Thr Gln
195 200 205
Val Ser Gly Thr Leu Ala Gly Thr Val Thr Val Thr Ser Arg Phe Thr
210 215 220
Leu Val Pro Ser Gly Arg Ala Asp Gly Val Thr Val Thr Cys Lys Val
225 230 235 240
Glu His Glu Ser Phe Glu Glu Pro Ala Leu Ile Pro Val Thr Leu Ser
245 250 255
Val Arg Tyr Pro Pro Glu Val Ser Ile Ser Gly Tyr Asp Asp Asn Trp
260 265 270
Tyr Leu Gly Arg Thr Asp Ala Thr Leu Ser Cys Asp Val Arg Ser Asn
275 280 285
Pro Glu Pro Thr Gly Tyr Asp Trp Ser Thr Thr Ser Gly Thr Phe Pro
290 295 300
Thr Ser Ala Val Ala Gln Gly Ser Gln Leu Val Ile His Ala Val Asp
305 310 315 320
Ser Leu Phe Asn Thr Thr Phe Val Cys Thr Val Thr Asn Ala Val Gly
325 330 335
Met Gly Arg Ala Glu Gln Val Ile Phe Val Arg Glu Thr Pro Asn Thr
340 345 350
Ala Gly Ala Gly Ala Thr Gly Gly Ile Leu Glu Asp Tyr Lys Asp Asp
355 360 365
Asp Asp Lys
370

<210> 32
<211> 1113
<212> DNA
<213> Homo sapiens

<400> 32
atggcccggg ccgctgccct cctgccgtcg agatcgccgc cgacgccgct gctgtggccg 60
ctgctgctgc tgctgctcct ggaaaccgga gcccaggatg tgcgagttca agtgctaccc 120
gaggtgcgag gccagctcgg gggcaccgtg gagctgccgt gccacctgct gccacctgtt 180
cctggactgt acatttccct ggtgacctgg cagcgcccag atgcacctgc gaaccaccag 240
aatgtggccg ccttccaccc taagatgggt cccagcttcc ccagcccgaa gcctggcagc 300
gagcggctgt ccttcgtctc tgccaagcag agcactgggc aagacacaga ggcagagctc 360
caggacgcca cgctggccct ccacgggctc acggtggagg acgagggcaa ctacacttgc 420
gagtttgcca ccttccccaa ggggtccgtc cgagggatga cctggctcag agtcatagcc 480
aagcccaaga accaagctga ggcccagaag gtcacgttca gccaggaccc tacgacagtg 540
gccctctgca tctccaaaga gggccgccca cctgcccgga tctcctggct ctcatccctg 600
gactgggaag ccaaagagac tcaggtgtca gggaccctgg ccggaactgt cactgtcacc 660
agccgcttca ccttggtgcc ctcgggccga gcagatggtg tcacggtcac ctgcaaagtg 720
gagcatgaga gcttcgagga accagccctg atacctgtga ccctctctgt acgctaccct 780
cctgaagtgt ccatctccgg ctatgatgac aactggtacc tcggccgtac tgatgccacc 840
ctgagctgtg acgtccgcag caacccagag cccacgggct atgactggag cacgacctca 900
ggcaccttcc cgacctccgc agtggcccag ggctcccagc tggtcatcca cgcagtggac 960
agtctgttca ataccacctt cgtctgcaca gtcaccaatg ccgtgggcat gggccgcgct 1020
gagcaggtca tctttgtccg agagaccccc aacacagcag gcgcaggggc cacaggcggc 1080
atcctcgagg attacaagga tgacgacgat aag 1113

<210> 33
<211> 30

<212> DNA
<213> Artificial Sequence

<220>
<223> Primer

<400> 33
aattgaattc cccaaatctt gtgacaaaac 30

<210> 34
<211> 29
<212> DNA
<213> Artificial Sequence

<220>
<223> Primer

<400> 34
aattctcgag tcatttaccc ggagacagg 29

<210> 35
<211> 25
<212> DNA
<213> Artificial Sequence

<220>
<223> Primer

<400> 35
aattaagctt atggcccggg ccgct 25

<210> 36
<211> 24
<212> DNA
<213> Artificial Sequence

<220>
<223> Primer

<400> 36
aattgaattc gggggtttct cgga 24

<210> 37
<211> 583
<212> PRT
<213> Homo sapiens

<400> 37
Met Ala Arg Ala Ala Ala Leu Leu Pro Ser Arg Ser Pro Pro Thr Pro
5 10 15
Leu Leu Trp Pro Leu Leu Leu Leu Leu Leu Leu Glu Thr Gly Ala Gln
20 25 30
Asp Val Arg Val Gln Val Leu Pro Glu Val Arg Gly Gln Leu Gly Gly

35 40 45
Thr Val Glu Leu Pro Cys His Leu Leu Pro Pro Val Pro Gly Leu Tyr
50 55 60
Ile Ser Leu Val Thr Trp Gln Arg Pro Asp Ala Pro Ala Asn His Gln
65 70 75 80
Asn Val Ala Ala Phe His Pro Lys Met Gly Pro Ser Phe Pro Ser Pro
85 90 95
Lys Pro Gly Ser Glu Arg Leu Ser Phe Val Ser Ala Lys Gln Ser Thr
100 105 110
Gly Gln Asp Thr Glu Ala Glu Leu Gln Asp Ala Thr Leu Ala Leu His
115 120 125
Gly Leu Thr Val Glu Asp Glu Gly Asn Tyr Thr Cys Glu Phe Ala Thr
130 135 140
Phe Pro Lys Gly Ser Val Arg Gly Met Thr Trp Leu Arg Val Ile Ala
145 150 155 160
Lys Pro Lys Asn Gln Ala Glu Ala Gln Lys Val Thr Phe Ser Gln Asp
165 170 175
Pro Thr Thr Val Ala Leu Cys Ile Ser Lys Glu Gly Arg Pro Pro Ala
180 185 190
Arg Ile Ser Trp Leu Ser Ser Leu Asp Trp Glu Ala Lys Glu Thr Gln
195 200 205
Val Ser Gly Thr Leu Ala Gly Thr Val Thr Val Thr Ser Arg Phe Thr
210 215 220
Leu Val Pro Ser Gly Arg Ala Asp Gly Val Thr Val Thr Cys Lys Val
225 230 235 240
Glu His Glu Ser Phe Glu Glu Pro Ala Leu Ile Pro Val Thr Leu Ser
245 250 255
Val Arg Tyr Pro Pro Glu Val Ser Ile Ser Gly Tyr Asp Asp Asn Trp
260 265 270
Tyr Leu Gly Arg Thr Asp Ala Thr Leu Ser Cys Asp Val Arg Ser Asn
275 280 285
Pro Glu Pro Thr Gly Tyr Asp Trp Ser Thr Thr Ser Gly Thr Phe Pro
290 295 300
Thr Ser Ala Val Ala Gln Gly Ser Gln Leu Val Ile His Ala Val Asp
305 310 315 320
Ser Leu Phe Asn Thr Thr Phe Val Cys Thr Val Thr Asn Ala Val Gly
325 330 335
Met Gly Arg Ala Glu Gln Val Ile Phe Val Arg Glu Thr Pro Glu Phe
340 345 350
Pro Lys Ser Cys Asp Lys Thr His Thr Cys Pro Pro Cys Pro Ala Pro
355 360 365
Glu Leu Leu Gly Gly Pro Ser Val Phe Leu Phe Pro Pro Lys Pro Lys
370 375 380
Asp Thr Leu Met Ile Ser Arg Thr Pro Glu Val Thr Cys Val Val Val
385 390 395 400
Asp Val Ser His Glu Asp Pro Glu Val Lys Phe Asn Trp Tyr Val Asp
405 410 415
Gly Val Glu Val His Asn Ala Lys Thr Lys Pro Arg Glu Glu Gln Tyr
420 425 430
Asn Ser Thr Tyr Arg Val Val Ser Val Leu Thr Val Leu His Gln Asp
435 440 445
Trp Leu Asn Gly Lys Glu Tyr Lys Cys Lys Val Ser Asn Lys Ala Leu
450 455 460
Pro Ala Pro Ile Glu Lys Thr Ile Ser Lys Ala Lys Gly Gln Pro Arg
465 470 475 480
Glu Pro Gln Val Tyr Thr Leu Pro Pro Ser Arg Asp Glu Leu Thr Lys
485 490 495

Asn Gln Val Ser Leu Thr Cys Leu Val Lys Gly Phe Tyr Pro Ser Asp
500 505 510
Ile Ala Val Glu Trp Glu Ser Asn Gly Gln Pro Glu Asn Asn Tyr Lys
515 520 525
Thr Thr Pro Pro Val Leu Asp Ser Asp Gly Ser Phe Phe Leu Tyr Ser
530 535 540
Lys Leu Thr Val Asp Lys Ser Arg Trp Gln Gln Gly Asn Val Phe Ser
545 550 555 560
Cys Ser Val Met His Glu Ala Leu His Asn His Tyr Thr Gln Lys Ser
565 570 575
Leu Ser Leu Ser Pro Gly Lys
580

<210> 38
<211> 1749
<212> DNA
<213> Homo sapiens

<400> 38
atggcccggg ccgctgccct cctgccgtcg agatcgccgc cgacgccgct gctgtggccg 60
ctgctgctgc tgctgctcct ggaaaccgga gcccaggatg tgcgagttca agtgctaccc 120
gaggtgcgag gccagctcgg gggcaccgtg gagctgccgt gccacctgct gccacctgtt 180
cctggactgt acatttccct ggtgacctgg cagcgcccag atgcacctgc gaaccaccag 240
aatgtggccg ccttccaccc taagatgggt cccagcttcc ccagcccgaa gcctggcagc 300
gagcggctgt ccttcgtctc tgccaagcag agcactgggc aagacacaga ggcagagctc 360
caggacgcca cgctggccct ccacgggctc acggtggagg acgagggcaa ctacacttgc 420
gagtttgcca ccttccccaa ggggtccgtc cgagggatga cctggctcag agtcatagcc 480
aagcccaaga accaagctga ggcccagaag gtcacgttca gccaggaccc tacgacagtg 540
gccctctgca tctccaaaga gggccgccca cctgcccgga tctcctggct ctcatccctg 600
gactgggaag ccaaagagac tcaggtgtca gggaccctgg ccggaactgt cactgtcacc 660
agccgcttca ccttggtgcc ctcgggccga gcagatggtg tcacggtcac ctgcaaagtg 720
gagcatgaga gcttcgagga accagccctg atacctgtga ccctctctgt acgctaccct 780
cctgaagtgt ccatctccgg ctatgatgac aactggtacc tcggccgtac tgatgccacc 840
ctgagctgtg acgtccgcag caacccagag cccacgggct atgactggag cacgacctca 900
ggcaccttcc cgacctccgc agtggcccag ggctcccagc tggtcatcca cgcagtggac 960
agtctgttca ataccacctt cgtctgcaca gtcaccaatg ccgtgggcat gggccgcgct 1020
gagcaggtca tctttgtccg agagaccccc gaattcccca aatcttgtga caaaactcac 1080
acatgcccac cgtgcccagc acctgaactc ctggggggac cgtcagtctt cctcttcccc 1140
ccaaaaccca aggacaccct catgatctcc cggacccctg aggtcacatg cgtggtggtg 1200
gacgtgagcc acgaagaccc tgaggtcaag ttcaactggt acgtggacgg cgtggaggtg 1260
cataatgcca agacaaagcc gcgggaggag cagtacaaca gcacgtaccg tgtggtcagc 1320
gtcctcaccg tcctgcacca ggactggctg aatggcaagg agtacaagtg caaggtctcc 1380
aacaaagccc tcccagcccc catcgagaaa accatctcca aagccaaagg gcagccccga 1440
gaaccacagg tgtacaccct gcccccatcc cgggatgagc tgaccaagaa ccaggtcagc 1500
ctgacctgcc tggtcaaagg cttctatccc agcgacatcg ccgtggagtg ggagagcaat 1560
gggcagccgg agaacaacta caagaccacg cctcccgtgc tggactccga cggctccttc 1620
ttcctctaca gcaagctcac cgtggacaag agcaggtggc agcaggggaa cgtcttctca 1680
tgctccgtga tgcatgaggc tctgcacaac cactacacgc agaagagcct ctccctgtct 1740
ccgggtaaa 1749

<210> 39
<211> 15
<212> PRT
<213> Artificial Sequence

<220>
<223> Peptide 1 used in Example 16

<400> 39
Cys Lys Met Gly Pro Ser Phe Pro Ser Pro Lys Pro Gly Ser Glu
5 10 15

<210> 40
<211> 15
<212> PRT
<213> Artificial Sequence

<220>
<223> Peptide 2 used in Example 16

<400> 40
Arg Glu Thr Pro Arg Ala Ser Pro Arg Asp Val Gly Pro Leu Cys
5 10 15

<210> 41
<211> 15
<212> PRT
<213> Artificial Sequence

<220>
<223> Peptide 3 used in Example 16

<400> 41
Cys Thr Leu Gly Ala Ser Glu His Ser Pro Leu Lys Thr Pro Tyr
5 10 15

<210> 42
<211> 20
<212> DNA
<213> Artificial Sequence

<220>
<223> Primer

<400> 42
gcccactcaa gaccccctac 20

<210> 43
<211> 19
<212> DNA
<213> Artificial Sequence

<220>
<223> Primer

<400> 43
tcgaggcatt tcctgctca 19

<210> 44
<211> 21
<212> DNA
<213> Artificial Sequence

<220>
<223> Primer

<400> 44
ttgatgctgg cgcctcatgc a 21

<210> 45
<211> 28
<212> DNA
<213> Artificial Sequence

<220>
<223> Primer

<400> 45
aattgaattc cccagagggc ccacaatc 28

<210> 46
<211> 27
<212> DNA
<213> Artificial Sequence

<220>
<223> Primer

<400> 46
aattctcgag tcatttaccc ggagtcc 27

<210> 47
<211> 29
<212> DNA
<213> Artificial Sequence

<220>
<223> Primer

<400> 47
agttaagctt atggcgcgga ccctgcggc 29

<210> 48
<211> 26
<212> DNA
<213> Artificial Sequence

<220>
<223> Primer

<400> 48
attgaattcc gtggcaattg tgtcat 26

<210> 49
<211> 637

<212> PRT
<213> Homo sapiens

<400> 49
Met Ala Arg Thr Leu Arg Pro Ser Pro Leu Cys Pro Gly Gly Gly Lys
5 10 15
Ala Gln Leu Ser Ser Ala Ser Leu Leu Gly Ala Gly Leu Leu Leu Gln
20 25 30
Pro Pro Thr Pro Pro Pro Leu Leu Leu Leu Leu Phe Pro Leu Leu Leu
35 40 45
Phe Ser Arg Leu Cys Gly Ala Leu Ala Gly Pro Ile Ile Val Glu Pro
50 55 60
His Val Thr Ala Val Trp Gly Lys Asn Val Ser Leu Lys Cys Leu Ile
65 70 75 80
Glu Val Asn Glu Thr Ile Thr Gln Ile Ser Trp Glu Lys Ile His Gly
85 90 95
Lys Ser Ser Gln Thr Val Ala Val His His Pro Gln Tyr Gly Phe Ser
100 105 110
Val Gln Gly Glu Tyr Gln Gly Arg Val Leu Phe Lys Asn Tyr Ser Leu
115 120 125
Asn Asp Ala Thr Ile Thr Leu His Asn Ile Gly Phe Ser Asp Ser Gly
130 135 140
Lys Tyr Ile Cys Lys Ala Val Thr Phe Pro Leu Gly Asn Ala Gln Ser
145 150 155 160
Ser Thr Thr Val Thr Val Leu Val Glu Pro Thr Val Ser Leu Ile Lys
165 170 175
Gly Pro Asp Ser Leu Ile Asp Gly Gly Asn Glu Thr Val Ala Ala Ile
180 185 190
Cys Ile Ala Ala Thr Gly Lys Pro Val Ala His Ile Asp Trp Glu Gly
195 200 205
Asp Leu Gly Glu Met Glu Ser Thr Thr Thr Ser Phe Pro Asn Glu Thr
210 215 220
Ala Thr Ile Ile Ser Gln Tyr Lys Leu Phe Pro Thr Arg Phe Ala Arg
225 230 235 240
Gly Arg Arg Ile Thr Cys Val Val Lys His Pro Ala Leu Glu Lys Asp
245 250 255
Ile Arg Tyr Ser Phe Ile Leu Asp Ile Gln Tyr Ala Pro Glu Val Ser
260 265 270
Val Thr Gly Tyr Asp Gly Asn Trp Phe Val Gly Arg Lys Gly Val Asn
275 280 285
Leu Lys Cys Asn Ala Asp Ala Asn Pro Pro Pro Phe Lys Ser Val Trp
290 295 300
Ser Arg Leu Asp Gly Gln Trp Pro Asp Gly Leu Leu Ala Ser Asp Asn
305 310 315 320
Thr Leu His Phe Val His Pro Leu Thr Phe Asn Tyr Ser Gly Val Tyr
325 330 335
Ile Cys Lys Val Thr Asn Ser Leu Gly Gln Arg Ser Asp Gln Lys Val
340 345 350
Ile Tyr Ile Ser Asp Pro Pro Thr Thr Thr Thr Leu Gln Pro Thr Ile
355 360 365
Gln Trp His Pro Ser Thr Ala Asp Ile Glu Asp Leu Ala Thr Glu Pro
370 375 380
Lys Lys Leu Pro Phe Pro Leu Ser Thr Leu Ala Thr Ile Lys Asp Asp
385 390 395 400
Thr Ile Ala Thr Glu Phe Pro Lys Ser Cys Asp Lys Thr His Thr Cys
405 410 415
Pro Pro Cys Pro Ala Pro Glu Leu Leu Gly Gly Pro Ser Val Phe Leu

```
                420                 425                 430
Phe Pro Pro Lys Pro Lys Asp Thr Leu Met Ile Ser Arg Thr Pro Glu
            435                 440                 445
Val Thr Cys Val Val Val Asp Val Ser His Glu Asp Pro Glu Val Lys
    450                 455                 460
Phe Asn Trp Tyr Val Asp Gly Val Glu Val His Asn Ala Lys Thr Lys
465                 470                 475                 480
Pro Arg Glu Glu Gln Tyr Asn Ser Thr Tyr Arg Val Val Ser Val Leu
                485                 490                 495
Thr Val Leu His Gln Asp Trp Leu Asn Gly Lys Glu Tyr Lys Cys Lys
            500                 505                 510
Val Ser Asn Lys Ala Leu Pro Ala Pro Ile Glu Lys Thr Ile Ser Lys
            515                 520                 525
Ala Lys Gly Gln Pro Arg Glu Pro Gln Val Tyr Thr Leu Pro Pro Ser
    530                 535                 540
Arg Asp Glu Leu Thr Lys Asn Gln Val Ser Leu Thr Cys Leu Val Lys
545                 550                 555                 560
Gly Phe Tyr Pro Ser Asp Ile Ala Val Glu Trp Glu Ser Asn Gly Gln
                565                 570                 575
Pro Glu Asn Asn Tyr Lys Thr Thr Pro Pro Val Leu Asp Ser Asp Gly
            580                 585                 590
Ser Phe Phe Leu Tyr Ser Lys Leu Thr Val Asp Lys Ser Arg Trp Gln
            595                 600                 605
Gln Gly Asn Val Phe Ser Cys Ser Val Met His Glu Ala Leu His Asn
    610                 615                 620
His Tyr Thr Gln Lys Ser Leu Ser Leu Ser Pro Gly Lys
625                 630                 635
```

<210> 50
<211> 1911
<212> DNA
<213> Homo sapiens

<400> 50

```
atggcgcgga ccctgcggcc gtccccgctg tgtcctggag gcggcaaagc acaactttcc   60
tccgcttctc tcctcggagc cgggctcctg ctgcagcccc cgacgccacc tccgctgctg  120
ctgctgctct tcccgctgct gctcttctcc aggctctgtg gtgccttagc tggaccaatt  180
attgtggagc cacatgtcac agcagtatgg ggaaagaatg tttcattaaa gtgtttaatt  240
gaagtaaatg aaaccataac acagatttca tgggagaaga tacatggcaa aagttcacag  300
actgttgcag ttcaccatcc ccaatatgga ttctctgttc aaggagaata tcagggaaga  360
gtcttgttta aaaattactc acttaatgat gcaacaatta ctctgcataa cataggattc  420
tctgattctg gaaaatacat ctgcaaagct gttacattcc cgcttggaaa tgcccagtcc  480
tctacaactg taactgtgtt agttgaaccc actgtgagcc tgataaaagg gccagattct  540
ttaattgatg gaggaaatga aacagtagca gccatttgca tcgcagccac tggaaaaccc  600
gttgcacata ttgactggga aggtgatctt ggtgaaatgg aatccactac aacttctttt  660
ccaaatgaaa cggcaacgat tatcagccag tacaagctat ttccaaccag atttgctaga  720
ggaaggcgaa ttacttgtgt tgtaaaacat ccagccttgg aaaaggacat ccgatactct  780
ttcatattag acatacagta tgctcctgaa gtttcggtaa caggatatga tggaaattgg  840
tttgtaggaa gaaaaggtgt taatctcaaa tgtaatgctg atgcaaatcc accacccttc  900
aaatctgtgt ggagcaggtt ggatggacaa tggcctgatg gtttattggc ttcagacaat  960
actcttcatt ttgtccatcc attgactttc aattattctg gtgtttatat ctgtaaagtg 1020
accaattccc ttggtcaaag aagtgaccaa aaagtcatct acatttcaga tcctcctact 1080
actaccaccc ttcagcctac aattcagtgg catccctcaa ctgctgacat cgaggatcta 1140
gcaacagaac ctaaaaaatt gcccttccca ttgtcaactt tggcaacaat taaggatgac 1200
acaattgcca cggaattccc caaatcttgt gacaaaactc acacatgccc accgtgccca 1260
gcacctgaac tcctgggggg accgtcagtc ttcctcttcc ccccaaaacc caaggacacc 1320
ctcatgatct cccggacccc tgaggtcaca tgcgtggtgg tggacgtgag ccacgaagac 1380
```

cctgaggtca agttcaactg gtacgtggac ggcgtggagg tgcataatgc caagacaaag 1440
ccgcgggagg agcagtacaa cagcacgtac cgtgtggtca gcgtcctcac cgtcctgcac 1500
caggactggc tgaatggcaa ggagtacaag tgcaaggtct ccaacaaagc cctcccagcc 1560
cccatcgaga aaaccatctc caaagccaaa gggcagcccc gagaaccaca ggtgtacacc 1620
ctgcccccat cccgggatga gctgaccaag aaccaggtca gcctgacctg cctggtcaaa 1680
ggcttctatc ccagcgacat cgccgtggag tgggagagca atgggcagcc ggagaacaac 1740
tacaagacca cgcctcccgt gctggactcc gacggctcct tcttcctcta cagcaagctc 1800
accgtggaca agagcaggtg gcagcagggg aacgtcttct catgctccgt gatgcatgag 1860
gctctgcaca accactacac gcagaagagc ctctccctgt ctccgggtaa a 1911

<210> 51
<211> 638
<212> PRT
<213> Homo sapiens

<400> 51
Met Ala Arg Thr Leu Arg Pro Ser Pro Leu Cys Pro Gly Gly Gly Lys
5 10 15
Ala Gln Leu Ser Ser Ala Ser Leu Leu Gly Ala Gly Leu Leu Leu Gln
20 25 30
Pro Pro Thr Pro Pro Pro Leu Leu Leu Leu Leu Phe Pro Leu Leu Leu
35 40 45
Phe Ser Arg Leu Cys Gly Ala Leu Ala Gly Pro Ile Ile Val Glu Pro
50 55 60
His Val Thr Ala Val Trp Gly Lys Asn Val Ser Leu Lys Cys Leu Ile
65 70 75 80
Glu Val Asn Glu Thr Ile Thr Gln Ile Ser Trp Glu Lys Ile His Gly
85 90 95
Lys Ser Ser Gln Thr Val Ala Val His His Pro Gln Tyr Gly Phe Ser
100 105 110
Val Gln Gly Glu Tyr Gln Gly Arg Val Leu Phe Lys Asn Tyr Ser Leu
115 120 125
Asn Asp Ala Thr Ile Thr Leu His Asn Ile Gly Phe Ser Asp Ser Gly
130 135 140
Lys Tyr Ile Cys Lys Ala Val Thr Phe Pro Leu Gly Asn Ala Gln Ser
145 150 155 160
Ser Thr Thr Val Thr Val Leu Val Glu Pro Thr Val Ser Leu Ile Lys
165 170 175
Gly Pro Asp Ser Leu Ile Asp Gly Gly Asn Glu Thr Val Ala Ala Ile
180 185 190
Cys Ile Ala Ala Thr Gly Lys Pro Val Ala His Ile Asp Trp Glu Gly
195 200 205
Asp Leu Gly Glu Met Glu Ser Thr Thr Thr Ser Phe Pro Asn Glu Thr
210 215 220
Ala Thr Ile Ile Ser Gln Tyr Lys Leu Phe Pro Thr Arg Phe Ala Arg
225 230 235 240
Gly Arg Arg Ile Thr Cys Val Val Lys His Pro Ala Leu Glu Lys Asp
245 250 255
Ile Arg Tyr Ser Phe Ile Leu Asp Ile Gln Tyr Ala Pro Glu Val Ser
260 265 270
Val Thr Gly Tyr Asp Gly Asn Trp Phe Val Gly Arg Lys Gly Val Asn
275 280 285
Leu Lys Cys Asn Ala Asp Ala Asn Pro Pro Pro Phe Lys Ser Val Trp
290 295 300
Ser Arg Leu Asp Gly Gln Trp Pro Asp Gly Leu Leu Ala Ser Asp Asn
305 310 315 320
Thr Leu His Phe Val His Pro Leu Thr Phe Asn Tyr Ser Gly Val Tyr

```
                    325                 330                 335
Ile Cys Lys Val Thr Asn Ser Leu Gly Gln Arg Ser Asp Gln Lys Val
                340                 345                 350
Ile Tyr Ile Ser Asp Pro Pro Thr Thr Thr Thr Leu Gln Pro Thr Ile
            355                 360                 365
Gln Trp His Pro Ser Thr Ala Asp Ile Glu Asp Leu Ala Thr Glu Pro
    370                 375                 380
Lys Lys Leu Pro Phe Pro Leu Ser Thr Leu Ala Thr Ile Lys Asp Asp
385                 390                 395                 400
Thr Ile Ala Thr Glu Phe Pro Arg Gly Pro Thr Ile Lys Pro Cys Pro
                405                 410                 415
Pro Cys Lys Cys Pro Ala Pro Asn Leu Leu Gly Gly Pro Ser Val Phe
            420                 425                 430
Ile Phe Pro Pro Lys Ile Lys Asp Val Leu Met Ile Ser Leu Ser Pro
        435                 440                 445
Ile Val Thr Cys Val Val Val Asp Val Ser Glu Asp Asp Pro Asp Val
    450                 455                 460
Gln Ile Ser Trp Phe Val Asn Asn Val Glu Val His Thr Ala Gln Thr
465                 470                 475                 480
Gln Thr His Arg Glu Asp Tyr Asn Ser Thr Leu Arg Val Val Ser Ala
                485                 490                 495
Leu Pro Ile Gln His Gln Asp Trp Met Ser Gly Lys Glu Phe Lys Cys
            500                 505                 510
Lys Val Asn Asn Lys Asp Leu Pro Ala Pro Ile Glu Arg Thr Ile Ser
        515                 520                 525
Lys Pro Lys Gly Ser Val Arg Ala Pro Gln Val Tyr Val Leu Pro Pro
    530                 535                 540
Pro Glu Glu Glu Met Thr Lys Lys Gln Val Thr Leu Thr Cys Met Val
545                 550                 555                 560
Thr Asp Phe Met Pro Glu Asp Ile Tyr Val Glu Trp Thr Asn Asn Gly
                565                 570                 575
Lys Thr Glu Leu Asn Tyr Lys Asn Thr Glu Pro Val Leu Asp Ser Asp
            580                 585                 590
Gly Ser Tyr Phe Met Tyr Ser Lys Leu Arg Val Glu Lys Lys Asn Trp
        595                 600                 605
Val Glu Arg Asn Ser Tyr Ser Cys Ser Val Val His Glu Gly Leu His
    610                 615                 620
Asn His His Thr Thr Lys Ser Phe Ser Arg Thr Pro Gly Lys
625                 630                 635
```

<210> 52
<211> 1914
<212> DNA
<213> Homo sapiens

<400> 52

```
atggcgcgga ccctgcggcc gtccccgctg tgtcctggag gcggcaaagc acaactttcc     60
tccgcttctc tcctcggagc cgggctcctg ctgcagcccc cgacgccacc tccgctgctg    120
ctgctgctct tcccgctgct gctcttctcc aggctctgtg gtgccttagc tggaccaatt    180
attgtggagc cacatgtcac agcagtatgg ggaaagaatg tttcattaaa gtgtttaatt    240
gaagtaaatg aaaccataac acagatttca tgggagaaga tacatggcaa agttcacag     300
actgttgcag ttcaccatcc ccaatatgga ttctctgttc aaggagaata tcagggaaga    360
gtcttgttta aaaattactc acttaatgat gcaacaatta ctctgcataa catagggattc   420
tctgattctg gaaaatacat ctgcaaagct gttacattcc cgcttggaaa tgcccagtcc    480
tctacaactg taactgtgtt agttgaaccc actgtgagcc tgataaaagg gccagattct    540
ttaattgatg gaggaaatga aacagtagca gccatttgca tcgcagccac tggaaaaccc    600
gttgcacata ttgactggga aggtgatctt ggtgaaatgg aatccactac aacttctttt    660
```

```
ccaaatgaaa cggcaacgat tatcagccag tacaagctat ttccaaccag atttgctaga   720
ggaaggcgaa ttacttgtgt tgtaaaacat ccagccttgg aaaaggacat ccgatactct   780
ttcatattag acatacagta tgctcctgaa gtttcggtaa caggatatga tggaaattgg   840
tttgtaggaa gaaaaggtgt taatctcaaa tgtaatgctg atgcaaatcc accacccttc   900
aaatctgtgt ggagcaggtt ggatggacaa tggcctgatg gtttattggc ttcagacaat   960
actcttcatt ttgtccatcc attgactttc aattattctg gtgtttatat ctgtaaagtg  1020
accaattccc ttggtcaaag aagtgaccaa aaagtcatct acatttcaga tcctcctact  1080
actaccaccc ttcagcctac aattcagtgg catccctcaa ctgctgacat cgaggatcta  1140
gcaacagaac ctaaaaaatt gcccttccca ttgtcaactt tggcaacaat taaggatgac  1200
acaattgcca cggaattccc cagagggccc acaatcaagc cctgtcctcc atgcaaatgc  1260
ccagcaccta acctcttggg tggaccatcc gtcttcatct tccctccaaa gatcaaggat  1320
gtactcatga tctccctgag ccccatagtc acatgtgtgg tggtggatgt gagcgaggat  1380
gacccagatg tccagatcag ctggtttgtg aacaacgtgg aagtacacac agctcagaca  1440
caaacccata gagaggatta caacagtact ctccgggtgg tcagtgccct ccccatccag  1500
caccaggact ggatgagtgg caaggagttc aaatgcaagg tcaacaacaa agacctccca  1560
gcgcccatcg agagaaccat ctcaaaaccc aaagggtcag taagagctcc acaggtatat  1620
gtcttgcctc caccagaaga agagatgact aagaaacagg tcactctgac ctgcatggtc  1680
acagacttca tgcctgaaga catttacgtg gagtggacca acaacgggaa aacagagcta  1740
aactacaaga acactgaacc agtcctggac tctgatggtt cttacttcat gtacagcaag  1800
ctgagagtgg aaaagaagaa ctgggtggaa agaaatagct actcctgttc agtggtccac  1860
gagggtctgc acaatcacca cacgactaag agcttctccc ggactccggg taaa        1914
```

<210> 53
<211> 102
<212> DNA
<213> Artificial

<220>
<223> DNA encoding flag protein

<400> 53

```
ctagtctcga gaattcacgc gtggtacctc tagagtcgac atggactaca aggacgacga     60

tgacaaggct agcgactaca aggacgacga tgacaagtga gc                       102
```

<210> 54
<211> 101
<212> DNA
<213> Artificial

<220>
<223> DNA encoding flag protein

<400> 54

```
ggccgctcac ttgtcatcgt cgtccttgta gtcgctagcc ttgtcatcgt cgtccttgta     60

gtccatgtcg actctagagg taccacgcgt aattctcgag a                        101
```

<210> 55
<211> 34
<212> DNA
<213> Artificial

<220>

<223> Primer

<400> 55
acgcgtcgac caccatggcg cggaccctgc ggcc 34

<210> 56
<211> 29
<212> DNA
<213> Artificial

<220>
<223> Primer

<400> 56
ctagctagcc gtggcaattg tgtcatcct 29

<210> 57
<211> 414
<212> PRT
<213> Artificial

<220>
<223> Nectin-3ED-FLAG protein

<400> 57

Met Ala Arg Thr Leu Arg Pro Ser Pro Leu Cys Pro Gly Gly Gly Lys
1 5 10 15

Ala Gln Leu Ser Ser Ala Ser Leu Leu Gly Ala Gly Leu Leu Leu Gln
20 25 30

Pro Pro Thr Pro Pro Pro Leu Leu Leu Leu Leu Phe Pro Leu Leu Leu
35 40 45

Phe Ser Arg Leu Cys Gly Ala Leu Ala Gly Pro Ile Ile Val Glu Pro
50 55 60

His Val Thr Ala Val Trp Gly Lys Asn Val Ser Leu Lys Cys Leu Ile
65 70 75 80

Glu Val Asn Glu Thr Ile Thr Gln Ile Ser Trp Glu Lys Ile His Gly
85 90 95

Lys Ser Ser Gln Thr Val Ala Val His His Pro Gln Tyr Gly Phe Ser
100 105 110

Val Gln Gly Glu Tyr Gln Gly Arg Val Leu Phe Lys Asn Tyr Ser Leu

115 120 125

Asn Asp Ala Thr Ile Thr Leu His Asn Ile Gly Phe Ser Asp Ser Gly
130 135 140

Lys Tyr Ile Cys Lys Ala Val Thr Phe Pro Leu Gly Asn Ala Gln Ser
145 150 155 160

Ser Thr Thr Val Thr Val Leu Val Glu Pro Thr Val Ser Leu Ile Lys
165 170 175

Gly Pro Asp Ser Leu Ile Asp Gly Gly Asn Glu Thr Val Ala Ala Ile
180 185 190

Cys Ile Ala Ala Thr Gly Lys Pro Val Ala His Ile Asp Trp Glu Gly
195 200 205

Asp Leu Gly Glu Met Glu Ser Thr Thr Thr Ser Phe Pro Asn Glu Thr
210 215 220

Ala Thr Ile Ile Ser Gln Tyr Lys Leu Phe Pro Thr Arg Phe Ala Arg
225 230 235 240

Gly Arg Arg Ile Thr Cys Val Val Lys His Pro Ala Leu Glu Lys Asp
245 250 255

Ile Arg Tyr Ser Phe Ile Leu Asp Ile Gln Tyr Ala Pro Glu Val Ser
260 265 270

Val Thr Gly Tyr Asp Gly Asn Trp Phe Val Gly Arg Lys Gly Val Asn
275 280 285

Leu Lys Cys Asn Ala Asp Ala Asn Pro Pro Pro Phe Lys Ser Val Trp
290 295 300

Ser Arg Leu Asp Gly Gln Trp Pro Asp Gly Leu Leu Ala Ser Asp Asn
305 310 315 320

Thr Leu His Phe Val His Pro Leu Thr Phe Asn Tyr Ser Gly Val Tyr
325 330 335

Ile Cys Lys Val Thr Asn Ser Leu Gly Gln Arg Ser Asp Gln Lys Val
340 345 350

Ile Tyr Ile Ser Asp Pro Pro Thr Thr Thr Thr Leu Gln Pro Thr Ile
355 360 365

Gln Trp His Pro Ser Thr Ala Asp Ile Glu Asp Leu Ala Thr Glu Pro
370 375 380

Lys Lys Leu Pro Phe Pro Leu Ser Thr Leu Ala Thr Ile Lys Asp Asp
385 390 395 400

Thr Ile Ala Thr Ala Ser Asp Tyr Lys Asp Asp Asp Asp Lys
405 410

<210> 58
<211> 1242
<212> DNA
<213> Artificial

<220>
<223> DNA encoding Nectin-3ED-FLAG protein

<400> 58
atggcgcgga ccctgcggcc gtccccgctg tgtcctggag gcggcaaagc acaactttcc 60

tccgcttctc tcctcggagc cgggctcctg ctgcagcccc cgacgccacc tccgctgctg 120

ctgctgctct tcccgctgct gctcttctcc aggctctgtg gtgccttagc tggaccaatt 180

attgtggagc cacatgtcac agcagtatgg ggaaagaatg tttcattaaa gtgtttaatt 240

gaagtaaatg aaaccataac acagatttca tgggagaaga tacatggcaa aagttcacag 300

actgttgcag ttcaccatcc ccaatatgga ttctctgttc aaggagaata tcagggaaga 360

gtcttgttta aaaattactc acttaatgat gcaacaatta ctctgcataa cataggattc 420

tctgattctg gaaaatacat ctgcaaagct gttacattcc cgcttggaaa tgcccagtcc 480

tctacaactg taactgtgtt agttgaaccc actgtgagcc tgataaaagg gccagattct 540

ttaattgatg gaggaaatga aacagtagca gccatttgca tcgcagccac tggaaaaccc 600

gttgcacata ttgactggga aggtgatctt ggtgaaatgg aatccactac aacttctttt 660

ccaaatgaaa cggcaacgat tatcagccag tacaagctat ttccaaccag atttgctaga 720

ggaaggcgaa ttacttgtgt tgtaaaacat ccagccttgg aaaaggacat ccgatactct 780

ttcatattag acatacagta tgctcctgaa gtttcggtaa caggatatga tggaaattgg 840

tttgtaggaa gaaaaggtgt taatctcaaa tgtaatgctg atgcaaatcc accacccttc 900

aaatctgtgt ggagcaggtt ggatggacaa tggcctgatg gtttattggc ttcagacaat 960

actcttcatt ttgtccatcc attgactttc aattattctg gtgtttatat ctgtaaagtg 1020

accaattccc ttggtcaaag aagtgaccaa aaagtcatct acatttcaga tcctcctact 1080

actaccaccc ttcagcctac aattcagtgg catccctcaa ctgctgacat cgaggatcta 1140

gcaacagaac ctaaaaaatt gcccttccca ttgtcaactt tggcaacaat taaggatgac 1200

acaattgcca cggctagcga ctacaaggac gacgatgaca ag 1242

<210> 59
<211> 28
<212> DNA
<213> Artificial

<220>
<223> Primer

<400> 59
aattgatatc atggctcgga tggggctt 28

<210> 60
<211> 28
<212> DNA
<213> Artificial

<220>
<223> Primer

<400> 60
aattctcgag cacgtaccac tccttctt 28

<210> 61
<211> 1551
<212> DNA
<213> Homo sapiens

<400> 61
atggctcgga tggggcttgc gggcgccgct ggacgctggt ggggactcgc tctcggcttg 60

accgcattct tcctcccagg cgtccactcc caggtggtcc aggtgaacga ctccatgtat 120

ggcttcatcg gcacagacgt ggttctgcac tgcagctttg ccaacccgct tcccagcgtg 180

aagatcaccc aggtcacatg gcagaagtcc accaatggct ccaagcagaa cgtggccatc 240

tacaacccat ccatgggcgt gtccgtgctg gctccctacc gcgagcgtgt ggaattcctg 300

cggccctcct tcaccgatgg cactatccgc ctctcccgcc tggagctgga ggatgagggt 360

gtctacatct gcgagtttgc taccttccct acgggcaatc gagaaagcca gctcaatctc 420

acggtgatgg ccaaacccac caattggata gagggtaccc aggcagtgct tcgagccaag 480

aaggggcagg atgacaaggt cctggtggcc acctgcacct cagccaatgg gaagcctccc 540

agtgtggtat cctgggaaac tcggttaaaa ggtgaggcag agtaccagga gatccggaac 600

cccaatggca cagtgacggt catcagccgc taccgcctgg tgcccagcag ggaagcccac 660

cagcagtcct tggcctgcat cgtcaactac cacatggacc gcttcaagga aagcctcact 720

ctcaacgtgc agtatgagcc tgaggtaacc attgaggggt ttgatggcaa ctggtacctg 780

cagcggatgg acgtgaagct cacctgcaaa gctgatgcta accccccagc cactgagtac 840

cactggacca cgctaaatgg ctctctcccc aagggtgtgg aggcccagaa cagaaccctc 900

ttcttcaagg gacccatcaa ctacagcctg gcagggacct acatctgtga ggccaccaac 960

cccatcggta cacgctcagg ccaggtggag gtcaatatca cagaattccc ctacaccccg 1020

tctcctcccg aacatgggcg gcgcgccggg ccggtgccca cggccatcat tgggggcgtg 1080

gcggggagca tcctgctggt gttgattgtg gtcggcggga tcgtggtcgc cctgcgtcgg 1140

cgccggcaca ccttcaaggg tgactacagc accaagaagc acgtgtatgg caacggctac 1200

agcaaggcag gcatccccca gcaccaccca ccaatggcac agaacctgca gtaccccgac 1260

gactcagacg acgagaagaa ggccggccca ctgggtggaa gcagctatga ggaggaggag 1320

gaggaggagg agggcggtgg agggggcgag cgcaaggtgg gcggcccccca ccccaaatat 1380

gacgaggacg ccaagcggcc ctacttcacc gtggatgagg ccgaggcccg tcaggacggc 1440

tacggggacc ggactctggg ctaccagtac gaccctgagc agctggactt ggctgagaac 1500

atggtttctc agaacgacgg gtctttcatt tccaagaagg agtggtacgt g 1551

<210> 62
<211> 517
<212> PRT
<213> Homo sapiens

<400> 62

Met Ala Arg Met Gly Leu Ala Gly Ala Ala Gly Arg Trp Trp Gly Leu
1 5 10 15

Ala Leu Gly Leu Thr Ala Phe Phe Leu Pro Gly Val His Ser Gln Val
20 25 30

Val Gln Val Asn Asp Ser Met Tyr Gly Phe Ile Gly Thr Asp Val Val
35 40 45

Leu His Cys Ser Phe Ala Asn Pro Leu Pro Ser Val Lys Ile Thr Gln
50 55 60

Val Thr Trp Gln Lys Ser Thr Asn Gly Ser Lys Gln Asn Val Ala Ile
65 70 75 80

Tyr Asn Pro Ser Met Gly Val Ser Val Leu Ala Pro Tyr Arg Glu Arg
85 90 95

Val Glu Phe Leu Arg Pro Ser Phe Thr Asp Gly Thr Ile Arg Leu Ser
100 105 110

Arg Leu Glu Leu Glu Asp Glu Gly Val Tyr Ile Cys Glu Phe Ala Thr
115 120 125

Phe Pro Thr Gly Asn Arg Glu Ser Gln Leu Asn Leu Thr Val Met Ala
130 135 140

Lys Pro Thr Asn Trp Ile Glu Gly Thr Gln Ala Val Leu Arg Ala Lys
145 150 155 160

Lys Gly Gln Asp Asp Lys Val Leu Val Ala Thr Cys Thr Ser Ala Asn
165 170 175

Gly Lys Pro Pro Ser Val Val Ser Trp Glu Thr Arg Leu Lys Gly Glu
180 185 190

Ala Glu Tyr Gln Glu Ile Arg Asn Pro Asn Gly Thr Val Thr Val Ile
195 200 205

Ser Arg Tyr Arg Leu Val Pro Ser Arg Glu Ala His Gln Gln Ser Leu
210 215 220

Ala Cys Ile Val Asn Tyr His Met Asp Arg Phe Lys Glu Ser Leu Thr
225 230 235 240

Leu Asn Val Gln Tyr Glu Pro Glu Val Thr Ile Glu Gly Phe Asp Gly
245 250 255

Asn Trp Tyr Leu Gln Arg Met Asp Val Lys Leu Thr Cys Lys Ala Asp
260 265 270

Ala Asn Pro Pro Ala Thr Glu Tyr His Trp Thr Thr Leu Asn Gly Ser
275 280 285

Leu Pro Lys Gly Val Glu Ala Gln Asn Arg Thr Leu Phe Phe Lys Gly
290 295 300

Pro Ile Asn Tyr Ser Leu Ala Gly Thr Tyr Ile Cys Glu Ala Thr Asn
305 310 315 320

Pro Ile Gly Thr Arg Ser Gly Gln Val Glu Val Asn Ile Thr Glu Phe
325 330 335

Pro Tyr Thr Pro Ser Pro Pro Glu His Gly Arg Arg Ala Gly Pro Val
340 345 350

Pro Thr Ala Ile Ile Gly Gly Val Ala Gly Ser Ile Leu Leu Val Leu
355 360 365

Ile Val Val Gly Gly Ile Val Val Ala Leu Arg Arg Arg Arg His Thr
370 375 380

Phe Lys Gly Asp Tyr Ser Thr Lys Lys His Val Tyr Gly Asn Gly Tyr
385 390 395 400

Ser Lys Ala Gly Ile Pro Gln His His Pro Pro Met Ala Gln Asn Leu
405 410 415

Gln Tyr Pro Asp Asp Ser Asp Asp Glu Lys Lys Ala Gly Pro Leu Gly
420 425 430

Gly Ser Ser Tyr Glu Glu Glu Glu Glu Glu Glu Glu Gly Gly Gly Gly
435 440 445

Gly Glu Arg Lys Val Gly Gly Pro His Pro Lys Tyr Asp Glu Asp Ala
450 455 460

Lys Arg Pro Tyr Phe Thr Val Asp Glu Ala Glu Ala Arg Gln Asp Gly
465 470 475 480

Tyr Gly Asp Arg Thr Leu Gly Tyr Gln Tyr Asp Pro Glu Gln Leu Asp
485 490 495

Leu Ala Glu Asn Met Val Ser Gln Asn Asp Gly Ser Phe Ile Ser Lys

500 505 510

Lys Glu Trp Tyr Val
515

<210> 63
<211> 29
<212> DNA
<213> Artificial

<220>
<223> Primer

<400> 63
atcgatatct caaacatacc actccctcc 29

<210> 64
<211> 29
<212> DNA
<213> Artificial

<220>
<223> Primer

<400> 64
aattctcgag aacataccac tccctcctg 29

<210> 65
<211> 25
<212> DNA
<213> Artificial

<220>
<223> Primer

<400> 65
aattgaattc atgcccctgt ccctg 25

<210> 66
<211> 29
<212> DNA
<213> Artificial

<220>
<223> Primer

<400> 66
ttaactcgag gaccaggtgt ccccgccca 29

<210> 67
<211> 1530

<212> DNA
<213> Homo sapiens

<400> 67
atgcccctgt ccctgggagc cgagatgtgg gggcctgagg cctggctgct gctgctgcta 60

ctgctggcat catttacagg ccggtgcccc gcgggtgagc tggagacctc agacgtggta 120

actgtggtgc tgggccagga cgcaaaactg ccctgcttct accgagggga ctccggcgag 180

caagtggggc aagtggcatg ggctcgggtg gacgcgggcg aaggcgccca ggaactagcg 240

ctactgcact ccaaatacgg gcttcatgtg agcccggctt acgagggccg cgtggagcag 300

ccgccgcccc cacgcaaccc cctggacggc tcagtgctcc tgcgcaacgc agtgcaggcg 360

gatgagggcg agtacgagtg ccgggtcagc accttccccg ccggcagctt ccaggcgcgg 420

ctgcggctcc gagtgctggt gcctcccctg ccctcactga atcctggtcc agcactagaa 480

gagggccagg gcctgaccct ggcagcctcc tgcacagctg agggcagccc agcccccagc 540

gtgacctggg acacggaggt caaaggcaca acgtccagcc gttccttcaa gcactcccgc 600

tctgctgccg tcacctcaga gttccacttg gtgcctagcc gcagcatgaa tgggcagcca 660

ctgacttgtg tggtgtccca tcctggcctg ctccaggacc aaaggatcac ccacatcctc 720

cacgtgtcct tccttgctga ggcctctgtg aggggccttg aagaccaaaa tctgtggcac 780

attggcagag aaggagctat gctcaagtgc ctgagtgaag ggcagccccc tccctcatac 840

aactggacac ggctggatgg gcctctgccc agtggggtac gagtggatgg ggacactttg 900

ggctttcccc cactgaccac tgagcacagc ggcatctacg tctgccatgt cagcaatgag 960

ttctcctcaa gggattctca ggtcactgtg gatgttcttg acccccagga agactctggg 1020

aagcaggtgg acctagtgtc agcctcggtg gtggtggtgg gtgtgatcgc cgcactcttg 1080

ttctgccttc tggtggtggt ggtggtgctc atgtcccgat accatcggcg caaggcccag 1140

cagatgaccc agaaatatga ggaggagctg accctgacca gggagaactc catccggagg 1200

ctgcattccc atcacacgga ccccaggagc cagccggagg agagtgtagg gctgagagcc 1260

gagggccacc ctgatagtct caaggacaac agtagctgct ctgtgatgag tgaagagccc 1320

gagggccgca gttactccac gctgaccacg gtgagggaga tagaaacaca gactgaactg 1380

ctgtctccag gctctgggcg ggccgaggag gaggaagatc aggatgaagg catcaaacag 1440

gccatgaacc attttgttca ggagaatggg accctacggg ccaagcccac gggcaatggc 1500

atctacatca atgggcgggg acacctggtc 1530

<210> 68
<211> 510
<212> PRT
<213> Homo sapiens

<400> 68

Met Pro Leu Ser Leu Gly Ala Glu Met Trp Gly Pro Glu Ala Trp Leu
1 5 10 15

Leu Leu Leu Leu Leu Leu Ala Ser Phe Thr Gly Arg Cys Pro Ala Gly
20 25 30

Glu Leu Glu Thr Ser Asp Val Val Thr Val Val Leu Gly Gln Asp Ala
35 40 45

Lys Leu Pro Cys Phe Tyr Arg Gly Asp Ser Gly Glu Gln Val Gly Gln
50 55 60

Val Ala Trp Ala Arg Val Asp Ala Gly Glu Gly Ala Gln Glu Leu Ala
65 70 75 80

Leu Leu His Ser Lys Tyr Gly Leu His Val Ser Pro Ala Tyr Glu Gly
85 90 95

Arg Val Glu Gln Pro Pro Pro Pro Arg Asn Pro Leu Asp Gly Ser Val
100 105 110

Leu Leu Arg Asn Ala Val Gln Ala Asp Glu Gly Glu Tyr Glu Cys Arg
115 120 125

Val Ser Thr Phe Pro Ala Gly Ser Phe Gln Ala Arg Leu Arg Leu Arg
130 135 140

Val Leu Val Pro Pro Leu Pro Ser Leu Asn Pro Gly Pro Ala Leu Glu
145 150 155 160

Glu Gly Gln Gly Leu Thr Leu Ala Ala Ser Cys Thr Ala Glu Gly Ser
165 170 175

Pro Ala Pro Ser Val Thr Trp Asp Thr Glu Val Lys Gly Thr Thr Ser
180 185 190

Ser Arg Ser Phe Lys His Ser Arg Ser Ala Ala Val Thr Ser Glu Phe
195 200 205

His Leu Val Pro Ser Arg Ser Met Asn Gly Gln Pro Leu Thr Cys Val
210 215 220

Val Ser His Pro Gly Leu Leu Gln Asp Gln Arg Ile Thr His Ile Leu
225 230 235 240

His Val Ser Phe Leu Ala Glu Ala Ser Val Arg Gly Leu Glu Asp Gln
245 250 255

Asn Leu Trp His Ile Gly Arg Glu Gly Ala Met Leu Lys Cys Leu Ser
260 265 270

Glu Gly Gln Pro Pro Pro Ser Tyr Asn Trp Thr Arg Leu Asp Gly Pro
275 280 285

Leu Pro Ser Gly Val Arg Val Asp Gly Asp Thr Leu Gly Phe Pro Pro
290 295 300

Leu Thr Thr Glu His Ser Gly Ile Tyr Val Cys His Val Ser Asn Glu
305 310 315 320

Phe Ser Ser Arg Asp Ser Gln Val Thr Val Asp Val Leu Asp Pro Gln
325 330 335

Glu Asp Ser Gly Lys Gln Val Asp Leu Val Ser Ala Ser Val Val Val
340 345 350

Val Gly Val Ile Ala Ala Leu Leu Phe Cys Leu Leu Val Val Val Val
355 360 365

Val Leu Met Ser Arg Tyr His Arg Arg Lys Ala Gln Gln Met Thr Gln
370 375 380

Lys Tyr Glu Glu Glu Leu Thr Leu Thr Arg Glu Asn Ser Ile Arg Arg
385 390 395 400

Leu His Ser His His Thr Asp Pro Arg Ser Gln Pro Glu Glu Ser Val
405 410 415

Gly Leu Arg Ala Glu Gly His Pro Asp Ser Leu Lys Asp Asn Ser Ser
420 425 430

Cys Ser Val Met Ser Glu Glu Pro Glu Gly Arg Ser Tyr Ser Thr Leu
435 440 445

Thr Thr Val Arg Glu Ile Glu Thr Gln Thr Glu Leu Leu Ser Pro Gly
450 455 460

Ser Gly Arg Ala Glu Glu Glu Glu Asp Gln Asp Glu Gly Ile Lys Gln
465 470 475 480

Ala Met Asn His Phe Val Gln Glu Asn Gly Thr Leu Arg Ala Lys Pro
485 490 495

Thr Gly Asn Gly Ile Tyr Ile Asn Gly Arg Gly His Leu Val
500 505 510

<210> 69
<211> 25
<212> DNA
<213> Artificial

<220>
<223> Primer

<400> 69
aattaagctt atggcccgag ccatg 25

<210> 70
<211> 25
<212> DNA
<213> Artificial

<220>
<223> Primer

<400> 70
aattgtcgac ccttgtgccc tctgt 25

<210> 71
<211> 417
<212> PRT
<213> Homo sapiens

<400> 71

Met Ala Arg Ala Met Ala Ala Ala Trp Pro Leu Leu Leu Val Ala Leu
1 5 10 15

Leu Val Leu Ser Trp Pro Pro Pro Gly Thr Gly Asp Val Val Val Gln

20 25 30

Ala Pro Thr Gln Val Pro Gly Phe Leu Gly Asp Ser Val Thr Leu Pro
35 40 45

Cys Tyr Leu Gln Val Pro Asn Met Glu Val Thr His Val Ser Gln Leu
50 55 60

Thr Trp Ala Arg His Gly Glu Ser Gly Ser Met Ala Val Phe His Gln
65 70 75 80

Thr Gln Gly Pro Ser Tyr Ser Glu Ser Lys Arg Leu Glu Phe Val Ala
85 90 95

Ala Arg Leu Gly Ala Glu Leu Arg Asn Ala Ser Leu Arg Met Phe Gly
100 105 110

Leu Arg Val Glu Asp Glu Gly Asn Tyr Thr Cys Leu Phe Val Thr Phe
115 120 125

Pro Gln Gly Ser Arg Ser Val Asp Ile Trp Leu Arg Val Leu Ala Lys
130 135 140

Pro Gln Asn Thr Ala Glu Val Gln Lys Val Gln Leu Thr Gly Glu Pro
145 150 155 160

Val Pro Met Ala Arg Cys Val Ser Thr Gly Gly Arg Pro Pro Ala Gln
165 170 175

Ile Thr Trp His Ser Asp Leu Gly Gly Met Pro Asn Thr Ser Gln Val
180 185 190

Pro Gly Phe Leu Ser Gly Thr Val Thr Val Thr Ser Leu Trp Ile Leu
195 200 205

Val Pro Ser Ser Gln Val Asp Gly Lys Asn Val Thr Cys Lys Val Glu
210 215 220

His Glu Ser Phe Glu Lys Pro Gln Leu Leu Thr Val Asn Leu Thr Val
225 230 235 240

Tyr Tyr Pro Pro Glu Val Ser Ile Ser Gly Tyr Asp Asn Asn Trp Tyr
245 250 255

Leu Gly Gln Asn Glu Ala Thr Leu Thr Cys Asp Ala Arg Ser Asn Pro
260 265 270

Glu Pro Thr Gly Tyr Asn Trp Ser Thr Thr Met Gly Pro Leu Pro Pro
275 280 285

Phe Ala Val Ala Gln Gly Ala Gln Leu Leu Ile Arg Pro Val Asp Lys
290 295 300

Pro Ile Asn Thr Thr Leu Ile Cys Asn Val Thr Asn Ala Leu Gly Ala
305 310 315 320

Arg Gln Ala Glu Leu Thr Val Gln Val Lys Glu Gly Pro Pro Ser Glu
325 330 335

His Ser Gly Met Ser Arg Asn Ala Ile Ile Phe Leu Val Leu Gly Ile
340 345 350

Leu Val Phe Leu Ile Leu Leu Gly Ile Gly Ile Tyr Phe Tyr Trp Ser
355 360 365

Lys Cys Ser Arg Glu Val Leu Trp His Cys His Leu Cys Pro Ser Ser
370 375 380

Thr Glu His Ala Ser Ala Ser Ala Asn Gly His Val Ser Tyr Ser Ala
385 390 395 400

Val Ser Arg Glu Asn Ser Ser Ser Gln Asp Pro Gln Thr Glu Gly Thr
405 410 415

Arg

<210> 72
<211> 1251
<212> DNA
<213> Homo sapiens

<400> 72
atggcccgag ccatggccgc cgcgtggccg ctgctgctgg tggcgctact ggtgctgtcc 60

tggccacccc caggaaccgg ggacgtcgtc gtgcaggcgc ccacccaggt gcccggcttc 120

ttgggcgact ccgtgacgct gccctgctac ctacaggtgc ccaacatgga ggtgacgcat 180

gtgtcacagc tgacttgggc gcggcatggt gaatctggca gcatggccgt cttccaccaa 240
acgcagggcc ccagctattc ggagtccaaa cggctggaat tcgtggcagc cagactgggc 300
gcggagctgc ggaatgcctc gctgaggatg ttcgggttgc gcgtagagga tgaaggcaac 360
tacacctgcc tgttcgtcac gttcccgcag ggcagcagga gcgtggatat ctggctccga 420
gtgcttgcca agccccagaa cacagctgag gttcagaagg tccagctcac tggagagcca 480
gtgcccatgg cccgctgcgt ctccacaggg ggtcgcccgc cagcccaaat cacctggcac 540
tcagacctgg gcgggatgcc caatacgagc caggtgccag ggttcctgtc tggcacagtc 600
actgtcacca gcctctggat attggtgccc tcaagccagg tggacggcaa gaatgtgacc 660
tgcaaggtgg agcacgagag ctttgagaag cctcagctgc tgactgtgaa cctcaccgtg 720
tactaccccc cagaggtatc catctctggc tatgataaca actggtacct tggccagaat 780
gaggccaccc tgacctgcga tgctcgcagc aacccagagc ccacaggcta taattggagc 840
acgaccatgg gtcccctgcc accctttgct gtggcccagg gcgcccagct cctgatccgt 900
cctgtggaca aaccaatcaa cacaacttta atctgcaacg tcaccaatgc cctaggagct 960
cgccaggcag aactgaccgt ccaggtcaaa gagggacctc ccagtgagca ctcaggcatg 1020
tcccgtaacg ccatcatctt cctggttctg ggaatcctgg tttttctgat cctgctgggg 1080
atcgggattt atttctattg gtccaaatgt tcccgtgagg tcctttggca ctgtcatctg 1140
tgtccctcga gtacagagca tgccagcgcc tcagctaatg ggcatgtctc ctattcagct 1200
gtgagcagag agaacagctc ttcccaggat ccacagacag agggcacaag g 1251

<210> 73
<211> 22
<212> DNA
<213> Artificial

<220>
<223> Primer

<400> 73
aattgatatc agcgcccccg ag 22

<210> 74
<211> 27
<212> DNA
<213> Artificial

<220>
<223> Primer

<400> 74
aattgatatc agggaacgtg accttct 27

<210> 75
<211> 27
<212> DNA
<213> Artificial

<220>
<223> Primer

<400> 75
aattgatatc cagaaggtca cgttcag 27

<210> 76
<211> 26
<212> DNA
<213> Artificial

<220>
<223> Primer

<400> 76
aattcctcga gtcacacata catggc 26

<210> 77
<211> 27
<212> DNA
<213> Artificial

<220>
<223> Primer

<400> 77
aattgatatc tccatctccg gctatga 27

<210> 78
<211> 421
<212> PRT
<213> Artificial

<220>
<223> Synthetic Protein

<400> 78

Met Ala Arg Ala Ala Ala Leu Leu Pro Ser Arg Ser Pro Pro Thr Pro
1 5 10 15

Leu Leu Trp Pro Leu Leu Leu Leu Leu Leu Leu Glu Thr Gly Ala Gln
20 25 30

Asp Val Arg Val Gln Val Leu Pro Glu Val Arg Gly Gln Leu Gly Gly
35 40 45

Ala Asp Ile Gln Lys Val Thr Phe Ser Gln Asp Pro Thr Thr Val Ala
50 55 60

Leu Cys Ile Ser Lys Glu Gly Arg Pro Pro Ala Arg Ile Ser Trp Leu
65 70 75 80

Ser Ser Leu Asp Trp Glu Ala Lys Glu Thr Gln Val Ser Gly Thr Leu
85 90 95

Ala Gly Thr Val Thr Val Thr Ser Arg Phe Thr Leu Val Pro Ser Gly
100 105 110

Arg Ala Asp Gly Val Thr Val Thr Cys Lys Val Glu His Glu Ser Phe
115 120 125

Glu Glu Pro Ala Leu Ile Pro Val Thr Leu Ser Val Arg Tyr Pro Pro
130 135 140

Glu Val Ser Ile Ser Gly Tyr Asp Asp Asn Trp Tyr Leu Gly Arg Thr
145 150 155 160

Asp Ala Thr Leu Ser Cys Asp Val Arg Ser Asn Pro Glu Pro Thr Gly
165 170 175

Tyr Asp Trp Ser Thr Thr Ser Gly Thr Phe Pro Thr Ser Ala Val Ala
180 185 190

Gln Gly Ser Gln Leu Val Ile His Ala Val Asp Ser Leu Phe Asn Thr
195 200 205

Thr Phe Val Cys Thr Val Thr Asn Ala Val Gly Met Gly Arg Ala Glu
210 215 220

Gln Val Ile Phe Val Arg Glu Thr Pro Asn Thr Ala Gly Ala Gly Ala
225 230 235 240

Thr Gly Gly Ile Ile Gly Gly Ile Ile Ala Ala Ile Ile Ala Thr Ala
245 250 255

Val Ala Ala Thr Gly Ile Leu Ile Cys Arg Gln Gln Arg Lys Glu Gln
260 265 270

Thr Leu Gln Gly Ala Glu Glu Asp Glu Asp Leu Glu Gly Pro Pro Ser
275 280 285

Tyr Lys Pro Pro Thr Pro Lys Ala Lys Leu Glu Ala Gln Glu Met Pro
290 295 300

Ser Gln Leu Phe Thr Leu Gly Ala Ser Glu His Ser Pro Leu Lys Thr
305 310 315 320

Pro Tyr Phe Asp Ala Gly Ala Ser Cys Thr Glu Gln Glu Met Pro Arg
325 330 335

Tyr His Glu Leu Pro Thr Leu Glu Glu Arg Ser Gly Pro Leu His Pro
340 345 350

Gly Ala Thr Ser Leu Gly Ser Pro Ile Pro Val Pro Pro Gly Pro Pro
355 360 365

Ala Val Glu Asp Val Ser Leu Asp Leu Glu Asp Glu Glu Gly Glu Glu
370 375 380

Glu Glu Glu Tyr Leu Asp Lys Ile Asn Pro Ile Tyr Asp Ala Leu Ser
385 390 395 400

Tyr Ser Ser Pro Ser Asp Ser Tyr Gln Gly Lys Gly Phe Val Met Ser
405 410 415

Arg Ala Met Tyr Val
420

<210> 79
<211> 1263
<212> DNA
<213> Artificial

<220>
<223> Synthetic DNA

<400> 79
atggcccggg ccgctgccct cctgccgtcg agatcgccgc cgacgccgct gctgtggccg 60

ctgctgctgc tgctgctcct ggaaaccgga gcccaggatg tgcgagttca agtgctaccc 120

gaggtgcgag gccagctcgg gggcgctgat atccagaagg tcacgttcag ccaggaccct 180

acgacagtgg ccctctgcat ctccaaagag ggccgcccac ctgcccggat ctcctggctc 240

tcatccctgg actgggaagc caaagagact caggtgtcag ggaccctggc cggaactgtc 300

actgtcacca gccgcttcac cttggtgccc tcgggccgag cagatggtgt cacggtcacc 360

tgcaaagtgg agcatgagag cttcgaggaa ccagccctga tacctgtgac cctctctgta 420

cgctaccctc ctgaagtgtc catctccggc tatgatgaca actggtacct cggccgtact 480

gatgccaccc tgagctgtga cgtccgcagc aacccagagc ccacgggcta tgactggagc 540

acgacctcag gcaccttccc gacctccgca gtggcccagg gctcccagct ggtcatccac 600

gcagtggaca gtctgttcaa taccaccttc gtctgcacag tcaccaatgc cgtgggcatg 660

ggccgcgctg agcaggtcat ctttgtccga gagaccccca acacagcagg cgcaggggcc 720

acaggcggca tcatcggggg catcatcgcc gccatcattg ctactgctgt ggctgccacg 780

ggcatcctta tctgccggca gcagcggaag gagcagacgc tgcagggggc agaggaggac 840

gaagacctgg agggacctcc ctcctacaag ccaccgaccc caaaagcgaa gctggaggca 900

caggagatgc cctcccagct cttcactctg gggcctcgg agcacagccc actcaagacc 960

ccctactttg atgctggcgc ctcatgcact gagcaggaaa tgcctcgata ccatgagctg 1020

cccaccttgg aagaacggtc aggacccttg caccctggag ccacaagcct ggggtccccc 1080

atcccggtgc ctccagggcc acctgctgtg gaagacgttt ccctggatct agaggatgag 1140

gagggggagg aggaggaaga gtatctggac aagatcaacc ccatctatga tgctctgtcc 1200

tatagcagcc cctctgattc ctaccagggc aaaggctttg tcatgtcccg ggccatgtat 1260

gtg 1263

<210> 80
<211> 451
<212> PRT
<213> Artificial

<220>
<223> Synthetic Protein

<400> 80

Met Ala Arg Ala Ala Ala Leu Leu Pro Ser Arg Ser Pro Pro Thr Pro
1               5                   10                  15

Leu Leu Trp Pro Leu Leu Leu Leu Leu Leu Leu Glu Thr Gly Ala Gln

20 25 30

Asp Val Arg Val Gln Val Leu Pro Glu Val Arg Gly Gln Leu Gly Gly
35 40 45

Thr Val Glu Leu Pro Cys His Leu Leu Pro Pro Val Pro Gly Leu Tyr
50 55 60

Ile Ser Leu Val Thr Trp Gln Arg Pro Asp Ala Pro Ala Asn His Gln
65 70 75 80

Asn Val Ala Ala Phe His Pro Lys Met Gly Pro Ser Phe Pro Ser Pro
85 90 95

Lys Pro Gly Ser Glu Arg Leu Ser Phe Val Ser Ala Lys Gln Ser Thr
100 105 110

Gly Gln Asp Thr Glu Ala Glu Leu Gln Asp Ala Thr Leu Ala Leu His
115 120 125

Gly Leu Thr Val Glu Asp Glu Gly Asn Tyr Thr Cys Glu Phe Ala Thr
130 135 140

Phe Pro Lys Gly Ser Val Arg Gly Met Thr Trp Leu Arg Val Ile Ala
145 150 155 160

Lys Pro Lys Asn Gln Ala Glu Ala Gln Lys Val Thr Phe Pro Asp Ile
165 170 175

Ser Ile Ser Gly Tyr Asp Asp Asn Trp Tyr Leu Gly Arg Thr Asp Ala
180 185 190

Thr Leu Ser Cys Asp Val Arg Ser Asn Pro Glu Pro Thr Gly Tyr Asp
195 200 205

Trp Ser Thr Thr Ser Gly Thr Phe Pro Thr Ser Ala Val Ala Gln Gly
210 215 220

Ser Gln Leu Val Ile His Ala Val Asp Ser Leu Phe Asn Thr Thr Phe
225 230 235 240

Val Cys Thr Val Thr Asn Ala Val Gly Met Gly Arg Ala Glu Gln Val
245 250 255

Ile Phe Val Arg Glu Thr Pro Asn Thr Ala Gly Ala Gly Ala Thr Gly
260 265 270

Gly Ile Ile Gly Gly Ile Ile Ala Ala Ile Ile Ala Thr Ala Val Ala
275 280 285

Ala Thr Gly Ile Leu Ile Cys Arg Gln Gln Arg Lys Glu Gln Thr Leu
290 295 300

Gln Gly Ala Glu Glu Asp Glu Asp Leu Glu Gly Pro Pro Ser Tyr Lys
305 310 315 320

Pro Pro Thr Pro Lys Ala Lys Leu Glu Ala Gln Glu Met Pro Ser Gln
325 330 335

Leu Phe Thr Leu Gly Ala Ser Glu His Ser Pro Leu Lys Thr Pro Tyr
340 345 350

Phe Asp Ala Gly Ala Ser Cys Thr Glu Gln Glu Met Pro Arg Tyr His
355 360 365

Glu Leu Pro Thr Leu Glu Glu Arg Ser Gly Pro Leu His Pro Gly Ala
370 375 380

Thr Ser Leu Gly Ser Pro Ile Pro Val Pro Pro Gly Pro Pro Ala Val
385 390 395 400

Glu Asp Val Ser Leu Asp Leu Glu Asp Glu Glu Gly Glu Glu Glu Glu
405 410 415

Glu Tyr Leu Asp Lys Ile Asn Pro Ile Tyr Asp Ala Leu Ser Tyr Ser
420 425 430

Ser Pro Ser Asp Ser Tyr Gln Gly Lys Gly Phe Val Met Ser Arg Ala
435 440 445

Met Tyr Val
450

<210> 81
<211> 1353
<212> DNA

<213> Artificial

<220>
<223> Synthetic DNA

<400> 81
atggcccggg ccgctgccct cctgccgtcg agatcgccgc cgacgccgct gctgtggccg 60

ctgctgctgc tgctgctcct ggaaaccgga gcccaggatg tgcgagttca agtgctaccc 120

gaggtgcgag gccagctcgg gggcaccgtg gagctgccgt gccacctgct gccacctgtt 180

cctggactgt acatttccct ggtgacctgg cagcgcccag atgcacctgc gaaccaccag 240

aatgtggccg ccttccaccc taagatgggt cccagcttcc ccagcccgaa gcctggcagc 300

gagcggctgt ccttcgtctc tgccaagcag agcactgggc aagacacaga ggcagagctc 360

caggacgcca cgctggccct ccacgggctc acggtggagg acgagggcaa ctacacttgc 420

gagtttgcca ccttccccaa ggggtccgtc cgagggatga cctggctcag agtcatagcc 480

aagcccaaga accaagctga ggcccagaag gtcacgttcc ctgatatctc catctccggc 540

tatgatgaca actggtacct cggccgtact gatgccaccc tgagctgtga cgtccgcagc 600

aacccagagc ccacgggcta tgactggagc acgacctcag gcaccttccc gacctccgca 660

gtggcccagg gctcccagct ggtcatccac gcagtggaca gtctgttcaa taccaccttc 720

gtctgcacag tcaccaatgc cgtgggcatg ggccgcgctg agcaggtcat ctttgtccga 780

gagaccccca acacagcagg cgcaggggcc acaggcggca tcatcggggg catcatcgcc 840

gccatcattg ctactgctgt ggctgccacg ggcatcctta tctgccggca gcagcggaag 900

gagcagacgc tgcagggggc agaggaggac gaagacctgg agggacctcc ctcctacaag 960

ccaccgaccc caaaagcgaa gctggaggca caggagatgc cctcccagct cttcactctg 1020

ggggcctcgg agcacagccc actcaagacc ccctactttg atgctggcgc ctcatgcact 1080

gagcaggaaa tgcctcgata ccatgagctg cccaccttgg aagaacggtc aggacccttg 1140

caccctggag ccacaagcct ggggtccccc atcccggtgc ctccagggcc acctgctgtg 1200

gaagacgttt ccctggatct agaggatgag gagggggagg aggaggaaga gtatctggac 1260

aagatcaacc ccatctatga tgctctgtcc tatagcagcc cctctgattc ctaccagggc 1320

aaaggctttg tcatgtcccg ggccatgtat gtg 1353

<210> 82
<211> 23
<212> DNA
<213> Artificial

<220>
<223> Primer

<400> 82
agctgggccg ggagagcaga aca 23

<210> 83
<211> 27
<212> DNA
<213> Artificial

<220>
<223> Primer

<400> 83
aaatgggatg cacgtgtgtt aactgac 27

<210> 84
<211> 23
<212> DNA
<213> Artificial

<220>
<223> Primer

<400> 84
agagcagaac agggaggcta gag 23

<210> 85
<211> 28
<212> DNA
<213> Artificial

<220>
<223> Primer

<400> 85
caagaaagct aagggatcaa caggtgag 28

<210> 86
<211> 538
<212> PRT
<213> Macaca fascicularis

<400> 86

Met Ala Arg Ala Val Ala Leu Leu Pro Ser Arg Ser Pro Pro Thr Pro
1 5 10 15

Leu Leu Trp Pro Leu Leu Leu Leu Leu Leu Arg Lys Thr Gly Ala Gln
20 25 30

Asp Val Arg Val Gln Val Leu Pro Glu Val Arg Gly Gln Leu Gly Gly
35 40 45

Thr Val Glu Leu Pro Cys His Leu Leu Pro Pro Val Pro Gly Leu Tyr
50 55 60

Ile Ser Leu Val Thr Trp Gln Arg Pro Asp Ala Pro Pro Asp His Gln
65 70 75 80

Asn Val Ala Ala Phe His Pro Lys Met Gly Pro Ser Phe Pro Ser Pro
85 90 95

Lys Pro Gly Ser Gln Arg Leu Ser Phe Val Ser Ala Lys Gln Ser Thr
100 105 110

Arg Gln Asp Thr Glu Ala Glu Leu Gln Asp Ala Thr Leu Ala Leu Arg
115 120 125

Gly Leu Thr Val Glu Asp Glu Gly Asn Tyr Thr Cys Glu Phe Ala Thr
130 135 140

Phe Pro Lys Gly Ser Val Arg Gly Met Thr Trp Leu Arg Val Ile Ala
145 150 155 160

Lys Pro Gln Asn His Ala Glu Ala Gln Glu Val Thr Phe Ser Gln Asp
165 170 175

Pro Val Pro Val Ala Arg Cys Ile Ser Lys Glu Gly Arg Pro Pro Ala
180 185 190

Arg Ile Ser Trp Leu Ser Ser Leu Asp Trp Glu Ala Lys Glu Thr Gln
195 200 205

Val Ser Gly Thr Leu Ala Gly Thr Val Thr Val Thr Ser Arg Phe Thr
210 215 220

Leu Val Pro Ser Gly Arg Ala Asp Gly Val Thr Val Thr Cys Lys Val
225 230 235 240

Glu His Glu Ser Phe Glu Glu Pro Ala Leu Ile Pro Val Thr Leu Ser
245 250 255

Val Arg Tyr Pro Pro Glu Val Ser Ile Ser Gly Tyr Asp Asp Asn Trp
260 265 270

Tyr Leu Gly Arg Thr Asp Ala Thr Leu Ser Cys Asp Val His Ser Asn
275 280 285

Pro Glu Pro Thr Gly Tyr Asp Trp Ser Thr Thr Ser Gly Ile Phe Pro
290 295 300

Thr Ser Ala Val Ala Gln Gly Ser Gln Leu Val Ile His Ala Val Asp
305 310 315 320

Ser Leu Phe Asn Thr Thr Phe Val Cys Thr Val Thr Asn Ala Val Gly
325 330 335

Met Gly Arg Ala Glu Gln Val Ile Phe Val Arg Glu Thr Pro Asn Thr
340 345 350

Ala Gly Ala Gly Ala Thr Gly Gly Ile Ile Gly Gly Ile Ile Ala Ala
355 360 365

Ile Ile Ala Thr Ala Val Ala Ala Thr Gly Ile Leu Ile Cys Arg Gln
370 375 380

Gln Arg Lys Glu Gln Thr Leu Gln Gly Ala Glu Glu Asp Glu Asp Leu
385 390 395 400

Glu Gly Pro Pro Ser Tyr Lys Pro Pro Thr Pro Lys Ala Lys Leu Glu
405 410 415

Glu Gln Glu Met Pro Ser Gln Leu Phe Thr Leu Gly Ala Ser Glu His
420 425 430

Ser Pro Leu Lys Thr Pro Tyr Phe Asp Ala Gly Ala Ser Cys Thr Glu
435 440 445

Gln Glu Met Pro Arg Tyr His Glu Leu Pro Thr Leu Glu Glu Arg Ser
450 455 460

Gly Pro Leu His Pro Gly Ala Thr Ser Leu Gly Ser Pro Ile Pro Val
465 470 475 480

Ile Ile Gly Ile Ile Val Val Glu Asp Val Ser Leu Asp Leu Glu Asp
485 490 495

Glu Glu Gly Glu Glu Glu Glu Glu Tyr Leu Asp Lys Ile Asn Pro Val
500 505 510

Tyr Asp Ala Leu Ser Tyr Ser Ser Pro Ser Asp Ser Tyr Gln Gly Lys
515 520 525

Gly Phe Val Met Ser Arg Ala Met Tyr Val
530 535

<210> 87
<211> 1614
<212> DNA
<213> Macaca fascicularis

<400> 87
atggcccggg ccgttgccct cctgccgtcg agatcgccgc cgacgccgct gctgtggccg 60

ctgctgctgc tgctgctccg gaaaaccgga gcccaggatg tgcgagttca agtactaccc 120

gaggtgcgag gccagctcgg gggcaccgtg gagctgccgt gccacctgct gccacctgtt 180

cctggactgt acatctccct ggtgacctgg cagcgcccag atgcacctcc agaccaccag 240

aatgtggccg ccttccaccc taagatgggt cccagcttcc ccagcccgaa gcccggcagc 300

cagcggctgt ccttcgtctc tgccaagcag agcactaggc aagacacaga ggcggagctc 360

caggacgcca cgctggccct ccgcgggctc acggtggagg acgaaggcaa ctacacctgc 420

gagtttgcca ccttccccaa ggggtcggtc cgagggatga cctggctcag agtcatagcc 480

aagccccaga accacgctga ggcccaggag gtcacgttca gccaggaccc tgtgccagtg 540

gcccgctgca tctccaaaga gggtcgccca cctgcccgga tctcctggct ctcatccctg 600

gactgggaag ccaaagagac ccaggtatca gggaccctgg ccggcactgt caccgtcacc 660

agccgcttca ccttggtccc ctcgggccga gcagatggtg tcacggtcac ctgcaaagtg 720

gagcatgaga gcttcgagga gccagccctg atacctgtga ccctctctgt acgctaccct 780

cctgaagtgt ccatctccgg ctatgatgac aactggtacc tcggccgtac tgatgccacc 840

ctgagctgtg acgtccacag caacccagag cccacgggct atgactggag cacgacctca 900

ggcatcttcc caacctccgc agtagcccag ggctcccagc tggtcatcca cgcggtggac 960

agtctgttca ataccacctt cgtctgcaca gtcaccaatg ccgtgggcat gggccgcgct 1020

gagcaggtca tctttgtccg agagaccccc aacacagcag gcgcaggggc cacgggcggc 1080

atcatcgggg gcatcatcgc cgccatcatt gcgactgctg tggctgccac gggcatcctt 1140

atctgccggc agcagcggaa ggagcagacg ctgcaggggg cagaggagga tgaagaccta 1200

gagggacctc cctcctacaa gccaccgacc ccaaaagcga agctggagga gcaggagatg 1260

ccctcccagc tcttcactct gggggcctcg gagcacagcc cactcaagac cccctacttt 1320

gacgctggcg cctcatgcac tgagcaggaa atgcctcgat accatgaatt gcccactttg 1380

gaagagcggt caggacccct gcaccctgga gccacaagcc tgggatcccc catcccggtg 1440

cctccagggc cacctgttgt ggaagacgtt tccctggatc tagaggatga ggagggggag 1500

gaggaggaag agtatctgga taagatcaac cccgtctacg atgctctgtc ctacagcagc 1560

ccctctgatt cctaccaggg caaaggcttt gtcatgtccc gggccatgta cgtg 1614

<210> 88
<211> 25
<212> DNA
<213> Artificial

<220>
<223> Primer

<400> 88
aattgaattc atggcccggg ccgtt 25

<210> 89
<211> 31
<212> DNA
<213> Artificial

<220>
<223> Primer

<400> 89
aattctcgag tcacacgtac atggcccggg a 31

<210> 90
<211> 36
<212> DNA
<213> Artificial

<220>
<223> Primer

<400> 90
agaagcttgc caccatggcc cgggccgctg ccctcc 36

<210> 91

<211> 25
<212> DNA
<213> Artificial

<220>
<223> Primer

<400> 91
cattctggtg gtctggaggt gcatc 25

<210> 92
<211> 33
<212> DNA
<213> Artificial

<220>
<223> Primer

<400> 92
gagaattctc acacatacat ggcccgggac atg 33

<210> 93
<211> 25
<212> DNA
<213> Artificial

<220>
<223> Primer

<400> 93
gatgcacctc cagaccacca gaatg 25

<210> 94
<211> 22
<212> DNA
<213> Artificial

<220>
<223> Primer

<400> 94
ctgtgtcttg cctagtgctc tg 22

<210> 95
<211> 22
<212> DNA
<213> Artificial

<220>
<223> Primer

<400> 95
cagagcacta ggcaagacac ag 22

<210> 96
<211> 21
<212> DNA
<213> Artificial

<220>
<223> Primer

<400> 96
cgtgagcccg cggagggcca g 21

<210> 97
<211> 21
<212> DNA
<213> Artificial

<220>
<223> Primer

<400> 97
ctggccctcc gcgggctcac g 21

<210> 98
<211> 28
<212> DNA
<213> Artificial

<220>
<223> Primer

<400> 98
ggatgtgcga gttgcagtgc tacccgag 28

<210> 99
<211> 28
<212> DNA
<213> Artificial

<220>
<223> Primer

<400> 99
ctcgggtagc actgcaactc gcacatcc 28

<210> 100
<211> 30
<212> DNA
<213> Artificial

<220>
<223> Primer

<400> 100
gagttcaagt gctaggcgag gtgcgaggcc 30

<210> 101
<211> 30
<212> DNA
<213> Artificial

<220>
<223> Primer

<400> 101
ggcctcgcac ctcgcctagc acttgaactc 30

<210> 102
<211> 30
<212> DNA
<213> Artificial

<220>
<223> Primer

<400> 102
ccgaggtgcg aggcgcgctc gggggcaccg 30

<210> 103
<211> 30
<212> DNA
<213> Artificial

<220>
<223> Primer

<400> 103
cggtgccccc gagcgcgcct cgcacctcgg 30

<210> 104
<211> 30
<212> DNA
<213> Artificial

<220>
<223> Primer

<400> 104
tggagctgcc gtgcgccctg ctgccacctg 30

<210> 105
<211> 30
<212> DNA
<213> Artificial

<220>
<223> Primer

<400> 105
caggtggcag cagggcgcac ggcagctcca 30

<210> 106
<211> 27
<212> DNA
<213> Artificial

<220>
<223> Primer

<400> 106
ctgctgccac ctgctcctgg actgtac 27

<210> 107
<211> 27
<212> DNA
<213> Artificial

<220>
<223> Primer

<400> 107
gtacagtcca ggagcaggtg gcagcag 27

<210> 108
<211> 30
<212> DNA
<213> Artificial

<220>
<223> Primer

<400> 108
ctgttcctgg actggccatc tccctggtga 30

<210> 109
<211> 30
<212> DNA
<213> Artificial

<220>
<223> Primer

<400> 109
tcaccaggga gatggccagt ccaggaacag 30

<210> 110

<211> 29
<212> DNA
<213> Artificial

<220>
<223> Primer

<400> 110
cctggtgacc tgggcgcgcc cagatgcac 29

<210> 111
<211> 29
<212> DNA
<213> Artificial

<220>
<223> Primer

<400> 111
gtgcatctgg gcgcgcccag gtcaccagg 29

<210> 112
<211> 29
<212> DNA
<213> Artificial

<220>
<223> Primer

<400> 112
gcagcgccca gatggacctg cgaaccacc 29

<210> 113
<211> 29
<212> DNA
<213> Artificial

<220>
<223> Primer

<400> 113
ggtggttcgc aggtccatct gggcgctgc 29

<210> 114
<211> 28
<212> DNA
<213> Artificial

<220>
<223> Primer

<400> 114
gcgcccagat gcaggtgcga accaccag 28

<210> 115
<211> 28
<212> DNA
<213> Artificial

<220>
<223> Primer

<400> 115
ctggtggttc gcacctgcat ctgggcgc 28

<210> 116
<211> 30
<212> DNA
<213> Artificial

<220>
<223> Primer

<400> 116
gcccagatgc acctgggaac caccagaatg 30

<210> 117
<211> 30
<212> DNA
<213> Artificial

<220>
<223> Primer

<400> 117
cattctggtg gttcccaggt gcatctgggc 30

<210> 118
<211> 30
<212> DNA
<213> Artificial

<220>
<223> Primer

<400> 118
cagatgcacc tgcggcccac cagaatgtgg 30

<210> 119
<211> 30
<212> DNA
<213> Artificial

<220>
<223> Primer

<400> 119
ccacattctg gtgggccgca ggtgcatctg 30

<210> 120
<211> 30
<212> DNA
<213> Artificial

<220>
<223> Primer

<400> 120
atgcacctgc gaacgcccag aatgtggccg 30

<210> 121
<211> 30
<212> DNA
<213> Artificial

<220>
<223> Primer

<400> 121
cggccacatt ctgggcgttc gcaggtgcat 30

<210> 122
<211> 26
<212> DNA
<213> Artificial

<220>
<223> Primer

<400> 122
cctgcgaacc acgcgaatgt ggccgc 26

<210> 123
<211> 26
<212> DNA
<213> Artificial

<220>
<223> Primer

<400> 123
gcggccacat tcgcgtggtt cgcagg 26

<210> 124
<211> 30
<212> DNA
<213> Artificial

<220>
<223> Primer

<400> 124
ctgcgaacca ccaggctgtg gccgccttcc 30

<210> 125
<211> 30
<212> DNA
<213> Artificial

<220>
<223> Primer

<400> 125
ggaaggcggc cacagcctgg tggttcgcag 30

<210> 126
<211> 30
<212> DNA
<213> Artificial

<220>
<223> Primer

<400> 126
ccgccttcca ccctgcgatg ggtcccagct 30

<210> 127
<211> 30
<212> DNA
<213> Artificial

<220>
<223> Primer

<400> 127
agctgggacc catcgcaggg tggaaggcgg 30

<210> 128
<211> 30
<212> DNA
<213> Artificial

<220>
<223> Primer

<400> 128
gtcccagctt ccccgccccg aagcctggca 30

<210> 129

<211> 30
<212> DNA
<213> Artificial

<220>
<223> Primer

<400> 129
tgccaggctt cggggcgggg aagctgggac 30

<210> 130
<211> 30
<212> DNA
<213> Artificial

<220>
<223> Primer

<400> 130
ccttcgtctc tgccgcgcag agcactgggc 30

<210> 131
<211> 30
<212> DNA
<213> Artificial

<220>
<223> Primer

<400> 131
gcccagtgct ctgcgcggca gagacgaagg 30

<210> 132
<211> 29
<212> DNA
<213> Artificial

<220>
<223> Primer

<400> 132
tgggcaagac acagcggcag agctccagg 29

<210> 133
<211> 29
<212> DNA
<213> Artificial

<220>
<223> Primer

<400> 133
cctggagctc tgccgctgtg tcttgccca 29

<210> 134
<211> 29
<212> DNA
<213> Artificial

<220>
<223> Primer

<400> 134
ggcagagctc caggccgcca cgctggccc 29

<210> 135
<211> 29
<212> DNA
<213> Artificial

<220>
<223> Primer

<400> 135
gggccagcgt ggcggcctgg agctctgcc 29

<210> 136
<211> 30
<212> DNA
<213> Artificial

<220>
<223> Primer

<400> 136
ccacgctggc cctcgccggg ctcacggtgg 30

<210> 137
<211> 30
<212> DNA
<213> Artificial

<220>
<223> Primer

<400> 137
ccaccgtgag cccggcgagg gccagcgtgg 30

<210> 138
<211> 28
<212> DNA
<213> Artificial

<220>
<223> Primer

<400> 138
ggaggacgag ggcgcctaca cttgcgag 28

<210> 139
<211> 28
<212> DNA
<213> Artificial

<220>
<223> Primer

<400> 139
ctcgcaagtg taggcgccct cgtcctcc 28

<210> 140
<211> 30
<212> DNA
<213> Artificial

<220>
<223> Primer

<400> 140
gcgagtttgc caccgccccc aaggggtccg 30

<210> 141
<211> 30
<212> DNA
<213> Artificial

<220>
<223> Primer

<400> 141
cggacccctt gggggcggtg gcaaactcgc 30

<210> 142
<211> 30
<212> DNA
<213> Artificial

<220>
<223> Primer

<400> 142
ttgccacctt ccccgcgggg tccgtccgag 30

<210> 143
<211> 30
<212> DNA
<213> Artificial

<220>
<223> Primer

<400> 143
ctcggacgga ccccgcgggg aaggtggcaa 30

<210> 144
<211> 27
<212> DNA
<213> Artificial

<220>
<223> Primer

<400> 144
cccaaggggt ccgccccgagg gatgacc 27

<210> 145
<211> 27
<212> DNA
<213> Artificial

<220>
<223> Primer

<400> 145
ggtcatccct cgggcggacc ccttggg 27

<210> 146
<211> 30
<212> DNA
<213> Artificial

<220>
<223> Primer

<400> 146
ggtccgtccg aggggcgacc tggctcagag 30

<210> 147
<211> 30
<212> DNA
<213> Artificial

<220>
<223> Primer

<400> 147
ctctgagcca ggtcgcccct cggacggacc 30

<210> 148

<211> 29
<212> DNA
<213> Artificial

<220>
<223> Primer

<400> 148
ccgtccgagg gatggcctgg ctcagagtc 29

<210> 149
<211> 29
<212> DNA
<213> Artificial

<220>
<223> Primer

<400> 149
gactctgagc caggccatcc ctcggacgg 29

<210> 150
<211> 30
<212> DNA
<213> Artificial

<220>
<223> Primer

<400> 150
ccaagcccaa gaacgcagct gaggcccaga 30

<210> 151
<211> 30
<212> DNA
<213> Artificial

<220>
<223> Primer

<400> 151
tctgggcctc agctgcgttc ttgggcttgg 30

<210> 152
<211> 30
<212> DNA
<213> Artificial

<220>
<223> Primer

<400> 152
aagctgaggc ccaggcggtc acgttcagcc 30

<210> 153
<211> 30
<212> DNA
<213> Artificial

<220>
<223> Primer

<400> 153
ggctgaacgt gaccgcctgg gcctcagctt 30

<210> 154
<211> 30
<212> DNA
<213> Artificial

<220>
<223> Primer

<400> 154
cccagaaggt cacggccagc caggacccta 30

<210> 155
<211> 30
<212> DNA
<213> Artificial

<220>
<223> Primer

<400> 155
tagggtcctg gctggccgtg accttctggg 30

<210> 156
<211> 30
<212> DNA
<213> Artificial

<220>
<223> Primer

<400> 156
cgttcagcca ggacggtacg acagtggccc 30

<210> 157
<211> 30
<212> DNA
<213> Artificial

<220>
<223> Primer

<400> 157
gggccactgt cgtaccgtcc tggctgaacg 30

<210> 158
<211> 30
<212> DNA
<213> Artificial

<220>
<223> Primer

<400> 158
cagtggccct ctgcgcctcc aaagagggcc 30

<210> 159
<211> 30
<212> DNA
<213> Artificial

<220>
<223> Primer

<400> 159
ggccctcttt ggaggcgcag agggccactg 30

<210> 160
<211> 30
<212> DNA
<213> Artificial

<220>
<223> Primer

<400> 160
ccctctgcat ctccgcagag ggccgcccac 30

<210> 161
<211> 30
<212> DNA
<213> Artificial

<220>
<223> Primer

<400> 161
gtgggcggcc ctctgcggag atgcagaggg 30

<210> 162
<211> 30
<212> DNA
<213> Artificial

<220>
<223> Primer

<400> 162
cccggatctc ctgggcctca tccctggact 30

<210> 163
<211> 30
<212> DNA
<213> Artificial

<220>
<223> Primer

<400> 163
agtccaggga tgaggcccag gagatccggg 30

<210> 164
<211> 30
<212> DNA
<213> Artificial

<220>
<223> Primer

<400> 164
tctcatccct ggacgcggaa gccaaagaga 30

<210> 165
<211> 30
<212> DNA
<213> Artificial

<220>
<223> Primer

<400> 165
tctctttggc ttccgcgtcc agggatgaga 30

<210> 166
<211> 29
<212> DNA
<213> Artificial

<220>
<223> Primer

<400> 166
ctgggaagcc aaagcgactc aggtgtcag 29

<210> 167

<211> 29
<212> DNA
<213> Artificial

<220>
<223> Primer

<400> 167
ctgacacctg agtcgctttg gcttcccag 29

<210> 168
<211> 29
<212> DNA
<213> Artificial

<220>
<223> Primer

<400> 168
ctcaggtgtc aggggccctg gccggaact 29

<210> 169
<211> 29
<212> DNA
<213> Artificial

<220>
<223> Primer

<400> 169
agttccggcc agggcccctg acacctgag 29

<210> 170
<211> 29
<212> DNA
<213> Artificial

<220>
<223> Primer

<400> 170
gagcagatgg tgtcgcggtc acctgcaaa 29

<210> 171
<211> 29
<212> DNA
<213> Artificial

<220>
<223> Primer

<400> 171
tttgcaggtg accgcgacac catctgctc 29

<210> 172
<211> 30
<212> DNA
<213> Artificial

<220>
<223> Primer

<400> 172
tcacggtcac ctgcgcagtg gagcatgaga 30

<210> 173
<211> 30
<212> DNA
<213> Artificial

<220>
<223> Primer

<400> 173
tctcatgctc cactgcgcag gtgaccgtga 30

<210> 174
<211> 29
<212> DNA
<213> Artificial

<220>
<223> Primer

<400> 174
cttcgaggaa ccaggcctga tacctgtga 29

<210> 175
<211> 29
<212> DNA
<213> Artificial

<220>
<223> Primer

<400> 175
tcacaggtat caggcctggt tcctcgaag 29

<210> 176
<211> 39
<212> DNA
<213> Artificial

<220>
<223> Primer

<400> 176
caaaagcttg ccgccaccat gaaacacctg tggttcttc 39

<210> 177
<211> 29
<212> DNA
<213> Artificial

<220>
<223> Primer

<400> 177
aaaatgaatt ctcatttacc cggagacag 29

<210> 178
<211> 33
<212> DNA
<213> Artificial

<220>
<223> Primer

<400> 178
ataaagcttg ccgccaccat ggacatgagg gtc 33

<210> 179
<211> 38
<212> DNA
<213> Artificial

<220>
<223> Primer

<400> 179
ccggaattcc taacactctc ccctgttgaa gctctttg 38

<210> 180
<211> 36
<212> DNA
<213> Artificial

<220>
<223> Primer

<400> 180
caaaagcttg ccgccaccat ggaarcccca gckcag 36

<210> 181
<211> 35
<212> DNA
<213> Artificial

<220>
<223> Primer

<400> 181
caaaagcttg ccgccaccat ggaactgggg ctccg 35

<210> 182
<211> 30
<212> DNA
<213> Artificial

<220>
<223> Primer

<400> 182
caaaagcttg ccgccaccat gttgccatca 30

<210> 183
<211> 19
<212> DNA
<213> Artificial

<220>
<223> Primer

<400> 183
aagtagtcct tgaccaggc 19

<210> 184
<211> 5
<212> PRT
<213> Homo sapiens

<400> 184

Ser Tyr Tyr Trp Ser
1 5

<210> 185
<211> 16
<212> PRT
<213> Homo sapiens

<400> 185

Tyr Ile Tyr Tyr Ser Gly Ser Thr Asn His Asn Pro Ser Leu Lys Ser
1 5 10 15

<210> 186
<211> 12
<212> PRT

<213> Homo sapiens

<400> 186

Asp Gly Gly Asp Asp Tyr Asn Tyr Gly Met Asp Val
1               5                   10

<210> 187
<211> 121
<212> PRT
<213> Homo sapiens

<400> 187

Gln Val Gln Leu Gln Glu Ser Gly Pro Gly Leu Val Lys Pro Ser Glu
1               5                   10                  15

Thr Leu Ser Leu Ile Cys Thr Val Ser Gly Gly Ser Ile Ser Ser Tyr
            20                  25                  30

Tyr Trp Ser Trp Ile Arg Gln Pro Pro Gly Lys Gly Leu Glu Trp Ile
        35                  40                  45

Gly Tyr Ile Tyr Tyr Ser Gly Ser Thr Asn His Asn Pro Ser Leu Lys
    50                  55                  60

Ser Arg Val Thr Ile Ser Val Asp Thr Ala Lys Asn Gln Phe Ser Leu
65                  70                  75                  80

Lys Leu Asn Ser Val Thr Ala Ala Asp Thr Ala Val Tyr Tyr Cys Ala
                85                  90                  95

Arg Asp Gly Gly Asp Asp Tyr Asn Tyr Gly Met Asp Val Trp Gly Gln
            100                 105                 110

Gly Thr Thr Val Thr Val Ser Ser Ala
        115                 120

<210> 188
<211> 15
<212> DNA
<213> Homo sapiens

<400> 188
agttactact ggagc                                                        15

<210> 189

<211> 48
<212> DNA
<213> Homo sapiens

<400> 189
tatatctatt acagtgggag caccaaccac aacccctccc tcaagagt 48

<210> 190
<211> 36
<212> DNA
<213> Homo sapiens

<400> 190
gatggtgggg acgactacaa ctacggtatg gacgtc 36

<210> 191
<211> 363
<212> DNA
<213> Homo sapiens

<400> 191
caggtgcagc tgcaggagtc gggcccagga ctggtgaagc cttcggagac cctgtccctc 60

atctgcactg tctctggtgg ctccatcagt agttactact ggagctggat ccggcagccc 120

ccagggaagg gactggagtg gattgggtat atctattaca gtgggagcac caaccacaac 180

ccctccctca agagtcgagt caccatatca gtagacacgg ccaagaacca gttctccctg 240

aagctgaact ctgtgaccgc tgcggacacg gccgtgtatt actgtgcgag agatggtggg 300

gacgactaca actacggtat ggacgtctgg ggccaaggga ccacggtcac cgtctcctca 360

gcc 363

<210> 192
<211> 11
<212> PRT
<213> Homo sapiens

<220>
<221> misc_feature
<222> (9)..(9)
<223> Xaa can be any naturally occurring amino acid

<400> 192

Arg Ala Ser Gln Gly Ile Ser Ser Xaa Leu Ala
1 5 10

<210> 193
<211> 7

<212> PRT
<213> Homo sapiens

<220>
<221> misc_feature
<222> (6)..(6)
<223> Xaa can be any naturally occurring amino acid

<400> 193

Asp Ala Ser Ser Leu Xaa Ser
1               5

<210> 194
<211> 9
<212> PRT
<213> Homo sapiens

<220>
<221> misc_feature
<222> (3)..(3)
<223> Xaa can be any naturally occurring amino acid

<220>
<221> misc_feature
<222> (8)..(8)
<223> Xaa can be any naturally occurring amino acid

<400> 194

Gln Gln Xaa Asn Ser Tyr Pro Xaa Thr
1               5

<210> 195
<211> 107
<212> PRT
<213> Homo sapiens

<220>
<221> misc_feature
<222> (32)..(32)
<223> Xaa can be any naturally occurring amino acid

<220>
<221> misc_feature
<222> (46)..(46)
<223> Xaa can be any naturally occurring amino acid

<220>
<221> misc_feature
<222> (55)..(55)
<223> Xaa can be any naturally occurring amino acid

<220>
<221> misc_feature
<222> (58)..(58)
<223> Xaa can be any naturally occurring amino acid

<220>
<221> misc_feature
<222> (91)..(91)
<223> Xaa can be any naturally occurring amino acid

<220>
<221> misc_feature
<222> (96)..(96)
<223> Xaa can be any naturally occurring amino acid

<400> 195

Ala Ile Gln Leu Thr Gln Ser Pro Ser Ser Leu Ser Ala Ser Val Gly
1 5 10 15

Asp Arg Val Thr Ile Thr Cys Arg Ala Ser Gln Gly Ile Ser Ser Xaa
20 25 30

Leu Ala Trp Tyr Gln Gln Lys Pro Gly Lys Ala Pro Lys Xaa Leu Ile
35 40 45

Tyr Asp Ala Ser Ser Leu Xaa Ser Gly Xaa Pro Ser Arg Phe Ser Gly
50 55 60

Ser Gly Ser Gly Thr Asp Phe Thr Leu Thr Ile Ile Ser Leu Gln Pro
65 70 75 80

Glu Asp Phe Ala Thr Tyr Tyr Cys Gln Gln Xaa Asn Ser Tyr Pro Xaa
85 90 95

Thr Phe Gly Gln Gly Thr Lys Val Glu Ile Lys
100 105

<210> 196
<211> 33
<212> DNA
<213> Homo sapiens

<400> 196
cgggcragtc agggcattag cagygsktta gcc 33

<210> 197
<211> 21

<212> DNA
<213> Homo sapiens

<400> 197
gatgcmtcca gtttgsaaag t 21

<210> 198
<211> 27
<212> DNA
<213> Homo sapiens

<400> 198
caacagtwta atagttaccc tyggacg 27

<210> 199
<211> 321
<212> DNA
<213> Homo sapiens

<400> 199
gccatccagt tgacccagtc tccatcctcc ctgtctgcat ctgtaggaga cagagtcacc 60

atcacttgyc gggcragtca gggcattagc agygskttag cctggtatca gcagaaacca 120

gggaaagctc ctaagytcct gatctatgat gcmtccagtt tgsaaagtgg grtcccatca 180

aggttcagcg gcagtggatc tgggacagat ttcactctca ccatcatcag cctgcagcct 240

gaagattttg caacttatta ctgtcaacag twtaatagtt accctyggac gttcggccaa 300

gggaccaagg tggaaatcaa a 321

<210> 200
<211> 5
<212> PRT
<213> Homo sapiens

<400> 200

Ser Tyr Tyr Trp Thr
1 5

<210> 201
<211> 16
<212> PRT
<213> Homo sapiens

<400> 201

Tyr Val Tyr Tyr Ser Gly Ser Thr Asn Tyr Asn Pro Ser Leu Lys Ser
1 5 10 15

<210> 202
<211> 12
<212> PRT
<213> Homo sapiens

<400> 202

Asp Pro Gly Glu Asp Tyr Tyr Tyr Gly Met Asp Val
1 5 10

<210> 203
<211> 121
<212> PRT
<213> Homo sapiens

<400> 203

Gln Val Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ser Ser Tyr
20 25 30

Tyr Trp Thr Trp Ile Arg Gln Pro Pro Gly Lys Gly Leu Glu Trp Ile
35 40 45

Gly Tyr Val Tyr Tyr Ser Gly Ser Thr Asn Tyr Asn Pro Ser Leu Lys
50 55 60

Ser Arg Val Thr Ile Ser Val Asp Thr Ser Lys Ser Gln Phe Ser Leu
65 70 75 80

Lys Leu Ser Ser Val Thr Ala Ala Asp Thr Ala Val Tyr Tyr Cys Thr
85 90 95

Arg Asp Pro Gly Glu Asp Tyr Tyr Tyr Gly Met Asp Val Trp Gly Gln
100 105 110

Gly Thr Thr Val Thr Val Ser Ser Ala
115 120

<210> 204
<211> 15
<212> DNA
<213> Homo sapiens

<400> 204
agttactact ggacc 15

<210> 205
<211> 48
<212> DNA
<213> Homo sapiens

<400> 205
tatgtctatt acagtgggag caccaactac aacccctccc tcaagagt 48

<210> 206
<211> 36
<212> DNA
<213> Homo sapiens

<400> 206
gaccctgggg aagactacta ctacggtatg gacgtc 36

<210> 207
<211> 363
<212> DNA
<213> Homo sapiens

<400> 207
caggtgcagc tgcaggagtc gggcccagga ctggtgaagc cttcggagac cctgtccctc 60

acctgcactg tctctggtgg ctccatcagt agttactact ggacctggat ccggcagccc 120

ccagggaagg gactggagtg gattggatat gtctattaca gtgggagcac caactacaac 180

ccctccctca agagtcgagt caccatatca gtagacacgt ccaagagcca gttctccctg 240

aagctgagct ctgtgaccgc tgcggacacg gccgtgtatt actgtacgag agaccctggg 300

gaagactact actacggtat ggacgtctgg ggccaaggga ccacggtcac cgtctcctca 360

gcc 363

<210> 208
<211> 11
<212> PRT
<213> Homo sapiens

<220>
<221> misc_feature
<222> (9)..(9)
<223> Xaa can be any naturally occurring amino acid

<400> 208

Arg Ala Ser Gln Gly Ile Ser Ser Xaa Leu Ala
1 5 10

<210> 209
<211> 7
<212> PRT
<213> Homo sapiens

<400> 209

Asp Ala Ser Ser Leu Glu Ser
1 5

<210> 210
<211> 9
<212> PRT
<213> Homo sapiens

<400> 210

Gln Gln Phe Asn Ser Tyr Pro Arg Thr
1 5

<210> 211
<211> 107
<212> PRT
<213> Homo sapiens

<220>
<221> misc_feature
<222> (32)..(32)
<223> Xaa can be any naturally occurring amino acid

<400> 211

Ala Ile Gln Leu Thr Gln Ser Pro Ser Ser Leu Ser Ala Ser Val Gly
1 5 10 15

Asp Arg Val Thr Ile Thr Cys Arg Ala Ser Gln Gly Ile Ser Ser Xaa
20 25 30

Leu Ala Trp Tyr Gln Gln Lys Pro Gly Lys Ala Pro Lys Leu Leu Ile
35 40 45

Tyr Asp Ala Ser Ser Leu Glu Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Phe Asn Ser Tyr Pro Arg

85 90 95

Thr Phe Gly Gln Gly Thr Lys Val Glu Ile Lys
100 105

<210> 212
<211> 33
<212> DNA
<213> Homo sapiens

<400> 212
cgggcaagtc agggcattag cagtgsttta gcc 33

<210> 213
<211> 21
<212> DNA
<213> Homo sapiens

<400> 213
gatgcctcca gtttggaaag t 21

<210> 214
<211> 27
<212> DNA
<213> Homo sapiens

<400> 214
caacagttta atagttaccc tcggacg 27

<210> 215
<211> 321
<212> DNA
<213> Homo sapiens

<400> 215
gccatccagt tgacccagtc tccatcctcc ctgtctgcat ctgtaggaga cagagtcacc 60

atcacttgcc gggcaagtca gggcattagc agtgstttag cctggtatca gcagaaacca 120

gggaaagctc ctaagctcct gatctatgat gcctccagtt tggaaagtgg ggtcccatca 180

aggttcagcg gcagtggatc tgggacagat ttcactctca ccatcagcag cctgcagcct 240

gaagattttg caacttatta ctgtcaacag tttaatagtt accctcggac gttcggccaa 300

gggaccaagg tggaaatcaa a 321

<210> 216
<211> 5
<212> PRT
<213> Homo sapiens

<400> 216

Ser Tyr Asn Met Asn
1 5

<210> 217
<211> 17
<212> PRT
<213> Homo sapiens

<400> 217

Ser Ile Ser Ser Ser Ser Ser Tyr Ile Tyr Tyr Ala Asp Ser Val Lys
1 5 10 15

Gly

<210> 218
<211> 13
<212> PRT
<213> Homo sapiens

<400> 218

Asp Tyr Tyr Gly Ser Gly Thr Tyr Tyr Leu Phe Asp Tyr
1 5 10

<210> 219
<211> 123
<212> PRT
<213> Homo sapiens

<400> 219

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Asn Met Asn Trp Val Arg Gln Ala Pro Gly Lys Gly Leu Glu Trp Val
35 40 45

Ser Ser Ile Ser Ser Ser Ser Ser Tyr Ile Tyr Tyr Ala Asp Ser Val
50 55 60

Lys Gly Arg Phe Thr Ile Ser Arg Asp Asn Ala Lys Asn Ser Leu Tyr

65 70 75 80

Leu Gln Met Asn Ser Leu Arg Ala Glu Asp Thr Ala Val Tyr Tyr Cys
85 90 95

Ala Arg Asp Tyr Tyr Gly Ser Gly Thr Tyr Tyr Leu Phe Asp Tyr Trp
100 105 110

Gly Gln Gly Thr Leu Val Thr Val Ser Ser Ala
115 120

<210> 220
<211> 15
<212> DNA
<213> Homo sapiens

<400> 220
agctataaca tgaac 15

<210> 221
<211> 51
<212> DNA
<213> Homo sapiens

<400> 221
tccattagta gtagtagtag ttacatatac tacgcagact cagtgaaggg c 51

<210> 222
<211> 39
<212> DNA
<213> Homo sapiens

<400> 222
gattactatg gttcggggac ttattatctc tttgactac 39

<210> 223
<211> 369
<212> DNA
<213> Homo sapiens

<400> 223
gaggtgcagc tggtggagtc tgggggaggc ctggtcaagc ctgggggtc cctgagactc 60

tcctgtgcag cctctggatt caccttcagt agctataaca tgaactgggt ccgccaggct 120

ccagggaagg ggctggagtg ggtctcatcc attagtagta gtagtagtta catatactac 180

gcagactcag tgaagggccg attcaccatc tccagagaca acgccaagaa ctcactgtat 240

ctgcaaatga acagcctgag agccgaggac acggctgtgt attactgtgc gagagattac 300

tatggttcgg ggacttatta tctctttgac tactggggcc agggaaccct ggtcaccgtc 360

tcctcagcc 369

<210> 224
<211> 11
<212> PRT
<213> Homo sapiens

<400> 224

Arg Ala Ser Gln Ser Ile Gly Ser Ser Leu His
1 5 10

<210> 225
<211> 7
<212> PRT
<213> Homo sapiens

<400> 225

Tyr Ala Ser Gln Ser Phe Ser
1 5

<210> 226
<211> 9
<212> PRT
<213> Homo sapiens

<400> 226

His Gln Ser Arg Ser Leu Pro Ile Thr
1 5

<210> 227
<211> 107
<212> PRT
<213> Homo sapiens

<400> 227

Glu Ile Val Leu Thr Gln Ser Pro Asp Phe Gln Ser Val Thr Pro Lys
1 5 10 15

Glu Lys Val Thr Ile Thr Cys Arg Ala Ser Gln Ser Ile Gly Ser Ser
20 25 30

Leu His Trp Tyr Gln Gln Lys Pro Asp Gln Ser Pro Lys Leu Leu Ile
35 40 45

Lys Tyr Ala Ser Gln Ser Phe Ser Gly Val Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Ala Tyr Tyr Cys His Gln Ser Arg Ser Leu Pro Ile
                85                  90                  95

Thr Phe Gly Gln Gly Thr Arg Leu Glu Ile Lys
            100                 105

<210> 228
<211> 33
<212> DNA
<213> Homo sapiens

<400> 228
cgggccagtc agagcattgg tagtagctta cac 33

<210> 229
<211> 21
<212> DNA
<213> Homo sapiens

<400> 229
tatgcttccc agtccttctc a 21

<210> 230
<211> 27
<212> DNA
<213> Homo sapiens

<400> 230
catcagagta ggagtttacc gatcacc 27

<210> 231
<211> 321
<212> DNA
<213> Homo sapiens

<400> 231
gaaattgtgc tgactcagtc tccagacttt cagtctgtga ctccaaagga gaaagtcacc 60

atcacctgcc gggccagtca gagcattggt agtagcttac actggtacca gcagaaacca 120

gatcagtctc caaagctcct catcaagtat gcttcccagt ccttctcagg ggtcccctcg 180

aggttcagtg gcagtggatc tgggacagat ttcaccctca ccatcaatag cctggaagct 240

gaagatgctg cagcgtatta ctgtcatcag agtaggagtt taccgatcac cttcggccaa 300

gggacacgac tggagattaa a 321

<210> 232
<211> 5
<212> PRT
<213> Homo sapiens

<400> 232

Ser Tyr Tyr Trp Thr
1 5

<210> 233
<211> 16
<212> PRT
<213> Homo sapiens

<400> 233

Tyr Ile Tyr Tyr Ser Gly Ser Thr Asn Ser Asn Pro Ser Leu Lys Ser
1 5 10 15

<210> 234
<211> 12
<212> PRT
<213> Homo sapiens

<400> 234

Asp Pro Gly Glu Asp Tyr Asn Tyr Gly Met Asp Val
1 5 10

<210> 235
<211> 121
<212> PRT
<213> Homo sapiens

<400> 235

Gln Val Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ser Ser Tyr
20 25 30

Tyr Trp Thr Trp Ile Arg Gln Pro Pro Gly Lys Gly Leu Glu Trp Ile
35 40 45

Gly Tyr Ile Tyr Tyr Ser Gly Ser Thr Asn Ser Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Thr
                85                  90                  95

Arg Asp Pro Gly Glu Asp Tyr Asn Tyr Gly Met Asp Val Trp Gly Gln
            100                 105                 110

Gly Thr Thr Val Thr Val Ser Ser Ala
        115                 120

<210> 236
<211> 15
<212> DNA
<213> Homo sapiens

<400> 236
agttactact ggacc 15

<210> 237
<211> 48
<212> DNA
<213> Homo sapiens

<400> 237
tatatctatt acagtgggag caccaactcc aacccctccc tcaagagt 48

<210> 238
<211> 36
<212> DNA
<213> Homo sapiens

<400> 238
gaccctgggg aagactacaa ctacggtatg gacgtc 36

<210> 239
<211> 363
<212> DNA
<213> Homo sapiens

<400> 239
caggtgcagc tgcaggagtc gggcccagga ctggtgaagc cttcggagac cttgtccctc 60

acctgcactg tctctggtgg ctccatcagt agttactact ggacctggat ccggcagccc 120

ccagggaagg gactggagtg gattgggtat atctattaca gtgggagcac caactccaac 180

ccctccctca agagtcgagt caccatatca gtagacacgt ccaagaacca gttctccctg 240

aagctgagtt ctgtgaccgc tgcggacacg gccgtgtatt actgtacgag agaccctggg 300

gaagactaca actacggtat ggacgtctgg ggccaaggga ccacggtcac cgtctcctca 360

gcc 363

<210> 240
<211> 11
<212> PRT
<213> Homo sapiens

<400> 240

Arg Ala Ser Gln Gly Ile Ser Ser Ala Leu Ala
1 5 10

<210> 241
<211> 7
<212> PRT
<213> Homo sapiens

<400> 241

Asp Ala Ser Ser Leu Glu Ser
1 5

<210> 242
<211> 9
<212> PRT
<213> Homo sapiens

<400> 242

Gln Gln Phe Asn Ser Tyr Pro Arg Thr
1 5

<210> 243
<211> 107
<212> PRT
<213> Homo sapiens

<400> 243

Ala Ile Gln Leu Thr Gln Ser Pro Ser Ser Leu Ser Ala Ser Val Gly
1 5 10 15

Asp Arg Val Thr Ile Thr Cys Arg Ala Ser Gln Gly Ile Ser Ser Ala

20 25 30

Leu Ala Trp Tyr Gln Gln Lys Pro Gly Lys Ala Pro Lys Leu Leu Ile
35 40 45

Tyr Asp Ala Ser Ser Leu Glu Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln Gln Phe Asn Ser Tyr Pro Arg
85 90 95

Thr Phe Gly Gln Gly Thr Lys Val Glu Ile Lys
100 105

<210> 244
<211> 33
<212> DNA
<213> Homo sapiens

<400> 244
cgggcaagtc agggcattag cagtgcttta gcc 33

<210> 245
<211> 21
<212> DNA
<213> Homo sapiens

<400> 245
gatgcctcca gtttggaaag t 21

<210> 246
<211> 27
<212> DNA
<213> Homo sapiens

<400> 246
caacagttta atagttaccc tcggacg 27

<210> 247
<211> 321
<212> DNA
<213> Homo sapiens

<400> 247
gccatccagt tgacccagtc tccatcctcc ctgtctgcat ctgtaggaga cagagtcacc 60

atcacttgcc gggcaagtca gggcattagc agtgctttag cctggtatca gcagaaacca 120

gggaaagctc ctaagctcct gatctatgat gcctccagtt tggaaagtgg ggtcccatca 180

aggttcagcg gcagtggatc tgggacagat ttcactctca ccatcagcag cctgcagcct 240

gaagattttg caacttatta ctgtcaacag tttaatagtt accctcggac gttcggccaa 300

gggaccaagg tggaaatcaa a 321

<210> 248
<211> 5
<212> PRT
<213> Homo sapiens

<400> 248

Ser Tyr Tyr Trp Ser
1 5

<210> 249
<211> 16
<212> PRT
<213> Homo sapiens

<400> 249

Tyr Ile Tyr Tyr Ser Gly Ser Thr Asn Tyr Asn Pro Ser Leu Lys Ser
1 5 10 15

<210> 250
<211> 12
<212> PRT
<213> Homo sapiens

<400> 250

Asp Ala Gly Glu Asp Tyr Ser Tyr Gly Met Asp Val
1 5 10

<210> 251
<211> 121
<212> PRT
<213> Homo sapiens

<400> 251

Gln Val Gln Leu Gln Glu Ser Gly Pro Gly Leu Val Lys Pro Ser Glu
1 5 10 15

Thr Leu Ser Leu Thr Cys Thr Val Ser Ser Ala Ser Ile Ser Ser Tyr

20 25 30

Tyr Trp Ser Trp Ile Arg Gln Pro Pro Gly Lys Gly Leu Glu Trp Ile
35 40 45

Gly Tyr Ile Tyr Tyr Ser Gly Ser Thr Asn Tyr Asn Pro Ser Leu Lys
50 55 60

Ser Arg Val Thr Ile Ser Ile Asp Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Asp Ala Gly Glu Asp Tyr Ser Tyr Gly Met Asp Val Trp Gly Gln
100 105 110

Gly Thr Thr Val Thr Val Ser Ser Ala
115 120

<210> 252
<211> 15
<212> DNA
<213> Homo sapiens

<400> 252
agttactact ggagc 15

<210> 253
<211> 48
<212> DNA
<213> Homo sapiens

<400> 253
tatatctatt acagtgggag caccaactac aacccctccc tcaagagt 48

<210> 254
<211> 36
<212> DNA
<213> Homo sapiens

<400> 254
gatgctgggg aggactactc ctacggtatg gacgtc 36

<210> 255
<211> 363
<212> DNA

<213> Homo sapiens

<400> 255
caggtgcagc tgcaggagtc gggcccagga ctggtgaagc cttcggagac cctgtccctc 60

acctgcactg tctctagtgc ctccatcagt agttactact ggagctggat ccggcagccc 120

ccagggaagg gactggagtg gattgggtat atctattaca gtgggagcac caactacaac 180

ccctccctca agagtcgagt caccatatca atagacacgt ccaagaacca gttctccctg 240

aagctgagct ctgtgaccgc tgcggacacg gccgtgtatt actgtgcgag agatgctggg 300

gaggactact cctacggtat ggacgtctgg ggccaaggga ccacggtcac cgtctcctca 360

gcc 363

<210> 256
<211> 11
<212> PRT
<213> Homo sapiens

<400> 256

Arg Ala Ser Gln Gly Ile Ser Ser Ala Leu Ala
1 5 10

<210> 257
<211> 7
<212> PRT
<213> Homo sapiens

<400> 257

Asp Ala Ser Ser Leu Glu Ser
1 5

<210> 258
<211> 8
<212> PRT
<213> Homo sapiens

<400> 258

Gln Gln Phe Asn Ser Tyr Arg Thr
1 5

<210> 259
<211> 106
<212> PRT
<213> Homo sapiens

<400> 259

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Arg Thr
                85                  90                  95

Phe Gly Gln Gly Thr Lys Val Glu Ile Lys
            100                 105

<210> 260
<211> 33
<212> DNA
<213> Homo sapiens

<400> 260
cgggcaagtc agggcattag cagtgcttta gcc 33

<210> 261
<211> 21
<212> DNA
<213> Homo sapiens

<400> 261
gatgcctcca gtttggaaag t 21

<210> 262
<211> 24
<212> DNA
<213> Homo sapiens

<400> 262
caacagttta atagttaccg gacg 24

<210> 263

<211> 318
<212> DNA
<213> Homo sapiens

<400> 263
gccatccagt tgacccagtc tccatcctcc ctgtctgcat ctgtaggaga cagagtcacc 60

atcacttgcc gggcaagtca gggcattagc agtgctttag cctggtatca gcagaaacca 120

gggaaagctc ctaagctcct gatctatgat gcctccagtt tggaaagtgg ggtcccatca 180

aggttcagcg gcagtggatc tgggacagat ttcactctca ccatcagcag cctgcagcct 240

gaagattttg caacttatta ctgtcaacag tttaatagtt accggacgtt cggccaaggg 300

accaaggtgg aaatcaaa 318

<210> 264
<211> 5
<212> PRT
<213> Homo sapiens

<400> 264

Ser Tyr Tyr Trp Thr
1 5

<210> 265
<211> 16
<212> PRT
<213> Homo sapiens

<400> 265

Tyr Ile Tyr Tyr Ser Gly Ser Thr Asn Tyr Asn Pro Ser Leu Lys Ser
1 5 10 15

<210> 266
<211> 12
<212> PRT
<213> Homo sapiens

<400> 266

Asp Pro Gly Glu Asp Tyr Asn Tyr Gly Met Asp Val
1 5 10

<210> 267
<211> 121
<212> PRT
<213> Homo sapiens

<400> 267

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Tyr
            20                  25                  30

Tyr Trp Thr Trp Ile Arg Gln Pro Pro Gly Lys Gly Leu Glu Trp Ile
        35                  40                  45

Gly Tyr Ile Tyr Tyr Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Thr
                85                  90                  95

Arg Asp Pro Gly Glu Asp Tyr Asn Tyr Gly Met Asp Val Trp Gly Gln
            100                 105                 110

Gly Thr Thr Val Thr Val Ser Ser Ala
        115                 120

<210> 268
<211> 15
<212> DNA
<213> Homo sapiens

<400> 268
agttactact ggacc 15

<210> 269
<211> 48
<212> DNA
<213> Homo sapiens

<400> 269
tatatctatt acagtgggag caccaactac aacccctccc tcaagagt 48

<210> 270
<211> 36
<212> DNA
<213> Homo sapiens

<400> 270

gaccctgggg aagactacaa ctacggtatg gacgtc 36

<210> 271
<211> 363
<212> DNA
<213> Homo sapiens

<400> 271
caggtgcagc tgcaggagtc gggcccagga ctggtgaagc cttcggagac cttgtccctc 60

acctgcactg tctctggtgg ctccatcagt agttactact ggacctggat ccggcagccc 120

ccagggaagg gactggagtg gattgggtat atctattaca gtgggagcac caactacaac 180

ccctccctca agagtcgagt caccatatca gtagacacgt ccaagaacca gttctccctg 240

aagctgagtt ctgtgaccgc tgcggacacg gccgtgtatt actgtacgag agaccctggg 300

gaagactaca actacggtat ggacgtctgg ggccaaggga ccacggtcac cgtctcctca 360

gcc 363

<210> 272
<211> 11
<212> PRT
<213> Homo sapiens

<400> 272

Arg Ala Ser Gln Gly Ile Ser Ser Ala Leu Ala
1 5 10

<210> 273
<211> 7
<212> PRT
<213> Homo sapiens

<400> 273

Asp Ala Ser Ser Leu Glu Ser
1 5

<210> 274
<211> 9
<212> PRT
<213> Homo sapiens

<400> 274

Gln Gln Phe Asn Ser Tyr Pro Arg Thr
1 5

<210> 275
<211> 107
<212> PRT
<213> Homo sapiens

<400> 275

```
Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro Arg
                85                  90                  95

Thr Phe Gly Gln Gly Thr Lys Val Glu Ile Lys
            100                 105
```

<210> 276
<211> 33
<212> DNA
<213> Homo sapiens

<400> 276
cgggcragtc agggyattag cagygcttta gcc 33

<210> 277
<211> 21
<212> DNA
<213> Homo sapiens

<400> 277
gatgcctcca gtttggaaag t 21

<210> 278
<211> 27
<212> DNA
<213> Homo sapiens

<400> 278
caacagttta atagttaccc tcggacg 27

<210> 279
<211> 321
<212> DNA
<213> Homo sapiens

<400> 279
gccatccagt tgacccagtc tccatcctcc ctgtctgcat ctgtaggaga cagagtcacc 60

atcacttgcc gggcragtca gggyattagc agygctttag cctggtatca gcagaaacca 120

gggaaagctc ctaagctcct gatctatgat gcctccagtt tggaaagtgg ggtcccatca 180

aggttcagcg gcagtggatc tgggacagat ttcactctca ccatcagcag cctgcagcct 240

gaagattttg caacttatta ctgtcaacag tttaatagtt accctcggac gttcggccaa 300

gggaccaagg tggaaatcaa a 321

<210> 280
<211> 5
<212> PRT
<213> Homo sapiens

<220>
<221> misc_feature
<222> (3)..(3)
<223> Xaa can be any naturally occurring amino acid

<400> 280

Ser Tyr Xaa Trp Thr
1 5

<210> 281
<211> 16
<212> PRT
<213> Homo sapiens

<400> 281

Tyr Ile Tyr Tyr Ser Gly Ser Thr Asn Tyr Asn Pro Ser Leu Lys Ser
1 5 10 15

<210> 282
<211> 12
<212> PRT
<213> Homo sapiens

<400> 282

Asp Pro Gly Glu Asp Tyr Asn Tyr Gly Met Asp Val
1 5 10

<210> 283
<211> 121
<212> PRT
<213> Homo sapiens

<220>
<221> misc_feature
<222> (33)..(33)
<223> Xaa can be any naturally occurring amino acid

<400> 283

Gln Val Gln Leu Gln Glu Ser Gly Pro Gly Leu Val Lys Pro Ser Glu
1 5 10 15

Thr Leu Ser Leu Thr Cys Thr Val Ser Gly Gly Ser Ile Ser Ser Tyr
20 25 30

Xaa Trp Thr Trp Ile Arg Gln Pro Pro Gly Lys Gly Leu Glu Trp Ile
35 40 45

Gly Tyr Ile Tyr Tyr Ser Gly Ser Thr Asn Tyr Asn Pro Ser Leu Lys
50 55 60

Ser Arg Val Thr Ile Ser Leu Asp Thr Ser Lys Asn Gln Phe Ser Leu
65 70 75 80

Lys Leu Ser Ser Val Thr Ala Ala Asp Thr Ala Val Tyr Tyr Cys Thr
85 90 95

Arg Asp Pro Gly Glu Asp Tyr Asn Tyr Gly Met Asp Val Trp Gly Gln
100 105 110

Gly Thr Thr Val Thr Val Ser Ser Ala
115 120

<210> 284
<211> 15
<212> DNA
<213> Homo sapiens

<400> 284

agttacwact ggacc 15

<210> 285
<211> 48
<212> DNA
<213> Homo sapiens

<400> 285
tatatctatt acagtgggag caccaactac aacccctccc tcaagagt 48

<210> 286
<211> 36
<212> DNA
<213> Homo sapiens

<400> 286
gaccctgggg aagactacaa ctacggtatg gacgtc 36

<210> 287
<211> 363
<212> DNA
<213> Homo sapiens

<400> 287
caggtgcagc tgcaggagtc gggcccagga ctggtgaagc cttcggagac cttgtccctc 60

acctgcactg tctctggtgg ctccatcagt agttacwact ggacctggat ccggcagccc 120

ccagggaagg gactggagtg gattgggtat atctattaca gtgggagcac caactacaac 180

ccctccctca agagtcgagt caccatatca ttagacacgt ccaagaacca gttctccctg 240

aagctgagtt ctgtgaccgc tgcggacacg gccgtgtatt actgtacgag agaccctggg 300

gaagactaca actacggtat ggacgtctgg ggccaaggga ccacggtcac cgtctcctca 360

gcc 363

<210> 288
<211> 11
<212> PRT
<213> Homo sapiens

<400> 288

Arg Ala Ser Gln Gly Ile Ser Ser Ala Leu Ala
1 5 10

<210> 289
<211> 7
<212> PRT
<213> Homo sapiens

<400> 289

Asp Ala Ser Ser Leu Glu Ser
1               5

<210> 290
<211> 9
<212> PRT
<213> Homo sapiens

<400> 290

Gln Gln Phe Asn Ser Tyr Pro Arg Thr
1               5

<210> 291
<211> 107
<212> PRT
<213> Homo sapiens

<400> 291

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro Arg
                85                  90                  95

Thr Phe Gly Gln Gly Thr Lys Val Glu Ile Lys
            100                 105

<210> 292
<211> 33
<212> DNA
<213> Homo sapiens

<400> 292
cgggcaagtc agggcattag cagtgcttta gcc 33

<210> 293
<211> 21
<212> DNA
<213> Homo sapiens

<400> 293
gatgcctcca gtttggaaag t 21

<210> 294
<211> 27
<212> DNA
<213> Homo sapiens

<400> 294
caacagttta atagttaccc tcggacg 27

<210> 295
<211> 321
<212> DNA
<213> Homo sapiens

<400> 295
gccatccagt tgacccagtc tccatcctcc ctgtctgcat ctgtaggaga cagagtcacc 60

atcacttgcc gggcaagtca gggcattagc agtgctttag cctggtatca gcagaaacca 120

gggaaagctc ctaagctcct gatctatgat gcctccagtt tggaaagtgg ggtcccatca 180

aggttcagcg gcagtggatc tgggacagat ttcactctca ccatcagcag cctgcagcct 240

gaagattttg caacttatta ctgtcaacag tttaatagtt accctcggac gttcggccaa 300

gggaccaagg tggaaatcaa a 321

<210> 296
<211> 5
<212> PRT
<213> Homo sapiens

<400> 296

Ser Tyr Tyr Trp Thr
1 5

<210> 297
<211> 16
<212> PRT
<213> Homo sapiens

<400> 297

Tyr Ile Phe Tyr Ser Gly Ser Thr Asn Tyr Asn Pro Ser Leu Lys Ser
1 5 10 15

<210> 298
<211> 7
<212> PRT
<213> Homo sapiens

<400> 298

Gly Ile Ala Gly Met Asp Val
1 5

<210> 299
<211> 116
<212> PRT
<213> Homo sapiens

<400> 299

Gln Val Gln Leu Gln Glu Ser Gly Pro Gly Leu Val Lys Pro Ser Glu
1 5 10 15

Thr Leu Ser Leu Thr Cys Thr Val Ser Gly Gly Ser Ile Arg Ser Tyr
20 25 30

Tyr Trp Thr Trp Ile Arg Gln Pro Pro Gly Lys Gly Leu Glu Trp Ile
35 40 45

Gly Tyr Ile Phe Tyr Ser Gly Ser Thr Asn Tyr Asn Pro Ser Leu Lys
50 55 60

Ser Arg Val Thr Ile Ser Val Asp Thr Ser Lys Asn Gln Cys Ser Leu
65 70 75 80

Lys Leu Thr Ser Val Thr Ala Ala Asp Thr Ala Val Tyr Tyr Cys Ala
85 90 95

Lys Gly Ile Ala Gly Met Asp Val Trp Gly Gln Gly Thr Thr Val Thr
100 105 110

Val Ser Ser Ala
115

<210> 300
<211> 15
<212> DNA
<213> Homo sapiens

<400> 300
agttactatt ggacc 15

<210> 301
<211> 48
<212> DNA
<213> Homo sapiens

<400> 301
tatatctttt acagtgggag caccaactac aacccctccc tcaagagt 48

<210> 302
<211> 21
<212> DNA
<213> Homo sapiens

<400> 302
ggtatagcag gtatggacgt c 21

<210> 303
<211> 348
<212> DNA
<213> Homo sapiens

<400> 303
caggtgcaac tgcaggagtc gggcccagga ctggtgaagc cttcggagac cctgtccctc 60

acctgcactg tctctggtgg ctccatcaga agttactatt ggacctggat ccggcagccc 120

ccagggaagg gactggagtg gattgggtat atcttttaca gtgggagcac caactacaac 180

ccctccctca agagtcgagt caccatatca gtagacacgt ccaagaacca gtgctccctg 240

aagctgacct ctgtgaccgc tgcggacacg gccgtgtatt actgtgcgaa aggtatagca 300

ggtatggacg tctggggcca agggaccacg gtcaccgtct cctcagcc 348

<210> 304
<211> 11
<212> PRT
<213> Homo sapiens

<400> 304

Arg Ala Ser Gln Ser Ile Gly Ser Ser Leu His
1               5                   10

<210> 305
<211> 7
<212> PRT
<213> Homo sapiens

<400> 305

Tyr Ala Ser Gln Ser Phe Ser
1 5

<210> 306
<211> 9
<212> PRT
<213> Homo sapiens

<400> 306

His Gln Ser Arg Ser Leu Pro Ile Thr
1 5

<210> 307
<211> 107
<212> PRT
<213> Homo sapiens

<400> 307

Glu Ile Val Leu Thr Gln Ser Pro Asp Phe Gln Ser Val Thr Pro Lys
1 5 10 15

Glu Lys Val Thr Ile Thr Cys Arg Ala Ser Gln Ser Ile Gly Ser Ser
20 25 30

Leu His Trp Tyr Gln Gln Lys Pro Asp Gln Ser Pro Lys Leu Leu Ile
35 40 45

Lys Tyr Ala Ser Gln Ser Phe Ser Gly Val Pro Ser Arg Phe Ser Gly
50 55 60

Ser Gly Ser Gly Thr Asp Phe Thr Leu Thr Ile Asn Ser Leu Glu Ala
65 70 75 80

Glu Asp Ala Ala Ala Tyr Tyr Cys His Gln Ser Arg Ser Leu Pro Ile
85 90 95

Thr Phe Gly Gln Gly Thr Arg Leu Glu Ile Lys
100 105

<210> 308
<211> 36
<212> DNA
<213> Homo sapiens

<400> 308
agggccagtc agagtgttag cagcagctac ttagcc 36

<210> 309
<211> 21
<212> DNA
<213> Homo sapiens

<400> 309
ggtgcatcca gcagggccac t 21

<210> 310
<211> 27
<212> DNA
<213> Homo sapiens

<400> 310
cagcagtatg gtagctcacc gtacact 27

<210> 311
<211> 324
<212> DNA
<213> Homo sapiens

<400> 311
gaaattgtgt tgacgcagtc tccaggcacc ctgtctttgt ctccagggga aagagccacc 60

ctctcctgca gggccagtca gagtgttagc agcagctact tagcctggta ccagcagaaa 120

cctggccagg ctcccaggct cctcatctat ggtgcatcca gcagggccac tggcatccca 180

gacaggttca gtggcagtgg gtctgggaca gacttcactc tcaccatcag cagactggag 240

cctgaagatt ttgcagtgta ttactgtcag cagtatggta gctcaccgta cacttttggc 300

caggggacca agctggagat caaa 324

<210> 312
<211> 11
<212> PRT
<213> Homo sapiens

<400> 312

Gln Val Gln Leu Gln Glu Ser Gly Pro Gly Leu
1 5 10

<210> 313
<211> 10
<212> PRT
<213> Homo sapiens

<400> 313

Glu Ile Val Leu Thr Gln Ser Pro Gly Thr
1 5 10

<210> 314
<211> 21
<212> DNA
<213> Homo sapiens

<400> 314
atgaaacacc tgtggttctt c 21

<210> 315
<211> 18
<212> DNA
<213> Homo sapiens

<400> 315
atggaaaccc cagcgcag 18

<210> 316
<211> 30
<212> DNA
<213> Artificial

<220>
<223> Primer

<400> 316
ggatgaattc tcatttaccc ggagacaggg 30

<210> 317
<211> 28
<212> DNA
<213> Artificial

<220>
<223> Primer

<400> 317
ccggaattcc taacactctc ccctgttg 28

## Description

TECHNICAL FIELD

The present invention relates to a monoclonal antibody against nectin-2 and use thereof, and more particularly, to an agent for preventing/treating cancer or a diagnostic agent for cancer, an apoptosis inducer of cancer cells, a growth inhibitor of cancer cells, and a cytotoxic agent against cancer cells through a host defense mechanism mediated by the Fc region of an antibody.

FIELD OF THE INVENTION

It is reported that in cancer its pathological conditions could be assessed by a gene microarray profiling data. Actually in leukemia, it is reported that leukemia can be classified by gene expression profiles. Also it is considered possible to predict response to a particular cancer therapy or discover a novel drug target protein for a particular cancer by clarifying the gene expression profile of each cancerous tissue and accumulating its classification. Specifically, where an enhanced expression of a certain protein is observed in a certain cancer, it becomes possible to induce an anti-tumor activity in patients newly diagnosed to be the antigen-positive, by means of (i) reducing the expression level of the protein, (ii) suppressing the function of the protein, (iii) eliciting immune response of a host to the protein, etc. At the same time, patients diagnosed to be the antigen-negative can immediately switch over to another cancer therapy, assuming to eliminate any concern of imposing a superfluous burden on patients. As such, it is expected that the expression profile analysis would greatly contribute to molecular diagnosis of a cancer and development of molecular target-based therapeutic drugs.

The nectin-2α gene (RefSeq Accession No. NM_002856) and the nectin-2δ gene (EMBLAccession No. X80038) are genes cloned from human leukemia cell line TF-1-derived cDNAand encode proteins consisting of 479 amino acids and 538 amino acids, respectively (RefSeq Accession No. NP_002847 and EMBL Accession No. CAA56342). The nectin-2δ gene is a splicing variant of the nectin-2α gene and the protein encoded by the nectin-2δ gene has an amino acid sequence corresponding to the 1st to 350th amino acid sequence of a protein encoded by the nectin-2α gene but is different in the amino acid sequence located on and after the 351st amino acid at the C-terminal portion. In addition, mouse genes (GenBank Accession No. BC009088 and RefSeq Accession No. NM_008990) showing homology to the nectin-2α gene and the nectin-2δ gene are cloned from a library derived from mouse ES cells, and encode proteins consisting of 467 amino acids and 530 amino acids, respectively (GenBank Accession No. AAH09088 and RefSeq Accession No. Nap _033016). These mouse nectin-2 genes have homology of about 72% and about 72% in terms of base sequence and about 69% and about 73% in terms of amino acid sequence, to the human nectin-2α gene and nectin-2δ gene, respectively Nectin-2α and nectin-2δ (hereinafter sometimes collectively referred to as nectin-2) are protein molecules also called PVRL2, PRR2, PVRR2, HveB, CD 112, etc. and belong to the nectin family consisting of four members, nectin-1, nectin-2, nectin-3 and nectin-4(hereinafter sometimes collectively referred to as nectin). Andnectin Necl-1, Necl-2, Necl-3, Necl-4 and Necl-5 are known as membrane proteins having a nectin-like structurenectin (J. Biol. Chem. (2004), 279 (17), p18015-p18025).

Nectin belongs to the immunoglobulin superfamily and is single transmembrane glycoprotein having 3 immunoglobulin-like loops in the extracellular region. It is considered that nectin molecules would form cis-dimers on the cell membranes, and the cis-dimers on the cell membranes trans-interact with one another to regulate cell-cell adhesion between epithelial cells or between spermatids and Sertoli cells in a $Ca^{2+}$ concentration-independent mode (Protein, Nucleic Acid and Enzyme (2003), 48 (2), p105-p112; Curr. Biol. (2002), 12, p1145-p1150). It is also reported that nectin-1 and nectin-3 play a part in the formation of synapses via trans-binding (J. Cell Biol. (2002), 156, p555-p565). It is known that the trans-binding of nectins is formed homophilically between the same molecules, whereas heterophilic trans-binding is also formed between nectin-1 and nectin-3, nectin-1 and nectin-4, nectin-2 and nectin-3 as well as nectin-3 and Necl-5 (J. Biol. Chem. (2002), 277 (30), p27006-p27013). It is also known that nectin in the intracellular C-terminal region binds to afadin and connects to the actin cytoskeleton through the molecule (J. Cell Sci. (2003), 116 (1), p17-p27).

As a physiological function of nectins other than cell adhesion, it is reported that for example, nectin-1 acts as a receptor for glycoprotein D expressed on herpes viruses to function as a scaffold of herpes viral entry into cells (J. Cell Sci. (2003), 116 (1), p17-p27). Also, nectin-2 is one of ligands for DNAM-1 (CD226) expressed on natural killer cells and natural killer cells expressing DNAM-1 are considered to induce cytotoxicity upon engagement with nectin-2 expressed on target cells (J. Exp. Med. (2003), 198 (4), p557-p567). Besides, it is reported that nectin-2 is one of genes involved in the tumor suppressor gene p53 pathway (WO 02/99040), a protein binding to nectin-3 which is a protein useful for treating angiogenesis disorders, cancer or viral infection (WO 02/28902), a receptor involved in viral infection (WO 99/63063), one of genes which are useful for diagnosis and treatment of breast cancer and ovarian cancer (WO 02/00677), one of 16 genes, which expression are enhanced in various cancers and are promising as a target for anti-tumor therapeutic antibodies (WO 03/088808) as well as one of genes, which expression are enhanced in cancer tissue and are promising for diagnosis and prevention of cancer (WO 04/030615). Nectin-2 gene was found as a gene, which expression was markedly increased in cancer tissues. It is reported that antisense oligonucleotide of the gene stimulates apoptosis in cancer cells (WO 2005/097204). As monoclonal antibodies against nectin-2, there are reports of mouse or rat monoclonal antibodies against human nectin-2 (Blood (1998), 92(12), p4602-p4611; J. Virol. (2000) 74 (3) p1267-p1274; Intl. Immunol. (2004) 16 (4), p533-p538; Mol. Immunol. (2005) 42, p463-p469; JEM (2003) 198 (4), p557-p567; Virol. (1998) 246, p179-p189; J. Virol. (2001) 75 (2) p11185-p11195; FEBS (2005) 579, p2243-2249) and monoclonal antibodies against mouse nectin-2 (J. Virol. (2001) 75(2) p11185-p11195; JBC (2001) 276, p48350-p48355; Oncogene (1999) 18, p1609-p1617; JCB (1999) 145, p539-p549; Exp. Cell Res. (1997) 235, p374-p384). However, none of these reports desribed as to the growth inhibitory activity of these antibodies against cancer cells.

DISCLOSURE OF THE INVENTION

The existing anti-cancer drugs are invariably accompanied by side effects. In clinical work sites, safe drugs that act specifically on cancer cells, have the least affect on normal tissues, and induce growth inhibition of cancer cells alone, are earnestly sought.

In order to solve the foregoing problems, the present inventors made extensive studies and as a result, found the nectin-2 gene as a gene whose expression markedly increases in cancer tissues, and also found that an antisense oligonucleotide of this gene induces apoptosis in cancer cells. The inventors have further succeeded in producing monoclonal antibodies against nectin-2 and found that the monoclonal antibodies have an excellent growth inhibitory activity and so on against cancer cells. As a result of further investigations based on these findings, the inventors have come to accomplish the present invention.

More specifically, the present invention relates to the following features:

[1] a monoclonal antibody against a protein comprising the same or substantially the same amino acid sequence as the amino acid sequence represented by SEQ ID NO: 1 (nectin-2$\alpha$)or SEQ ID NO: 3 (nectin-2$\delta$), its partial peptide, or a salt thereof;

[2] the antibody according to [1] above, which is a monoclonal antibody against a protein comprising the amino acid sequence represented by SEQ ID NO: 3 (nectin-2$\delta$), its partial peptide, or a salt thereof;

[3] the antibody according to [1] above, which is a human monoclonal antibody;

[4] the antibody according to [1] above, which is a chimeric monoclonal antibody;

[5] the antibody according to [1] above, which is a humanized monoclonal antibody;

[6] the antibody according to [1] above, which is a monoclonal antibody wherein the constant region of the antibody belongs to human $IgG_1$ subclass;

[7] the antibody according to [1] above, which has a cancer cell growth inhibitory activity;

[8] the antibody according to [1] above, which has antibody-dependent cellular cytotoxicity (ADCC);

[9] the antibody according to [1] above, which has a nectin-2/nectin-3 or nectin-2/nectin-2 trans-binding inhibitory activity;

[10] the antibody according to [1] above, which is a monoclonal antibody capable of recognizing the epitope present in the 1st-350th (extracellular region) amino acid sequence in the amino acid sequence represented by SEQ ID NO: 1 (nectin-2$\alpha$) or SEQ ID NO: 3(nectin-2$\delta$);

[11] the antibody according to [1] above, which is a monoclonal antibody capable of recognizing the epitope present in the 47th-142nd (first immunoglobulin-like domain) or 175th-240th (second immunoglobulin-like domain) amino acid sequence in the amino acid sequence represented by SEQ ID NO: 1 (nectin-2$\alpha$) or SEQ ID NO: 3 (nectin-2$\delta$);

[12] the antibody according to [1] above, which is a monoclonal antibody capable of recognizing the amino acid sequence containing at least one amino acid residue of the 75th, 76th, 77th, 78th, 95th, 137th, 145th, 173rd, 184th, 186th and 212th amino acid residues in the amino acid sequence represented by SEQ ID NO: 1 (nectin-2$\alpha$) or SEQ ID NO: 3 (nectin-2$\delta$);

[13] the antibody according to [1] above, wherein the antibody bind competitively with a monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1(FERM BP-10428), Nec1-964-1 (FERM BP-10683), Nec1-1302-2 (FERM BP-10684), Nec1-554-1 (FERM BP-10681), Necl-769-2 (FERM BP-10682) or Nec8-4116-8 (FERM BP-10685), to the protein comprising the same or substantially the same amino acid sequence as the amino acid sequence represented by SEQ ID NO: 1 or SEQ ID NO: 3, its partial peptide, or a salt thereof;

[14] the antibody according to [1] above, which is capable of recognizing the same or substantially the same amino acid sequence as the amino acid sequence recognized by a monoclonal antibody produced by the hybridoma cell represented by Necl-803-2 (FERM BP-10417), Necl-244-3 (FERM BP-10423), Nec1-530-1 (FERM BP-10424), Necl-903-1 (FERM BP-10425), Necl-520-1 (FERM BP-10426), Nec1-845-2 (FERM BP-10427) or Necl-834-1 (FERM BP-10428), Nec1-964-1 (FERM BP-10683), Necl-1302-2 (FERM BP-10684), Necl-554-1 (FERM BP-10681), Necl-769-2 (FERM BP-10682) or Nec8-4116-8 (FERM BP-10685);

[15] a hybridoma cell, which is capable of producing the antibody according to [1] above;

[16] the hybridoma cell according to [15] above, which is represented by Nec1-803-2 (FERM BP-10417), Nec1-244-3 (FERM BP-10423), Necl-530-1 (FERM BP-10424), Nec1-903-1 (FERM BP-10425), Necl-520-1 (FERM BP-10426), Necl-845-2 (FERM BP-10427), Necl-834-1 (FERM BP-10428), Necl-964-1 (FERM BP-10683), Necl-1302-2 (FERM BP-10684), Necl-554-1 (FERM BP-10681), Nec1-769-2 (FERM BP-10682) or Nec8-4116-8 (FERM BP-10685);

[17] a monoclonal antibody produced by the hybridoma cell according to [16];

[18] the antibody according to [1] above, which is a recombinant monoclonal antibody;

[19] the antibody according to [1] above, wherein the amino acid sequences of a first complementarity determining region (CDR1), a second complementarity determining region (CDR2) and a third complementarity determining region (CDR3) in a heavy chain variable region of said antibody comprise the same or substantially the same amino acid sequence as the amino acid sequence represented by (i) the sequence identification number selected from the group consisting of SEQ ID NOS: 184, 200, 216, 232, 248, 264, 280 and 296, (ii) the sequence identification number selected from the group consisting of SEQ ID NOS: 185, 201, 217, 233, 249, 265, 281 and 297, and (iii) the sequence identification number selected from the group consisting of SEQ ID NOS:186, 202, 218, 234, 250, 266, 282 and 298, respectively;

[19a] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 184, SEQ ID NO: 185 and SEQ ID NO: 186, respectively;

[19b] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 200, SEQ ID NO: 201 and SEQ ID NO: 202, respectively;

[19c] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 216, SEQ ID NO: 217 and SEQ ID NO: 218, respectively;

[19d] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 232, SEQ ID NO: 233 and SEQ ID NO: 234, respectively;

[19e] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 248, SEQ ID NO: 249 and SEQ ID NO: 250, respectively;

[19f] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 264, SEQ ID NO: 265 and SEQ ID NO: 266, respectively;

[19g] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 280, SEQ ID NO: 281 and SEQ ID NO: 282, respectively;

[19h] the antibody according to [19] above, wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by SEQ ID NO: 296, SEQ ID NO: 297 and SEQ ID NO: 298, respectively;

[20] the antibody according to [1] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) the sequence identification number selected from the group consisting of SEQ ID NOS: 192, 208, 224, 240, 256, 272, 288 and 304, (v) the sequence identification number selected from the group consisting of SEQ ID NOS: 193, 209, 225, 241, 257, 273, 289 and 305, and (vi) the sequence identification number selected from the group consisting of SEQ ID NOS: 194, 210, 226, 242, 258, 274, 290 and 306, respectively;
[20a] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 192, SEQ ID NO: 193 and SEQ ID NO: 194, respectively;
[20b] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 208, SEQ ID NO: 209 and SEQ ID NO: 210, respectively;
[20c] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 224, SEQ ID NO: 225 and SEQ ID NO: 226, respectively;
[20d] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 240, SEQ ID NO: 241 and SEQ ID NO: 242, respectively;
[20e] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 256, SEQ ID NO: 257 and SEQ ID NO: 258, respectively;
[20f] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 272, SEQ ID NO: 273 and SEQ ID NO: 274, respectively;
[20g] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 288, SEQ ID NO: 289 and SEQ ID NO: 290, respectively;
[20h] the antibody according to [20] above, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by SEQ ID NO: 304, SEQ ID NO: 305 and SEQ ID NO: 306, respectively;
[21] a diagnostic agent, which comprises a monoclonal antibody against a protein comprising the same or substantially the same amino acid sequence as the amino acid sequence represented by SEQ ID NO: 1 or SEQ ID NO: 3, its partial peptide, or a salt thereof;
[22] the diagnostic agent according to [21] above, which is a diagnostic agent for cancer;
[23] a medicament, which comprises a monoclonal antibody against a protein comprising the same or substantially the same amino acid sequence as the amino acid sequence represented by SEQ ID NO: 1 or SEQ ID NO: 3, its partial peptide, or a salt thereof;
[24] the medicament according to [23] above, which is an agent for preventing/treating cancer;
[25] the medicament according to [23] above, which is an apoptosis inducer of cancer cells;
[26] the medicament according to [23] above, which is a growth inhibitor of cancer cells;

[27] the medicament according to [23] above, which is a cytotoxic agent against cancer cells wherein a host defense mechanism mediated by the Fc region of an antibody is utilized;
[28] a method for preventing/treating cancer, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof;
[29] a method for inducing apoptosis of cancer cells, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof;
[30] a method for inhibiting growth of cancer cells, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof;
[31] a method for killing cancer cells through a host defense mechanism mediated by the Fc region of an antibody, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof;
[32] use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of an agent for preventing/treating cancer;
[33] use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of an apoptosis inducer of cancer cells;
[34] use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of a growth inhibitor of cancer cells;
[35] use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of a cytotoxic agent against cancer cells through a host defense mechanism mediated by the Fc region of an antibody;
[36] An agent for preventing or treating breast cancer which comprises a monoclonal antibody produced by the hybridoma cell represented by Nec1-803-2 (FERM BP-10417), Necl-244-3 (FERM BP-10423), Nec1-530-1 (FERM BP-10424), Necl-903-1 (FERM BP-10425), Nec1-520-1 (FERM BP-10426), Necl-845-2 (FERM BP-10427), Necl-834-1 (FERM BP-10428), Nec1-964-1 (FERM BP-10683), Necl-1302-2 (FERM BP-10684), Necl-554-1 (FERM BP-10681), Necl-769-2 (FERM BP-10682) or Nec8-4116-8 (FERM BP-10685);
[37] An agent for preventing or treating breast cancer which comprises a monoclonal antibody binding to nectin-2 competitively with a monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685);
[38] The agent for preventing or treating breast cancer according to [37] above, wherein the monoclonal antibody binding to nectin-2 competitively with the monoclonal antibody produced by the hybridoma cell represented by Nec1-554-1 (FERM BP-10681) is Nec1-1044-4 (FERM BP-10805) or Nec1-1302-2 (FERM BP-10684);
[39] The agent for preventing or treating breast cancer according to [37] above, wherein the monoclonal antibody binding to nectin-2 competitively with the monoclonal antibody produced by the hybridoma cell represented by Nec8-4116-8 (FERM BP-10685) is Nec8-3704-7 (FERM BP-10807) or Nec8-3517-11 (FERM BP-10806);
[40] A method for preventing or treating breast cancer according to [36] or [37] above, wherein a host defense mechanism mediated by the Fc region of the antibody is utilized;
[41] An agent for preventing or treating breast cancer, which comprises an antibody wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) a sequence identification number selected from the group consisting of SEQ ID NOS: 184, 200, 216, 232, 248, 264, 280 and 296, (ii) a sequence identification number selected from the group consisting of SEQ ID NOS: 185, 201, 217, 233, 249, 265, 281 and 297, and (iii) a sequence identification number selected from the group consisting of SEQ ID NOS: 186, 202, 218, 234, 250, 266, 282 and 298, respectively;
[42] An agent for preventing or treating breast cancer, which comprises an antibody wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) a sequence identification number selected from the group consisting of SEQ ID NOS: 192, 208, 224, 240, 256, 272, 288 and 304, (v) a sequence identification number selected from the group consisting of SEQ ID NOS: 193, 209, 225, 241, 257, 273, 289 and 305, and (vi) a sequence identification number selected from the group consisting of SEQ ID NOS: 194, 210, 226, 242, 258, 274, 290 and 306, respectively;

[43] An agent for preventing or treating breast cancer, which comprises an antibody wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) a sequence identification number selected from the group consisting of SEQ ID NOS: 184, 200, 216, 232, 248, 264, 280 and 296, (ii) a sequence identification number selected from the group consisting of SEQ ID NOS: 185, 201, 217, 233, 249, 265, 281 and 297, and (iii) a sequence identification number selected from the group consisting of SEQ ID NOS: 186, 202, 218, 234, 250, 266, 282 and 298, respectively; wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) a sequence identification number selected from the group consisting of SEQ ID NOS: 192, 208, 224, 240, 256, 272, 288 and 304, (v) a sequence identification number selected from the group consisting of SEQ ID NOS: 193, 209, 225, 241, 257, 273, 289 and 305, and (vi) a sequence identification number selected from the group consisting of SEQ ID NOS: 194, 210, 226, 242, 258, 274, 290 and 306, respectively; and, a constant region of said antibody;

[44] A hybridoma cell represented by Nec1-1044-4 (FERM BP-10805), Nec8-3517-11 (FERM BP-10806) or Nec8-3704-7 (FERM BP-10807);

[45] A monoclonal antibody produced by the hybridoma cell according to [44] above;

[46] A monoclonal antibody binding competitively with a monoclonal antibody produced by the hybridoma cell represented by Nec1-1044-4 (FERM BP-10805), Nec8-3517-11 (FERM BP-10806) or Nec8-3 704-7 (FERM BP-10807);

[47] An agent for preventing or treating breast cancer which comprises the monoclonal antibody according to [45] or [46] above;

[48] An agent for preventing or treating breast cancer which comprises an antibody binding competitively with an antibody wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) a sequence identification number selected from the group consisting of SEQ ID NOS: 184, 200, 216, 232, 248, 264, 280 and 296, (ii) a sequence identification number selected from the group consisting of SEQ ID NOS: 185, 201, 217, 233, 249, 265, 281 and 297, and (iii) a sequence identification number selected from the group consisting of SEQ ID NOS: 186, 202, 218, 234, 250, 266, 282 and 298, respectively;

[49] An agent for preventing or treating breast cancer which comprises an antibody binding competitively with an antibody wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) a sequence identification number selected from the group consisting of SEQ ID NOS: 192, 208, 224, 240, 256, 272, 288 and 304, (v) a sequence identification number selected from the group consisting of SEQ ID NOS: 193, 209, 225, 241, 257, 273, 289 and 305, and (vi) a sequence identification number selected from the group consisting of SEQ ID NOS: 194, 210, 226, 242, 258, 274, 290 and 306, respectively;

[50] An agent for preventing or treating breast cancer, which comprises an antibody binding competitively with an antibody wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) a sequence identification number selected from the group consisting of SEQ ID NOS: 184, 200, 216, 232, 248, 264, 280 and 296, (ii) a sequence identification number selected from the group consisting of SEQ ID NOS: 185, 201, 217, 233, 249, 265, 281 and 297, and (iii) a sequence identification number selected from the group consisting of SEQ ID NOS: 186, 202, 218, 234, 250, 266, 282 and 298, respectively; wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) a sequence identification number selected from the group consisting of SEQ ID NOS: 192, 208, 224, 240, 256, 272, 288 and 304, (v) a sequence identification number selected from the group consisting of SEQ ID NOS: 193, 209, 225, 241, 257, 273, 289 and 305, and (vi) a sequence identification number selected from the group consisting of SEQ ID NOS: 194, 210, 226, 242, 258, 274, 290 and 306, respectively; and, a constant region of said antibody, and so on. The monoclonal antibody against the protein comprising same or substantially the same amino acid sequence as the amino acid sequence represented by SEQ ID NO: 1 or SEQ ID NO: 3, its partial peptide, or a salt thereof, can be safely used as, for example, an agent for preventing/treating cancer (e.g., colorectal cancer, breast cancer, lung cancer, prostate cancer, esophageal cancer, gastric cancer, liver cancer, biliary tract cancer, spleen cancer, renal cancer, bladder cancer, uterine cancer, ovarian cancer, testicular cancer, thyroid cancer, pancreatic cancer, brain tumor, blood tumor, etc.) (preferably an agent for preventing/treating breast cancer, lung cancer, colorectal cancer, prostate cancer, ovarian cancer, pancreatic cancer, etc.), an apoptosis inducer of cancer cells, a growth inhibitor of cancer cells, an inducer of cell cycle change in cancer cells, a cytotoxic agent against cancer cells utilizing a host defense mechanism mediated by the Fc region of an antibody, an antibody-dependent cytotoxic agent against cancer cells, etc.

BRIEF DESCRIPTION OF THE DRAWINGS

FIG. 1 shows the amino acid sequences (SEQ ID NOS: 187, 203, 219, 235, 251, 267, 283 and 299) in the H chain variable region and the amino acid sequences (SEQ ID NOS: 195, 211, 227, 243, 259, 275, 291 and 307) in the L chain variable region, of the antibody of the present invention obtained in EXAMPLE 1.

FIG 2 shows the base sequences (SEQ ID NOS: 191, 207, 223, 239, 255, 271, 287 and 303) in the H chain variable region of the antibody of the present invention obtained in EXAMPLE 1.

FIG 3 shows the base sequences (SEQ ID NOS: 199, 215, 231, 247, 263, 279, 295 and 311) in the L chain variable region of the antibody of the present invention obtained in EXAMPLE 1.

FIG 4 shows changes in the mean tumor volume with passage of time after the cancer cell line transplantation in EXAMPLE 23.

EMBODIMENT OF THE INVENTION

The protein comprising the same or substantially the same amino acid sequence as the amino acid sequence represented by SEQ ID NO: 1 (hereinafter referred to as nectin-2α) or the protein comprising the same or substantially the same amino acid sequence as the amino acid sequence represented by SEQ ID NO: 3 (hereinafter referred to as nectin-2δ) (hereinafter, these proteins are sometimes collectively referred to as nectin-2 or the protein of the present invention), may be any protein derived from any cells (e.g., hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes, etc.), megakaryocyte, synovial cells, chondrocytes, bone cells, osteoblasts, osteoclasts, mammary gland cells, hepatocytes or interstitial cells, or the corresponding precursor cells, stem cells, cancer cells, etc.), or any tissues where such cells are present, e.g., brain or any region of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc., from human and other warm-blooded animals (e.g., guinea pigs, rats, mice, fowl, rabbits, swine, sheep, bovine, monkeys, etc.). The protein may also be a synthetic protein.
The amino acid sequence substantially identical to the same amino acid sequence as that represented by SEQ ID NO: 1 or SEQ ID NO: 3 includes amino acid sequences having at least about 50% homology, preferably at least about 60% homology, more preferably at least about 70% homology, still more preferably at least about 80% homology, much more preferably at least about 90% homology and most preferably at least about 95% homology, to the amino acid sequence shown by SEQ ID NO: 1 or SEQ ID NO: 3.
Preferred examples of the protein comprising substantially the same amino acid sequence as the amino acid sequence represented by SEQ ID NO: 1 or SEQ ID NO: 3 include proteins comprising substantially the same amino acid sequence as the amino acid sequence represented by SEQ ID NO: 1 or SEQ ID NO: 3 and having a property substantially equivalent to that of the protein containing the amino acid sequence represented by SEQ ID NO: 1, SEQ ID NO: 3, etc.
Homology of the amino acid sequences can be measured using a homology scoring algorithm NCBI BLAST (National Center for Biotechnology Information Basic Local Alignment Search Tool) under the following conditions (an expectation value = 10; gaps are allowed; matrix = BLOSUM62; filtering = OFF).

The substantially equivalent is used to mean that the nature of these properties is equivalent in terms of quality (e.g., physiologically or pharmacologically). Thus, the activity of the protein of the present invention is preferably equivalent (e.g., about 0.01 to 100 times, preferably about 0.1 to 10 times, more preferably 0.5 to 2 times), but differences in quantitative factors such as degree of these activities and a molecular weight of the protein may be allowable.

Examples of nectin-2 include so-called muteins such as proteins having (i) the amino acid sequence represented by SEQ ID NO: 1 or SEQ ID NO: 3, of which at least 1 or 2 (e.g., about 1 to about 50, preferably about 1 to about 30, more preferably about 1 to about 10 and most preferably several (1 to 5)) amino acids are deleted; (ii) the amino acid sequence represented by SEQ ID NO: 1 or SEQ ID NO: 3, to which at least 1 or 2 (e.g., about 1 to about 50, preferably about 1 to about 30, more preferably about 1 to about 10 and most preferably several (1 to 5)) amino acids are added; (iii) the amino acid sequence represented by SEQ ID NO: 1 or SEQ ID NO: 3, in which at least 1 or 2 (e.g., about 1 to about 50, preferably about 1 to about 30, more preferably about 1 to about 10 and most preferably several (1 to 5)) amino acids are inserted; (iv) the amino acid sequence represented by SEQ ID NO: 1 or SEQ ID NO: 3, in which at least 1 or 2 (e.g., about 1 to about 50, preferably about 1 to about 30, more preferably about 1 to about 10 and most preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Throughout the specification, the proteins are represented in accordance with the conventional way of describing peptides, that is, the N-terminus (amino terminus) at the left hand and the C-terminus (carboxyl terminus) at the right hand. In the protein used in the present invention including the protein comprising the amino acid sequence represented by SEQ ID NO: 1, the C-terminus may be in any form of a carboxyl group (-COOH), a carboxylate ($-COO^-$), an amide ($-CONH_2$) and an ester (-COOR).

Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group such as benzyl, phenethyl, etc.; an $\alpha$-naphthyl-$C_{1-2}$ alkyl group such as $\alpha$-naphthylmethyl, etc.; pivaloyloxymethyl and the like.

Where nectin-2 contains a carboxyl group (or a carboxylate) at a position other than the C-terminus, the carboxyl group may be amidated or esterified and such an amide or ester is also included within nectin-2 used in the present invention. Examples of the ester group in this case may be the C-terminal esters described above, etc.

Furthermore, examples of nectin-2 include variants wherein the amino group at the N-terminal amino acid residues (e.g., methionine residue) is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains; etc.

Specific examples of nectin-2 include a protein (nectin-2$\alpha$) comprising the amino acid sequence represented by SEQ ID NO: 1, a protein (nectin-2$\delta$) comprising the amino acid sequence represented by SEQ ID NO: 3, and so on.

The partial peptide of nectin-2 may be any peptide as long as it is a partial peptide of nectin-2 described above and preferably has the property equivalent to that of nectin-2 described above.

For example, in the constituent amino acid sequence of nectin-2, peptides containing, e.g., at least 20, preferably at least 50, more preferably at least 70, much more preferably at least 100 and most preferably at least 200 amino acids, can be used.

The partial peptide of nectin-2 used in the present invention may be peptides containing the amino acid sequence, of which at least 1 or 2 (preferably about 1 to about 20, more preferably about 1 to about 10 and most preferably several (1 to 5)) amino acids may be deleted; peptides containing the amino acid sequence, to which at least 1 or 2 (preferably about 1 to about 20, more preferably about 1 to about 10 and most preferably several (1 to 5)) amino acids may be added; peptides containing the amino acid sequence, in which at least 1 or 2 (preferably about 1 to about 20, more preferably about 1 to about 10 and most preferably several (1 to 5)) amino acids may be inserted; or peptides containing the amino acid sequence, in which at least 1 or 2 (preferably about 1 to about 20, more preferably several and most preferably about 1 to about 5) amino acids may be substituted by other amino acids.

In the partial peptide of nectin-2, the C-terminus may be in any form of a carboxyl group (-COOH), a carboxylate ($-COO^-$), an amide ($-CONH_2$) or an ester (-COOR).

Furthermore, the partial peptide of nectin-2 includes those having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated peptides such as so-called glycopeptides in which sugar chains are conjugated; etc., as in nectin-2 described above.

As salts of nectin-2 or its partial peptides, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

The monoclonal antibodies against nectin-2, its partial peptide or salts thereof (hereinafter sometimes briefly referred to as the antibody of the present invention) may be any of monoclonal antibodies, as long as they are antibodies capable of recognizing nectin-2, its partial peptide or salts thereof. Among them, human monoclonal antibodies are preferably used.

Also, examples of the antibodies of the present invention include a monoclonal antibodies (specifically, human monoclonal antibodies), against nectin-2δ, its partial peptide or salts thereof.

Furthermore, antibodies having at least one of the following properties (1) to (8) are preferably employed as the antibody of the present invention.

(1) An antibody having the growth inhibitory activity against cancer cells (e.g., human cancer cell OV-90)
(2) An antibody having the antibody-dependent cellular cytotoxicity (ADCC)
(3) An antibody having the inhibitory activity against the cis-binding of nectin-2
Specifically, these antibodies:

- (i) inhibit homo-cis-binding of nectin-2α;
- (ii) inhibit homo-cis-binding of nectin-2δ; or,
- (iii) inhibit hetero-cis-binding of nectin-2α and nectin-2δ.

(4) An antibody having an inhibitory activity against the nectin-2/nectin-3 or nectin-2/nectin-2 trans-binding
Specifically, these antibodies:

- (i) inhibit trans-binding of a homo-cis-dimer of nectin-2α and a homo-cis-dimer of nectin-2α;
- (ii) inhibit trans-binding of a homo-cis-dimer of nectin-2α and a homo-cis-dimer of nectin-2δ;
- (iii) inhibit trans-binding of a homo-cis-dimer of nectin-2α and a hetero-cis-dimer of nectin-2α and nectin-2δ;
- (iv) inhibit trans-binding of a homo-cis-dimer of nectin-2α and a homo-cis-dimer of the nectin-3;
- (v) inhibit trans-binding of a homo-cis-dimer of nectin-2δ and a homo-cis-dimer of nectin-2δ;
- (vi) inhibit trans-binding of a homo-cis-dimer of nectin-2δ and a hetero-cis-dimer of nectin-2α and nectin-2δ;
- (vii) inhibit trans-binding of a homo-cis-dimer of nectin-2δ and a homo-cis-dimer of nectin-3;
- (viii) inhibit trans-binding of a hetero-cis-dimer of nectin-2α and nectin-2δ and a homo-cis-dimer of nectin-3; or,
- (ix) inhibit trans-binding of a hetero-cis-dimer of nectin-2α and nectin-2δ and a hetero-cis-dimer of nectin-2α and nectin-2δ.

(5) An antibody belonging to any of the epitope groups I to VII shown in EXAMPLE 4 or the epitope subgroup shown in EXAMPLE 18
Preferably, the antibody belongs to the epitope group IV, VI or VII shown in EXAMPLE 4. More preferably, the antibody belongs to the epitope subgroup IVb, VIb or VIIa shown in EXAMPLE 18.
(6) An antibody recognizing the same or substantially the same amino acid sequence as the amino acid sequence which is recognized by a monoclonal antibody (antibody belonging to the epitope group I, IV, V, VI or VII in Table. 4) produced by the hybridoma cell shown by:

Nec1-803-2 (FERM BP-10417),
Nec1-244-3 (FERM BP-10423),
Nec1-530-1 (FERM BP-10424),
Nec1-903-1 (FERM BP-10425),
Nec1-520-1 (FERM BP-10426),
Nec1-845-2 (FERM BP-10427),
Nec1-834-1 (FERM BP-10428),
Nec1-964-1 (FERM BP-10683),
Nec1-1302-2 (FERM BP-10684),
Nec1-554-1 (FERM BP-10681),

Nec1-769-2 (FERM BP-10682) or,
Nec8-4116-8 (FERM BP-10685)

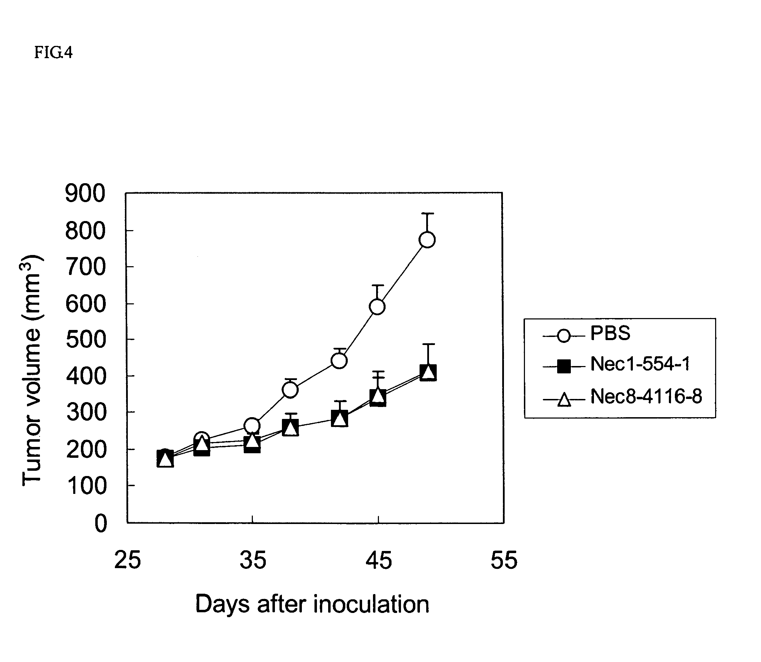

In "the same or substantially the same amino acid sequence as the amino acid sequence (hereinafter the latter amino acid is merely referred to as the epitope) which is recognized by a monoclonal antibody (antibody belonging to the epitope I, IV, V, VI or VII in FIG 4) produced by a hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1(FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685)), the term "substantially the same amino acid sequence as the epitope " includes (i) the amino acid sequence, of which at least 1 or 2 (e.g., about 1 to about 10, preferably several (1 to 5)) amino acids in the epitope are deleted; (ii) the amino acid sequence, to which at least 1 or 2 (e.g., about 1 to about 10, preferably several (1 to 5)) amino acids s in the epitope are added; (iii) the amino acid sequence, in which at least 1 or 2 (e.g., about 1 to about 10, preferably several (1 to 5)) amino acids s in the epitope are inserted; (iv) the amino acid sequence, in which at least 1 or 2 (e.g., about 1 to about 10, preferably several (1 to 5)) amino acids s in the epitope are substituted by other amino acids; or (v) a combination of (i)-(iv) amino acid sequences; and the like. The position of its insertion, addition, deletion or substitution described above is not particularly limited.
More specifically, the term "substantially the same amino acid sequence as an epitope" includes amino acid sequences near the epitope and includes, for example, (i) the amino acid sequence in which at least 1 or 2 (e.g., about 1 to about 10, preferably several (1 to 5)) amino acids are added to the N-terminal side of the epitope site; (ii) the amino acid sequence in which at least 1 or 2 (e.g., about 1 to about 10, preferably several (1 to 5)) amino acids are added to the C-terminal side of the epitope; (iii) the amino acid sequence in which at least 1 or 2 (e.g., about 1 to about 10, preferably several (1 to 5)) amino acids are added to the amino acid sequence of amino acid sequences (e.g., about 1 to about 10, preferably several (1 to 5)) at the N-terminal side within the epitope; or (iv) the amino acid sequence in which at least 1 or 2 (e.g., about 1 to about 10, preferably several (1 to 5)) amino acids are added to the amino acid sequence of amino acid sequences (e.g., about 1 to about 10, preferably several (1 to 5)) at the C-terminal side within the epitope; and the like.
For example, each monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685) and other monoclonal antibodies belonging to the same group as the group to which the aforesaid monoclonal antibody belongs are considered to recognize the same or substantially the same amino acid sequence as said monoclonal antibody.
(7) Antibody which is competitive with a monoclonal antibody produced by the hybridoma cell shown by:

Nec1-803-2 (FERM BP-10417),
Nec1-244-3 (FERM BP-10423),
Nec1-530-1 (FERM BP-10424),
Nec1-903-1 (FERM BP-10425),
Nec1-520-1 (FERM BP-10426),
Nec1-845-2 (FERM BP-10427),
Nec1-834-1 (FERM BP-10428),
Nec1-964-1 (FERM BP-10683),
Nec1-1302-2 (FERM BP-10684),
Nec1-554-1 (FERM BP-10681),
Nec1-769-2 (FERM BP-10682), or
Nec8-4116-8 (FERM BP-10685),
for binding to nectin-2α or nectin-2δ.

Herein, the term "antibody which is competitive with a monoclonal antibody produced by each hybridoma cell for binding to nectin-2α or nectin-2δ" refers to an antibody, which binding to nectin-2α or nectin-2δ is competitively inhibited by adding an excess of any one of the 12 antibodies described above. Specifically, the antibody refers to, for example, an antibody showing approximately 50-100% binding inhibition, when 50-fold molar amount of any one of the 12 antibodies described above is added to said antibody.
(8) A monoclonal antibody comprising the same or substantially the same amino acid sequence as the amino acid sequence of a monoclonal antibody produced by hybridoma cell shown by:

Nec1-803-2 (FERM BP-10417),
Nec1-244-3 (FERM BP-10423),
Nec1-530-1 (FERM BP-10424),
Nec1-903-1 (FERM BP-10425),
Nec1-520-1 (FERM BP-10426),
Nec1-845-2 (FERM BP-10427),
Nec1-834-1 (FERM BP-10428),
Nec1-964-1 (FERM BP-10683),
Nec1-1302-2 (FERM BP-10684),
Nec1-554-1 (FERM BP-10681),
Nec1-769-2 (FERM BP-10682), or
Nec8-4116-8 (FERM BP-10685).

Herein, the same or substantially the same amino acid sequence as the amino acid sequence (hereinafter amino acid sequence A) of the monoclonal antibody described above includes amino acid sequences having at least about 50% homology, preferably at least about 60% homology, more preferably at least about 70% homology, still more preferably at least about 80% homology, much more preferably at least about 90% homology and most preferably at least about 95% homology, to the amino acid sequence A; etc.

Examples of the antibody comprising substantially the same amino acid sequence as the amino acid sequence A include an antibody comprising substantially the same amino acid sequence as the amino acid sequence A and having an activity substantially equivalent to the protein comprising the amino acid sequence A; and the like.

Homology of the amino acid sequences can be measured using a homology scoring algorithm NCBI BLAST (National Center for Biotechnology Information Basic Local Alignment Search Tool) under the following conditions (an expectation value = 10; gaps are allowed; matrix = BLOSUM62; filtering = OFF).

The term substantially equivalent is used to mean that the nature of these properties is equivalent in terms of quality (e.g., physiologically or pharmacologically). Thus, the activity of protein used in the present invention is preferably equivalent (e.g., about 0.01 to 100 times, preferably about 0.1 to 10 times, more preferably 0.5 to 2 times), but differences in quantitative factors such as degree of these activities and a molecular weight of the protein may be allowable.

Examples of the monoclonal antibody comprising the same or substantially the same amino acid sequence as the amino acid sequence A include antibody containing (i) an amino acid sequence wherein at least 1 or 2 (e.g., about 1 to about 50, preferably about 1 to about 30, more preferably about 1 to about 10 and most preferably several (1 to 5)) amino acids are deleted of the amino acid sequence A; (ii) an amino acid sequence wherein at least 1 or 2 (e.g., about 1 to about 50, preferably about 1 to about 30, more preferably about 1 to about 10 and most preferably several (1 to 5)) amino acids are added to the amino acid sequence A; (iii) an amino acid sequence wherein at least 1 or 2 (e.g., about 1 to about 50, preferably about 1 to about 30, more preferably about 1 to about 10 and most preferably several (1 to 5)) amino acids are inserted into the amino acid sequence A; (iv) an amino acid sequence wherein at least 1 or 2 (e.g., about 1 to about 50, preferably about 1 to about 30, more preferably about 1 to about 10 and most preferably several (1 to 5)) amino acids in the amino acid sequence A are substituted by other amino acids; or (v) a combination of these amino acid sequences; and the like.

The antibody of the present invention includes a chimeric antibody, humanized antibody, human antibody and antibody fragment. The "chimeric antibody" means an antibody which has the variable regions derived from antibody of different species and constant regions of human antibody (see, e.g., EP 0125023, etc.). The "humanized antibody" refers to an antibody designed to modify a heterologous antibody like a mouse antibody by replacing its primary structure other than the complementarity determining regions of H chain and L chain with the corresponding primary structure of a human antibody. The "human antibody" refers to a monoclonal antibody prepared using a transgenic animal carrying human antibody genes (see EP0546073) and a monoclonal antibody prepared using a library in which a human antibody gene is presented on the cell surface of bacteriophage, Escherichia coli, yeast, animal cells, etc., a so-called antibody display technology (Nature Biotechnology 23, 1105 (2005)) and a monoclonal antibody isolated from human B cells producing an antibody against nectin-2 using cell fusion method, phage display method, or the like.

In the present invention, the "antibody fragment" refers to a part of the full-length antibody, and generally means a fragment containing antigen-binding regions or variable regions. The antibody fragment includes, for example, Fab, Fab', $F(ab')_2$, a single chain antibody (scFv), disulfide-stabilized antibody (dsFv), etc.

The antibody in accordance with a preferred embodiment of the present invention recognizes an epitope present in the 1st-350th (extracellular domain) amino acid sequence in the amino acid sequence represented by SEQ ID NO: 1 (nectin-2$\alpha$) or SEQ ID NO: 3 (nectin-2$\delta$); an epitope present in the 47th-142nd (first immunoglobulin-like domain) or 175th-240th (second immunoglobulin-like domain) amino acid sequence in the amino acid sequence represented by SEQ ID NO: 1 (nectin-2α) or SEQ ID NO: 3 (nectin-2δ); or the amino acid sequence containing at least one amino acid residue from the 75th, 76th, 77th, 78th, 95th, 137th, 145th, 173rd, 184th, 186th and 212th amino acid residues in the amino acid sequence represented by SEQ ID NO: 1 (nectin-2α) or SEQ ID NO: 3 (nectin-2δ).

The present invention further provides a monoclonal antibody comprising a specific CDR amino acid sequence or a variable region amino acid sequence. The present invention still further provides a monoclonal antibody light chain or its fragment, and a monoclonal antibody heavy chain or its fragment, comprising a specific CDR amino acid sequence.

At the N-terminal sides of the heavy and light chains, there are variable regions which are called a heavy chain variable region (VH) and a light chain variable region (VL), respectively. In the variable region, there is a complementarity determining region (CDR) and this part is responsible for the specificity of antigen recognition. A part of the variable region other than CDR functions to retain the structure of CDR and is called a framework region (FR). At the C-terminal sides of the heavy and light chains, there are constant regions which are called a heavy chain constant region (CH) and a light chain constant region (CL), respectively. In the heavy chain variable region, there are three complementarity determining regions: the first complementarity determining region (CDR1), the second complementarity determining region (CDR2), and the third complementarity determining region (CDR3). The three complementarity determining regions in the heavy chain variable region are collectively called a heavy chain complementarity determining region. Likewise, there are three complementarity determining regions in the light chain variable region, which are the first complementarity determining region (CDR1), the second complementarity determining region (CDR2), and the third complementarity determining region (CDR3). These three complementarity determining regions in the light chain variable region are collectively called a light chain complementarity determining region.

Specifically, in the antibody in accordance with a preferred embodiment of the present invention, the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) the sequence identification number selected from the group consisting of SEQ ID NOS: 184, 200, 216, 232, 248, 264, 280 and 296, (ii) the sequence identification number selected from the group consisting of SEQ ID NOS: 185, 201, 217, 233, 249, 265, 281 and 297, and (iii) the sequence identification number selected from the group consisting of SEQ ID NOS: 186, 202, 218, 234, 250, 266, 282 and 298, respectively.

Furthermore, in the antibody in accordance with another preferred embodiment of the present invention, the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) the sequence identification number selected from the group consisting of SEQ ID NOS: 192, 208, 224, 240, 256, 272, 288 and 304, (v) the sequence identification number selected from the group consisting of SEQ ID NOS: 193, 209, 225, 241, 257, 273, 289 and 305, and (vi) the sequence identification number selected from the group consisting of SEQ ID NOS: 194, 210, 226, 242, 258, 274, 290 and 306, respectively.

The CDR sequences from the antibody of the present invention are not necessarily limited but include those given in TABLES 21 and 22 later described, as suitable combinations of amino acid sequences of VH CDR1, VH CDR2 and VH CDR3 and suitable combinations of amino acid sequences ofVL CDR1, VL CDR2 and VL CDR3. Amino acid sequences other than CDR are not particularly limited but the antibody of the present invention includes a so-called CDR grafted antibody in which amino acid sequences other than CDR are derived from another antibody, especially from an antibody of different species. Human-derived amino acid sequences are preferred as the amino acid sequences other than CDR and may be accompanied, if necessary, by the addition, deletion, substitution and/or insertion of one or more amino acid residues in the framework region (FR).

The amino acid sequence and base sequence in the variable regions of the antibody of the present invention are preferably those given in TABLE 25.

The monoclonal antibody comprising a specific CDR amino acid sequence or variable region amino acid sequence of the antibody of the present invention can be prepared using known methods.

The antibody of the present invention includes preferably a monoclonal antibody, in which the constant regions of the antibody belong to preferably a human antibody, more preferably human IgG and most preferably human IgG1 subclass.

The antibody against nectin-2, its partial peptide, or salts thereof (which are sometimes briefly referred to as nectin-2 in the following description of the antibody) can be prepared by publicly known methods for manufacturing antibodies or antisera.

The preparation of an antigen for the antibody of the present invention and preparation of the antibody will be described below.

(1) Preparation of antigen

As the antigen used to prepare the antibody of the present invention, for example, any one of the antigens such as a protein comprising the amino acid sequence represented by SEQ ID NO: 1 or SEQ ID NO: 3 (nectin-2), its partial peptide, or salts thereof, a cell line or its membrane fraction wherein the protein comprising the amino acid sequence represented by SEQ ID NO: 1 or SEQ ID NO: 3 (nectin-2) is highly expressed naturally or artificially, a fusion protein of the extracellular domain protein of nectin-2 and the other protein or peptide, or salts thereof, a (synthetic) peptide having one or more antigenic determinants, which are the same as in nectin-2, etc. can be used (hereinafter these antigens are sometimes merely referred to as the antigen of the present invention).
Specific examples of the antigen of the present invention, which can be preferably used, include a cell line or its membrane fraction wherein nectin-2 is highly expressed naturally or artificially, an extracellular domain protein of nectin-2 or salts thereof, a fusion protein of the extracellular domain protein of nectin-2 and the other protein or peptide, or a (synthetic) peptide having one or more antigenic determinants, which are the same as in nectin-2, etc.
Examples of the other protein or peptide include FLAG-tag, His-tag, Myc-tag, V5-tag, GST-tag, S-tag, T7-tag, or the Fc regions of human antibody, mouse antibody, etc., and so on.
Although the length of such (synthetic) peptide is not limited so long as it is such a length as having immunogenicity, the peptide is preferably a peptide having, e.g., 6, preferably 10 and more preferably 12 consecutive amino acid residues.
Nectin-2 or its partial peptide, or salts thereof may be manufactured by publicly known methods or their modifications used to purify proteins from human or warm-blooded animal cells or tissues described above. Alternatively, they may also be manufactured by culturing transformants bearing DNAs encoding these proteins. And, they may also be manufactured according to methods for peptide synthesis described below. In addition, they may also be manufactured by culturing transformants bearing DNAs encoding a fusion protein of extracellular domain protein of nectin-2 and the other protein or peptide.

(a) Where the antigen of the present invention or salts thereof are prepared from tissues or cells of human or warm-blooded animals (e.g., guinea pigs, rats, mice, fowl, rabbits, swine, sheep, bovine, monkeys, etc.), the tissues or cells are homogenized and the crude fraction (e.g., its membrane fraction or soluble fraction) can be used as an antigen in its intact form. Alternatively, the homogenate is extracted with an acid, a surfactant, an alcohol, etc. and the extract is then purified and isolated by a combination of salting out, dialysis, gel filtration, chromatography techniques such as reverse phase chromatography, ion exchange chromatography, affinity chromatography, and the like.
(b) Where nectin-2 or fusion protein of the extracellular domain protein of nectin-2 and the other protein (peptide), or salts thereof, are prepared using transformants bearing DNA, the DNA can be prepared by publicly known method [e.g., the method described in Molecular Cloning, (2nd ed.; J. Sambrook et al., Cold Spring Harbor Lab. Press, 1989), etc.].

For cloning of DNAs that completely encode nectin-2 or its partial peptide (hereinafter sometimes merely referred to as nectin-2 in the description of cloning of DNAs encoding the same and their expression), the DNA can be either amplified by PCR using synthetic DNA primers containing a part of the base sequence encoding nectin-2, or the DNA inserted into an appropriate vector can be selected by hybridization with a labeled DNA fragment or synthetic DNA that encodes a part or entire region of nectin-2. A template polynucleotide used for PCR may be any one so long as it contains a base sequence encoding nectin-2. The polynucleotide may also be any one of genomic DNA, genomic DNA library, cDNA derived from the cells or tissues described above, cDNA library derived from the cells or tissues described above and a synthetic DNA.
The hybridization can be carried out by publicly known methods or modifications thereof, for example, by the method described in Molecular Cloning, 2nd ed. (J. Sambrook et al., Cold Spring Harbor Lab. Press, 1989), etc. A commercially available library can also be used according to the instructions of the attached manufacturer's protocol. More preferably, the hybridization can be carried out under high stringent conditions.
The high stringent conditions are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.
More specifically, there are employed (i) a DNA comprising the base sequence represented by SEQ ID NO: 2, etc. as the DNA encoding the protein comprising the amino acid sequence represented by SEQ ID NO: 1 (nectin-2α); and (ii) a DNA comprising the base sequence represented by SEQ ID NO: 4, etc. as the DNA encoding the protein comprising the amino acid sequence represented by SEQ ID NO: 3 (nectin-2δ).
Substitution of the base sequence of DNA can be performed by publicly known methods such as the ODA-LA PCR method, the Gapped duplex method, the Kunkel method, etc., or its modification, using PCR, a publicly known kit available as Mutan™-super Express Km (TaKaRa Shuzo Co., Ltd.) or Mutan™-K (TaKaRa Shuzo Co., Ltd.), etc.

The cloned DNA encoding nectin-2 can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof These translation initiation and termination codons may also be added by using an appropriate synthetic DNA adapter. Where the DNA encoding the fusion protein of the extracellular domain of nectin-2 and the other protein (peptide) or its salt, the DNA encoding the nectin-2 extracellular domain cloned or synthesized by the same method as described above is ligated with a DNA encoding the other protein (peptide) by publicly known methods or their modifications.

The expression vector for nectin-2 can be manufactured, for example, by (a) excising the desired DNA fragment from the DNA encoding nectin-2, and then (b) ligating the DNA fragment with an appropriate expression vector downstream a promoter in the vector.

Examples of the vector include plasmids derived form E. coli (e.g., pBR322, pBR325, pUC12, pUC 13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, insect viruses such as baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, $\lambda P_L$ promoter, lpp promoter, T7 promoter, etc. In the case of using bacteria of the genus Bacillus as the host, preferred example of the promoter are SPO1 promoter, SP02 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches a host is added to the N-terminal side of nectin-2. Examples of the signal sequence that can be used are PhoA signal sequence, OmpA signal sequence, etc. when the host is a bacterium of the genus Escherichia; α-amylase signal sequence, subtilisin signal sequence, etc. when the host is a bacterium of the genus Bacillus; MFα signal sequence, SUC2 signal sequence, etc. when the host is yeast; and insulin signal sequence, α-interferon signal sequence, antibody molecule signal sequence, etc. when the host is an animal cell, respectively.

Using the vector containing the DNA encoding nectin-2 thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine ofTrichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include simian cell COS-7, Vero, Chinese hamster cell CHO (hereinafter referred to as CHO cell), dhfr gene-deficient Chinese hamster cell CHO (hereinafter simply referred to as CHO ($dhfr^-$) cell), mouse L cell, mouse AtT-20, mouse myeloma cell, mouse ATDC5 cell, mouse NS0 cell, mouse FM3A cell, rat GH3 cell, human FL cell, human embryonic HEK293 cell, human embryonic 293F cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors bearing the DNAs encoding nectin-2 can be obtained.

Where the host is a bacterium belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the host is a bacterium belonging to the genus Escherichia, the transformant is usually cultivated at about 15 to 43°C for about 3 to 24 hours. If necessary, the culture may be aerated or agitated.

Where the host is a bacterium belonging to the genus Bacillus, the transformant is cultured generally at about 30 to 40°C for about 6 to 24 hours. If necessary, the culture can be aerated or agitated.

Where the host is yeast, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated. Where the host is an insect cell or insect, the transformant is cultivated in, for example, Grace's Insect Medium (Nature, 195, 788 (1962)) to which an appropriate additive such as 10% heat-inactivated bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where the host is an animal cell, the transformant is cultured in, for example, MEM medium [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc., containing about 5 to 20% fetal bovine serum. Preferably, pH of the medium is adjusted to about 6 to about 8. The transformant is usually cultivated at about 30°C to about 40°C for about 15 to 60 hours and, if necessary, the culture can be aerated or agitated.

As described above, nectin-2 can be produced in the transformant, on the cell membrane of the transformant, or outside of the transformant.

Nectin-2 can be separated and purified from the culture described above by the following procedures.

When nectin-2 is extracted from the bacteria or cells, the bacteria or cells are collected after culturing by publicly known methods and suspended in an appropriate buffer. The bacteria or cells are then disrupted by publicly known methods such as ultrasonication, a treatment with lysozyme and/or freeze-thaw cycling, followed by centrifugation, filtration, etc to produce crude extract of the protein. The buffer used for the procedures may contain a protein modifier such as urea or guanidine hydrochloride, or a surfactant such as Triton X-100™, etc. When nectin-2 is secreted in the culture broth, the supernatant can be separated, after completion of the cultivation, from the bacteria or cells to collect the supernatant by publicly known methods.

Nectin-2 contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, etc.; a method utilizing difference in electric charge such as ion exchange chromatography, etc.; a method utilizing difference in specific affinity such as affinity chromatography, etc.; a method utilizing difference in hydrophobicity such as reverse phase chromatography, etc.; a method utilizing difference in isoelectric point such as chromatofocusing; and the like.

When nectin-2 thus obtained is in a free form, nectin-2 can be converted into its salt by publicly known methods or modifications thereof On the other hand, when nectin-2 is obtained in the form of a salt, it can be converted into its free form or in the form of a different salt by publicly known methods or modifications thereof.

Nectin-2 produced by the recombinant can be treated, prior to or after the purification, with an appropriate protein-modifying enzyme so that nectin-2 can be optionally modified or the polypeptide may be partially removed. Examples of the protein-modifying enzyme include trypsin, chymotrypsin, arginyl endopeptidase, protein kinase, glycosidase, and the like.

The presence of the thus produced nectin-2 can be determined by enzyme immunoassay, western blotting using a specific antibody, etc.

(c) Mammalian cells which express nectin-2 can also be used directly as the antigen of the present invention. As the mammalian cells, there can be used the naturally occurring cells as described in (a) above, cells transformed by the methods as described in (b) above, etc. Hosts used for the transformation may be any cells as far as they are cells collected from human, simian, rat, mouse, hamster, etc. and preferably used are HEK293, COS7, CHO-K1, NIH3T3, Balb3T3, FM3A, L929, SP2/0, P3U1, NS0, B16, P388, or the like.

(d) The (synthetic) peptide having one or more antigenic determinants, which are the same as in nectin-2, or its salt, can be manufactured by publicly known methods for peptide synthesis or by cleaving nectin-2 with an appropriate peptidase. For the methods for peptide synthesis, for example, either solid phase synthesis or liquid phase synthesis may be used. That is, the partial peptide or amino acids that can construct the peptide are condensed with the remaining part. Where the product contains protecting groups, these protecting groups are removed to give the desired peptide. Publicly known methods for condensation and elimination of the protecting groups are described in (i) to (v) below.

(i) M. Bodanszky & M.A. Ondetti: Peptide Synthesis, Interscience Publishers, New York (1966)

(ii) Schroeder & Luebke: The Peptide, Academic Press, New York (1965)

(iii) Nobuo Izumiya, et al.: Peptide Gosei-no-Kiso to Jikken (Basics and experiments of peptide synthesis), published by Maruzen Co. (1975)

(iv) Haruaki Yajima & Shunpei Sakakibara: Seikagaku Jikken Koza (Biochemical Experiment) 1, Tanpakushitsu no Kagaku (Chemistry of Proteins) IV, 205 (1977) (v) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the partial peptide used in the present invention may be purified and isolated by a combination of conventional purification methods such as solvent extraction, distillation, column chromatography, liquid chromatography and recrystallization. When the partial peptide obtained by the above methods is in a free form, the partial peptide can be converted into an appropriate salt by a publicly known method or its modification; conversely when the partial peptide is obtained in a salt form, it can be converted into a free form or other different salt form by a publicly known method or its modification.

(2) Production of monoclonal antibody

(a) Establishment of monoclonal antibody-producing cells by the hybridoma method

The antigen of the present invention is administered to warm-blooded animals. Immunization may be done by any method, as long as it can stimulate antibody production, and preferably used are intravenous injection, intraperitoneal injection, intramuscular injection, subcutaneous injection, intradermal injection, footpad injection, etc.

Naturally occurring mammalian cells or transformed mammalian cells, which express the protein of the present invention, can be injected to animal for immunization as a suspension of the cells in a medium used for tissue culture (e.g., RPMI 1640) or buffer (e.g., Hanks' balanced salt solution).

The antigen of the present invention may be provided for direct immunization in its immobilized form. The antigen of the present invention may also be bound or adsorbed to an appropriate carrier and the complex produced can be provided for immunization. A mixing ratio of the carrier to the antigen of the present invention (hapten) may be in any ratio of any type, as long as the antibody can be efficiently produced to the antigen of the present invention which is bound or adsorbed to the carrier. A naturally occurring or synthetic high molecular carrier conventionally used to produce an antibody against a hapten may be used in a weight ratio of 0.1 to 100 against 1 of hapten. Examples of the naturally occurring high molecular carrier, which can be used, are serum albumin from mammals such as bovine, rabbit, human, etc., thyroglobulins from mammals such as bovine, rabbit, etc., hemoglobins from mammals such as bovine, rabbit, human, sheep, etc or keyhole limpet hemocyanin. Examples of the synthetic high molecular carrier, which can be used, are various latexes including polymers, copolymers, etc., such as polyamino acids, polystyrenes, polyacryls, polyvinyls, polypropylenes, etc.

For coupling of the hapten and the carrier, a variety of condensing agents can be used. Examples of the condensing agents, which are advantageously employed, are diazonium compounds such as bis-diazotized benzidine capable of crosslinking tyrosines, histidines or tryptophans; dialdehyde compounds such as glutaraldehyde and diisocyanate compounds such as toluene-2,4-diisocyanatecapable of crosslinking amino groups with each other;dimaleimide compounds such as N,N'-o-phenylenedimaleimide, etc., capable of crosslinking thiols with each other; maleimide activated ester compounds capable of crosslinking an amino group with a thiol group; carbodiimide compounds capable of crosslinking an amino group with a carboxyl group; etc. In the crosslinking of amino groups with each other, a thiol group is introduced into one amino group by reacting with an activated ester reagent (e.g., N-succinimidyl 3-(2-pyridyldithio) propionate (SPDP), etc.) having dithiopyridyl group, followed by reduction, whereas a maleimide group in introduced into another amino group using a maleimide activated ester reagent, and the two groups may be reacted with each other.

When the antigen of the present invention is administered, in order to potentiate the antibody productivity of an immune animal, the antigen of the present invention may be mixed with an adjuvant such as complete Freund's adjuvant or incomplete Freund's adjuvant, Alum, a Ribi adjuvant, etc. and the resulting mixture or emulsion may be administered to the animal. The administration is usually made once every about 2 to 6 weeks and about 2 to 10 times in total. Further in producing the monoclonal antibody of the present invention, DNA immunization may be used (see, e.g., Nature, 356, 152-154). Examples of the applicable warm-blooded animals are monkeys, rabbits, dogs, guinea pigs, mice, rats, hamster, sheep, goats, camel, lama and fowl, with the use of mice and rats being preferred for producing the monoclonal antibody. These warm-blooded animals may be wild or KO animals wherein the warm-blooded animal ortholog genes of antigen proteins are knockout to achieve a stronger immune response against the antigen. Also, transgenic animals wherein antibody genes of warm-blooded animals are knockout and human antibody genes are introduced (see EP0546073), knock-in animals (WO 02/098217, WO 03/020743), etc. may be used to produce human monoclonal antibodies.

In preparation of the monoclonal antibody-producing cells, an warm-blooded animal, e.g., a mouse, wherein the antibody titer is noted is selected from animals immunized with the antigen, then spleen or lymph node is collected 2 to 5 days after the final immunization. The antibody-producing B cells contained therein are fused with myeloma cells derived from the same or different species, whereby hybridomas producing the monoclonal antibody can be established. The antibody titer in antisera may be determined by any method, so long as the amount of antibody specifically binding to the antigen can be quantified. As will be later described, the antibody titer can be determined, for example, by reacting an immobilized protein antigen or antigen-expressing cell line with antiserum and then measuring the level of antibody bound to them using a labeled anti-immunoglobulin antibody. The fusion may be carried out in accordance with known methods, e.g., by Koehler and Milstein [Nature, 256, 495 (1975)]. Examples of the fusion accelerator are polyethylene glycol (PEG), Sendai virus, etc., and PEG is preferably employed.

Examples of the myeloma cells are those collected from warm-blooded animals such as NS-1, P3U1, SP2/0, AP-1, etc. In particular, SP2/0 and P3U1 are preferably employed. A preferred ratio of the number of the antibody-producing cells used (spleen cells) to the number of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

For cell fusion operations to establish the monoclonal antibody-producing cells, electrofusion may also be employed.

The screening of hybridomas can be performed by publicly known methods or their modifications. The screening of hybridomas can be performed normally in a medium for animal cells supplemented with HAT (hypoxanthine, aminopterin and thymidine). As a screening and growth medium, any medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for culture of a hybridoma (SFM-101, Nissui Seiyaku Co., Ltd.) and the like, can be used. The culture is carried out generally at a temperature of 20 to 40°C, preferably at 37°C, for about 5 days to about 3 weeks, preferably 1 to 2 weeks. Culture can be carried out normally in 5% carbon dioxide gas.

Various methods can be used for screening of the monoclonal antibody-producing hybridomas. Examples of such methods include a method which involves adding the supernatant of a hybridoma to a solid phase (e.g., microplate) adsorbed with a soluble protein antigen or protein antigen-expressing cell, directly or together with a carrier, followed by the reaction with an anti-immunoglobulin antibody (for example, when spleen cells used for the cell fusion are from mouse, an anti-mouse immunoglobulin antibody is used) labeled with a radioactive substance, an enzyme, a fluorescent substance, etc., or with Protein A, and detecting the monoclonal antibody bound to the solid phase; a method which involves adding the hybridoma supernatant to a solid phase adsorbed with an anti-immunoglobulin antibody or Protein A, followed by the reaction with a soluble protein antigen labeled with a radioactive substance, an enzyme, or a fluorescent substance, etc. and detecting the antigen-specific monoclonal antibody bound to the solid phase; etc. When the protein antigen-expressing cell is used, the hybridoma culture supernatant is added to the cell, followed by the reaction with a fluorescence-labeled anti-immunoglobulin antibody, and the fluorescence intensity of the cell is assayed on a fluorescence detector such as a flow cytometer, etc. Thus, the monoclonal antibody bound to the protein antigen on the cell membrane can be detected.

(b) Production of monoclonal antibody by other methods

The method for producing the antibody of the present invention is not limited to the method described in (a), but for example, a so-called antibody display technology, which involves presenting an antibody gene library prepared by publicly known methods using B lymphocyte of human or warm-blooded animal (e.g., monkey, rabbit, dog, guinea pig, mouse, rat, hamster, sheep, goat, camel, lama, fowl, etc.) as a material, on the cell surface of bacteriophage, Escherichia coli, yeast, animal cells, etc or on ribosome [Nature Biotechnology 23, 1105 (2005)] can be used. Human or warm-blooded animals may be naive ones, patients carrying cancer which highly expressing the antigen of the present invention , or warm-blooded animals which are immunized with the antigen of the present invention by the method described in (a). The form of antibodies presented on the cell surface includes but is not limited to IgG molecules, IgM molecules, Fab fragments, single chain Fv (scFv) fragments, etc.

The gene for the monoclonal antibody (fragment) capable of specifically binding to the antigen of the present invention can be obtained as follows. The aforesaid antibody (fragment)-presenting cell or antibody (fragment)-presenting ribosome carrying antibody gene library is reacted with the antigen of the present invention for a given period of time, followed by removing non-specifically bound substances by washing. After eluting and recovering the product bound specifically to the antigen of the present invention, the antibody (fragment)-presenting cell or antibody (fragment)-presenting ribosome is allowed to grow. The same procedure is repeated several times, and finally the aimed gene can be isolated from the cloned antibody (fragment)-presenting cell or antibody (fragment)-presenting ribosome by publicly known methods. The thus obtained monoclonal antibody fragment gene is recombined with said region of the IgG antibody gene to acquire the monoclonal antibody IgG antibody gene.

The antibody of the present invention can also be obtained by immunizing antibody-producing cells isolated from human or the warm-blooded animals described above with the antigen of the present invention in vitro by publicly known methods and then establishing hybridomas as in the method described in (a).

(c) Manufacturing of monoclonal antibody

The monoclonal antibody of the present invention can be manufactured by culturing the monoclonal antibody-producing hybridoma obtained in (a) and the recombinant cell line in which the antibody gene isolated from the monoclonal antibody-producing hybridoma obtained in (a) by publicly known methods, or the monoclonal antibody gene obtained in (b) is artificially expressed. The monoclonal antibody can also be manufactured by incorporating the antibody gene into a chromosome of warm-blooded animal or plant by publicly known methods, and producing the monoclonal antibody in blood, milk and egg of warm-blooded animals, in plant body, in mold, etc. [Curr. Opin. Biotechnol., 7, 536 (1996), Nature Rev. Genet., 4, 794 (2003), Appl. Environ. Microbiol., 70, 2567 (2004)]. Examples of warm-blooded animals used are bovine, goat, sheep, swine, fowl, mouse, rabbit, etc. Examples of plant bodies are tobacco, sweet corn, potato, duckweed, etc.

The monoclonal antibody of the present invention can be purified from the above-described raw materials containing the monoclonal antibody, for example, by publicly known methods for separation and purification of immunoglobulins [e.g., salting out, alcohol precipitation, isoelectric precipitation, various chromatographies such as ion exchange chromatography, hydrophobic interaction chromatography, reverse phase chromatography, gel filtration chromatography, hydroxyapatite chromatography, affinity chromatography in which only antibody can be separated and purified with a carrier to which a substance having affinity to the antibody such as antigen, protein A, and protein G, etc is immobilized.], and the like.

(3) Medicament comprising the antibody of the present invention

The antibody of the present invention described above can be used as a medicament such as an agent for, for example, preventing/treating cancer (e.g., colon cancer, breast cancer, lung cancer, prostate cancer, esophageal cancer, gastric cancer, liver cancer, biliary tract carcinoma, spleen cancer, renal cancer, bladder cancer, uterine cancer, ovarian cancer, testicular cancer, thyroid cancer, pancreatic cancer, brain tumor, blood tumor, etc.) (preferably, an agent for preventing/treating breast cancer, lung cancer, colon cancer, prostate cancer, ovarian cancer, pancreatic cancer, etc.), an apoptosis inducer of cancer cells, a growth inhibitor of cancer cells, an inducer of cell cycle change in a cancer cell, an agent for suppressing cancer metastasis, a cancer cell adhesion inhibitor, a cytotoxic agent against cancer cells using a host defense mechanism mediated by the Fc region of an antibody, an antibody-dependent cytotoxic agent in cancer cells, etc. As the method for damaging cancer cells using a host defense mechanism mediated by the Fc region of an antibody, antibody-dependent cell-mediated cytotoxicity (ADCC) by effector cells of living body and complement-dependent cytotoxicity (CDC) are given, and ADCC is preferably used.

The medicament comprising the antibody of the present invention is low toxic, and can be administered orally or parenterally (e.g., intravascular administration, subcutaneous administration, etc.) to human or mammals (e.g., rats, rabbits, sheep, swine, bovine, cats, dogs, monkeys, etc.) as it is in the form of liquid preparation or as a pharmaceutical composition of appropriate dosage form.

The antibody of the present invention may be administered in itself, or may be administered as an appropriate pharmaceutical composition. The pharmaceutical composition used for the administration may contain the antibody of the present invention or its salt, a pharmacologically acceptable carrier, and a diluent or excipient. Such a pharmaceutical composition is provided in the dosage form suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody of the present invention or its salt in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, for example, there are e.g., sesame oil, soybean oil, etc., which may be used in combination with a solubilizing agent such as benzyl benzoate, benzyl alcohol, etc. The injectable preparaion thus prepared is usually filled in an appropriate ampoule. The suppository used for rectal administration may be prepared by blending the antibody of the present invention or its salt with conventional bases for suppositories.

The composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and may contain a vehicle, a diluent or excipient conventionally used in the field of pharmaceutical preparations. Examples of the vehicle or excipient for tablets are lactose, starch, sucrose, magnesium stearate, etc.

Favorably, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the antibody described above is contained in about 5 to about 100 mg especially in the form of injection, and in 10 to 250 mg for the other forms.

The dose of the aforesaid preventive/therapeutic agent or regulator comprising the antibody of the present invention may favorably be an intravenous administration of about 0.01 to about 20 mg/kg body weight, preferably about 0.1 to about 10 mg/kg body weight and more preferably about 0.1 to about 5 mg/kg body weight per administration as the antibody of the present invention, about 1 to 5 times/day, preferably about 1 to 3 times/day, for example when it is used for treating/preventing breast cancer in adult, although the dose may vary depending upon subject to be administered, target disease, conditions, route of administration, etc.. Also in other parenteral and oral administration, the agent can be administered in a dose corresponding to the dose given above. When the condition is especially severe, the dose may be increased according to the condition.

The antibody of the present invention may be administered as it stands or in the form of an appropriate pharmaceutical composition. The pharmaceutical composition used for the aforesaid administration contains the aforesaid antibody or its salts, a pharmacologically acceptable carrier, and a diluent or excipient. Such a composition is provided in the dosage form suitable for oral or parenteral administration (e.g., intravascular injection, subcutaneous injection, etc.).

Each composition described above may further contain other active components unless formulation causes any adverse interaction by compounding with the antibody described above.

Furthermore, the antibody of the present invention may be used in combination with other drugs, for example, alkylating agents (e.g., cyclophosphamide, ifosfamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, Taxol, etc.), cisplatin, carboplatin, etoposide, irinotecan, etc. The antibody of the present invention and the drugs described above may be administered simultaneously or at staggered times to the patient.

(4) Quantification of nectin-2 using the antibody of the present invention

The antibody of the present invention is capable of specifically recognizing nectin-2 and therefore can be used for quantification of nectin-2 in a test sample fluid, in particular, for quantification by sandwich immunoassay; etc.

That is, the present invention provides:

(i) a method of quantifying nectin-2 in a test sample fluid, which is characterized by competitively reacting the antibody of the present invention, a test sample fluid and a labeled nectin-2, and measuring the ratio of the labeled nectin-2 bound to said antibody;
(ii) a method of quantifying nectin-2 in a test sample fluid, which is characterized by reacting a test sample fluid with the antibody of the present invention immobilized on a carrier and another labeled antibody of the present invention simultaneously or sequentially, and then measuring the activity of the labeling agent on the insoluble carrier; and,
(iii) a method of quantifying nectin-2 in a test sample fluid, which is characterized by reacting a test sample fluid with the antibody of the present invention immobilized on a carrier, and then measuring the quantitative change of nectin-2 bound to insoluble carrier by e.g., detection method such as surface plasmon resonance (SPR) etc.

In the quantification method (ii) described above, an antibody having different binding sites to nectin-2 is preferably used.
The antibody of the present invention can be used not only for the quantification of nectin-2 but also for the detection of nectin-2 by means of a tissue staining, etc. For these purposes, the antibody molecule per se may be used, and F $(ab')_2$, Fab' or Fab fractions of the antibody molecule may also be used.
The method of quantifying nectin-2 using the antibody of the present invention is not particularly limited. Any quantification method may be used, so long as the level of an antibody, antigen or antibody-antigen complex corresponding to the level of antigen (e.g., the level of protein) in a test sample fluid can be detected by chemical or physical means and the level of the antigen can be calculated from a standard curve prepared from standard solutions containing known levels of the antigen. For such an assay method, for example, nephrometry, the competitive method, the immunometric method, the SPR method, the sandwich method, etc. are suitably used. However, it is particularly preferred to use the sandwich method in terms of sensitivity and specificity described later.
Examples of labeling agents, which are employed for the assay methods using labeling agents, are radioisotopes, enzymes, fluorescent substances, luminescent substances, etc. Examples of radioisotopes include [$^{125}I$], [$^{131}I$], [$^{3}H$], [$^{14}C$], etc. Preferred examples of the enzymes are those that are stable and have a higher specific activity, which include e.g., β-galactosidase, β-glucosidase, alkaline phosphatase, peroxidase, malate dehydrogenase, etc. Examples of the fluorescent substances include e.g., cyanine fluorescent dyes (e.g., Cy2, Cy3, Cy5, Cy5.5, Cy7 (manufactured by Amersham Biosciences), etc.), fluorescamine, fluorescein isothiocyanate, Alexa Fluor dye (Invitrogen), europium fluorescence complex (Perkin Elmer), etc. Examples of the luminescent substances are e.g., luminol, a luminol derivative, luciferin, lucigenin, etc. Furthermore, a biotin-avidin system may be used for combining an antibody or antigen with a labeling agent.
For immobilization of the antigen or antibody, physical adsorption may be used, and method using chemical binding which is conventionally used for insolubilization or immobilization of proteins, enzymes, etc. may also be used. To immobilize the antigen or antibody, these proteins may be labeled with biotin, which can be bound to a carrier on which streptoavidin (avidin) is previously immobilized. Immobilization of the antibody may be performed by capturing it to a carrier on which protein A, protein G, anti-immunoglobulin antibody, etc is previously immobilized. For carriers, e.g., insoluble polysaccharides such as agarose, dextran, cellulose, etc.; synthetic resin such as polystyrene, polyacrylamide, silicon, etc., and glass or the like are used.
In the sandwich method, the immobilized monoclonal antibody of the present invention is reacted with a test sample fluid (primary reaction), then with a labeled form of another monoclonal antibody of the present invention (secondary reaction), and the activity of the label on the immobilizing carrier is measured, whereby the level of the protein of the present invention in the test sample fluid can be quantified. The order of the primary and secondary reactions may be reversed, and the reactions may be performed simultaneously or at staggered times. The methods of labeling and immobilization can be performed by the methods described above. In the immunoassay by the sandwich method, the antibody used for immobilized or labeled antibodies is not necessarily one, but a mixture of two or more of antibodies may be used to increase the assay sensitivity.
In the assay for nectin-2 of the present invention by the sandwich method, the antibodies used in the primary and secondary reactions are preferably antibodies having different binding sites for nectin-2.
The antibodies of the present invention can be used for the assay systems other than the sandwich method, for example, the competitive method, the immunometric method, the SPR method, nephrometry, etc.
In the competitive method, the amount of the antigen in the test sample fluid is quantified by competitively reacting antigen in a test sample fluid and the labeled antigen with antibody, separating the unreacted labeled antigen (F) and the labeled antigen bound to the antibody (B) (B/F separation), followed by measurement of the amount of the label in B or F. This reaction method includes a liquid phase method using a soluble antibody as an antibody, and polyethylene glycol, a secondary antibody against the soluble antibody, etc. for B/F separation, and an solid method either using an immobilized antibody as the primary antibody, or using a soluble antibody as the primary antibody and immobilized antibody as the secondary antibody.
In the immunometric method, the amount of the antigen in the test sample fluid is quantified by competitively reacting antigen in a test sample fluid and immobilized antigen with a definite amount of labeled antibody, followed by separation of the immobilized phase from the liquid phase, or by reacting antigen in a test sample fluid and an excess amount of labeled antibody, adding immobilized antigen to capture the unreacted labeled antibody, followed by separation of the immobilized phase from the liquid phase, both of which were followed by measurement of the amount of the label in either phase.

In the SPR method, the antibody is insolubilized on the surface of a gold thin film formed on a glass substrate, and a test sample fluid is applied onto the thin film. A change in quantity of the protein analyte bound to the antibody on the thin layer is quantified using the principle of surface plasmon resonance (SPR) (Protein, Nucleic Acid and Enzyme, 37, 2977-2984 (1992)).

And in the nephrometry, the insoluble precipitate produced after the antigen-antibody reaction in gel or solution is quantified. When the amount of antigen in the test sample fluid is small and only a small amount of the precipitate is obtained, laser nephrometry using laser scattering is favorably employed.

For applying each of these immunological assays to the quantification method of the present invention, it is not required to set forth any particular conditions, procedures, etc. Quantification system for nectin-2 is established by adding conventional technical consideration in the art to the conventional conditions, procedures, etc. For details of these general technical means, the following reviews and texts may be refered.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Enzyme immunoassay" (2nd ed.) (Igakushoin, published in 1982), Eiji Ishikawa, et al. ed. " Enzyme immunoassay " (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc. may be referred.

As described above, nectin-2 can be quantified with high sensitivity, using the antibody of the present invention.

(5) Diagnostic agent and diagnostic method using the antibody of the present invention

Furthermore, when an increased level of nectin-2 is detected by quantifying the level of nectin-2 using the antibody of the present invention, it can be diagnosed that one suffers from diseases, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovarian cancer, testicular cancer, thyroid cancer, pancreatic cancer, brain tumor, blood tumor, etc.) or the like, or it is highly likely that one would suffer from these diseases in the future.

Besides, the antibody of the present invention may be used for detecting nectin-2 present in test samples such as body fluids, tissues, etc. The antibody may also be used for preparation of antibody columns used to purify nectin-2, for detection of nectin-2 in each fraction upon purification, for analysis of the behavior of nectin-2 in test cells; etc.

(6) Antibody used in the agent of the invention for preventing/treating breast cancer

A monoclonal antibody (hereinafter reffered to as the antibody used in the present invention) produced by hybridoma cell shown by:

Nec1-803-2 (FERM BP-10417),
Nec1-244-3 (FERM BP-10423),
Nec1-530-1 (FERM BP-10424),
Nec1-903-1 (FERM BP-10425),
Nec1-520-1 (FERM BP-10426),
Nec1-845-2 (FERM BP-10427),
Nec1-834-1 (FERM BP-10428),
Nec1-964-1 (FERM BP-10683),
Nec1-1302-2 (FERM BP-10684),
Nec1-554-1 (FERM BP-10681),
Nec1-769-2 (FERM BP-10682), or
Nec8-4116-8 (FERM BP-10685) can be used.

The antibody used in the present invention further includes antibodies (including antibody fragments) produced by genetic engineering and having specific CDR amino acid sequences or amino acid sequences in the variable region of the antibodies produced by these hybridomas.

At the N-terminal sides of the heavy and light chains, there are variable regions which are called a heavy chain variable region (VH) and a light chain variable region (VL), respectively. In the variable region, there is a complementarity determining region (CDR) and this part is responsible for the specificity of antigen recognition. A part of the variable region other than CDR functions to retain the structure of CDR and is called a framework region (FR). At the C-terminal sides of the heavy and light chains, there are constant regions which are called a heavy chain constant region (CH) and a light chain constant region (CL), respectively. In the heavy chain variable region, there are three complementarity determining regions: the first complementarity determining region (CDR1), the second complementarity determining region (CDR2), and the third complementarity determining region (CDR3). The three complementarity determining regions in the heavy chain variable region are collectively called a heavy chain complementarity determining region. Likewise, there are three complementarity determining regions in the light chain variable region, which are the first complementarity determining region (CDR1), the second complementarity determining region (CDR2), and the third complementarity determining region (CDR3). These three complementarity determining regions in the light chain variable region are collectively called a light chain complementarity determining region.

CDR Sequence (amino acid sequence and base sequence) of the antibody used in the present invention are shown in the following TABLEs 21-24.

The amino acid sequences of the first complementarity determining region (CDR1), the second complementarity determining region (CDR2) and the third complementarity determining region (CDR3) in a heavy chain variable region of said antibody comprise the same amino acid sequence as the amino acid sequence represented by (i) the sequence identification number selected from the group consisting of SEQ ID NOS: 184, 200, 216, 232, 248, 264, 280 and 296, (ii) the sequence identification number selected from the group consisting of SEQ ID NOS: 185, 201, 217, 233, 249, 265, 281 and 297, and (iii) the sequence identification number selected from the group consisting of SEQ ID NOS:186, 202, 218, 234, 250, 266, 282 and 298, respectively.

The amino acid sequences of the first complementarity determining region (CDR1), the second complementarity determining region (CDR2) and the third complementarity determining region (CDR3) in a light chain variable region of said antibody comprise the same amino acid sequence as the amino acid sequence represented by (iv) the sequence identification number selected from the group consisting of SEQ ID NOS: 192, 208, 224, 240, 256, 272, 288 and 304, (v) the sequence identification number selected from the group consisting of SEQ ID NOS: 193, 209, 225, 241, 257, 273, 289 and 305, and (vi) the sequence identification number selected from the group consisting of SEQ ID NOS: 194, 210, 226, 242, 258, 274, 290 and 306, respectively.

As for the antibody used in the present invention, amino acid sequences other than CDR are not particularly limited but the antibody of the present invention includes a so-called CDR grafted antibody in which amino acid sequences other than CDR are derived from another antibody, especially from an antibody of different species. Human-derived amino acid sequences are preferred as the amino acid sequences other than CDR and may be accompanied, if necessary, by the addition, deletion, substitution and/or insertion of one or more amino acid residues in the framework region (FR).

As for the antibody used in the present invention, the amino acid sequence and base sequence in the variable regions of the antibody of the present invention are preferably those given in TABLE 25. The monoclonal antibody comprising a specific CDR amino acid sequence or variable region amino acid sequence of the antibody used in the present invention can be prepared using known methods.

(7) Monoclonal antibody binding to nectin-2 competitively with the antibody of the present invention used as the agent for preventing/treating breast cancer:

The monoclonal antibody which can be used includes the monoclonal antibody produced by the hybridoma cell represented by:

Nec1-803-2 (FERM BP-10417),
Nec1-244-3 (FERM BP-10423),
Nec1-530-1 (FERM BP-10424),
Nec1-903-1 (FERM BP-10425),
Nec1-520-1 (FERM BP-10426),
Nec1-845-2 (FERM BP-10427),
Nec1-834-1 (FERM BP-10428),
Nec1-964-1 (FERM BP-10683),
Nec1-1302-2 (FERM BP-10684),
Nec1-554-1 (FERM BP-10681),
Nec1-769-2 (FERM BP-10682), or,
Nec8-4116-8 (FERM BP-10685) or the monoclonal antibody, which binds to nectin-2 competitively with the antibodies (including antibody fragments) having specific CDR amino acid sequences or amino acid sequences in the variable region of the antibodies produced by these hybridomas (hereinafter also referred to as the antibody binding competitively with the antibody used in the present invention).

(7)-(i) Preparation of antigen

As the antigen used to prepare the antibody competitively binding to the antigen with the antibody used in the present invention, for example, a cell line or its membrane fraction wherein any one of the antigens such as a protein comprising the amino acid sequence represented by SEQ ID NO: 1 or SEQ ID NO: 3 (nectin-2), its partial peptide, or salts thereof, is highly expressed naturally or artificially; a fusion protein of the extracellular domain protein of nectin-2 and the other protein or peptide, or salts thereof; a (synthetic) peptide having one or more antigenic determinants, which are the same as in nectin-2; an animal cell expression vector comprising the nucleotide sequence represented by SEQ ID NO: 2 or SEQ ID NO: 4, or a part of the nucleotide sequence thereof, etc. can be used (hereinafter these antigens are sometimes merely referred to as the antigen used in the present invention).

Examples of the "other protein or peptide" to produce the fusion protein with extracellular domain of nectin-2 include FLAG-tag, His-tag, Myc-tag, V5-tag, GST-tag, S-tag, T7-tag, or the Fc regions of human antibody, mouse antibody, etc., and so on.

Although the length of a peptide having the same antigenic determinant as nectin-2 used to produce an antibody competitively binding to nectin-2 with the antibody used in the present invention is not limited so long as it has such a length as exhibiting immunogenicity, the peptide includes, for example, a peptide having, e.g., 6, preferably 10 and more preferably 12 consecutive amino acid residues.

A peptide having the sequence of at least 20, preferably at least 50, more preferably at least 70, still more preferably at least 100 and most preferably at least 200 amino acids in the constituent amino acid sequence of nectin-2, and the like are used as the protein comprising the amino acid sequence represented by SEQ ID NO: 1 or SEQ ID NO: 3, or its partial peptide, which is used to produce the antibody competitively binding to nectin-2 with the antibody used in the present invention.

Also, the antigenic determinant of the antibody used in the present invention is described in PCT/JP2006/320429, based on which the present application was filed. The description of PCT/JP2006/320429 is also incorporated in the present invention by reference.

Nectin-2 or its partial peptides, or salts thereof may be manufactured by publicly known methods or their modifications used to purify proteins from human or warm-blooded animal cells or tissues described above. Alternatively, they may also be manufactured by culturing transformants bearing DNAs encoding these proteins. And, they may also be manufactured according to a modification of the methods for peptide synthesis described later described. In addition, a fusion protein of the extracellular domain of nectin-2 and the other protein or peptide may also be manufactured by culturing transformants bearing DNAs encoding the fusion protein.

(7)-(ii) Production of monoclonal antibody

(a) Production of monoclonal antibody-producing cells by the hybridoma method

The antigen described in (2)-(i) is administered to warm-blooded animals. Immunization may be done by any method, as long as it can stimulate antibody production, and preferably used are intravenous injection, intraperitoneal injection, intramuscular injection, subcutaneous injection, intradermal injection, footpad injection, etc. The antigen used in the present invention may directly be used for immunization in its insolublized form. Alternatively, the antigen may be bound or adsorbed to an appropriate carrier and the resulting conjugate may be used for immunization.

When the antigen used in the present invention is administered, in order to potentiate the antibody productivity of an immununized animal, the antigen used in the present invention may be mixed with an adjuvant such as complete Freund's adjuvant or incomplete Freund's adjuvant, alum, a Ribi adjuvant, etc. and the resulting mixture or emulsion may be administered to the animal. Examples of warm-blooded animals used are monkeys, rabbits, dogs, guinea pigs, mice, rats, hamster, sheep, goats, camels, llamas and fowl, with the use of mice and rats being preferred. These warm-blooded animals may be KO animals wherein the warm-blooded animal ortholog genes of antigen proteins are knockout to achieve a more potent immune response against the antigen. Also, transgenic animals wherein antibody genes of warm-blooded animals are knockout and human antibody genes are introduced (see European Patent Application EPA 0546073), knock-in animals (WO 02/098217, WO 03/020743), etc. may be used to produce human monoclonal antibodies.

In producing the monoclonal antibody-producing cells, a warm-blooded animal, e.g., a mouse, wherein the antibody titer is observed is selected from animals immunized with the antigen, then spleen or lymph node is collected 2 to 5 days after the final immunization. The antibody-producing B cells contained therein are fused with myeloma cells derived from the same or different species, whereby hybridomas producing the monoclonal antibody can be produced. Examples of the myeloma cells are those collected from warm-blooded animals such as NS-1, P3U1, SP2/0, AP-1, etc. In particular, SP2/0 and P3U1 are preferably employed. Screening of hybridomas can be performed by publicly known methods or their modifications.

(b) Production of monoclonal antibody by other methods

The method for producing the antibody is not limited to the methods described above, but a so-called antibody display technology, which involves presenting an antibody gene library prepared by publicly known methods using B lymphocytes of, e.g., human or warm-blooded animal (e.g., monkey, rabbit, dog, guinea pig, mouse, rat, hamster, sheep, goat, camel, llama, fowl, etc.) as a material on the cell surface of bacteriophage, Escherichia coli, yeast, animal cells, etc. or on ribosome [Nature Biotechnology: 23, 1105 (2005)] can be used. Human or warm-blooded animals may be naive ones, patients carrying cancer which overexpresses the antigen used in the present invention, or warm-blooded animals which are immunized with the antigen used in the present invention by the method described in (a). The form of antibodies presented on the cell surface includes, but not limited to, IgG molecules, IgM molecules, Fab fragments, single chain Fv (scFv) fragments, etc.

The gene for the monoclonal antibody (fragment) capable of specifically binding to the antigen used in the present invention can be produced as follows. The aforesaid antibody (fragment)-presenting cell or antibody (fragment)-presenting ribosome carrying an antibody gene library is reacted with the antigen used in the present invention for a given period of time, followed by removing non-specifically bound substances by washing. After eluting and recovering the product bound specifically to the antigen used in the present invention, the antibody (fragment)-presenting cell or antibody (fragment)-presenting ribosome is allowed to grow. The same procedure is repeated several times, and finally the aimed gene can be isolated from the cloned antibody (fragment)-presenting cell or antibody (fragment)-presenting ribosome by publicly known methods. The thus obtained monoclonal antibody fragment gene is recombined with said region of the IgG antibody gene by publicly known methods to obtain the monoclonal antibody IgG antibody gene.

The monoclonal antibody comprising substantially the same amino acid sequence as a specific CDR amino acid sequence or a variable region amino acid sequence of the antibody used in the present invention can also be obtained by genetic engineering.

Herein, the aforesaid amino acid sequence which is substantially the same as a specific CDR amino acid sequence or a variable region amino acid sequence (hereinafter amino acid sequence A) of the monoclonal antibody used in the present invention includes amino acid sequences having at least about 50% homology, preferably at least about 60% homology, more preferably at least about 70% homology, still more preferably at least about 80% homology, much more preferably at least about 90% homology and most preferably at least about 95% homology, to the amino acid sequence A; etc.

Homology of the amino acid sequences can be measured using a homology scoring algorithm NCBI BLAST (National Center for Biotechnology Information Basic Local Alignment Search Tool) under the following conditions (an expectation value = 10; gaps are allowed; matrix = BLOSUM62; filtering = OFF).

Examples of the monoclonal antibody comprising the same or substantially the same amino acid sequence as the amino acid sequence A include antibody containing (i) an amino acid sequence wherein at least 1 or 2 (e.g., about 1 to about 50, preferably about 1 to about 30, more preferably about 1 to about 10 and most preferably several (1 to 5)) amino acids are deleted of the amino acid sequence A; (ii) an amino acid sequence wherein at least 1 or 2 (e.g., about 1 to about 50, preferably about 1 to about 30, more preferably about 1 to about 10 and most preferably several (1 to 5)) amino acids are added to the amino acid sequence A; (iii) an amino acid sequence wherein at least 1 or 2 (e.g., about 1 to about 50, preferably about 1 to about 30, more preferably about 1 to about 10 and most preferably several (1 to 5)) amino acids are inserted into the amino acid sequence A; (iv) an amino acid sequence wherein at least 1 or 2 (e.g., about 1 to about 50, preferably about 1 to about 30, more preferably about 1 to about 10 and most preferably several (1 to 5)) amino acids in the amino acid sequence A are substituted by other amino acids; or (v) an amino acid sequence which are in combination of abovementhions; and the like.

The antibody used in the present invention can also be obtained by immunizing antibody-producing cells isolated from human or the warm-blooded animals described above with the antigen used in the present invention in vitro by publicly known methods and then producing hybridomas.

The monoclonal antibody used in the present invention can be manufactured by culturing the monoclonal antibody-producing hybridoma obtained in (a) and the recombinant cell line in which the antibody gene isolated from the monoclonal antibody-producing hybridoma obtained in (a) by publicly known methods, or the monoclonal antibody gene obtained in (b) is artificially expressed. The monoclonal antibody can also be manufactured by incorporating the antibody gene into a chromosome of warm-blooded animal or plant by publicly known methods, and producing the monoclonal antibody in blood, milk and egg of warm-blooded animals, in plant body, in mold, etc. [Curr. Opin. Biotechnol., 7, 536 (1996), Nature Rev. Genet., 4, 794 (2003), Appl. Environ. Microbiol., 70, 2567 (2004)]. Examples of warm-blooded animals used are bovine, goat, sheep, swine, fowl, mouse, rabbit, etc. Examples of plant bodies are tobacco, sweet corn, potato, duckweed, etc.

Various methods can be used for screening of the monoclonal antibody-producing hybridomas. Examples of such methods include a method which involves adding the culture supernatant of a hybridoma to a solid phase (e.g., microplate) adsorbed with a soluble protein antigen or protein antigen-expressing cell, directly or together with a carrier, followed by the reaction with an anti-immunoglobulin antibody (for example, when spleen cells used for the cell fusion are from mouse, an anti-mouse immunoglobulin antibody is used) labeled with a radioactive substance, an enzyme, a fluorescent substance, etc., or with Protein A, and detecting the monoclonal antibody bound to the solid phase; a method which involves adding the hybridoma supernatant to a solid phase adsorbed with an anti-immunoglobulin antibody or Protein A, followed by the reaction with a soluble protein antigen labeled with a radioactive substance, an enzyme, or a fluorescent substance, etc. and detecting the antigen-specific monoclonal antibody bound to the solid phase; etc. When the protein antigen-expressing cell is used, the hybridoma culture supernatant is added to the cell, followed by the reaction with a fluorescence-labeled anti-immunoglobulin antibody, and the fluorescence intensity of the cell is assayed on a fluorescence detector such as a flow cytometer, etc. Thus, the monoclonal antibody bound to the protein antigen on the cell membrane can be detected.

The monoclonal antibody used in the present invention can be purified from the above-described raw materials containing the monoclonal antibody, for example, by publicly known methods for separation and purification of immunoglobulins [e.g., salting out, alcohol precipitation, isoelectric precipitation, various chromatographies such as ion exchange chromatography, hydrophobic interaction chromatography, reverse phase chromatography, gel filtration chromatography, hydroxyapatite chromatography, affinity chromatography in which only antibody can be separated and purified with a carrier to which a substance having affinity to the antibody such as antigen, protein A, and protein G, etc is immobilized.], and the like.

(7)-(iii) Screening of antibody competitively binding to nectin-2 with the antibody used as the agent of this invention for preventing/treating breast cancer

The antibody which binds competitively with the antibody used in the present invention can be obtained by screening in an assay to determine if the antibody binds to nectin-2 competitively with the antibody used in the present invention.

The "nectin-2" used for the screening is the amino acid sequence represented by SEQ ID NO: 1 (hereinafter briefly referred to as nectin-2α) or the amino acid sequence represented by SEQ ID NO: 3 (hereinafter briefly referred to as nectin-2δ) (hereinafter both nectins are sometimes collectively referred to as nectin-2 or the protein used in the present invention). The nectin-2 may be proteins derived from human or warm-blooded animal cells or tissues, or recombinant proteins. They may also be peptides shown by a partial sequence of the amino acid sequence represented by SEQ ID NO: 1 or SEQ ID NO: 3. Examples of the nectin-2, which can be used, are a peptide shown by the 1st-350th (extracellular domain) amino acid sequence in the amino acid sequence represented by SEQ ID NO: 1 (nectin-2α) or SEQ ID NO: 3 (nectin-2δ), a peptide shown by the 47th-142nd (the first IG-like domain) amino acid sequence in the amino acid sequence represented by SEQ ID NO: 1 (nectin-2α) or SEQ ID NO: 3 (nectin-2δ), or a peptide shown by the 175th-240th (the second IG-like domain) amino acid sequence in the amino acid sequence represented by SEQ ID NO: 1 (nectin-2α) or SEQ ID NO: 3 (nectin-2δ) and the like. Nectin-2 further includes peptides wherein the C-terminal carboxyl group is esterified or amidated, those wherein the amino group at the N-terminal amino acid residue (e.g., methionine residue) is protected with a protecting group, those wherein the N-terminal region is cleaved *in vivo* and the glutamyl residue thus formed is pyroglutamated; those wherein a substituent on the side chain of an amino acid in the molecule is protected with a suitable protecting group, or conjugated peptides such as glycopeptides bound to sugar chains, etc. These peptides may be in the form of salts with physiologically acceptable acids (e.g., inorganic acids, organic acids) or bases (e.g., alkaline metal salts), etc.

As used herein, the term "antibody binding competitively" refers to an antibody, which binding to nectin-2 is competitively inhibited by adding an excess of any one of the antibodies used in the present invention. Specifically, the antibody refers to, for example, an antibody showing at least 50% inhibition against the binding of a test antibody to nectin-2, when 50-fold molar amount of any one of the antibodies used in the present invention is added to said antibody under test.

As used herein, the antibody used in the present invention includes a chimeric antibody, a humanized antibody and a human antibody. The "chimeric antibody" means an antibody which has the variable regions derived from an antibody of different species and constant regions of human antibody (see, e.g., EP 0125023, etc.). The "humanized antibody" refers to an antibody designed to modify a human-heterologous antibody like a mouse antibody, by replacing its primary structure other than the complementarity determining regions of H and L chains with the corresponding primary structure of a human antibody. The "human antibody" refers to a monoclonal antibody prepared using a transgenic animal carrying human antibody genes (see EP 0546073) and a monoclonal antibody prepared using a library in which a human antibody gene is presented on the cell surface of bacteriophage, Escherichia coli, yeast, animal cells, etc., a so-called antibody display technology (Nature Biotechnology, 23, 1105 (2005)), and a monoclonal antibody isolated from human B cells producing an antibody against nectin-2 using cell fusion, phage display, or the like. The antibody used in the present invention is preferably a monoclonal antibody with the constant regions of the antibody belonging to a human antibody, more preferably human IgG; and most preferably human $IgG_1$ subclass.

(8) Agent for preventing/treating breast cancer of the present invention

The medicament comprising the aforesaid antibody used in the present invention or the antibody binding to nectin-2 competitively with the antibody used in the present invention can be used as an agent for preventing/treating breast cancer, an agent for preventing or treating breast cancer using a host defense mechanism mediated by the Fc region of the antibody, an antibody-dependent cytotoxic agent against breast cancer cells, etc. The method for damaging breast cancer cells using a host defense mechanism mediated by the Fc region of the antibody includes antibody-dependent cellular cytotoxicity (ADCC) by effector cells of living body and complement-dependent cytotoxicity (CDC), and ADCC is preferably used.

The antibody used in the present invention or the antibody competitively binding to nectin-2 with the antibody used in the present invention is low toxic, and can be administered orally or parenterally (e.g., intravascular administration, subcutaneous administration, etc.) to human or mammals (e.g., rats, rabbits, sheep, swine, bovine, cats, dogs, monkeys, etc.) as it is in the form of liquid preparation or as a pharmaceutical composition of appropriate dosage form.

The antibody used in the present invention or the antibody competitively binding to nectin-2 with the antibody used in the present invention may be administered directly, or may be administered as an appropriate pharmaceutical composition. The pharmaceutical composition used for the administration may contain the antibody used in the present invention and its salt, a pharmacologically acceptable carrier, and a diluent or excipient. Such a pharmaceutical composition is provided in the dosage form suitable for oral or parenteral administration.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. The injectable preparations may be prepared, for example, by dissolving, suspending or emulsifying the antibody of the present invention or its salt in a sterile aqueous medium or an oily medium conventionally used for injections. As the aqueous medium for injections, there are used, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, for example, there are e.g., sesame oil, soybean oil, etc., which may be used in combination with a solubilizing agent such as benzyl benzoate, benzyl alcohol, etc. The injectable preparation thus prepared is usually filled in an appropriate ampoule. The suppository used for rectal administration may be prepared by blending the antibody of the present invention or its salt with conventional bases for suppositories.

The composition for oral administration includes solid or liquid dosage forms, specifically, tablets (including dragees and film-coated tablets), pills, granules, powdery preparations, capsules (including soft capsules), syrup, emulsions, suspensions, etc. Such a composition is manufactured by publicly known methods and may contain a vehicle, a diluent or excipient conventionally used in the field of pharmaceutical preparations. Examples of the vehicle or excipient for tablets are lactose, starch, sucrose, magnesium stearate, etc.

Advantageously, the pharmaceutical compositions for oral or parenteral use described above are prepared into pharmaceutical preparations with a unit dosage form suitable for a dose of the active ingredients. Such unit dosage forms include, for example, tablets, pills, capsules, injections (ampoules), suppositories, etc. The amount of the antibody used in the present invention or the antibody competitively binding to nectin-2 with the antibody used in the present invention contained is generally 5 to 500 mg per dosage unit form; it is preferred that the antibody described above is contained in about 5 to about 100 mg especially in the form of injection, and in 10 to 250 mg for the other dosage forms.

The dose of the aforesaid preventive/therapeutic agent or regulator comprising the antibody used in the present invention or the antibody competitively binding to nectin-2 with the antibody used in the present invention may vary depending upon subject to be administered, target disease, conditions, route of administration, etc. but the agent or regulator may advantageously be administered intravenously in a single dose of about 0.01 to about 20 mg/kg body weight, preferably about 0.1 to about 10 mg/kg body weight and more preferably about 0.1 to about 5 mg/kg body weight per administration as the antibody of the present invention, about 1 to 5 times/day, preferably about 1 to 3 times/day when it is used for treating/preventing, for example, breast cancer in adult. Also in other parenteral and oral administration, the agent or regulator can be administered in a dose corresponding to the dose given above. When the condition is especially severe, the dose may be increased according to the condition.

The antibody used in the present invention or the antibody competitively binding to nectin-2 with the antibody used in the present invention may be administered as it stands or in the form of an appropriate pharmaceutical composition. The pharmaceutical composition used for the aforesaid administration contains the aforesaid antibody or its salts, a pharmacologically acceptable carrier, and a diluent or excipient. Such a composition is provided in the dosage form suitable for oral or parenteral administration (e.g., intravascular injection, subcutaneous injection, etc.).

Each composition described above may further contain other active components unless formulation causes any adverse interaction by formulating with the antibody described above.

Furthermore, the antibody used in the present invention or the antibody competitively binding to nectin-2 with the antibody used in the present invention may be used in combination with other drugs, for example, alkylating agents (e.g., cyclophosphamide, ifosfamide, etc.), metabolic antagonists (e.g., methotrexate, 5-fluorouracil, gemcitabine, etc.), antitumor antibiotics (e.g., mitomycin, adriamycin, etc.), plant-derived antitumor agents (e.g., vincristine, vindesine, paclitaxel, docetaxel, etc.), hormone therapeutic agents (e.g., tamoxifen, anastrozole, letrozole, etc.), platinum preparations (e.g., cisplatin, carboplatin, etc.), molecular targeting agents (e.g., herceptin, gefitinib and imatinib, etc.), etoposide, irinotecan, etc. The antibody used in the present invention and the drugs described above may be administered simultaneously or at staggered times to the patient.

(9) This invention further includes the following features.

That is, the present invention is further directed to the hybridoma cell represented by Nec1-1044-4 (FERM BP-10805), Nec8-3517-11 (FERM BP-10806) or Nec8-3 704-7 (FERM BP-10807); the monoclonal antibody produced from the hybridoma cell represented by Nec1-1044-4 (FERM BP-10805), Nec8-3517-11 (FERM BP-10806) or Nec8-3 704-7 (FERM BP-10807); the monoclonal antibody binding competitively with the monoclonal antibody produced from the hybridoma cell represented by Nec1-1044-4 (FERM BP-10805), Nec8-3517-11 (FERM BP-10806) or Nec8-3 704-7 (FERM BP-10807); and the agent for preventing or treating breast cancer comprising these monoclonal antibodies.

These features can be implemented as in the embodiments (6) to (8) described above.

Hybridoma Nec1-1044-4 has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10805 since April 3, 2007.

Hybridoma Nec8-3517-11 has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10806 since April 3, 2007.

Hybridoma Nec8-3704-7 has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10807 since April 3, 2007.

In the specification and drawings, where bases, amino acids, etc. are denoted by their abbreviations, they are based on conventional codes in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A | : adenine |
| T | : thymine |
| G | : guanine |
| C | : cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP | : adenosine triphosphate |
| EDTA : | ethylenediaminetetraacetic acid |
| SDS | : sodium dodecyl sulfate |
| Gly | : glycine |
| Ala | : alanine |
| Val | : valine |
| Leu | : leucine |
| Ile | : isoleucine |

Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln : glutamine
pGlu : pyroglutamic acid
Sec : selenocysteine

The sequence identification numbers in the sequence listing of the specification indicate the following sequences.

[SEQ ID NO: 1]

This shows the amino acid sequence of nectin-2α.

[SEQ ID NO: 2]

This shows the base sequence of DNA encoding nectin-2α having the amino acid sequence represented by SEQ ID NO: 1.

[SEQ ID NO: 3]

This shows the amino acid sequence of nectin-2δ.

[SEQ ID NO: 4]

This shows the base sequence of DNA encoding nectin-2δ having the amino acid sequence represented by SEQ ID NO: 3.

[SEQ ID NO: 5]

This shows the amino acid sequence of nectin-3.

[SEQ ID NO: 6]

This shows the base sequence of DNA encoding nectin-3 having the amino acid sequence represented by SEQ ID NO: 5.

[SEQ ID NO: 7]

This shows the base sequence of the antisense oligonucleotide 1 used in REFERENCE EXAMPLES 1 and 2.

[SEQ ID NO: 8]

This shows the base sequence of the control oligonucleotide 1 used in REFERENCE EXAMPLES 1 and 2.

[SEQ ID NO: 9]

This shows the base sequence of primer 1 used in REFERENCE EXAMPLE 2.

[SEQ ID NO: 10]

This shows the base sequence of primer 2 used in REFERENCE EXAMPLE 2.

[SEQ ID NO: 11]

This shows the base sequence of TaqMan probe 1 used in REFERENCE EXAMPLE 2.

[SEQ ID NO: 12]

This shows the base sequence of primer 3 used in REFERENCE EXAMPLE 2.

[SEQ ID NO: 13]

This shows the base sequence of primer 4 used in REFERENCE EXAMPLE 2.

[SEQ ID NO: 14]

This shows the base sequence of TaqMan probe 2 used in REFERENCE EXAMPLE 2.

[SEQ ID NO: 15]

This shows the base sequence of primer 5 used in REFERENCE EXAMPLES 3 and 4.

[SEQ ID NO: 16]

This shows the base sequence of primer 6 used in REFERENCE EXAMPLE 3.

[SEQ ID NO: 17]

This shows the base sequence of primer 7 used in REFERENCE EXAMPLE 4.

[SEQ ID NO: 18]

This shows the base sequence of primer 8 used in REFERENCE EXAMPLE 5.

[SEQ ID NO: 19]

This shows the base sequence of siRNA-1 used in REFERENCE EXAMPLES 5, 6 and 7.

[SEQ ID NO: 20]

This shows the base sequence of siRNA-1 used in REFERENCE EXAMPLES 5, 6 and 7.

[SEQ ID NO: 21]

This shows the base sequence of siRNA-2 used in REFERENCE EXAMPLES 5, 6 and 7.

[SEQ ID NO: 22]

This shows the base sequence of siRNA-2 used in REFERENCE EXAMPLES 5, 6 and 7.

[SEQ ID NO: 23]

This shows the base sequence of siRNA-3 used in REFERENCE EXAMPLES 5, 6 and 7.

[SEQ ID NO: 24]

This shows the base sequence of siRNA-3 used in REFERENCE EXAMPLES 5, 6 and 7.

[SEQ ID NO: 25]

This shows the base sequence of siRNA-4 used in REFERENCE EXAMPLES 5, 6 and 7.

[SEQ ID NO: 26]

This shows the base sequence of siRNA-4 used in REFERENCE EXAMPLES 5, 6 and 7.

[SEQ ID NO: 27]

This shows the base sequence of siRNA-5 used in REFERENCE EXAMPLES 5, 6 and 7.

[SEQ ID NO: 28]

This shows the base sequence of siRNA-5 used in REFERENCE EXAMPLES 5, 6 and 7.

[SEQ ID NO: 29]

This shows the base sequence of primer 33 used in REFERENCE EXAMPLE 12.

[SEQ ID NO: 30]

This shows the base sequence of primer 34 used in REFERENCE EXAMPLE 12.

[SEQ ID NO: 31]

This shows the amino acid sequence of nectin-2ED-FLAG protein.

[SEQ ID NO: 32]

This shows the base sequence of DNA encoding the amino acid sequence of nectin-2ED-FLAG protein represented by SEQ ID NO: 31.

[SEQ ID NO: 33]

This shows the base sequence of primer 33 used in REFERENCE EXAMPLE 15.

[SEQ ID NO: 34]

This shows the base sequence of primer 34 used in REFERENCE EXAMPLE 15.

[SEQ ID NO: 35]

This shows the base sequence of primer 35 used in REFERENCE EXAMPLE 15.

[SEQ ID NO: 36]

This shows the base sequence of primer 36 used in REFERENCE EXAMPLE 15.

[SEQ ID NO: 37]

This shows the amino acid sequence of nectin-2ED-hFc protein.

[SEQ ID NO: 38]

This shows the base sequence of DNA encoding the amino acid sequence of nectin-2ED-FLAG protein represented by SEQ ID NO: 37.

[SEQ ID NO: 39]

This shows the amino acid sequence of the peptide 1 used in REFERENCE EXAMPLE 17.

[SEQ ID NO: 40]

This shows the amino acid sequence of the peptide 2 used in REFERENCE EXAMPLE 17.

[SEQ ID NO: 41]

This shows the amino acid sequence of the peptide 3 used in REFERENCE EXAMPLE 17.

[SEQ ID NO: 42]

This shows the base sequence of primer 42 used in REFERENCE EXAMPLES 18 and 19.

[SEQ ID NO: 43]

This shows the base sequence of primer 43 used in REFERENCE EXAMPLES 18 and 19.

[SEQ ID NO: 44]

This shows the base sequence of TaqMan probe 3 used in REFERENCE EXAMPLES 18 and 19.

[SEQ ID NO: 45]

This shows the base sequence of primer 45 used in REFERENCE EXAMPLE 21.

[SEQ ID NO: 46]

This shows the base sequence of primer 46 used in REFERENCE EXAMPLE 21.

[SEQ ID NO: 47]

This shows the base sequence of primer 47 used in REFERENCE EXAMPLE 21.

[SEQ ID NO: 48]

This shows the base sequence of primer 48 used in REFERENCE EXAMPLE 21.

[SEQ ID NO: 49]

This shows the amino acid sequence of nectin-3ED-hFc protein.

[SEQ ID NO: 50]

This shows the base sequence of DNA encoding the amino acid sequence of nectin-3ED-hFc protein represented by SEQ ID NO: 49.

[SEQ ID NO: 51]

This shows the amino acid sequence of nectin-3ED-mFc protein.

[SEQ ID NO: 52]

This shows the base sequence of DNA encoding the amino acid sequence of nectin-3ED-mFc protein represented by SEQ ID NO: 51.

[SEQ ID NO: 53]

This shows the base sequence of DNA encoding the amino acid sequence of FLAG protein (FLAG-FSALNOT) used in REFERENCE EXAMPLE 25.

[SEQ ID NO: 54]

This shows the base sequence of DNA encoding the amino acid sequence of FLAG protein (FLAG-RSALNOT) used in REFERENCE EXAMPLE 25.

[SEQ ID NO: 55]

This shows the base sequence of primer 55 used in REFERENCE EXAMPLE 25.

[SEQ ID NO: 56]

This shows the base sequence of primer 56 used in REFERENCE EXAMPLE 25.

[SEQ ID NO: 57]

This shows the amino acid sequence of nectin-3ED-FLAG protein.

[SEQ ID NO: 58]

This shows the base sequence of DNA encoding the amino acid sequence of nectin-3ED-FLAG protein.

[SEQ ID NO: 59]

This shows the base sequence of primer 59 used in REFERENCE EXAMPLE 29.

[SEQ ID NO: 60]

This shows the base sequence of primer 60 used in REFERENCE EXAMPLE 29.

[SEQ ID NO: 61]

This shows the base sequence of DNA encoding the amino acid sequence of nectin-1 protein.

[SEQ ID NO: 62]

This shows the amino acid sequence of nectin-1 protein.

[SEQ ID NO: 63]

This shows the base sequence of primer 63 used in REFERENCE EXAMPLE 29.

[SEQ ID NO: 64]

This shows the base sequence of primer 64 used in REFERENCE EXAMPLE 29.

[SEQ ID NO: 65]

This shows the base sequence of primer 65 used in REFERENCE EXAMPLE 29.

[SEQ ID NO: 66]

This shows the base sequence of primer 66 used in REFERENCE EXAMPLE 29.

[SEQ ID NO: 67]

This shows the base sequence of DNA encoding the amino acid sequence of nectin-4 protein.

[SEQ ID NO: 68]

This shows the amino acid sequence of nectin-4 protein.

[SEQ ID NO: 69]

This shows the base sequence of primer 69 used in REFERENCE EXAMPLE 29.

[SEQ ID NO: 70]

This shows the base sequence of primer 70 used in REFERENCE EXAMPLE 29.

[SEQ ID NO: 71]

This shows the amino acid sequence of Nec1-5 protein.

[SEQ ID NO: 72]

This shows the base sequence of DNA encoding the amino acid sequence of Nec1-5 protein.

[SEQ ID NO: 73]

This shows the base sequence of primer 73 used in REFERENCE EXAMPLE 30.

[SEQ ID NO: 74]

This shows the base sequence of primer 74 used in REFERENCE EXAMPLE 30.

[SEQ ID NO: 75]

This shows the base sequence of primer 75 used in REFERENCE EXAMPLE 30.

[SEQ ID NO: 76]

This shows the base sequence of primer 76 used in REFERENCE EXAMPLE 30.

[SEQ ID NO: 77]

This shows the base sequence of primer 77 used in REFERENCE EXAMPLE 30.

[SEQ ID NO: 78]

This shows the amino acid sequence of Ig1 domain-deficient protein in nectin-2.

[SEQ ID NO: 79]

This shows the base sequence of DNA encoding the amino acid sequence of Ig1 domain-deficient protein in nectin-2.

[SEQ ID NO: 80]

This shows the amino acid sequence of Ig2 domain-deficient protein in nectin-2.

[SEQ ID NO: 81]

This shows the base sequence of DNA encoding the amino acid sequence of Ig2 domain-deficient protein in nectin-2.

[SEQ ID NO: 82]

This shows the base sequence of primer 82 used in REFERENCE EXAMPLE 31.

[SEQ ID NO: 83]

This shows the base sequence of primer 83 used in REFERENCE EXAMPLE 31.

[SEQ ID NO: 84]

This shows the base sequence of primer 84 used in REFERENCE EXAMPLE 31.

[SEQ ID NO: 85]

This shows the base sequence of primer 85 used in REFERENCE EXAMPLE 31.

[SEQ ID NO: 86]

This shows the amino acid sequence of cynomolgus monkey nectin-2 protein.

[SEQ ID NO: 87]

This shows the base sequence of DNA encoding the amino acid sequence of cynomolgus monkey nectin-2 protein.

[SEQ ID NO: 88]

This shows the base sequence of primer 88 used in REFERENCE EXAMPLE 32.

[SEQ ID NO: 89]

This shows the base sequence of primer 89 used in REFERENCE EXAMPLE 32.

[SEQ ID NO: 90]

This shows the base sequence of primer 90 used in REFERENCE EXAMPLE 33.

[SEQ ID NO: 91]

This shows the base sequence of primer 91 used in REFERENCE EXAMPLE 33.

[SEQ ID NO: 92]

This shows the base sequence of primer 92 used in REFERENCE EXAMPLE 33.

[SEQ ID NO: 93]

This shows the base sequence of primer 93 used in REFERENCE EXAMPLE 33.

[SEQ ID NO: 94]

This shows the base sequence of primer 94 used in REFERENCE EXAMPLE 33.

[SEQ ID NO: 95]

This shows the base sequence of primer 95 used in REFERENCE EXAMPLE 33.

[SEQ ID NO: 96]

This shows the base sequence of primer 96 used in REFERENCE EXAMPLE 33.

[SEQ ID NO: 97]

This shows the base sequence of primer 97 used in REFERENCE EXAMPLE 33.

[SEQ ID NO: 98]

This shows the base sequence of primer Q37A used in REFERENCE EXAMPLE 34.

[SEQ ID NO: 99]

This shows the base sequence of primer Q3 7A R used in REFERENCE EXAMPLE 34.

[SEQ ID NO: 100]

This shows the base sequence of primer P40G used in REFERENCE EXAMPLE 34.

[SEQ ID NO: 101]

This shows the base sequence of primer P40G R used in REFERENCE EXAMPLE 34.

[SEQ ID NO: 102]

This shows the base sequence of primer Q45A used in REFERENCE EXAMPLE 34.

[SEQ ID NO: 103]

This shows the base sequence of primer Q45A R used in REFERENCE EXAMPLE 34.

[SEQ ID NO: 104]

This shows the base sequence of primer H55A used in REFERENCE EXAMPLE 34.

[SEQ ID NO: 105]

This shows the base sequence of primer H55A R used in REFERENCE EXAMPLE 34.

[SEQ ID NO: 106]

This shows the base sequence of primer V60A used in REFERENCE EXAMPLE 34.

[SEQ ID NO: 107]

This shows the base sequence of primer V60A R used in REFERENCE EXAMPLE 34.

[SEQ ID NO: 108]

This shows the base sequence of primer Y64A used in REFERENCE EXAMPLE 34.

[SEQ ID NO: 109]

This shows the base sequence of primer Y64A R used in REFERENCE EXAMPLE 34.

[SEQ ID NO: 110]

This shows the base sequence of primer Q71A used in REFERENCE EXAMPLE 34.

[SEQ ID NO: 111]

This shows the base sequence of primer Q71AR used in REFERENCE EXAMPLE 34.

[SEQ ID NO: 112]

This shows the base sequence of primer A75G used in REFERENCE EXAMPLE 34.

[SEQ ID NO: 113]

This shows the base sequence of primer A75G R used in REFERENCE EXAMPLE 34.

[SEQ ID NO: 114]

This shows the base sequence of primer P76G used in REFERENCE EXAMPLE 34.

[SEQ ID NO: 115]

This shows the base sequence of primer P76G R used in REFERENCE EXAMPLE 34.

[SEQ ID NO: 116]

This shows the base sequence of primer A77G used in REFERENCE EXAMPLE 34.

[SEQ ID NO: 117]

This shows the base sequence of primer A77G R used in REFERENCE EXAMPLE 34.

[SEQ ID NO: 118]

This shows the base sequence of primer N78A used in REFERENCE EXAMPLE 34.

[SEQ ID NO: 119]

This shows the base sequence of primer N78A R used in REFERENCE EXAMPLE 34.

[SEQ ID NO: 120]

This shows the base sequence of primer H79A used in REFERENCE EXAMPLE 34.

[SEQ ID NO: 121]

This shows the base sequence of primer H79AR used in REFERENCE EXAMPLE 34.

[SEQ ID NO: 122]

This shows the base sequence of primer Q80A used in REFERENCE EXAMPLE 34.

[SEQ ID NO: 123]

This shows the base sequence of primer Q80A R used in REFERENCE EXAMPLE 34.

[SEQ ID NO: 124]

This shows the base sequence of primer N81 A used in REFERENCE EXAMPLE 34.

[SEQ ID NO: 125]

This shows the base sequence of primer N81A R used in REFERENCE EXAMPLE 34.

[SEQ ID NO: 126]

This shows the base sequence of primer K88A used in REFERENCE EXAMPLE 34.

[SEQ ID NO: 127]

This shows the base sequence of primer K88A R used in REFERENCE EXAMPLE 34.

[SEQ ID NO: 128]

This shows the base sequence of primer S95A used in REFERENCE EXAMPLE 34.

[SEQ ID NO: 129]

This shows the base sequence of primer S95A R used in REFERENCE EXAMPLE 34.

[SEQ ID NO: 130]

This shows the base sequence of primer K109A used in REFERENCE EXAMPLE 34.

[SEQ ID NO: 131]

This shows the base sequence of primer K109A R used in REFERENCE EXAMPLE 34.

[SEQ ID NO: 132]

This shows the base sequence of primer E117Aused in REFERENCE EXAMPLE 34.

[SEQ ID NO: 133]

This shows the base sequence of primer E117A R used in REFERENCE EXAMPLE 34.

[SEQ ID NO: 134]

This shows the base sequence of primer D122A used in REFERENCE EXAMPLE 34.

[SEQ ID NO: 135]

This shows the base sequence of primer D122A R used in REFERENCE EXAMPLE 34.

[SEQ ID NO: 136]

This shows the base sequence of primer H128A used in REFERENCE EXAMPLE 34.

[SEQ ID NO: 137]

This shows the base sequence of primer H128A R used in REFERENCE EXAMPLE 34.

[SEQ ID NO: 138]

This shows the base sequence of primer N137A used in REFERENCE EXAMPLE 34.

[SEQ ID NO: 139]

This shows the base sequence of primer N137A R used in REFERENCE EXAMPLE 34.

[SEQ ID NO: 140]

This shows the base sequence of primer F 145A used in REFERENCE EXAMPLE 34.

[SEQ ID NO: 141]

This shows the base sequence of primer F 145A R used in REFERENCE EXAMPLE 34.

[SEQ ID NO: 142]

This shows the base sequence of primer K147A used in REFERENCE EXAMPLE 34.

[SEQ ID NO: 143]

This shows the base sequence of primer K147A R used in REFERENCE EXAMPLE 34.

[SEQ ID NO: 144]

This shows the base sequence of primer V150A used in REFERENCE EXAMPLE 34.

[SEQ ID NO: 145]

This shows the base sequence of primer V150A R used in REFERENCE EXAMPLE 34.

[SEQ ID NO: 146]

This shows the base sequence of primer M153A used in REFERENCE EXAMPLE 34.

[SEQ ID NO: 147]

This shows the base sequence of primer M153A R used in REFERENCE EXAMPLE 34.

[SEQ ID NO: 148]

This shows the base sequence of primer T154A used in REFERENCE EXAMPLE 34.

[SEQ ID NO: 149]

This shows the base sequence of primer T154AR used in REFERENCE EXAMPLE 34.

[SEQ ID NO: 150]

This shows the base sequence of primer Q165A used in REFERENCE EXAMPLE 35.

[SEQ ID NO: 151]

This shows the base sequence of primer Q165AR used in REFERENCE EXAMPLE 35.

[SEQ ID NO: 152]

This shows the base sequence of primer K170A used in REFERENCE EXAMPLE 35.

[SEQ ID NO: 153]

This shows the base sequence of primer K170A R used in REFERENCE EXAMPLE 35.

[SEQ ID NO: 154]

This shows the base sequence of primer F173A used in REFERENCE EXAMPLE 35.

[SEQ ID NO: 155]

This shows the base sequence of primer F173A R used in REFERENCE EXAMPLE 35.

[SEQ ID NO: 156]

This shows the base sequence of primer P 177G used in REFERENCE EXAMPLE 35.

[SEQ ID NO: 157]

This shows the base sequence of primer P177G R used in REFERENCE EXAMPLE 35.

[SEQ ID NO: 158]

This shows the base sequence of primer I184A used in REFERENCE EXAMPLE 35.

[SEQ ID NO: 159]

This shows the base sequence of primer I184A R used in REFERENCE EXAMPLE 35.

[SEQ ID NO: 160]

This shows the base sequence of primer K186A used in REFERENCE EXAMPLE 35.

[SEQ ID NO: 161]

This shows the base sequence of primer K186A R used in REFERENCE EXAMPLE 35.

[SEQ ID NO: 162]

This shows the base sequence of primer L197A used in REFERENCE EXAMPLE 35.

[SEQ ID NO: 163]

This shows the base sequence of primer L197A R used in REFERENCE EXAMPLE 35.

[SEQ ID NO: 164]

This shows the base sequence of primer W202A used in REFERENCE EXAMPLE 35.

[SEQ ID NO: 165]

This shows the base sequence of primer W202A R used in REFERENCE EXAMPLE 35.

[SEQ ID NO: 166]

This shows the base sequence of primer E206A used in REFERENCE EXAMPLE 35.

[SEQ ID NO: 167]

This shows the base sequence of primer E206A R used in REFERENCE EXAMPLE 35.

[SEQ ID NO: 168]

This shows the base sequence of primer T212A used in REFERENCE EXAMPLE 35.

[SEQ ID NO: 169]

This shows the base sequence of primer T212A R used in REFERENCE EXAMPLE 35.

[SEQ ID NO: 170]

This shows the base sequence of primer T235A used in REFERENCE EXAMPLE 35.

[SEQ ID NO: 171]

This shows the base sequence of primer T23 5A R used in REFERENCE EXAMPLE 35.

[SEQ ID NO: 172]

This shows the base sequence of primer K239A used in REFERENCE EXAMPLE 35.

[SEQ ID NO: 173]

This shows the base sequence of primer K239A R used in REFERENCE EXAMPLE 35.

[SEQ ID NO: 174]

This shows the base sequence of primer A249G used in REFERENCE EXAMPLE 35.

[SEQ ID NO: 175]

This shows the base sequence of primer A249G R used in REFERENCE EXAMPLE 35.

[SEQ ID NO: 176]

This shows the base sequence of primer 176 used in EXAMPLE 19.

[SEQ ID NO: 177]

This shows the base sequence of primer 177 used in EXAMPLE 19.

[SEQ ID NO: 178]

This shows the base sequence of primer 178 used in EXAMPLE 19.

[SEQ ID NO: 179]

This shows the base sequence of primer 179 used in EXAMPLE 19.

[SEQ ID NO: 180]

This shows the base sequence of primer 180 used in EXAMPLE 19.

[SEQ ID NO: 181]

This shows the base sequence of primer 181 used in EXAMPLE 19.

[SEQ ID NO: 182]

This shows the base sequence of primer 182 used in EXAMPLE 19.

[SEQ ID NO: 183]

This shows the base sequence of primer 183 used in EXAMPLE 19.

[SEQ ID NO: 184]

This shows CDR1 in the heavy chain of Nec1-244-3 (amino acid sequence).

[SEQ ID NO: 185]

This shows CDR2 in the heavy chain of Nec1-244-3 (amino acid sequence).

[SEQ ID NO: 186]

This shows CDR3 in the heavy chain of Nec1-244-3 (amino acid sequence).

[SEQ ID NO: 187]

This shows the amino acid sequence in the heavy chain variable region of Nec1-244-3.

[SEQ ID NO: 188]

This shows CDR1 in the heavy chain of Nec1-244-3 (base sequence).

[SEQ ID NO: 189]

This shows CDR2 in the heavy chain of Nec1-244-3 (base sequence).

[SEQ ID NO: 190]

This shows CDR3 in the heavy chain of Nec1-244-3 (base sequence).

[SEQ ID NO: 191]

This shows the base sequence in the heavy chain variable region of Nec1-244-3.

[SEQ ID NO: 192]

This shows CDR1 in the light chain of Nec1-244-3 (amino acid sequence).

[SEQ ID NO: 193]

This shows CDR2 in the light chain of Nec1-244-3 (amino acid sequence).

[SEQ ID NO: 194]

This shows CDR3 in the light chain of Nec1-244-3 (amino acid sequence).

[SEQ ID NO: 195]

This shows the amino acid sequence in the light variable region of Nec1-244-3.

[SEQ ID NO: 196]

This shows CDR1 in the light chain of Nec1-244-3 (base sequence).

[SEQ ID NO: 197]

This shows CDR2 in the light chain of Nec1-244-3 (base sequence).

[SEQ ID NO: 198]

This shows CDR3 in the light chain of Nec1-244-3 (base sequence).

[SEQ ID NO: 199]

This shows the base sequence in the light variable region of Nec1-244-3.

[SEQ ID NO: 200]

This shows CDR1 in the heavy chain of Nec1-530-1 (amino acid sequence).

[SEQ ID NO: 201]

This shows CDR2 in the heavy chain of Nec1-530-1 (amino acid sequence).

[SEQ ID NO: 202]

This shows CDR3 in the heavy chain of Nec1-530-1 (amino acid sequence).

[SEQ ID NO: 203]

This shows the amino acid sequence in the heavy chain variable region of Nec1-530-1.

[SEQ ID NO: 204]

This shows CDR1 in the heavy chain of Nec1-530-1 (base sequence).

[SEQ ID NO: 205]

This shows CDR2 in the heavy chain of Nec1-530-1 (base sequence).

[SEQ ID NO: 206]

This shows CDR3 in the heavy chain of Nec1-530-1 (base sequence).

[SEQ ID NO: 207]

This shows the base sequence in the heavy chain variable region of Nec1-530-1.

[SEQ ID NO: 208]

This shows CDR1 in the light chain of Nec1-530-1 (amino acid sequence).

[SEQ ID NO: 209]

This shows CDR2 in the light chain of Nec1-530-1 (amino acid sequence).

[SEQ ID NO: 210]

This shows CDR3 in the light chain of Nec1-530-1 (amino acid sequence).

[SEQ ID NO: 211]

This shows the amino acid sequence in the light variable region of Nec1-530-1.

[SEQ ID NO: 212]

This shows CDR1 in the light chain of Nec1-530-1 (base sequence).

[SEQ ID NO: 213]

This shows CDR2 in the light chain of Nec1-530-1 (base sequence).

[SEQ ID NO: 214]

This shows CDR3 in the light chain of Nec1-530-1 (base sequence).

[SEQ ID NO: 215]

This shows the base sequence in the light variable region of Nec1-530-1.

[SEQ ID NO: 216]

This shows CDR1 in the heavy chain of Nec1-554-1 (amino acid sequence).

[SEQ ID NO: 217]

This shows CDR2 in the heavy chain of Nec1-554-1 (amino acid sequence).

[SEQ ID NO: 218]

This shows CDR3 in the heavy chain of Nec1-554-1 (amino acid sequence).

[SEQ NO: 219]

This shows the amino acid sequence in the heavy chain variable region of Nec1-554-1.

[SEQ ID NO: 220]

This shows CDR1 in the heavy chain of Nec1-554-1 (base sequence).

[SEQ ID NO: 221]

This shows CDR2 in the heavy chain of Nec1-554-1 (base sequence).

[SEQ ID NO: 222]

This shows CDR3 in the heavy chain of Nec1-554-1 (base sequence).

[SEQ ID NO: 223]

This shows the base sequence in the heavy chain variable region of Nec1-554-1.

[SEQ ID NO: 224]

This shows CDR1 in the light chain of Nec1-554-1 (amino acid sequence).

[SEQ ID NO: 225]

This shows CDR2 in the light chain of Nec1-554-1 (amino acid sequence).

[SEQ ID NO: 226]

This shows CDR3 in the light chain of Nec1-554-1 (amino acid sequence).

[SEQ ID NO: 227]

This shows the amino acid sequence in the light variable region of Nec1-554-1.

[SEQ ID NO: 228]

This shows CDR1 in the light chain of Nec1-554-1 (base sequence).

[SEQ ID NO: 229]

This shows CDR2 in the light chain of Nec1-554-1 (base sequence).

[SEQ ID NO: 230]

This shows CDR3 in the light chain of Nec1-554-1 (base sequence).

[SEQ ID NO: 231]

This shows the base sequence in the light variable region of Nec1-554-1.

[SEQ ID NO: 232]

This shows CDR1 in the heavy chain of Nec1-803-2 (amino acid sequence).

[SEQ ID NO: 233]

This shows CDR2 in the heavy chain of Nec1-803-2 (amino acid sequence).

[SEQ ID NO: 234]

This shows CDR3 in the heavy chain of Nec1-803-2 (amino acid sequence).

[SEQ ID NO: 235]

This shows the amino acid sequence in the heavy chain variable region of Nec1-803-2.

[SEQ ID NO: 236]

This shows CDR1 in the heavy chain of Nec1-803-2 (base sequence).

[SEQ ID NO: 237]

This shows CDR2 in the heavy chain of Nec1-803-2 (base sequence).

[SEQ ID NO: 238]

This shows CDR3 in the heavy chain of Nec1-803-2 (base sequence).

[SEQ ID NO: 239]

This shows the base sequence in the heavy chain variable region of Nec1-803-2.

[SEQ ID NO: 240]

This shows CDR1 in the light chain of Nec1-803-2 (amino acid sequence).

[SEQ ID NO: 241]

This shows CDR2 in the light chain of Nec1-803-2 (amino acid sequence).

[SEQ ID NO: 242]

This shows CDR3 in the light chain of Nec1-803-2 (amino acid sequence).

[SEQ ID NO: 243]

This shows the amino acid sequence in the light variable region of Nec1-803-2.

[SEQ ID NO: 244]

This shows CDR1 in the light chain of Nec1-803-2 (base sequence).

[SEQ ID NO: 245]

This shows CDR2 in the light chain of Nec1-803-2 (base sequence).

[SEQ ID NO: 246]

This shows CDR3 in the light chain of Nec1-803-2 (base sequence).

[SEQ ID NO: 247]

This shows the base sequence in the light variable region of Nec1-803-2.

[SEQ ID NO: 248]

This shows CDR1 in the heavy chain of Nec1-834-1 (amino acid sequence).

[SEQ ID NO: 249]

This shows CDR2 in the heavy chain of Nec1-834-1 (amino acid sequence).

[SEQ ID NO: 250]

This shows CDR3 in the heavy chain of Nec1-834-1 (amino acid sequence).

[SEQ ID NO: 25]

This shows the amino acid sequence in the heavy chain variable region of Nec1-834-1.

[SEQ ID NO: 252]

This shows CDR1 in the heavy chain of Necl-834-1 (base sequence).

[SEQ ID NO: 253]

This shows CDR2 in the heavy chain of Nec1-834-1 (base sequence).

[SEQ ID NO: 254]

This shows CDR3 in the heavy chain of Nec1-834-1 (base sequence).

[SEQ ID NO: 255]

This shows the base sequence in the heavy chain variable region of Nec1-834-1.

[SEQ ID NO: 256]

This shows CDR1 in the light chain of Nec1-834-1 (amino acid sequence).

[SEQ ID NO: 257]

This shows CDR2 in the light chain of Nec1-834-1 (amino acid sequence).

[SEQ ID NO: 258]

This shows CDR3 in the light chain of Nec1-834-1 (amino acid sequence).

[SEQ ID NO: 259]

This shows the amino acid sequence in the light variable region of Nec1-834-1.

[SEQ ID NO: 260]

This shows CDR1 in the light chain of Nec1-834-1 (base sequence).

[SEQ ID NO: 261]

This shows CDR2 in the light chain of Nec1-834-1 (base sequence).

[SEQ ID NO: 262]

This shows CDR3 in the light chain of Nec1-834-1 (base sequence).

[SEQ ID NO: 263]

This shows the base sequence in the light variable region of Nec1-834-1.

[SEQ ID NO: 264]

This shows CDR1 in the heavy chain of Nec1-845-2 (amino acid sequence).

[SEQ ID NO: 265]

This shows CDR2 in the heavy chain of Nec1-845-2 (amino acid sequence).

[SEQ ID NO: 266]

This shows CDR3 in the heavy chain of Nec1-845-2 (amino acid sequence).

[SEQ ID NO: 267]

This shows the amino acid sequence in the heavy chain variable region of Nec1-845-2.

[SEQ ID NO: 268]

This shows CDR1 in the heavy chain of Nec1-845-2 (base sequence).

[SEQ ID NO: 269]

This shows CDR2 in the heavy chain of Nec1-845-2 (base sequence).

[SEQ ID NO: 270]

This shows CDR3 in the heavy chain of Nec1-845-2 (base sequence).

[SEQ ID NO: 271]

This shows the base sequence in the heavy chain variable region of Nec1-845-2.

[SEQ ID NO: 272]

This shows CDR1 in the light chain of Nec1-845-2 (amino acid sequence).

[SEQ ID NO: 273]

This shows CDR2 in the light chain of Nec1-845-2 (amino acid sequence).

[SEQ ID NO: 274]

This shows CDR3 in the light chain of Nec1-845-2 (amino acid sequence).

[SEQ ID NO: 275]

This shows the amino acid sequence in the light variable region of Nec1-845-2.

[SEQ ID NO: 276]

This shows CDR1 in the light chain of Nec1-845-2 (base sequence).

[SEQ ID NO: 277]

This shows CDR2 in the light chain of Nec1-845-2 (base sequence).

[SEQ ID NO: 278]

This shows CDR3 in the light chain of Nec1-845-2 (base sequence).

[SEQ ID NO: 279]

This shows the base sequence in the light variable region of Nec1-845-2.

[SEQ ID NO: 280]

This shows CDR1 in the heavy chain of Nec1-903-1 (amino acid sequence).

[SEQ ID NO: 281]

This shows CDR2 in the heavy chain of Nec1-903-1 (amino acid sequence).

[SEQ ID NO: 282]

This shows CDR3 in the heavy chain of Nec1-903-1 (amino acid sequence).

[SEQ ID NO: 283]

This shows the amino acid sequence in the heavy chain variable region of Nec1-903-1.

[SEQ ID NO: 284]

This shows CDR1 in the heavy chain of Nec1-903-1 (base sequence).

[SEQ ID NO: 285]

This shows CDR2 in the heavy chain of Nec1-903-1 (base sequence).

[SEQ ID NO: 286]

This shows CDR3 in the heavy chain of Nec1-903-1 (base sequence).

[SEQ ID NO: 287]

This shows the base sequence in the heavy chain variable region of Nec1-903-1.

[SEQ ID NO: 288]

This shows CDR1 in the light chain of Nec1-903-1 (amino acid sequence).

[SEQ ID NO: 289]

This shows CDR2 in the light chain of Nec1-903-1 (amino acid sequence.

[SEQ ID NO: 290]

This shows CDR3 in the light chain of Nec1-903-1 (amino acid sequence).

[SEQ ID NO: 291]

This shows the amino acid sequence in the light variable region of Nec1-903-1.

[SEQ ID NO: 292]

This shows CDR1 in the light chain of Nec1-903-1 (base sequence).

[SEQ ID NO: 293]

This shows CDR2 in the light chain of Nec1-903-1 (base sequence).

[SEQ ID NO: 294]

This shows CDR3 in the light chain of Nec1-903-1 (base sequence).

[SEQ ID NO: 295]

This shows the base sequence in the light variable region of Nec1-903-1.

[SEQ ID NO: 296]

This shows CDR1 in the heavy chain of Nec8-4116-8 (amino acid sequence).

[SEQ ID NO: 297]

This shows CDR2 in the heavy chain of Nec8-4116-8 (amino acid sequence).

[SEQ ID NO: 298]

This shows CDR3 in the heavy chain of Nec8-4116-8 (amino acid sequence).

[SEQ ID NO: 299]

This shows the amino acid sequence in the heavy chain variable region of Nec8-4116-8.

[SEQ ID NO: 300]

This shows CDR1 in the heavy chain of Nec8-4116-8 (base sequence).

[SEQ ID NO: 301]

This shows CDR2 in the heavy chain of Nec8-4116-8 (base sequence).

[SEQ ID NO: 302]

This shows CDR3 in the heavy chain of Nec8-4116-8 (base sequence).

[SEQ ID NO: 303]

This shows the amino acid sequence in the heavy chain variable region of Nec8-4116-8.

[SEQ ID NO: 304]

This shows CDR1 in the light chain of Nec8-4116-8 (amino acid sequence).

[SEQ ID NO: 305]

This shows CDR2 in the light chain of Nec8-4116-8 (amino acid sequence).

[SEQ ID NO: 306]

This shows CDR3 in the light chain of Nec8-4116-8 (amino acid sequence).

[SEQ ID NO: 307]

This shows the amino acid sequence in the light variable region of Nec8-4116-8.

[SEQ ID NO: 308]

This shows CDR1 in the light chain of Nec8-4116-8 (base sequence).

[SEQ ID NO: 309]

This shows CDR2 in the light chain of Nec8-4116-8 (base sequence).

[SEQ ID NO: 310]

This shows CDR3 in the light chain of Nec8-4116-8 (base sequence).

[SEQ ID NO: 311]

This shows the amino acid sequence in the light variable region of Nec8-4116-8.

[SEQ ID NO: 312]

This shows the N-terminal amino acid sequence of H chain in the antibody preparation obtained in REFERENCE EXAMPLE 38.

[SEQ ID NO: 313]

This shows the N-terminal amino acid sequence of L chain in the antibody preparation obtained in REFERENCE EXAMPLE 38.

[SEQ ID NO: 314]

This shows the base sequence encoding the N terminus of putative signal sequence from the germline coincident with the amino acid sequence of SEQ ID NO: 312.

[SEQ ID NO: 315]

This shows the base sequence encoding the N terminus of putative signal sequence from the germline coincident with the amino acid sequence of SEQ ID NO: 313.

[SEQ ID NO: 316]

This shows the base sequence of the primer used in REFERENCE EXAMPLE 38.

[SEQ ID NO: 317]

This shows the base sequence of the primer used in REFERENCE EXAMPLE 38.
Hybridoma Nec1-803-2 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10417 since September 16, 2005.
Hybridoma Nec1-244-3 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10423 since October 4, 2005.
Hybridoma Nec1-530-1 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10424 since October 4, 2005.
Hybridoma Nec1-903-1 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10425 since October 4, 2005.
Hybridoma Nec1-520-1 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10426 since October 4, 2005.
Hybridoma Nec1-845-2 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10427 since October 4, 2005.
Hybridoma Nec1-834-1 obtained in EXAMPLE 1 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10428 since October 4, 2005.
Hybridoma Nec1-554-1 obtained in EXAMPLE 8 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10681 since September 20, 2006.
Hybridoma Nec1-769-2 obtained in EXAMPLE 8 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10682 since September 20, 2006.
Hybridoma Nec1-964-1 obtained in EXAMPLE 8 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10683 since September 20, 2006.
Hybridoma Nec1-1302-2 obtained in EXAMPLE 8 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10684 since September 20, 2006.
Hybridoma Nec8-4116-8 obtained in EXAMPLE 8 later described has been deposited on International Patent Organisms Depository, National Institute of Advanced Industrial Science and Technology, located at Central 6, 1-1-1 Higashi, Tsukuba, Ibaraki (postal code: 305-8566) under Accession Number FERM BP-10685 since September 20, 2006.

## EXAMPLES

Hereinafter the present invention will be described specifically by referring to REFERENCE EXAMPLES and EXAMPLES but is not deemed to be limited thereto.

### REFERENCE EXAMPLE 1

Apoptosis induction in human colon cancer cell line HT-29 by the antisense oligonucleotide of the nectin-2α gene and nectin-2δ gene

After the antisense oligonucleotide sequence (SEQ ID NO: 7) hybridizable to the coding region of nectin-2α gene or to the intron region of nectin-2δ gene was designed, the phosphorothioated oligonucleotide was synthesized to obtain the HPLC-purified product (hereinafter merely referred to as antisense oligonucleotide 1). As a control, the oligonucleotide (SEQ ID NO: 8) having a reverse sequence of the base sequence represented by SEQ ID NO: 7 was likewise phosphorothioated to obtain the HPLC-purified authentic product (hereinafter merely referred to as control oligonucleotide 1).

Human colon cancer cell line HT-29 purchased from American Type Culture Collection (ATCC) was suspended in McCoy's 5A medium (Invitrogen) supplemented with 10% fetal bovine serum (FBS) (JRH) [hereinafter sometimes abbreviated as M5 medium], and plated on a 96-well flat bottom tissue culture plate (Becton Dickinson) at a cell density of 1 x $10^4$ cells/well, followed by incubation at 37°C overnight in a 5% carbon dioxide gas flow. The antisense oligonucleotide 1, 200 ng, or 200 ng of the control oligonucleotide 1 was mixed with 50 μL of Opti-MEM I (Invitrogen) together with 0.5 μL of Lipofectamine 2000 (Invitrogen) and the mixture was allowed to stand at room temperature for 20 minutes. The whole volume of the solution mixture above was added to the HT-29 cell culture, which medium had previously been exchanged with 50 μL of Opti-MEM I, the incubation was continued for further 3 hours at 37°C in a 5% carbon dioxide gas flow. Thereafter, the medium was again exchanged with M5 medium. After the incubation was continued for further 2 days, the apoptosis induction activity of the oligonucleotide above was measured using Caspase-Glo 3/7 Assay (Promega) in accordance with the protocol attached. As a result, the antisense oligonucleotide 1 (SEQ ID NO: 7) of the nectin-2α gene and nectin-2δ gene showed the apoptosis induction activity of approximately 1.9 times higher than the control oligonucleotide 1 (SEQ ID NO: 8), indicating that there was a statistically significant difference ($P<0.05$).

REFERENCE EXAMPLE 2

Reduction in mRNA expression levels of nectin-2α gene and nectin-2δ gene in human colon cancer cell line HT-29 by antisense oligonucleotide of nectin-2α gene and nectin-2δ gene

Human colon cancer cell line HT-29 used in REFERENCE EXAMPLE 1 was suspended in M5 medium and plated on a 24-well flat bottom tissue culture plate (Becton Dickinson) at a cell density of 6 x $10^4$ cells/well. After the cells were incubated overnight at 37°C in a 5% carbon dioxide gas flow, the antisense oligonucleotide 1 or the control oligonucleotide 1 was transfected by the procedure of REFERENCE EXAMPLE 1, except that the weight or volume of all additives was scaled up to 6 times in proportion to the count of cells plated. After these cells were incubated at 37°C for 24 hours in a 5% carbon dioxide gas flow, the total RNA was extracted by RNeasy Mini Total RNA Kit (QIAGEN). Using about 400 ng of the total RNA as a template, reverse transcription was carried out to prepare cDNA using TaqMan Reverse Transcription Reagents (Applied Biosystems) in accordance with the protocol attached. Expression level of the nectin-2α gene was measured by quantitative PCR using the cDNA as a template in an amount corresponding to 5 ng when calculated as the total RNA; the reaction solution was made up to 15 μL by adding 7.5 μL of TaqMan Universal PCR Master Mix (Applied Biosystems), 500 nM each of primer 1 (SEQ ID NO: 9) and primer 2 (SEQ ID NO: 10) and 100 nM of FAM-labeled TaqMan probe 1 (SEQ ID NO: 11). PCR was carried out by reacting at 50°C for 2 minutes and 95°C for 10 minutes and then repeating 40 times the cycle set to include 95°C for 15 seconds and 60°C for 1 minute. The expression level of nectin-2δ was measured as in the nectin-2α gene by quantitative PCR, in which the cDNA as a template was used in an amount corresponding to 5 ng when calculated as the total RNA and the reaction solution was made up to 15 μL by adding 7.5 μL of TaqMan Universal PCR Master Mix, 500 nM each of primer 3 (SEQ ID NO: 12) and primer 4 (SEQ ID NO: 13) and 100 nM of FAM-labeled TaqMan probe 2 (SEQ ID NO: 14). PCR was carried out by reacting at 50°C for 2 minutes and 95°C for 10 minutes and then repeating 40 times the cycle set to include 95°C for 15 seconds and 60°C for 1 minute. The expression level of mRNA for the β-actin gene contained in the same amount of template cDNA was assayed using TaqMan β-actin Control Reagents (Applied Biosystems), which was used as the internal standard.

Where no oligonucleotide was transfected, the expression levels of the nectin-2α and nectin-2δ genes were 0.15% and 0.76% of the β-actin gene expression levels, respectively. In the groups given with the antisense oligonucleotide 1 (SEQ ID NO: 7), expression levels of the nectin-2α and nectin-2δ genes were 0.095% and 0.45%, respectively, indicating that a statistically significant ($P<0.01$) reduction in the expression level was observed when compared to the case where no oligonucleotide was transfected. On the other hand, in the group given with the control oligonucleotide 1 (SEQ ID NO: 8) used as negative control, expression levels of the nectin-2α and nectin-2δ genes were 0.18% and 0.76%, respectively, indicating that it was the same as in the case where no oligonucleotide was transfected.

These results and the results of REFERENCE EXAMPLE 1 suggest that reduction in expression levels of the nectin-2α and nectin-2δ genes induced the apoptosis of human colon cancer cell line HT-29.

REFERENCE EXAMPLE 3

Cloning and base sequencing of cDNA encoding nectin-2α

Using human lung cancer cell line A549-derived Marathon-Ready cDNA (BD Biosciences) as a template, PCR was carried out by using primer 5 (SEQ ID NO: 15) tagged with the recognition site of restriction enzyme EcoRI and primer 6 (SEQ ID NO: 16) tagged with the recognition site of restriction enzyme EcoRV In this reaction, the reaction solution was composed of 1 μL of the cDNA described above, 1 U of PfuTurbo Hotstart DNA Polymerase (STRATAGENE), 1 μM each of primer 5 (SEQ ID NO: 15) and primer 6 (SEQ ID NO: 16), 200 μM dNTPs and 10 μL of 2 x GC Buffer I (TaKaRa Bio) to make the total 20 μL. PCR was carried out by reacting at 94°C for 1 minute and then repeating 5 times the cycle set to include 94°C for 5 seconds and 72°C for 4 minutes, 5 times the cycle set to include 94°C for 5 seconds and 70°C for 4 minutes and 35 times the cycle set to include 94°C for 5 seconds and 68°C for 4 minutes. Next, the PCR product was purified using PCR Purification Kit (QIAGEN). The purified product was digested with restriction enzymes EcoRI and EcoRV. pcDNA3.1(+) (Invitrogen) was also treated with restriction enzymes EcoRI and EcoRV These products were purified usign PCR Purification Kit. The respective DNA fragments were ligated using DNA Ligation Kit ver. 2 (TaKaRa Bio) and then transfected to Escherichia coli TOP 10 (Invitrogen), followed by incubation for selection in ampicillin-containing LB agar medium. As a result of sequencing analysis of individual gene clones recovered from the grown colony of Escherichia coli, the animal cell expression vector pcDNA3.1 (+)-Nectin-2α bearing the cDNA sequence (SEQ ID NO: 2) encoding the nectin-2α protein (SEQ ID NO: 1) was obtained.

## REFERENCE EXAMPLE 4

### Cloning and base sequencing of cDNA encoding nectin-2δ

Using human lung cancer cell line A549-derived Marathon-Ready cDNA (BD Biosciences) as a template, PCR was carried out by using primer 5 (SEQ ID NO: 15) tagged with the recognition site of restriction enzyme EcoRI and primer 7 (SEQ ID NO: 17) tagged with the recognition site of restriction enzyme EcoRV In this reaction, the reaction solution was composed of 1 μL of the above cDNA used as a template, 1 U of PfuTurbo Hotstart DNA Polymerase (STRATAGENE), 1 μM each of primer 5 (SEQ ID NO: 15) and primer 7 (SEQ ID NO: 17), 200 μM dNTPs and 10 μL of 2 x GC Buffer I (TaKaRa Bio) to make the total 20 μL. PCR was carried out by reacting at 94°C for 1 minute, then repeating 5 times the cycle set to include 94°C for 5 seconds and 72°C for 4 minutes, 5 times the cycle set to include 94°C for 5 seconds and 70°C for 4 minutes and 35 times the cycle set to include 94°C for 5 seconds and 68°C for 4 minutes. The purified product was then eluted with 50 μL of water using PCR Purification Kit (QIAGEN), and using the same as a template, PCR was carried out. In this reaction, the reaction solution was composed of 1 μL of the above PCR product used as a template, 1 U of Pfu Turbo Hotstart DNA Polymerase, 1 μM each of primer 5 (SEQ ID NO: 15) and primer 8 (SEQ ID NO: 18) tagged with the recognition site of restriction enzyme EcoRV, 200 μM dNTPs and 10 μL of 2 x GC Buffer I to make the total 20 μL. PCR was carried out by reacting at 94°C for 1 minute, then repeating 25 times the cycle set to include 94°C for 20 seconds, 60°C for 15 seconds and 72°C for 2 minutes. Next, the PCR product was purified using PCR Purification Kit. The purified product was then digested with restriction enzymes EcoRI and EcoRV Similarly, pcDNA3.1(+) (Invitrogen) was digested with restriction enzymes EcoRI and EcoRV After these products were purified using PCR Purification Kit, the two DNA fragments were ligated using DNA Ligation Kit ver. 2 (TaKaRa Bio) and then transfected to Escherichia coli TOP 10 (Invitrogen), followed by incubation for selection in ampicillin-containing LB agar medium. As a result of sequencing analysis of the individual gene clones recovered from the grown colony of Escherichia coli, the animal cell expression vector pcDNA3.1(+)-Nectin-2δ bearing the cDNA sequence (SEQ ID NO: 4) encoding the nectin-2δ protein (SEQ ID NO: 3) was obtained.

## REFERENCE EXAMPLE 5

### Cell growth inhibition of human colon cancer cell line HT-29 by nectin-2 siRNA

Mixtures were prepared by mixing five siRNAs (siRNA-1, siRNA-2, siRNA-3, siRNA-4 and siRNA-5) specific to mRNA of the nectin-2α gene or nectin-2δ gene (hereinafter they are collectively referred to as the nectin-2 gene) on an equal volume basis (hereinafter the mixtures are referred to as the nectin-2-siRNA). The siRNA-1, siRNA-2, siRNA-3, siRNA-4 and siRNA-5 were prepared by hybridizing two RNA fragments, respectively (siRNA-1 was prepared by hybridizing RNA having the base sequence represented by SEQ ID NO: 19 to RNA having the base sequence represented by SEQ ID NO: 20, siRNA-2 by hybridizing RNA having the base sequence represented by SEQ ID NO: 21 to RNA having the base sequence represented by SEQ ID NO: 22, siRNA-3 by hybridizing RNA having the base sequence represented by SEQ ID NO: 23 to RNA having the base sequence represented by SEQ ID NO: 24, siRNA-4 by hybridizing RNA having the base sequence represented by SEQ ID NO: 25 to RNA having the base sequence represented by SEQ ID NO: 26, and siRNA-5 by hybridizing RNA having the base sequence represented by SEQ ID NO: 27 to RNA having the base sequence represented by SEQ ID NO: 28). Non-specific Control IX (hereinafter referred to as non-silencing dsRNA) purchased from Dharmacon was used as a negative control.

Specifically, human colon cancer cell line HT-29 purchased from American Type Culture Collection (ATCC) was suspended in M5A medium supplemented with 10% FBS (JRH) and plated on a 10-cm tissue culture Petri dish (Becton Dickinson) at a cell density of 5 x $10^5$ cells/dish. After incubation overnight at 37°C in a 5% carbon dioxide gas flow, the cells were detached using a trypsin/EDTA solution and recovered by centrifugal operation. The HT-29 cells, 1 x $10^6$, were suspended in 100 μL of solution V included in Cell Line Nucleofector Kit V (Amaxa), which solution contained 150 pmol of nectin-2-siRNA or 150 pmol of non-silencing dsRNA, and transfected using Nucleofector program T-20, followed by incubation at 37°C for 24 hours in a 5% carbon dioxide gas flow These cells were again plated on a 96-well flat bottom tissue culture plate at a cell density of 3,000 cells/well and the incubation was continued for 5 days at 37°C in a 5% carbon dioxide gas flow. After the medium was removed from each well, the plate was cooled at -80°C for 5 minutes and allowed to stand at room temperature for 5 minutes to disrupt the cells. Next, 100 μl of an aqueous solution containing 1% PicoGreen (Invitrogen) and 1% IGEPAL-CA630 (ICN) was added to each well, which was allowed to stand at room temperature for 20 minutes. Then, the fluorescence intensity was measured (excitation wavelength at 485 nm and emission wavelength at 535 nm) using Multilabel Counter (Perkin Elmer) to determine the DNA level in the cells. As a result, the fluorescence intensity decreased by about 38% in the nectin-2-siRNA group, when compared to the non-silencing dsRNA group, indicating that there was a statistically significant difference (P<0.001). This reveals that growth of the HT-29 cell line was significantly inhibited by addition of the nectin-2-siRNA.

REFERENCE EXAMPLE 6

Change in cell cycle of human colon cancer cell line HT-29 by nectin-2-siRNA

Human colon cancer cell line HT-29 used in REFERENCE EXAMPLE 1 was suspended in M5A medium and plated on a 10 cm tissue culture Petri dish at a cell density of 5 x $10^5$ cells/dish. After incubation overnight at 37°C in a 5% carbon dioxide gas flow, nectin-2-siRNA or non-silencing dsRNA as a negative control was transfected by a modification of the procedure of REFERENCE EXAMPLE 5. The incubation was continued at 37°C for 24 hours in a 5% carbon dioxide gas flow. These cells were again plated on a 6-well flat bottom tissue culture plate (Becton Dickinson) at a cell density of 2 x $10^5$ cells/well and incubated at 37°C for 5 days in a 5% carbon dioxide gas flow. After the culture cells in each well were detached by trypsin-EDTA treatment, cell cycle analysis was performed using FACScan (Becton Dickinson) using CycleTEST Plus DNA Reagent Kit (Becton Dickinson). As a result, the ratio of cells in the G0/G1 phase increased by about 13% and the ratio of cells in the S-phase decreased by about 11% in the nectin-2-siRNA group, as compared to the non-silencing dsRNA group used as the negative control. The results suggest that the change in cell cycle of human colon cancer cell line HT-29 was induced by nectin-2-siRNA.

REFERENCE EXAMPLE 7

Reduction in mRNA expression level of nectin-2 in human colon cancer cell line HT-29 by nectin-2-siRNA

Human colon cancer cell line HT-29 used in REFERENCE EXAMPLE 1 was suspended in M5A medium and plated on a 10 cm tissue culture Petri dish at a cell density of 5 x $10^5$ cells/dish. After incubation overnight at 37°C in a 5% carbon dioxide gas flow, nectin-2-siRNA or non-silencing dsRNA as a negative control was transfected by a modification of the procedure of REFERENCE EXAMPLE 5, followed by incubation at 37°C for 24 hours in a 5% carbon dioxide gas flow. The total RNA was extracted from these cells using RNeasy Mini Total RNA Kit (QIAGEN). Using about 100 ng of the total RNA as a template, reverse transcription was performed by using TaqMan Reverse Transcription Reagents (Applied Biosystems). The expression level of mRNA of the nectin-2α gene was measured by quantitative PCR, in which the cDNA as a template was used in an amount corresponding to 10 ng when calculated as the total RNA, and the reaction solution was made up to 10 μL by adding 5 μL of TaqMan Universal PCR Master Mix (Applied Biosystems), 500 nM each of primer 1 (SEQ ID NO: 9) and primer 2 (SEQ ID NO: 10) and 100 nM of FAM-labeled TaqMan probe 1 (SEQ ID NO: 11). On the other hand, the expression level of mRNA of the nectin-2δ gene was measured by quantitative PCR, in which the cDNA as a template was used in an amount corresponding to 10 ng when calculated as the total RNA, and the reaction solution was made up to 10 μL by adding 5 μL of TaqMan Universal PCR Master Mix, 500 nM each of primer 3 (SEQ ID NO: 12) and primer 4 (SEQ ID NO: 13) and 100 nM of FAM-labeled TaqMan probe 2 (SEQ ID NO: 14). PCR was carried out by reacting at 50°C for 2 minutes and 95°C for 10 minutes and then repeating 40 times the cycle set to include 95°C for 15 seconds and 60°C for 1 minute. The expression level of mRNA for β-actin contained in the same amount of the template cDNA was measured using TaqMan β-actin Control Reagents (Applied Biosystems) and used as the internal standard.

The expression levels of mRNA of nectin-2α and nectin-2δ decreased by 69% and 73%, respectively, in the nectin-2-siRNA group, when compared to the non-silencing dsRNA group used as a negative control, indicating that there was a statistically significant difference (P<0.001). These results indicate that the reduction in expression levels of mRNA for the nectin-2α and nectin-2δ genes was induced by nectin-2-siRNA.

REFERENCE EXAMPLE 8

Enhanced expression of nectin-2 mRNA in human cancer tissue

The expression levels of mRNA for the nectin-2 gene between human cancer tissues and human normal tissues were compared and studied by quantitative PCR. As templates for PCR, cDNA CeHAT-SD Breast Tumor 1 (Cosmo Bio), cDNA CeHAT-SD Breast Tumor 2 (Cosmo Bio), Human Colon Matched cDNA Pair Panel (BD Biosciences), Human Lung Matched cDNA Pair Panel (BD Biosciences) and Human Ovary Matched cDNA Pair Panel (BD Biosciences) were used. The expression level of mRNA for the nectin-2α gene was measured as follows: 1 μL of cDNA was used as a template and the reaction solution was made up to 15 μL by adding 7.5 μL of TaqMan Universal PCR Master Mix (Applied Biosystems), 500 nM each of primer 1 (SEQ NO: 9) and primer 2 (SEQ ID NO: 10) and 100 nM of FAM-labeled TaqMan probe 1 (SEQ ID NO: 11). The expression level of mRNA for the nectin-2δ gene was measured as follows: 1 μL of cDNA was used as a template and the reaction solution was made up to 15 μL by adding 7.5 μL of TaqMan Universal PCR Master Mix, 500 nM each of primer 3 (SEQ ID NO: 12) and primer 4 (SEQ ID NO: 13) and 100 nM of FAM-labeled TaqMan probe 2 (SEQ ID NO: 14), except that the amount of the template was 0.2 μL for cDNA CeHAT-SD Breast Tumor 1 (Cosmo Bio) and cDNA CeHAT-SD Breast Tumor 2 (Cosmo Bio). PCR was performed by reacting at 50°C for 2 minutes and 95°C for 10 minutes and then repeating 40 times the cycle set to include 95°C for 15 seconds and 60°C for 1 minute. On the other hand, the expression level of mRNA for β-actin contained in the same amount of the template cDNA was measured and used as the internal standard. As a result, the expression level of mRNA for the nectin-2α gene increased in cancer tissues of 3 donors included in cDNA CeHAT-SD Breast Tumor 1 (Cosmo Bio) by 1.1 times, 10 times and 4.3 times, respectively, and increased in cancer tissues of 3 donors included in cDNA CeHAT-SD Breast Tumor 2 (Cosmo Bio) by 12 times, 3.5 times and 21 times, respectively, when compared to the expression level in human normal tissues. Likewise, the expression level of mRNA for the nectin-2α gene increased in cancer tissues of 5 donors included in Human Colon Matched cDNA Pair Panel (BD Biosciences) by 4.8 times, 3.2 times, 2.6 times, 1.9 times and 1.8 times, respectively, as compared to normal tissues; in cancer tissues of 5 donors included in Human Lung Matched cDNA Pair Panel (BD Biosciences) by 11 times, 3.7 times, 4.1 times, 3.2 times and 1.3 times, respectively, as compared to normal tissues; and, in cancer tissues of 4 out of 5 donors included in Human Ovary Matched cDNA Pair Panel (BD Biosciences) by 1.3 times, 1.8 times, 2.6 times and 2.4 times, respectively, as compared to normal tissues. The expression level of mRNA for the nectin-2δ gene in cancer tissues increased in cancer tissues of 2 out of 3 donors included in cDNA CeHAT-SD Breast Tumor 1 (Cosmo Bio), which was 1.1 times and 5.3 times, respectively, and in cancer tissues of 2 out of 3 donors included in cDNA CeHAT-SD Breast Tumor 2 (Cosmo Bio), which was 2.0 times and 2.5 times, respectively, as compared to the expression level in normal tissues. Likewise, the expression level of mRNA for the nectin-2δ gene was found in cancer tissues with 3 out of 5 donors included in Human Colon Matched cDNA Pair Panel (BD Biosciences) to be 1.3 times, 1.8 times and 1.5 times, respectively, as compared to normal tissues; in cancer tissues of 4 out of 5 donors included in Human Lung Matched cDNA Pair Panel (BD Biosciences) to be 4.8 times, 3.7 times, 1.1 times and 1.3 times, respectively, as compared to normal tissues; and in cancer tissues of 4 out of 5 donors included in Human Ovary Matched cDNA Pair Panel (BD Biosciences) to be 4.2 times, 2.1 times, 2.4 times and 4.2 times, respectively. From these results, it was confirmed that mRNA for the nectin-2α gene and nectin-2δ gene was overexpressed in cancer tissues, as compared to normal tissues.

REFERENCE EXAMPLE 9

Comparison in expression level of mRNA of nectin-2 gene in human cancer cell line

Osteosarcoma cell line Saos-2; brain tumor cell lines SK-N-MC, SK-N-AS, SK-N-BE, SK-N-DZ, SK-N-FI, SK-N-SH, D341 Med, Daoy, DBTRG-05MG, U-118 MG, U-87 MG, CCF-STTG1 and SW 1088; breast cancer cell lines HCC1937, ZR-75-1, AU565, MCF-7, MDA-MB-231, SKBR-3, BT474, MDA-MB-435s, MDA-MB-436, MDA-MB-468, MDA-MB-175VII and T-47D; colon cancer cell lines Caco-2, COLO 201, COLO 205, COLO 320DM, DLD-1, HAT-15, HCT-8, HT-29, LoVo, LS 180, LS123, LS174T, NCI-H548, NCI-SNU-C 1, SK-CO-1, SW 403, SW 48, SW 480, SW 620, SW 837, SW 948, HCT 116 and WiDr; non-small cell lung cancer cell lines A549, NCI-H23, NCI-H226, NCI-H358, NCI-H460, NCI-H522, NCI-H661, NCI-H810, NCI-H1155, NCI-H1299, NCI-H1395, NCI-H1435, NCI-H1581, NCI-H1651, NCI-H1703, NCI-H1793, NCI-H2073, NCI-H2085, NCI-H2106, NCI-H2228, NCI-H2342, NCI-H2347, SK-LU-1, NCI-H2122, SK-MES-1 and NCI-H292; small cell lung cancer cell lines NCI-H187, NCI-H378, NCI-H526, NCI-H889, NCI-H1417, NCI-H1672, NCI-H1836, NCI-H1963, NCI-H2227, NCI-N417 and SHP-77; ovary cancer cell lines ES-2, Caov-3, MDAH2774, NIH:OVCAR3, OV-90, SK-OV-3, TOV-112D and TOV-21G; prostate cancer cell lines DU 145 and LNCaP; human retinoblastoma cell lines WERI-Rb-1 and Y79, testicular cancer cell line Cates-1B (all purchased from ATCC); colon cancer cell line COCM1, non-small cell lung cancer cell line VMRC-LCD and prostate cancer cell line PC3 (all purchased from Japanese Collection of Research Bioresources (JCRB)) were cultured, respectively, in accordance with the culture protocol recommended by ATCC or JCRB The total RNA was prepared from the cultured cells using RNeasy Mini Total RNA Kit (QIAGEN). Using this total RNA as a template, reverse transcription was performed to prepare cDNA. Using this cDNA as a template, quantitative PCR was carried out to measure the expression level of mRNA for the nectin-2 gene.

The expression level of mRNA for the nectin-2 gene was quantified by the procedure described in REFERENCE EXAMPLE 2, using as a template the cDNA obtained from 5 ng of the total RNA described above. On the other hand, the expression level of a gene for β-actin contained in the same amount of the template cDNA was measured and used as the internal standard.

Relative expression levels obtained by standardization of the expression level of mRNA for the nectin-2α gene or the nectin-2δ gene with the expression level of mRNA for the β-actin gene are shown in [TABLE 1]. The results reveal that the expression level of mRNA for the nectin-2α gene was 1% or higher in 2 strains of all the cancer cell lines under investigation and the expression level of mRNA for the nectin-2δ gene was 1% or higher in 12 strains of the cancer cell lines, when compared to the expression level of β-actin mRNA.

TABLE 1

| Cell name | Nectin -2 α | Nectin-2 δ | Cell name | Nectin -2 α | Nectin -2 δ | Cell name | Nectin -2 α | Nectin-2 δ |
|---|---|---|---|---|---|---|---|---|
| Saos-2 | 0. 04 | 0. 10 | HCT-8 | 0. 36 | 1. 44 | NCI-H1155 | 0. 06 | 0. 06 |
| CCF-STTG1 | 0. 19 | 0. 39 | HT-29 | 0. 35 | 1. 93 | NCI-H1299 | 0. 57 | 0. 82 |
| SW 1088 | 0. 17 | 0. 11 | LoVo | 0. 16 | 0. 46 | NCI-H1581 | 0. 19 | 0. 61 |
| DBTRG-05MG | 0. 08 | 0. 18 | LS 180 | 0. 17 | 0. 36 | NCI-H2106 | 0. 05 | 0. 13 |
| U-118 MG | 0. 26 | 0. 08 | LS123 | 0.29 | 0. 90 | NCI-H187 | 0. 00 | 0.01 |
| U-87 MG | 0. 13 | 0. 11 | LS174T | 0. 08 | 0. 44 | NCI-H378 | 0. 06 | 0.11 |
| D341 Med | 0. 11 | 0. 09 | NCI-H548 | 1. 11 | 2. 07 | NCI-H526 | 0. 26 | 0. 41 |
| Daoy | 0. 13 | 0. 11 | NCI-SNU-C 1 | 0. 19 | 0. 30 | NCI-H889 | 0. 07 | 0. 19 |
| SK-N-AS | 0. 06 | 0. 04 | SK-C0-1 | 0. 57 | 1. 35 | NCI-H1417 | 0. 08 | 0. 20 |
| SK-N-BE | 0. 03 | 0. 04 | SW 403 | 0. 12 | 0. 49 | NCI-H1672 | 0. 07 | 0. 51 |
| SK-N-DZ | 0. 03 | 0. 03 | SW 48 | 0. 21 | 0. 22 | NCI-H1836 | 0. 12 | 0. 27 |
| SK-N-FI | 0. 12 | 0. 17 | SW 480 | 0. 14 | 0. 26 | NCI-H1963 | 0. 04 | 0. 05 |
| SK-N-SH | 0. 09 | 0. 19 | SW 620 | 0. 10 | 0. 38 | NCI-H2227 | 0. 12 | 0. 36 |
| SK-N-MC | 0. 10 | 0. 09 | SW 837 | 0.36 | 1. 27 | NCI-N417 | 0. 00 | 0. 00 |
| AU565 | 0. 05 | 0. 13 | SW 948 | 0. 56 | 1. 19 | SHP-77 | 0. 10 | 0. 33 |
| MCF-7 | 0. 08 | 0. 68 | WiDr | 0. 21 | 1. 92 | NCI-H226 | 0. 04 | 0. 26 |
| MDA-MB-231 | 0. 08 | 0. 11 | A549 | 0. 24 | 0. 25 | NCI-H1703 | 0. 30 | 0. 48 |
| SK-BR-3 | 0. 31 | 0. 65 | NCI-H23 | 0. 15 | 0. 24 | NCI-H2122 | 0. 02 | 0. 17 |
| BT474 | 0.19 | 0. 58 | NCI-H358 | 0. 12 | 0. 46 | SK-MES-1 | 0. 04 | 0. 12 |
| HCC1937 | 0. 15 | 0. 29 | NCI-H522 | 0. 20 | 0. 18 | NCI-H292 | 0. 00 | 1. 03 |
| MDA-MB-435s | 0. 08 | 0. 12 | NCI-H1395 | 0. 16 | 0. 39 | Caov-3 | 0. 08 | 0.30 |

(continued)

| Cell name | Nectin -2 α | Nectin-2 δ | Cell name | Nectin -2 α | Nectin -2 δ | Cell name | Nectin -2 α | Nectin-2 δ |
|---|---|---|---|---|---|---|---|---|
| ZR-75-1 | 0. 63 | 1. 57 | NCI-H1435 | 0. 40 | 0. 46 | MDAH2774 | 0. 12 | 0. 17 |
| MDA-MB-436 | 0. 08 | 0. 15 | NCI-H1651 | 0. 07 | 0. 21 | NIH:0VCR3 | 0. 17 | 0. 43 |
| MDA-MB-468 | 0. 04 | 0. 26 | NCI-H1793 | 0. 13 | 0. 25 | 0V-90 | 1. 09 | 5. 06 |
| MDA-MB-175 VII | 0. 03 | 0. 12 | NCI-H2073 | 0. 15 | 0. 34 | SK-0V-3 | 0. 32 | 0. 72 |
| T-47D | 0. 08 | 0. 40 | NCI-H2085 | 0. 20 | 0. 34 | T0V-112D | 0. 46 | 0. 45 |
| C0CM1 | 0. 22 | 0. 77 | NCI-H2228 | 0. 34 | 0. 44 | T0V-21G | 0. 24 | 0. 25 |
| Caco-2 | 0. 37 | 0. 99 | NCI-H2342 | 0. 64 | 2. 45 | ES-2 | 0. 20 | 0. 28 |
| C0L0 201 | 0. 16 | 0. 40 | NCI-H2347 | 0. 05 | 0. 12 | DU 145 | 0. 14 | 0. 60 |
| C0L0 205 | 0. 23 | 0. 63 | SK-LU-1 | 0. 04 | 0. 10 | LNCaP | 0. 29 | 0. 60 |
| C0L0 320DM | 0. 15 | 0.24 | VMRC-LCD | 0. 12 | 0. 10 | PC3 | 0. 14 | 0.24 |
| DLD-1 | 0. 35 | 1. 26 | NCI-H460 | 0. 12 | 0. 15 | Y79 | 0. 11 | 0. 19 |
| HCT 116 | 0. 40 | 0. 71 | NCI-H661 | 0. 13 | 0. 44 | WERI-Rb-1 | 0. 25 | 0. 54 |
| HCT-15 | 0. 43 | 0. 76 | NCI-H810 | 0. 09 | 0. 20 | Cates-1B | 0.16 | 0. 18 |

REFERENCE EXAMPLE 10

Preparation of anti-nectin-2 rabbit polyclonal antibody by immunization with nectin-2δ cDNA

Preparation of anti-nectin-2 rabbit polyclonal antibody by DNA immunization using gene gun was entrusted to Genovac (Nosan Corporation). A vector for animal cell expression wherein cDNA encoding the amino acid sequence of nectin-2δ (SEQ ID NO: 3) was incorporated was coated onto microparticles by the method described in patent literature (WO 00/29442) filed by Genovac, with which two rabbits were immunized using a gene gun. Blood for testing was collected from the ear vein. After increased antibody titer was confirmed in the serum, blood was collected via the carotid artery under anesthesia to obtain 127 mL and 115 mL, respectively.

These anti-sera were diluted in PBS to 2-fold and centrifuged. The supernatant was applied on an antigen column prepared by immobilizing the nectin-2ED-FLAG protein to HiTrap NHS-Activated HP (Amersham Biosciences). After washing with PBS, the column was eluted with 0.1 M glycine-HCl (pH 3) containing 0.15 M NaCl. The eluate was immediately neutralized with 1 M Tris-HCl (pH 8) and then dialyzed against PBS at 4°C overnight to purify and obtain anti-nectin-2 rabbit polyclonal antibodies. The anti-nectin-2 rabbit polyclonal antibodies obtained herein were named N2-R1 and N2-R2, respectively.

REFERENCE EXAMPLE 11

Expression level of nectin-2 protein in human cancer cell lines

The expression level of nectin-2 protein in human cancer cell lines was analyzed by flow cytometry. Human cancer cell lines NCI-H1703, HT-29, OV-90, SKBR-3, SK-OV-3, NCI-H2342, TOV-112D, NCI-H2122, NCI-H292, Capan-2, MDA-MB-231, BxPC-3, HCT-8, SK-N-DZ, Caov-3, DU 145, A549, Caco-2, WiDr, ZR-75-1, HAT-15, NCI-H1299, NCI-H2228 and BT474 (all purchased from ATCC) were cultured, respectively, according to the procedure recommended by ATCC. The cell suspension was prepared at 1 x $10^6$/mL using Stain buffer (BD Biosciences), respectively. The anti-nectin-2 rabbit polyclonal antibody (N2-R2) prepared in REFERENCE EXAMPLE 10 was added to the cell suspension at a final concentration of 3 μg/mL, which was reacted at 4°C for an hour. In a similar manner, non-immune rabbit IgG (Jackson ImmunoResearch Laboratories) was added in a final concentration of 3 μg/mL, which was used as a negative control. After this cell suspension was centrifuged, the cells were washed with Stain buffer and Alexa488-labeled anti-rabbit IgG antibody (Invitrogen) was added thereto at a final concentration of 10 μg/mL, which was reacted at 4°C for an hour. Again, the cells were washed with Stain buffer and provided for FACScan (Becton Dickinson) to measure the expression level of nectin-2 protein in the respective cells. A ratio of the median value of fluorescence intensities for the N2-R2-stained cells to the median value of fluorescence intensities of the negative control for each cell line is shown in [TABLE 2]. The results reveal that the nectin-2 protein was highly expressed in human cancer cell lines from plural kinds of cancer such as lung cancer, breast cancer, ovarian cancer, etc.

TABLE 2

| Cell name | Ratio | Cell name | Ratio |
|---|---|---|---|
| NCI-H1703 | 72.5 | HCT-8 | 48.8 |
| HT-29 | 65.5 | SK-N-DZ | 6.0 |
| 0V-90 | 133.4 | Caov-3 | 28.4 |
| SKBR-3 | 61.6 | DU 145 | 17.9 |
| SK-0V-3 | 65.0 | A549 | 16.9 |
| NCI-H2342 | 62.1 | Caco-2 | 50.5 |
| TOV-112D | 44.1 | WDr | 50.0 |
| NCI-H2122 | 28.1 | ZR-75-1 | 25.5 |
| NCI-H292 | 10.6 | HCT-15 | 25.9 |
| Capan-2 | 83.6 | NCI-H1299 | 36.9 |
| MDA-MB-231 | 17.0 | NCI-H2228 | 20.9 |
| BxPC-3 | 46.6 | BT474 | 42.0 |

REFERENCE EXAMPLE 12

Construction of animal cell expression vector for recombinant nectin-2 extracellular domain-FLAG protein

Using as a template the animal cell expression vector (pcDNA3.1(+)-Nectin-2δ) prepared in REFERENCE EXAMPLE 4, PCR was carried out by using primer 33 (SEQ ID NO: 29) tagged with the recognition site of restriction enzyme EcoRI and primer 34 (SEQ ID NO: 30) tagged with the recognition site of restriction enzyme XhoI. In this reaction, the reaction solution was composed of 10 ng of pcDNA3.1(+)-Nectin-2δ, 2.5 U of PfuUltra Hotstart DNA Polymerase (STRATAGENE), 0.2 μM each of primer 33 (SEQ ID NO: 29) and primer 34 (SEQ ID NO: 30), 200 μM dNTPs and 5 μL of 10 x Pfu Ultra Buffer (STRATAGENE), which was made the total 50 μL. PCR was carried out by reacting at 95°C for 2 minutes and then repeating 30 times the cycle set to include 95°C for 30 seconds, 60°C for 30 seconds and 72°C for 1 minute and 15 seconds, followed by reacting at 72°C for 10 minutes. Next, the PCR product was purified using PCR Purification Kit (QIAGEN). The purified product was then digested with restriction enzymes EcoRI and XhoI. Similarly, pCMV-Tag4 (STRATAGENE) was also digested with restriction enzymes EcoRI and XhoI. Each DNA fragment was purified using Wizard SV Gel and PCR Clean-Up System (Promega). The two fragments were ligated using Ligation High (TOYOBO). The plasmid obtained was transfected to Escherichia coli TOP10 (Invitrogen) and incubated for selection in kanamycin-containing LB agar medium. As a result of sequencing analysis of individual gene clones recovered from the grown colony of Escherichia coli, the animal cell expression vector pCMV-Tag4-Nectin-2ED-FLAG having the cDNA sequence (SEQ ID NO: 32) encoding the nectin-2ED-FLAG protein (SEQ ID NO: 31) with a FLAG tag at the C terminus of the extracellular domain (1st-361st amino acid sequence of nectin-2δ represented by SEQ ID NO: 3) of the nectin-2δ protein was obtained.

REFERENCE EXAMPLE 13

Preparation of recombinant nectin-2ED-FLAG protein

The nectin-2ED-FLAG protein encoded by the animal cell expression vector (pCMV-Tag4-Nectin-2ED-FLAG) prepared in REFERENCE EXAMPLE 12 was prepared using FreeStyle293 Expression System (Invitrogen). Specifically, the animal cell expression vector pCMV-Tag4-Nectin-2ED-FLAG was transfected to the 293F cell line using 293 Fectin (Invitrogen), followed by spinner culture at 37°C for 3 days in an 8% carbon dioxide gas flow. The cell suspension was centrifuged and the resulting culture supernatant was filtrated through a 0.45 μm filter. The filtrate was applied on an anti-FLAG antibody column (Sigma), which had been equilibrated with phosphate buffered saline (PBS). After washing the column with PBS, the nectin-2ED-FLAG protein was eluted with PBS containing 0.1 mg/mL of the FLAG peptide. After this eluted fraction was concentrated by ultrafiltration using Vivaspin (VIVA SCIENCE), the contaminated FLAG peptide was removed by using a gel filtration column PD-10 (Amersham Biosciences, which name was changed to GE Healthcare Biosciences) equilibrated with PBS and concentrated again to obtain the recombinant nectin-2ED-FLAG protein with high purity.

REFERENCE EXAMPLE 14

Preparation of anti-nectin-2 rabbit polyclonal antibody

Anti-nectin-2 rabbit polyclonal antibody was prepared using the recombinant nectin-2ED-FLAG protein prepared in REFERENCE EXAMPLE 13 as an immunogen. A PBS solution of the nectin-2ED-FLAG protein and Freund's complete adjuvant were mixed in equal volumes. Using the emulsion thus prepared, three domestic rabbits (Japanese white rabbit, female, 3 kg) were immunized with 0.1 mg/animal of the nectin-2ED-FLAG protein subcutaneously and intracutaneously at the back of the animal. For the second and subsequent immunization, the protein emulsion was likewise prepared using Freund's incomplete adjuvant and booster was repeated 7 times every 2 weeks.

Prior to the immunization and a week after the fourth and sixth booster, blood was collected for testing through the ear vein. An increase in the antibody titer in sera was confirmed by ELISA using an immunoplate coated with the nectin-2ED-FLAG protein. A week after the last booster, blood was collected from the three rabbits through the carotid artery under anesthesia to obtain the anti-sera of 78.9 ml, 78.2 ml and 78.8 ml, respectively.

These anti-sera were diluted in PBS to 2-fold and centrifuged. The supernatant was applied onto an antigen column prepared by immobilizing the nectin-2ED-FLAG protein to HiTrap NHS-Activated HP (Amersham Biosciences, which name was changed to GE Healthcare Bio-sciences). After washing with PBS, the column was eluted with 0.1 M glycine-HCl (pH 3) containing 0.15 M NaCl. The eluate was immediately neutralized with 1 M Tris-HCl (pH 8) and then dialyzed against PBS at 4°C overnight to obtain anti-nectin-2 rabbit polyclonal antibodies (N2-No. 1, N2-No. 2 and N2-No. 3).

REFERENCE EXAMPLE 15

Construction of animal cell expression vector for recombinant nectin-2 extracellular domain-Fc protein

(1) Cloning of human IgG1-Fc fragment gene

Using human spleen-derived Marathon-Ready cDNA (BD BIOSCIENCES) as a template, PCR was carried out using primer 33 (SEQ ID NO: 33) tagged with the recognition site of restriction enzyme EcoRI and primer 34 (SEQ ID NO: 34) tagged with the recognition site of restriction enzyme XhoI. In this reaction, the reaction solution was composed of 1 μL of the cDNA described above, 1 U of PfuTurbo Hotstart DNA Polymerase (STRATAGENE), 1 μM each of primer 33 (SEQ ID NO: 33) and primer 34 (SEQ ID NO: 34), 200 μM dNTPs and 10 μL of 2 x GC Buffer I (TaKaRa Bio) to make the total 20 μL. PCR was carried out by reacting at 95°C for 1 minutes and then repeating 30 times the cycle set to include 95°C for 20 seconds, 60°C for 15 seconds and 72°C for 2 minute. Next, the PCR product was purified with PCR Purification Kit (QIAGEN). The purified product was then digested with restriction enzymes EcoRI and XhoI. The pcDNA3.1(+) (Invitrogen) was also digested with restriction enzymes EcoRI and XhoI. These products were purified using PCR Purification Kit. The two DNA fragments were ligated using DNA Ligation Kit ver. 2 (TaKaRa Bio) and then transfected to Escherichia coli TOP10 (Invitrogen), followed by incubation for selection in ampicillin-containing LB agar medium. As a result of sequencing analysis of individual gene clones recovered from the grown colony of Escherichia coli, the animal cell expression vector pcDNA3.1(+)-hFc bearing a cDNA sequence encoding the Fc region of human IgG1 was obtained.

(2) Construction of nectin-2 extracellular domain-human Fc chimeric protein expression vector

Using the pcDNA3. 1(+)-Nectin-2δ prepared in REFERENCE EXAMPLE 4 as a template, PCR was carried out by using primer 35 (SEQ ID NO: 35) tagged with the recognition site of restriction enzyme HindIII and primer 36 (SEQ ID NO: 36) tagged with the recognition site of restriction enzyme EcoRI. In this reaction, the reaction solution was composed of 10 ng of pcDNA3.1 (+)-Nectin-2δ, 2.5 U of PfuTurbo Hotstart DNA Polymerase, 0.2 μM each of primer 35 (SEQ ID NO: 35) and primer 36 (SEQ ID NO: 36), 200 μM dNTPs and 10 μL of 2 x GC Buffer I (TaKaRa Bio) to make the total 20 μL. PCR was performed by reacting at 95°C for 1 minute and then repeating 35 times the cycle set to include 95°C for 20 seconds, 60°C for 15 seconds and 72°C for 2 minutes and 30 seconds. The PCR product was separated by agarose gel electrophoresis, purified using Gel Extraction Kit (QIAGEN) and digested with restriction enzymes HindIII and EcoRI. Similarly, pcDNA3.1 (+)-hFc was also digested with restriction enzymes HindIII and EcoRI. The two DNA fragments were ligated using DNA Ligation Kit ver. 2 and then transfected to Escherichia coli TOP10, followed by incubation for selection in ampicillin-containing LB agar medium. As a result of sequencing analysis of individual gene clones recovered from the grown colony of Escherichia coli, the animal cell expression vector (pcDNA3.1(+)-Nectin-2ED-hFc) bearing the cDNA sequence (SEQ ID NO: 38) encoding the fused protein (SEQ ID NO: 37) of the extracellular domain of nectin-2δ protein (1st-350th in the amino acid sequence of nectin-2δ represented by SEQ ID NO: 3) to the Fc region of human IgG1 was obtained.

REFERENCE EXAMPLE 16

Preparation of recombinant nectin-2ED-Fc protein

The nectin-2ED-hFc protein encoded by the animal cell expression vector (pcDNA3. 1(+)-Nectin-2ED-hFc) prepared in REFERENCE EXAMPLE 15 was prepared using FreeStyle293 Expression System (Invitrogen). Specifically, pcDNA3.1(+)-Nectin-2ED-hFc was transfected to the 293F cell line using 293 Fectin (Invitrogen), followed by spinner culture at 37°C for 3 days in an 8% carbon dioxide gas flow. The cell suspension was centrifuged and the resulting culture supernatant was filtrated through a 0.22 μm filter and the filtrate was applied onto a rProteinA Sepharose FF column (Amersham Biosciences, which name was changed to GE Healthcare Bio-sciences) equilibrated with PBS. After washing the column with PBS, elution was performed with 0.1 M glycine-HCl (pH 3.5) containing 0.15 M NaCl and the eluate was immediately neutralized with 1 M Tris-HCl (pH 8). After the eluted nectin-2ED-hFc fraction was dialyzed against PBS at 4°C overnight, the fraction was concentrated by ultrafiltration using Amicon Ultra15 30MWCO (MILLIPORE) to obtain the recombinant nectin-2ED-Fc protein.

REFERENCE EXAMPLE 17

Preparation of anti-nectin-2 rabbit polyclonal antibody using peptide antigen

Based on the amino acid sequences of nectin-2α protein (SEQ ID NO: 1) and nectin-2δ protein (SEQ ID NO: 3), the following 3 peptides (peptides 1 - 3) consisting of 15 amino acids were synthesized.

Amino acid sequence of peptide 1

[Cys-Lys-Met-Gly-Pro-Ser-Phe-Pro-Ser-Pro-Lys-Pro-Gly-Ser-Glu (SEQ ID NO: 39)] is a sequence wherein Cys residue is added to the 88th-101st amino acid sequence of nectin-2α protein (SEQ ID NO: 1) and nectin-2δ protein (SEQ ID NO: 3) at its N terminus.

Amino acid sequence of peptide 2

[Arg-Glu-Thr-Pro-Arg-Ala-Ser-Pro-Arg-Asp-Val-Gly-Pro-Leu-Cys (SEQ ID NO: 40)] is a sequence wherein Cys residue is added to the 347th-360th amino acid sequence of nectin-2α protein (SEQ ID NO: 1) at its C terminus.

Amino acid sequence of peptide 3

[Cys-Thr-Leu-Gly-Ala-Ser-Glu-His-Ser-Pro-Leu-Lys-Thr-Pro-Tyr (SEQ ID NO: 41)] is a sequence wherein Cys residue is added to the 426th-439th amino acid sequence of nectin-2δ protein (SEQ ID NO: 3) at its N terminus.

Each of peptide 1, peptide 2 and peptide 3 described above was chemically bound to maleimide keyhole limpet hemocyanin (KLH) (Pierce) as a carrier protein, which was used as an immunogen. Male rabbit KBL: JW (11 weeks old, Kitayama Labes) was used as an animal for immunization. An emulsion composed of the immunogen above and Freund's complete adjuvant (Difco) was used for primary immunization and an emulsion composed of the immunogen above and Freund's incomplete adjuvant (Difco) was used for the second and subsequent immunization. These emulsions were injected subcutaneously at the back in 0.5 mg each as the protein 4 times in total every 2 weeks. On day 52 after the primary immunization, blood was collected through the carotid artery under anesthesia and the serum of 70 ml, 66 ml or 72 ml was obtained from the rabbit immunized with peptide 1, peptide 2 or peptide 3, respectively. The immunoglobulin fraction was concentrated from the sera thus obtained by ammonium sulfate salting-out, and purified by a protein A affinity column (Amersham Biosciences, which name was changed to GE Healthcare Bio-sciences) to give the IgG antibody fraction. The IgG antibody thus obtained was applied onto the anitigen-immobilized column, in which each peptide was coupled to a Activated Thiol Sepharose 4B column (Amersham Biosciences, which name was changed to GE Healthcare) via the Cys residue. After the column was washed with PBS, the peptide-specific antibody was eluted using 8M urea-containing PBS. The eluates were dialyzed against PBS to remove urea, which was followed by ultraconcentration and sterilization by filtering. Thus, the purified anti-nectin-2 rabbit polyclonal antibodies (AS-2704, AS-2705 and AS-2706) against peptides 1, 2 and 3 were obtained.

REFERENCE EXAMPLE 18

Establishment of NS0 cell line stably expressing the recombinant full-length nectin-2δ

The NS0 cell line stably expressing the nectin-2δ protein (SEQ ID NO: 3) was established. To obtain the animal cell expression vector pEE12.4-Nectin-2δ, pcDNA3.1(+)-Nectin-2δ prepared in REFERENCE EXAMPLE 4 was digested with restriction enzymes EcoRI and EcoRV Likewise, the animal cell expression vector pEE12.4 (Lonza Biologics) was digested with restriction enzymes EcoRI and SmaI. These products were subjected to agarose gel electrophoresis and the desired fragments were excised out to purify using MinElute Gel Extraction Kit (QIAGEN). The two DNA fragments were ligated using Ligation High (TOYOBO), which was then transfected to Competent High DH5α (TOYOBO) and incubated for selection in ampicillin-containing LB agar medium. The grown colony of Escherichia coli was incubated in ampicillin-containing LB medium, and the cells was collected by centrifugation. The plasmid was prepared from the thus recovered cells using QIAfilter Plasmid Maxi Kit (QIAGEN). After the plasmid was digested with restriction enzyme EcoRI, the insertion of the nectin-2δ gene was confirmed by an agarose gel electrophoresis. Thus, the animal cell expression vector pEE12.4-Nectin-2δ having the cDNA sequence (SEQ ID NO: 4) encoding the nectin-2δ protein (SEQ ID NO: 3) was obtained. Using Gene Pulser (Bio-Rad), 40 μg of the pEE12.4-Nectin-2δ linearized by restriction enzyme (PvuI) digestion was transfected (250V, 400 μF) to NS0 cells (2 x $10^7$). The cells were resuspended in DMEM medium (JRH) supplemented with 10% dialyzed FBS (Invitrogen) and 2 mM L-glutamine, and plated on 96-well flat bottom tissue culture plates at 8,000 cells/50 μL per well on 16 plates, at 2,000 cells/50 μL per well on 20 plates and at 400 counts/ 50 μL per well on 40 plates. After incubation at 37°C for 24 hours in an 8% carbon dioxide gas flow, 150 μL each of GS-selection DMEM medium (JRH) supplemented with 10% dialyzed FBS and GS supplement (JRH) was added to each well. The incubation was continued at 37°C for 3 to 4 weeks in an 8% carbon dioxide gas flow. The grown colony in the abovementioned selection medium was again plated on a 24-well flat bottom tissue culture plate. The total RNA was extracted from the grown cells using RNeasy 96 Kit (QIAGEN). Quantitative PCR was then performed using TaqMan One Step PCR Master Mix Reagents Kit (Applied Biosystems) to select the cell line highly expressing nectin-2δ mRNA. The total RNA, 100 ng, was used as a template, and the reaction solution for quantitative PCR was made up to 50 μL by adding 25 μL of 2X Master Mix (Applied Biosystems), 1.25 μL of 40X MultiScribe (Applied Biosystems), 50 nM each of primer 42 (SEQ ID NO: 42) and primer 43 (SEQ ID NO: 43) and 50 nM of FAM-labeled TaqMan probe 3 (SEQ ID NO: 44). PCR was performed by reacting at 48°C for 30 minutes and 95°C for 10 minutes and then repeating 40 times the cycle set to include 95°C for 15 seconds and 60°C for 1 minute. As a result, 12 strains of NS0 cells highly expressiing the nectin-2δ mRNA were selected and obtained.

Expression levels of the nectin-2δ protein in these 12 strains were compared by flow cytometry using anti-nectin-2 peptide rabbit polyclonal antibody (AS-2704) prepared in REFARENCE EXAMPLE 17 and among them, the NS0 cell line (#2-75) highly expressing the nectin-2δ protein was selected and obtained.

REFERENCE EXAMPLE 19

Establishment of the FM3A cell line stably expressing the recombinant full-length nectin-2δ

The FM3A cell line stably expressing the nectin-2δ protein (SEQ ID NO: 3) was established. To obtain the animal cell expression vector pEF1-Nectin-2δ, pcDNA3.1(+)-Nectin-2δ prepared in REFERENCE EXAMPLE 4 was digested with restriction enzymes EcoRI and EcoRV Likewise, the animal cell expression vector pEF1/myc-His A (Invitrogen) was digested with restriction enzymes EcoRI and EcoRV These products were purified by PCR Purification Kit (QIAGEN) and the two DNA fragments were ligated using DNA Ligation Kit ver. 2 (TaKaRa Bio), which was then transfected to Competent High JM109 (TOYOBO) and incubated for selection in ampicillin-containing LB agar medium. The grown colony of Escherichia coli was incubated in ampicillin-containing LB medium, and the cells was collected by centrifugation. The plasmid was prepared from the thus recovered cells using QIAprep Turbo Miniprep Kit (QIAGEN). After the plasmid was digested with restriction enzymes EcoRI and EcoRV, the insertion of the nectin-2δ gene was confirmed by agarose gel electrophoresis, and the animal cell expression vector pEF1-Nectin-2δ having the cDNA sequence (SEQ ID NO: 4) encoding the nectin-2δ protein (SEQ ID NO: 3) was obtained.

Using Gene Pulser (Bio-Rad), 40 μg of the pEF1-Nectin-2δ was transfected (350V, 950 μF) to FM3A cells (1 x $10^7$). The cells were resuspended in RPMI 1640 medium (Invitrogen) supplemented with 10% FBS (Invitrogen), and incubated at 37°C for 18 hours in a 5% carbon dioxide gas flow. The gene-transfected cells were resuspended in RPMI 1640 medium supplemented with 10% FBS and 1 mg/mL Geneticine (Invitrogen), and plated on 96-well flat bottom tissue culture plates at 1,000 cells/200 μL/well and 100 cells/200 μL/well on 20 plates each. The culture was continued at 37°C for 1 to 2 weeks in a 5% carbon dioxide gas flow. The grown colony in the abovementioned selection medium was again plated on a 24-well flat bottom tissue culture plate. The total RNA was extracted from the grown cells using RNeasy 96 Kit (QIAGEN). Quantitative PCR was then performed using TaqMan One Step PCR Master Mix Reagents Kit (Applied Biosystems) to select the cell line highly expressing nectin-2δ mRNA. The total RNA, 100 ng, was used as a template, and the reaction solution for quantitative PCR was made up to 50 μL by adding 25 μL of 2X Master Mix (Applied Biosystems), 1.25 μL of 40X MultiScribe (Applied Biosystems), 50 nM each of primer 1 (SEQ ID NO: 9) and primer 2 (SEQ ID NO: 10) and 50 nM of FAM-labeled TaqMan probe 1 (SEQ ID NO: 11). PCR was performed by reacting at 48°C for 30 minutes and 95°C for 10 minutes and then repeating 40 times the cycle set to include 95°C for 15 seconds and 60°C for 1 minute. As a result, 7 strains of FM3A cells highly expressing the nectin-2δ mRNA were obtained.

Expression levels of the nectin-2δ protein in these 7 strains were compared by flow cytometry using anti-nectin-2 peptide rabbit polyclonal antibody (AS-2704) prepared in REFARENCE EXAMPLE 17 and among them, the FM3A cell lines (#58, #60) that highly expressed the nectin-2δ protein were selected and obtained. Moreover, these cell lines were resuspended in the selection medium described above and plated on a 96-well flat bottom tissue culture plate at 3 counts/200 μL, 1 cell/200 μL/well and 0.3 cell/200 μL/well, respectively. The culture was continued at 37°C in a 5% carbon dioxide gas flow. A portion of the cell line grown as a single colony was provided for the flow cytometry described above, and the clone (#60-6) showing a high expression level of the nectin-2δ protein was selected and obtained.

REFERENCE EXAMPLE 20

Establishment of the CHO-K1 cell line stably expressing the recombinant full-length nectin-2δ

The CHO-K1 cell line stably expressing the nectin-2δ protein (SEQ ID NO: 3) was established. The nectin-2δ animal cell expression vector (pEE12.4-Nectin-2δ) constructed in REFERENCE EXAMPLE 18 was linearized by digestion with restriction enzyme PvuI, and 40 μg of the linearized vector was transfected to CHO-K1 cells (1 x $10^7$) using Gene Pulser. The cells were resuspended in DMEM medium (JRH) supplemented with 10% dialyzed FBS and GS supplement (JRH) and plated on 96-well flat bottom tissue culture plates at 2,500 cells/50 μL/well on 40 plates. After incubation at 37°C for 24 hours in a 5% carbon dioxide gas flow, 150 μL each of the abovementioned medium containing 33.3 μM or 66.6 μM MSX (ICN) was added to 20 plates, respectively. The selection culture was continued at 37°C for 3 to 4 weeks in a 5% carbon dioxide gas flow. The colony grown in the selection medium described above was again plated on a 24-well flat bottom tissue culture plate. The total RNA was obtained from the cells using RNeasy 96 Kit (QIAGEN),, and reverse transcription was performed using the RNA as a template. A quantitative PCR was further conducted using this reaction product as a template, and the top 60 clones highly expressing nectin-2δ mRNA were selected and obtained.

Expression levels of the nectin-2δ protein of these 60 clones were compared by flow cytometry using anti-nectin-2 peptide rabbit polyclonal antibody (AS-2704) prepared in REFARENCE EXAMPLE 17 to select and obtain the CHO-K1 cell line (43-2) highly expressing the nectin-2δ protein.

REFERENCE EXAMPLE 21

Construction of animal cell expression vector for recombinant nectin-3 extracellular domain-Fc protein

(1) Cloning of mouse IgG2a-Fc fragment

Using mouse spleen-derived Marathon-Ready cDNA (BD Biosciences) as a template, PCR was carried out by using primer 45 (SEQ ID NO: 45) tagged with the recognition site of restriction enzyme EcoRI and primer 46 (SEQ ID NO: 46) tagged with the recognition site of restriction enzyme XhoI. In this reaction, the reaction solution was composed of 1 μL of the above cDNA, 1 U of PfuTurbo Hotstart DNA Polymerase (STRATAGENE), 1 μM each of primer 45 (SEQ ID NO: 45) and primer 46 (SEQ ID NO: 46), 200 μM dNTPs and 10 μL of 2 x GC Buffer I (TaKaRa Bio) to make the

total 20 μL. PCR was carried out by reacting at 95°C for 1 minute and then repeating 30 times the cycle set to include 95°C for 20 seconds, 60°C for 15 seconds and 72°C for 2 minutes. Next, the PCR product was purified with PCR Purification Kit (QIAGEN). The product was then digested with restriction enzymes EcoRI and XhoI. Similarly, pcDNA3.1 (+) (Invitrogen) was also digested with restriction enzymes EcoRI and XhoI. The two DNA fragments were ligated using DNA Ligation Kit ver. 2 (TaKaRa Bio) and then transfected to Escherichia coli TOP10 (Invitrogen), followed by incubation for selection in ampicillin-containing LB agar medium. As a result of sequencing analysis of individual gene clones recovered from the grown colony of Escherichia coli, the animal cell expression vector pcDNA3.1(+)-mFc bearing a cDNA sequence encoding the Fc region of mouse IgG2a was obtained.

(2) Construction of nectin-3 extracellular domain-human Fc chimeric protein expression vector

Using human lung cancer cell line A549-derived Marathon-Ready cDNA (BD Biosciences) as a template, PCR was carried out by using primer 47 (SEQ ID NO: 47) tagged with the recognition site of restriction enzyme HindIII and primer 48 (SEQ ID NO: 48) tagged with the recognition site of restriction enzyme EcoRI. In this reaction, the reaction solution was composed of 1 μL of the above cDNA, 2.5 U of PfuTurbo Hotstart DNA Polymerase, 1 μM each of primer 47 (SEQ ID NO: 47) and primer 48 (SEQ ID NO: 48), 200 μM dNTPs and 25 μL of 2 x GC Buffer I (TaKaRa Bio) to make the total 50 μL. PCR was carried out by reacting at 95°C for 1 minute and then repeating 35 times the cycle set to include 95°C for 20 seconds, 60°C for 15 seconds and 72°C for 2 minutes. Next, the PCR product was purified using PCR Purification Kit. The purified product was then digested with restriction enzymes HindIII and EcoRI. Similarly, pcDNA3.1(+)-hFc prepared in REFARENCE EXAMPLE 15 was digested with restriction enzymes HindIII and EcoRI. The reaction product was separated by agarose gel electrophoresis and purified using Gel Extraction Kit (QIAGEN). The two DNA fragments were ligated using DNA Ligation Kit ver. 2 and then transfected to Escherichia coli TOP 10, followed by incubation for selection in ampicillin-containing LB agar medium. As a result of sequencing analysis of individual gene clones recovered from the grown colony of Escherichia coli, the animal cell expression vector pcDNA3.1 (+)-Nectin-3ED-hFc bearing the cDNA sequence (SEQ ID NO: 50) encoding the fusion protein (SEQ ID NO: 49) of the extracellular domain (1st to 404th in the amino acid sequence of nectin-3 represented by SEQ ID NO: 5) of nectin-3 protein to the Fc region of human IgG1 was obtained.

(3) Construction of nectin-3 extracellular domain-mouse Fc chimeric protein expression vector

The animal cell expression vector pcDNA3.1(+)-Nectin-3ED-hFc obtained in (2) was digested with restriction enzymes HindIII and EcoRI, the products were separated by agarose gel electrophoresis, and the DNA fragment encoding the extracellular domain of the nectin-3 protein was excised out and purified with Gel Extraction Kit. Similarly, pcDNA3.1 (+)-mFc obtained in (1) was digested with restriction enzymes HindIII and EcoRI, the reaction products were separated by agarose gel electrophoresis, and the DNA fragment was excised out and purified with Gel Extraction Kit. The two DNA fragments were ligated using DNA Ligation Kit ver. 2, which was then transfected to Escherichia coli TOP 10 and incubated for selection in ampicillin-containing LB agar medium. As a result of sequencing analysis of individual gene clones recovered from the grown colony of Escherichia coli, the animal cell expression vector pcDNA3.1(+)-Nectin-3ED-mFc bearing the cDNA sequence (SEQ ID NO: 52) encoding the fused protein (SEQ ID NO: 51) of the extracellular domain of nectin-3 protein (1st-404th in the amino acid sequence of nectin-3 represented by SEQ ID NO: 5) to the mouse Fc region was obtained.

REFERENCE EXAMPLE 22

Preparation of recombinant nectin-3ED-mFc protein

The nectin-3ED-mFc protein encoded by pcDNA3.1(+)-Nectin-3ED-mFc prepared in REFERENCE EXAMPLE 21 was prepared using FreeStyle293 Expression System (Invitrogen). Specifically, pcDNA3.1(+)-Nectin-3ED-mFc was transfected to the 293F cell line using 293 Fectin (Invitrogen), followed by spinner culture at 37°C for 3 days in an 8% carbon dioxide gas flow. The cell suspension was centrifuged and the resulting culture supernatant was filtrated through a 0.22 μm filter and the filtrate was applied onto an rProteinA Sepharose FF column (Amersham Biosciences, which name was changed to GE Healthcare Biosciences) equilibrated with PBS. After washing the column with PBS, elution was performed with 0.1 M glycine-HCl (pH 3.5) containing 0.15 M NaCl. The eluate was immediately neutralized with 1 M Tris-HCl (pH 8). After the eluted nectin-3ED-Fc fraction was dialyzed against PBS at 4°C overnight, the fraction was concentrated by ultrafiltration using Amicon Ultra15 30MACO (MILLIPORE) to obtain the recombinant nectin-3ED-mFc protein.

REFERENCE EXAMPLE 23

Purification of anti-nectin-2 rabbit polyclonal antibody

To reduce non-specific reactions in immunohistochemical staining (IHC), the anti-nectin-2 rabbit polyclonal antibody N2-No. 2 prepared in REFERENCE EXAMPLE 14 was applied onto the nectin-3ED-FLAG column prepared by immobilizing the nectin-3ED-FLAG protein prepared in REFERENCE EXAMPLE 26 onto HiTrap NHS-Activated HP (GE Healthcare) to remove the antibody fraction bound to the FLAG tag. The nectin-2-specific rabbit polyclonal antibody fraction which passed through the column was recovered as N2-No. 2-2, which was used for the IHC test.

REFERENCE EXAMPLE 24

Enhanced expression of nectin-2 protein in human cancer tissues

Expression of the nectin-2 protein in human cancer tissues was examined by IHC. Specifically, human breast cancer tissue array (Cybrdi), human ovarian cancer tissue array (Cybrdi) and human normal tissue array (Biomax) were subjected to a paraffin removal treatment, immersed in an antigen retrieval solution (DAKO) and then treated at 121°C for 15 minutes in an autoclave. Next, these tissue arrays were washed with water, treated with 3% hydrogen peroxide at room temperature for 7.5 minutes and washed with PBS, followed by blocking with goat serum (Vectastain) at room temperature for 20 minutes. After the goat serum was removed, the arrays were reacted with an antibody-dilution buffer (DAKO) containing 1 $\mu$g/mL of N2-No. 2-2 purified in REFERENCE EXAMPLE 23 at 4°C overnight. The arrays were washed with PBS and then reacted with ENVISION+ (DAKO) at room temperature for 30 minutes. After washing again with PBS, a DAB substrate (Merck) solution containing 0.01% hydrogen peroxide was added to the tissue arrays and reacted at room temperature for 3 minutes. Thereafter, the arrays were washed with water and immersed in hematoxylin for a minute, followed by dehydration-treatment. Microscopic observation of the tissues spotted on each array revealed that expression of the nectin-2 protein was significantly enhanced in the cancer tissues at the rates shown in TABLES 2A and 2B.

TABLE 2A

| Breast tissues | | |
|---|---|---|
| Classification | Positive Rate | |
| Infiltrating ductal carcinoma | 89% | (16/18) |
| Ductal carcinoma | 100% | (7/7) |
| Infiltrating lobular carcinoma | 100% | (9/9) |
| Medullary carcinoma | 50% | (3/6) |
| Mucinous adenocarcinoma | 71% | (5/7) |
| Paget's disease | 100% | (7/7) |
| Normal breast | 0% | (0/5) |

TABLE 2B

| Ovarian Tissues | | | |
|---|---|---|---|
| Classification | | Positive rate | |
| Epithelial | Serous carcinoma | 55% | (22/40) |
| | Granular carcinoma | 100% | (3/3) |
| | Clear cell carcinoma | 50% | (1/2) |
| | Endometrioid carcinoma | 0% | (0/1) |
| | Mucinous carcinoma | 25% | (1/4) |
| | Brenner tumor | 100% | (1/1) |
| Germinoma | | 0% | (0/4) |

(continued)

| Ovarian Tissues | | |
|---|---|---|
| Classification | Positive rate | |
| Theca cell carcinoma | 100% | (1/1) |
| Metastatic carcinoma | 17% | (1/6) |
| Normal ovary | 0% | (0/3) |

REFERENCE EXAMPLE 25

Construction of animal cell expression vector for recombinant nectin-3 extracellular domain-FLAG protein

In preparing the animal cell expression vector of recombinant nectin-3 extracellular domain-FLAG protein, a vector to which the FLAG tag sequence could be added was first constructed. Specifically, two synthetic DNAs, FLAG-FSALNOT (SEQ ID NO: 53) or FLAG-RSALNOT (SEQ ID NO: 54), 5' end of which was phosphorylated was diluted in TE (1 mM EDTA-containing Tris-HCl (pH 8)) to be 50 μM, respectively, mixed in equal volumes, heated at 95°C for 10 minutes and then gradually cooled for annealing. Next, an animal cell expression vector pCI-neo (Promega) was digested with restriction enzymes NheI and NotI, and the reaction products were subjected to agarose gel electrophoresis and the vector fragment was extracted with Gel Extraction Kit (QIAGEN). The annealed synthetic DNA described above was mixed with this restriction enzyme-treated pCI-neo and the mixture was subjected to ligation using Ligation High (TOYOBO) to construct the animal cell expression vector pCI-FLAG which contained FLAG tag sequence.

Next, using the pcDNA3.1(+)-Nectin-3ED-hFc prepared in REFERENCE EXAMPLE 21 as a template, PCR was carried out by using primer 55 (SEQ ID NO: 55) tagged with the recognition site of restriction enzyme SalI and primer 56 (SEQ ID NO: 56) tagged with the recognition site of restriction enzyme NheI. In this reaction, the reaction solution was composed of the pcDNA3.1 (+)-Nectin-3ED-hFc used as a template, 2.5 U ofPyrobest DNA Polymerase (Takara Bio), 0.5 μM each of primer 55 (SEQ ID NO: 55) and primer 56 (SEQ ID NO: 56), 200 μM dNTPs and 5μ L of 10 x GC Buffer I (TaKaRa Bio) to make the total 50 μL. PCR was performed by reacting at 94°C for 2 minutes and then repeating 30 times the cycle set to include 94°C for 30 seconds, 60°C for 30 seconds and 68°C for 1 minute and 30 seconds, followed by reacting at 68°C for 2 minutes. Next, the PCR product was purified using Gel Extraction Kit (QIAGEN) and then digested with restriction enzymes SalI and NheI. Similarly, the pCI-FLAG described above was digested with restriction enzymes SalI and NheI. After the two DNA fragments were purified using PCR Purification Kit, the two DNA fragments were ligated using Ligation High (TOYOBO). The product obtained was transfected to Escherichia coli TOP 10 (Invitrogen), followed by incubation for selection in ampicillin-containing LB agar medium. As a result of sequencing analysis of the individual gene clones recovered from the grown colony of Escherichia coli, the animal cell expression vector pCI-Nectin-3ED-FLAG bearing the cDNA sequence (SEQ ID NO: 58) encoding the nectin-3ED-FLAG protein (SEQ ID NO: 57), which was the N terminal 404 amino acid sequence of the nectin-3 protein (SEQ ID NO: 5) FLAG-tagged at the C terminus was obtained.

REFERENCE EXAMPLE 26

Preparation of recombinant nectin-3ED-FLAG protein

The nectin-3ED-FLAG protein encoded by the animal cell expression vector (pCI-Nectin-3ED-FLAG) constructed in REFERENCE EXAMPLE 25 was prepared using FreeStyle293 Expression System (Invitrogen). Specifically, the animal cell expression vector pCI-Nectin-3ED-FLAG was transfected to the 293F cell line using 293 Fectin (Invitrogen), followed by spinner culture at 37°C for 3 days in an 8% carbon dioxide gas flow. The cell suspension was centrifuged and the resulting culture supernatant was filtrated through a 0.45 μm filter and the filtrate was applied onto an anti-FLAG antibody column (Sigma) equilibrated with 0.1 M Tris-HCl (pH 7.5) containing 0.3 M NaCl. After washing the column with 0.1 M Tris-HCl (pH 7.5) containing 0.3 M NaCl, the nectin-3ED-FLAG protein was eluted with the same buffer containing 0.1 mg/mL of the FLAG peptide. After the eluted fraction was concentrated by ultrafiltration using Amicon Ultra 15 (30K MWCO) (Millipore), the fraction was applied onto a PD-10 Desalting column (GE Healthcare Biosciences) equilibrated with PBS to remove the contaminated FLAG peptide, and concentrated again to obtain the recombinant nectin-3ED-FLAG protein of high purity.

REFERENCE EXAMPLE 27

Preparation of anti-nectin-3 rabbit polyclonal antibody

Anti-nectin-3 rabbit polyclonal antibody was prepared using the recombinant nectin-3ED-FLAG protein prepared in REFERENCE EXAMPLE 26 as an immunogen. A PBS solution of the nectin-3ED-FLAG protein was mixed with Freund's complete adjuvant (Difco) in equal volumes. Using the emulsion thus prepared, two domestic rabbits (Japanese white rabbit, female, 3 kg) were immunized subcutaneously and intracutaneously with the nectin-3ED-FLAG protein at 0.1 mg each/animal at the back of the animal. For the second and subsequent immunization, the protein emulsion was likewise prepared using Freund's incomplete adjuvant (Difco) and booster was repeated 7 times every 2 weeks.
Prior to the immunization and a week after the fourth and sixth booster, blood was collected for testing through the ear vein. An increase in the antibody titer in sera was confirmed by ELISA using an immunoplate coated with the nectin-3ED-FLAG protein. A week after the last booster, blood was collected from the two rabbits through the carotid artery under anesthesia to give the anti-sera of 78.9 ml and 78.8 ml, respectively.
These anti-sera were diluted in PBS to 2-fold and centrifuged. The supernatant was applied onto an antigen column prepared by immobilizing the nectin-3ED-FLAG protein to HiTrap NHS-Activated HP (GE Healthcare Bio-sciences). After washing with PBS, the column was eluted with 0.1 M glycine-HCl (pH 3) containing 0.15 M NaCl. The eluate was immediately neutralized with 1 M Tris-HCl (pH 8) and then dialyzed against PBS at 4°C overnight to obtain anti-nectin-3 rabbit polyclonal antibodies (N3-No. 1 and N3-No. 3).

REFERENCE EXAMPLE 28

Large scale production of anti-nectin-2 human monoclonal antibody

Eleven anti-nectin-2 human monoclonal antibodies used for the *in vivo* assay of anti-tumor activity were prepared from the antibody-producing hybridomas (Nec1-803-2, Nec1-964-1, Nec1-303-2, Nec1-554-1, Nec1-1302-2, Nec1-769-2, Nec1-1305-1, Nec1-141-3, Nec1-209-2, Nec1-909-1 and Nec1-847-2). Atypical example of the method for production is described below. After expanding the culture of the hybridoma cell line described above at 37°C in a 5% carbon dioxide gas flow in IH medium (Iscove's Modified Dulbecco's Medium : Ham's F-12 = 1:1, 0.1 mM MEM non-essential amino acid solution, 1 mM sodium pyruvate solution, 2 mM L-glutamine solution; Invitrogen) containing 10% FBS, Ultra-Low IgG (Invitrogen), the cells were further expanded with a primary adaption medium (IH medium: CD Hybridoma Medium, 8 mM L-glutamine solution = 1:1, Invitrogen), followed by incubation for one day. These were further expanded with the medium for main culture (IH medium: CD Hybridoma Medium, 8 mM L-glutamine solution = 1:3, Invitrogen), followed by the incubation for 5 to 7 days in a spinner flask at 37°C in a 5% carbon dioxide gas flow, or in an agitation culture tank of 50 L volume, under the conditions of 2 ppm dissolved oxygen concentration, pH 7.0 and 40 rpm rotation number of the agitation. In the latter case, a glucose solution and an L-glutamine solution was added during the incubation based on analysis of the medium components. The main culture was terminated at a cell viability of about 50%.. After termination of the culture, the culture supernatant was harvested by centrifugation (7,460 x g, 20 minutes)..
Each hybridoma supernatant thus obtained was concentrated and applied onto an ultrafiltration membrane (Hydrosart membrane, molecular weight cut-off, 10,000; Sartorius AG) for buffer exchange to 20 mM phosphate buffer (pH 7.0) containing 0.15 M NaCl , followed by a centrifugation (14,300 x g, 20 minutes) to obtain the supernatant. The supernatant was further microfiltrated (Stericup HV, 0.45 μm; Millipore) to obtain the concentrate, and it was adsorbed to a Protein A Sepharose column (22 mm ID x 79 mm, GE Healthcare Biosciences) equilibrated with 20 mM phosphate buffer (pH 7.0) containing 0.15 M NaCl. After washing the column with 20 column volume of the same buffer, the antibody fraction adsorbed to the column was eluted out with 20 column volume of 0.1 M sodium citrate buffer (pH 3.0). Immediately thereafter, the fraction was neutralized by adding 1/10 volume of 1 M Tris-HCl buffer (pH 9.0). After concentrating the antibody solution using an ultrafiltration membrane (AmiconUltra, molecular weight cut-off, 30,000; Millipore), the concentrate was applied onto the Superdex 200 column (26 mmID x 60 cm, GE Healthcare Biosciences) equilibrated with PBS and eluted with the same buffer to give the antibody monomer fraction. It was passed through an ActiClean ETox column (25 mmID x 59 mm, Sterogene Bioseparations) to remove endotoxin, followed by a concentration using an ultrafiltration membrane (AmiconUltra, molecular weight cut-off, 10,000; Millipore). The concentrate was further filtrated aseptically (Millex GV, 0.22 μm; Millipore) to give the purified antibody preparation.
Since the antibody fraction purified from the culture supernatant of hybridoma Nec1-554-1 with Protein A column was found to be exceptionally a mixture of the active and inactive antibodies, the antibody fraction was further separated on a cation exchange column to collect the active fraction. Specifically, the Protein A-purified antibody fraction described above was diluted 3-time with 20 mM sodium acetate buffer (pH 5.0) and adsorbed to an SP-5PW column (21.5 mmID x 150 mm, Toso) equilibrated with 20 mM sodium acetate buffer (pH 5.0) containing 100 mM NaCl. After washing the column with about 2 column volume of 20 mM sodium acetate buffer (pH 5.0) containing 100 mM NaCl, the active antibody fraction and the inactive antibody fraction was separately eluted with a linear gradient of the NaCl concentration from 100 mM to 300 mM over 70 minutes, and the active antibody fraction (SP3) was collected.. Thus obtained antibody eluate was concentrated using an ultrafiltration membrane (AmiconUltra, molecular weight cut-off, 30,000; Millipore), and was applied onto a Superdex 200 column (26 mmID x 60 cm, GE Healthcare Biosciences) equilibrated with PBS and eluted with the same buffer to give the antibody monomer fraction. It was passed through the ActiClean ETox column (25 mmID x 59 mm, Sterogene Bioseparations) equilibrated with PBS to remove endotoxin, followed by a concentration using an ultrafiltration membrane (AmiconUltra, molecular weight cut-off, 10,000; Millipore), and the concentrate was further filtrated aseptically (Millex GV, 0.22 μm; Millipore) to give the purified antibody. The purified antibody was named Nec1-554-1 SP3.

All of the purified antibodies thus obtained demonstrated purity of 95% or higher on SDS-PAGE and gel filtration HPLC using Superdex 200 column. The endotoxin content in the antibody preparation was found to be 0.1 EU/mg antibody or less by the analyses using both Endospecy ES-24S Set (Seikagaku Corp.) and Toxicolor DIA Set (Seikagaku Corp.).

REFERENCE EXAMPLE 29

Construction of animal cell expression vectors for nectin-1, nectin-3, nectin-4 and Nec1-5

Human nectin-1 gene was obtained by performing PCR using Marathon-Ready cDNA (Takara Bio) of human brain as a template, primer 59 (SEQ ID NO: 59) tagged with the recognition site of restriction enzyme EcoRV, and primer 60 (SEQ ID NO: 60) tagged with the recognition site of restriction enzyme XhoI. In this reaction, the reaction solution was composed of 1 μL of the cDNA solution described above used as a template, 1 U of PfuTurbo Hotstart DNA Polymerase (STRATAGENE), 1 μM each of primer 59 (SEQ ID NO: 59) and primer 60 (SEQ ID NO: 60), 200 μM dNTPs and 2 μL of 10x Pfu Ultra Buffer (STRATAGENE) to make the total 20 μL. PCR was carried out by reacting at 95°C for 1 minute and then repeating 40 times the cycle set to include 95°C for 20 seconds, 60°C for 15 seconds and 72°C for 3 minutes. The PCR product was purified using PCR Purification Kit (QIAGEN) and then digested with restriction enzymes EcoRV and XhoI. Similarly, pCMV-Tag4 (STRATAGENE) was digested with restriction enzymes EcoRV and XhoI. These DNA fragments were purified, respectively, using PCR Purification Kit and the two DNA fragments were ligated using DNA Ligation Kit ver. 2 (TaKaRa Bio). The reaction mixture was transfected to Escherichia coli TOP 10 (Invitrogen), followed by incubation for selection in kanamycin-containing LB agar medium. As a result of sequencing of the individual gene clones recovered from the grown colony of Escherichia coli, the animal cell expression vector pCMV-Tag4-Nectin-1 bearing the cDNA sequence (SEQ ID NO: 61) encoding the nectin-1 protein (SEQ ID NO: 62) was obtained.

Human nectin-3 gene was obtained by performing PCR using the cDNA of human placenta contained in MTC Multiple Tissue cDNA Panels (Takara Bio) as a template, primer 47 (SEQ ID NO: 47) tagged with the recognition site of restriction enzyme HindIII, and primer 63 (SEQ ID NO: 63) tagged with the recognition site of restriction enzyme EcoRV In this reaction, the reaction solution was composed of 1 μL of the cDNA solution described above, 1 U of PfuTurbo Hotstart DNA Polymerase, 1 μM each of primer 47 (SEQ ID NO: 47) and primer 63 (SEQ ID NO: 63), 200 μM dNTPs and 10 μL of 2x GC Buffer I (TaKaRa Bio) to make the total 20 μL. PCR was carried out by reacting at 95°C for 1 minute and then repeating 40 times the cycle set to include 95°C for 20 seconds, 60°C for 15 seconds and 72°C for 2 minutes. The PCR product was purified using PCR Purification Kit and then digested with restriction enzymes HindIII and EcoRV Similarly, cDNA3.1(+) (Invitrogen) was digested with restriction enzymes HindIII and EcoRV These DNA fragments were purified, respectively, using PCR Purification Kit, and the two DNA fragments were ligated using DNA Ligation Kit ver. 2. The reaction mixture was transfected to Escherichia coli TOP 10, followed by incubation for selection in ampicillin-containing LB agar medium. As a result of sequencing of the individual gene clones recovered from the grown colony of Escherichia coli, the animal cell expression vector pcDNA3.1(+)-Nectin-3 bearing the cDNA sequence (SEQ ID NO: 6) encoding the nectin-3 protein (SEQ ID NO: 5) was obtained. Using pcDNA3.1(+)-Nectin-3 obtained herein as a template, PCR was carried out by using primer 47 (SEQ ID NO: 47) tagged with the recognition site of restriction enzyme HindIII and primer 64 (SEQ ID NO: 64) tagged with the recognition site of restriction enzyme XhoI. In this reaction, the reaction solution was composed of 100 ng of pcDNA3.1(+)-Nectin-3, 2.5 U of PfuTurbo Hotstart DNA Polymerase, 1 μM each of primer 47 (SEQ ID NO: 47) and primer 64 (SEQ ID NO: 64), 200 μM dNTPs and 25 μL of 2x GC Buffer I to make the total 50 μL. PCR was carried out by reacting at 95°C for 1 minute and then repeating 30 times the cycle set to include 95°C for 20 seconds, 60°C for 15 seconds and 72°C for 2 minutes. The PCR product was purified using PCR Purification Kit and then digested with restriction enzymes HindIII and XhoI. Similarly, pCMV-Tag4 was digested with restriction enzymes HindIII and XhoI. These DNA fragments were purified, respectively, using MinElute PCR Purification Kit (QIAGEN), and the two DNA fragments were ligated using DNA Ligation Kit ver. 2. The reaction mixture was transfected to Escherichia coli TOP 10, followed by incubation for selection in kanamycin-containing LB agar medium. As a result of sequencing of the individual gene clones recovered from the grown colony of Escherichia coli, the animal cell expression vector pCMV-Tag4-Nectin-3 bearing the cDNA sequence (SEQ ID NO: 6) encoding the nectin-3 protein (SEQ ID NO: 5) was obtained.

Human nectin-4 gene was obtained by performing PCR using Marathon-Ready cDNA (Takara Bio) of human lung as a template, primer 65 (SEQ ID NO: 65) tagged with the recognition site of restriction enzyme EcoRI, and primer 66 (SEQ ID NO: 66) tagged with the recognition site of restriction enzyme XhoI. In this reaction, the reaction solution was composed of 1 μL of the cDNA solution described above, 1 U of PfuTurbo Hotstart DNA Polymerase, 1 μM each of primer 65 (SEQ ID NO: 65) and primer 66 (SEQ ID NO: 66), 200 μM dNTPs and 2 μL of 10x Pfu Ultra Buffer to make the total 20 μL. PCR was carried out by reacting at 95°C for 1 minute and then repeating 40 times the cycle set to include 95°C for 20 seconds, 60°C for 15 seconds and 72°C for 3 minutes. Next, the PCR product was purified using PCR Purification Kit and then digested with restriction enzymes EcoRI and XhoI. Similarly, pCMV-Tag4 was digested with restriction enzymes EcoRI and XhoI. These DNA fragments were purified using PCR Purification Kit, and the insert DNA fragment and the vector fragment were ligated using DNA Ligation Kit ver. 2. The reaction mixture was transfected to Escherichia coli TOP 10, followed by incubation for selection in kanamycin-containing LB agar medium. As a result of sequencing of the individual gene clones recovered from the grown colony of Escherichia coli, the animal cell expression vector pCMV-Tag4-Nectin-4 bearing the cDNA sequence (SEQ ID NO: 67) encoding the nectin-4 protein (SEQ ID NO: 68) was obtained.

Human Necl-5 gene was obtained by performing PCR using as a template Marathon-Ready cDNA (Takara Bio) of human small intestine, primer 69 (SEQ ID NO: 69) tagged with the recognition site of restriction enzyme HindIII, and primer 70 (SEQ ID NO: 70) tagged with the recognition site of restriction enzyme SalI. In this reaction, the reaction solution was composed of 1 μL of the cDNA solution above, 1 U of PfuTurbo Hotstart DNA Polymerase, 1 μM each of primer 69 (SEQ ID NO: 69) and primer 70 (SEQ ID NO: 70), 200 μM dNTPs and 2 μL of 10x Pfu Ultra Buffer to make the total 20 μL. PCR was carried out by reacting at 95°C for 1 minute and then repeating 40 times the cycle set to include 95°C for 20 seconds, 60°C for 15 seconds and 72°C for 3 minutes. Next, the PCR product was purified on PCR Purification Kit and then ligated with pCR-BluntII-TOPO (Invitrogen). The reaction mixture was transfected to Escherichia coli TOP10, followed by incubation for selection in kanamycin-containing LB agar medium. As a result of sequencing of the individual gene clones recovered from the grown colony of Escherichia coli, the cloning vector pCR-BluntII-Necl-5 bearing the cDNA sequence (SEQ ID NO: 72) encoding the Necl-5 protein (SEQ ID NO: 71) was obtained. The pCR-BluntII-Necl-5 obtained herein was digested with restriction enzymes HindIII and SalI. Similarly, pCMV-Tag4 was digested with restriction enzymes HindIII and SalI. These DNA fragments were purified using MinElute PCR Purification Kit, and the two DNA fragments were ligated using DNA Ligation Kit ver. 2. The reaction mixture was transfected to Escherichia coli TOP10, followed by incubation for selection in kanamycin-containing LB agar medium. As a result of sequencing of the individual gene clones recovered from the grown colony of Escherichia coli, the animal cell expression vector pCMV-Tag4-Necl-5 bearing the cDNA sequence (SEQ ID NO: 72) encoding the Necl-5 protein (SEQ ID NO: 71) was obtained.

## REFERENCE EXAMPLE 30

### Construction of animal cell expression vector of Ig1 or Ig2 domain-deficient nectin-2 variant

The nectin-2 variant gene in which Ig1 domain or Ig2 domain in the extracellular region of nectin-2 was deficient was obtained by the following procedure. PCR was carried out using pcDNA3.1(+)-Nectin-2δ obtained in REFERENCE EXAMPLE 4 as a template. In the reaction, the reaction solution was composed of 10 ng of pcDNA3.1(+)-Nectin-2δ described above, 1U of PfuTurbo Hotstart DNA Polymerase (STRATAGENE), 1 μM each of primer 5 (SEQ ID NO: 15) tagged with the recognition sequence of restriction enzyme EcoRI and primer 73 (SEQ ID NO: 73) tagged with the recognition sequence of restriction enzyme EcoRV for Ig1 domain-deficient variant, or primer 5 (SEQ ID NO: 15) tagged with the recognition sequence of restriction enzyme EcoRI and primer 74 (SEQ ID NO: 74) tagged with the recognition sequence of restriction enzyme EcoRV for Ig2 domain-deficient variant, 200 μM dNTPs and 10 μL of 2x GC Buffer I (TaKaRa Bio), to make the total 20 μL. PCR was carried out by reacting at 95°C for 1 minute and then repeating 30 times the cycle set to include 95°C for 20 seconds, 60°C for 15 seconds and 72°C for 1.5 minutes. The PCR product was purified using PCR Purification Kit (QIAGEN) and then digested with restriction enzymes EcoRI and EcoRV Similarly, pcDNA3.1 (+) (Invitrogen) was digested with restriction enzymes EcoRI and EcoRV These fragments were purified using PCR Purification Kit, and digested with the restriction enzymes. And after the vector fragment were ligated using DNA Ligation Kit ver. 2 (TaKaRa Bio), the ligation product was transfected to Escherichia coli TOP 10 (Invitrogen), followed by an incubation for selection in ampicillin-containing LB agar medium to obtain plasmids pcDNA3.1(+)-Nectin-2ΔIg1-1 and pcDNA3.1(+)-Nectin-2ΔIG2-1 having the respective PCR fragments amplified above.

Next, PCR was performed using 10 ng of pcDNA3.1(+)-Nectin-2δ as a template, 1U of PfuTurbo Hotstart DNA Polymerase, 1 μM each of primer 75 (SEQ ID NO: 75) tagged with the recognition sequence of restriction enzyme EcoRV and primer 76 (SEQ ID NO: 76) tagged with the recognition sequence of restriction enzyme XhoI for Ig1 domain-deficient variant, or primer 77 (SEQ ID NO: 77) tagged with the recognition sequence of restriction enzyme EcoRV and primer 76 (SEQ ID NO: 76) tagged with the recognition sequence of restriction enzyme XhoI for Ig2 domain-deficient

variant, 200 μM dNTPs and 10 μL of 2x GC Buffer I to make the total 20 μL. PCR was carried out by reacting at 95°C for 1 minute and then repeating 30 times the cycle set to include 95°C for 20 seconds, 60°C for 15 seconds and 72°C for 1.5 minutes. Next, the PCR product was purified using PCR Purification Kit and then digested with restriction enzymes EcoRV and XhoI. Similarly, pcDNA3.1(+)-Nectin-2ΔIg1-1 and pcDNA3.1(+)-Nectin-2ΔIg2-1 constructed above were digested with restriction enzymes EcoRV and XhoI. These fragments were purified using PCR Purification Kit, and the restriction enzyme digests of the PCR product using primer 75 (SEQ ID NO: 75) and primer 76 (SEQ ID NO: 76) was ligated with the restriction enzyme digests of pcDNA3.1(+)-Nectin-2ΔIg1-1 using DNA Ligation Kit ver. 2. Likewise, the restriction enzyme digests of the PCR product using primer 77 (SEQ ID NO: 77) and primer 76 (SEQ ID NO: 76) was ligated with the restriction enzyme digests of pcDNA3.1(+)-Nectin-2ΔIg2-1 using DNA Ligation Kit ver. 2. They were transfected to Escherichia coli TOP10, followed byincubation for selection in ampicillin-containing LB agar medium to give the animal cell expression vector pcDNA3.1(+)-Nectin-2ΔIg bearing the cDNA sequence (SEQ ID NO: 79) encoding the nectin-2 Ig1 domain-deficient protein (SEQ ID NO: 78) and the animal cell expression vector pcDNA3.1(+)-Nectin-2ΔIg2 bearing the cDNA sequence (SEQ ID NO: 81) encoding the nectin-2 Ig2 domain-deficient protein (SEQ ID NO: 80).

REFERENCE EXAMPLE 31

Cloning and base sequencing of cDNA encoding cynomolgus monkey nectin-2

A gene of cynomolgus monkey nectin-2 was obtained as follows. A cDNA library was prepared by a reverse transcription reaction using the TaqMan Reverse Transcription Reagents (Applied Biosystems) and about 1 μg of the total RNA (UNITECH) prepared from the testis of cynomolgus monkey as a template, in accordance with the protocol attached to the kit. Using the cDNA library as a template, PCR was performed using primer 82 (SEQ ID NO: 82) and primer 83 (SEQ ID NO: 83). In the reaction, the reaction solution was composed of the above cDNA corresponding to about 40 ng of the total RNA, 1U of Pfu Turbo Hotstart DNA Polymerase (STRATAGENE), 1 μM each of primer 82 (SEQ ID NO: 82) and primer 83 (SEQ ID NO: 83), 200 μM dNTPs and 2 μL of 10x Pfu Ultra Buffer to make the total 20 μL. PCR was carried out by reacting at 96°C for 1 minute and then repeating 40 times the cycle set to include 96°C for 20 seconds, 60°C for 15 seconds and 72°C for 2.5 minutes. Next, using this PCR product as a template, PCR was performed using primer 84 (SEQ ID NO: 84) and primer 85 (SEQ ID NO: 85). In the reaction, the reaction solution was composed of 1 μL of the above PCR product, 1U of PfuTurbo Hotstart DNA Polymerase, 1 μM each of primer 84 (SEQ ID NO: 84) and primer 85 (SEQ ID NO: 85), 200 μM dNTPs and 2 μL of 10x Pfu Ultra Bufferto make the total 20 μL. PCR was carried out by reacting at 96°C for 1 minute and then repeating 40 times the cycle set to include 96°C for 20 seconds, 60°C for 15 seconds and 72°C for 2.5 minutes. Next, the PCR product was purified using MinElute PCR Purification Kit (QIAGEN) and ligated with pCR-BluntII-TOPO (Invitrogen). The ligation product was transfected to Escherichia coli competent cell TOP10 (Invitrogen), followed by incubation for selection in kanamycin-containing LB agar medium. As a result of sequencing of the individual gene clones recovered from the grown colony of Escherichia coli, the vector pCR-BluntII-maNectin-2 bearing the cDNA sequence (SEQ ID NO: 87) encoding the cynomolgus monkey nectin-2 protein (SEQ ID NO: 86) was obtained.

REFERENCE EXAMPLE 32

Construction of animal cell expression vector for cynomolgus monkey nectin-2

Using the pCR-BluntII-maNectin-2 having the cynomolgus monkey nectin-2 gene prepared in REFERENCE EXAMPLE 31 as a template, PCR was carried out by using primer 88 (SEQ ID NO: 88) tagged with the recognition site of restriction enzyme EcoRI and primer 89 (SEQ ID NO: 89) tagged with the recognition site of restriction enzyme XhoI. In this reaction, the reaction solution was composed of 10 ng of pCR-BluntII-maNectin-2, 1 U of KOD-Plus-DNA Polymerase (TOYOBO), 0.3 μM each of primer 88 (SEQ ID NO: 88) and primer 89 (SEQ ID NO: 89), 200 μM dNTPs and 5 μL of 10x PCR Buffer (TOYOBO) to make the total 50 μL. PCR was carried out by reacting at 95°C for 3 minutes and then repeating 35 times the cycle set to include 95°C for 30 seconds, 60°C for 30 seconds and 68°C for 1 minute and 30 seconds. Next, the PCR product was purified using MinElute PCR Purification Kit (QIAGEN) and then digested with restriction enzymes EcoRI and XhoI, followed by purification with MinElute PCR Purification Kit (QIAGEN). Similarly, pcDNA3.1(+) (Invitrogen) was digested with restriction enzymes EcoRI and XhoI. After separation by agarose electrophoresis, the vector fragment was purified using MinElute Gel Extraction Kit (QIAGEN). The insert DNA fragment and the vector fragment thus obtained were ligated using Ligation High (TOYOBO) and then transfected to Escherichia coli Competent High DH5α (TOYOBO), followed by incubation for selection in ampicillin-containing LB agar medium. As a result of sequencing of the individual gene clones recovered from the grown colony of Escherichia coli, the animal cell expression vector pcDNA3.1(+)-maNectin-2 having the cDNA sequence (SEQ ID NO: 87) encoding the cynomolgus monkey nectin-2 protein (SEQ ID NO: 86) was obtained.

REFERENCE EXAMPLE 33

Construction of animal cell expression vector for human nectin-2 in which a cynomolgus monkey-type mutation was introduced

Using the animal cell expression vector pcDNA3.1(+)-Nectin-2δ prepared in REFERENCE EXAMPLE 4 as a template, PCR was carried out by using the pair of primer 90 (SEQ ID NO: 90) tagged with the recognition site of restriction enzyme HindIII and primer 91 (SEQ ID NO: 91), or the pair of primer 92 (SEQ ID NO: 92) tagged with the recognition site of restriction enzyme EcoRI and primer 93 (SEQ ID NO: 93). In this reaction, the reaction solution was composed of 10 ng of pcDNA3.1(+)-Nectin-2δ, 1 U of KOD-Plus-DNA Polymerase (TOYOBO), 0.3 μM each of primer 90 (SEQ ID NO: 90) and primer 91 (SEQ ID NO: 91), or primer 92 (SEQ ID NO: 92) and primer 93 (SEQ ID NO: 93), 200 μM dNTPs and 5 μL of 10x PCR Buffer (TOYOBO) to make the total 50 μL. PCR was carried out by reacting at 95°C for 3 minutes and then repeating 35 times the cycle set to include 95°C for 30 seconds, 60°C for 30 seconds and 68°C for 1 minute. Next, the PCR products were purified using MinElute PCR Purification Kit (QIAGEN). Next, using the mixture of the PCR products thus obtained as a template, PCR was carried out using primer 90 (SEQ ID NO: 90) and primer 92 (SEQ ID NO: 92). In this reaction, the reaction solution was composed of 5 μL each of the purified PCR products described above, 1 U of KOD-Plus-DNA Polymerase, 0.3 μM each of primer 90 (SEQ ID NO: 90) and primer 92 (SEQ ID NO: 92), 200 μM dNTPs and 5 μL of 10x PCR Bufferto make the total 50 μL. PCR was carried out by reacting at 95°C for 3 minutes and then repeating 20 times the cycle set to include 95°C for 30 seconds, 60°C for 30 seconds and 68°C for 1 minute. Next, the PCR product was purified using MinElute PCR Purification Kit and then digested with restriction enzymes HindIII and EcoRI, followed by purification with MinElute PCR Purification Kit (QIAGEN). Similarly, pcDNA3.1(+) (Invitrogen) was digested with restriction enzymes HindIII and EcoRI. After separation by agarose electrophoresis, the objective vector fragment was purified using MinElute Gel Extraction Kit (QIAGEN). The insert DNA fragment and the vector fragment were ligated using Ligation High (TOYOBO) and then transfected to Escherichia coli Competent High DH5α (TOYOBO), followed by incubation for selection in ampicillin-containing LB agar medium. As a result of sequencing of the individual clones recovered from the grown colony of Escherichia coli, the animal cell expression vector pcDNA3.1(+)-Nectin-2 (AN77-78PD) having a cDNA sequence encoding AN77-78PD with mutation of Ala to Pro at position 77 and Asn to Asp at position 78 in the human nectin-2δ protein (SEQ ID NO: 3) was obtained.

Next, the animal cell expression vector pcDNA3.1(+)-Nectin-2δ prepared in REFERENCE EXAMPLE 4 using as a template, PCR was performed by using the pair of primer 90 (SEQ ID NO: 90) tagged with the recognition site of restriction enzyme HindIII and primer 94 (SEQ ID NO: 94), or the pair of primer 92 (SEQ ID NO: 2) tagged with the recognition site of restriction enzyme EcoRI and primer 95 (SEQ ID NO: 95). In this reaction, the reaction solution was composed of 10 ng of pcDNA3.1(+)-Nectin-2δ, 1 U of KOD-Plus-DNA Polymerase, 0.3 μM each of primer 90 (SEQ ID NO: 90) and primer 94 (SEQ ID NO: 94) or primer 92 (SEQ ID NO: 92) and primer 95 (SEQ ID NO: 95), 200 μM dNTPs and 5 μL of 10x PCR Buffer to make the total 50 μL. PCR was carried out by reacting at 95°C for 3 minutes and then repeating 35 times the cycle set to include 95°C for 30 seconds, 60°C for 30 seconds and 68°C for 1 minute. Next, the PCR products were purified using MinElute PCR Purification Kit. Using the mixture of the PCR products thus obtained as a template, PCR was carried out using primer 90 (SEQ ID NO: 90) and primer 92 (SEQ ID NO: 92). In this reaction, the reaction solution was composed of 5 μL each of the purified PCR products described above, 1 U of KOD-Plus-DNA Polymerase, 0.3 μM each of primer 90 (SEQ ID NO: 90) and primer 92 (SEQ ID NO: 92), 200 μM dNTPs and 5 μL of 10x PCR Buffer to make the total 50 μL. PCR was carried out by reacting at 95°C for 3 minutes and then repeating 20 times the cycle set to include 95°C for 30 seconds, 60°C for 30 seconds and 68°C for 1 minute. Hereinafter, the same procedure as described above was performed to give the animal cell expression vector pcDNA3.1(+)-Nectin-2 (G113R) having a cDNA sequence encoding the protein G113R with mutation of Gly to Arg at position 113 in human nectin-2δ protein (SEQ ID NO: 3).

Next, using the animal cell expression vector pcDNA3.1(+)-Nectin-2δ prepared in REFERENCE EXAMPLE 4 as a template, PCR was performed by using the pair of primer 90 (SEQ ID NO: 90) tagged with the recognition site of restriction enzyme HindIII and primer 96 (SEQ ID NO: 96), or the pair of primer 92 (SEQ ID NO: 2) tagged with the recognition site of restriction enzyme EcoRI and primer 97 (SEQ ID NO: 97). In this reaction, the reaction solution was composed of 10 ng of pcDNA3.1(+)-Nectin-2δ, 1 U of KOD-Plus-DNA Polymerase, 0.3 μM each of primer 90 (SEQ ID NO: 90) and primer 96 (SEQ ID NO: 96) or primer 96 (SEQ ID NO: 96) and primer 97 (SEQ ID NO: 97), 200 μM dNTPs and 5 μL of 10x PCR Buffer to make the total 50 μL. PCR was carried out by reacting at 95°C for 3 minutes and then repeating 35 times the cycle set to include 95°C for 30 seconds, 60°C for 30 seconds and 68°C for 1 minute. Next, the PCR products were purified using MinElute PCR Purification Kit. Using the mixture of the PCR products thus obtained as a template, PCR was carried out using primer 90 (SEQ ID NO: 90) and primer 92 (SEQ ID NO: 92). In this reaction, the reaction solution was composed of 5 μL each of the purified PCR products described above, 1 U of KOD-Plus-DNA Polymerase, 0.3 3 μM each of primer 90 (SEQ ID NO: 90) and primer 92 (SEQ ID NO: 92), 200 μM dNTPs and 5 μL of 10x PCR Buffer to make the total 50 μL. PCR was carried out by reacting at 95°C for 3 minutes and then repeating 20 times the cycle set to include 95°C for 30 seconds, 60°C for 30 seconds and 68°C for 1 minute. Hereinafter, the same procedure as described above was performed to give the animal cell expression vector pcDNA3.1(+)-Nectin-2 (H128R) having a cDNA sequence encoding the protein H128R with mutation of His to Arg at position 128 in human nectin-2δ protein (SEQ ID NO: 3).

REFERENCE EXAMPLE 34

Construction of animal cell expression vectors for human Nectin-2ED-Fc protein with a single amino acid substitution in the Ig1 domain

Using the animal cell expression vector pcDNA3.1(+)-Nectin-2ED-hFc prepared in REFERENCE EXAMPLE 15 as a template, the DNA sequence encoding 26 amino acid residues in the Ig1 domain of nectin-2 was mutated to replace each of the amino acid residues with alanine residue or glycine residue, by using Quick Change XL Site-Directed Mutagenesis Kit (Stratagene). In the reaction, the reaction solution composed of 7 μL of the expression plasmid described above (20 ng/ μL), 35 μL of 10x Buffer, 7 μL of dNTP mix, 21 μL of Quick Solution and 7 μL of PfuTurbo DNA Polymerase (2.5U/μL) to make the total volume 300 μL by adding distilled water thereto. A 9 μL aliquot of the solution was separately dispensed into 26 tubes for PCR, to which each combination of 0.5 μL of the respective primers (3.7 μM) was added: primer Q37A (SEQ ID NO: 98) and primer Q37A R (SEQ ID NO: 99), primer P40G (SEQ ID NO: 100) and primer P40G R (SEQ ID NO: 101), primer Q45A (SEQ ID NO: 102) and primer Q45A R (SEQ ID NO: 103), primer H55A (SEQ ID NO: 104) and primer H55AR (SEQ ID NO: 105), primer V60A (SEQ ID NO: 106) and primer V60A R (SEQ ID NO: 107), primer Y64A (SEQ ID NO: 108) and primer Y64A R (SEQ ID NO: 109), primer Q71A (SEQ ID NO: 110) and primer Q71A R (SEQ ID NO: 111), primer A75G (SEQ ID NO: 112) and primer A75G R (SEQ ID NO: 113), primer P76G (SEQ ID NO: 114) and primer P76G R (SEQ ID NO: 115), primer A77G (SEQ ID NO: 116) and primer A77G R (SEQ ID NO: 117), primer N78A (SEQ ID NO: 118) and primer N78A R (SEQ ID NO: 119), primer H79A (SEQ ID NO: 120) and primer H79A R (SEQ ID NO: 121), primer Q80A (SEQ ID NO: 122) and primer Q80AR (SEQ ID NO: 123), primer N81A (SEQ ID NO: 124) and primer N81AR (SEQ ID NO: 125), primer K88A (SEQ ID NO: 126) and primer K88A R (SEQ ID NO: 127), primer S95A (SEQ ID NO: 128) and primer S95A R (SEQ ID NO: 129), primer K109A (SEQ ID NO: 130) and primer K109A R (SEQ ID NO: 131), primer E117A(SEQ ID NO: 132) and primer E117A R (SEQ ID NO: 133), primer D122A (SEQ ID NO: 134) and primer D122AR (SEQ ID NO: 135), primer H128A (SEQ ID NO: 136) and primer H128AR (SEQ ID NO: 137), primer N137A (SEQ ID NO: 138) and primer N 13 7A R (SEQ ID NO: 139), primer F145A (SEQ ID NO: 140) and primer F145A R (SEQ ID NO: 141), primer K147A (SEQ ID NO: 142) and primer K147A R (SEQ ID NO: 143), primer V150A (SEQ ID NO: 144) and primer V150A R (SEQ ID NO: 145), primer M153A (SEQ ID NO: 146) and primer M153A R (SEQ ID NO: 147), or, primer T154A (SEQ ID NO: 148) and primer T154AR (SEQ ID NO: 149). PCR was carried out by reacting at 95°C for 1 minute and then repeating 18 times the cycle set to include 95°C for 50 seconds, 60°C for 50 seconds and 68°C for 7 minutes and 40 seconds, followed by reaction at 68°C for 7 minutes. After the reaction, 1 μL each of restriction enzyme DpnI (2U/μL) was added to 26 tubes of the PCR solution, followed by reacting them at 37°C for an hour. These reaction mixtures, 2 μL, were transfected to 20 μL of Escherichia coli XL10-Gold ultracompetent cells and incubated for selection in ampicillin-containing LB agar medium. As a result of sequencing of the individual gene clones recovered from the grown colony of Escherichia coli, the following animal cell expression vectors were obtained, respectively: the animal cell expression vector pcDNA3.1(+)-Nectin-2ED-Fc (Q37A) having a cDNA sequence encoding the protein Q37A with mutation of Gln to Ala at position 37 in Nectin-2ED-Fc (SEQ ID NO: 37), the animal cell expression vector pcDNA3.1(+)-Nectin-2ED-Fc (P40G) having a cDNA sequence encoding the protein P40G with mutation of Pro to Gly at position 40 in Nectin-2ED-Fc (SEQ ID NO: 37), the animal cell expression vector pcDNA3.1(+)-Nectin-2ED-Fc (Q45A) having a cDNA sequence encoding the protein Q45A with mutation of Gln to Ala at position 45 in Nectin-2ED-Fc (SEQ ID NO: 37), the animal cell expression vector pcDNA3.1(+)-Nectin-2ED-Fc (H55A) having a cDNA sequence encoding the protein H55A with mutation of His to Ala at position 55 in Nectin-2ED-Fc (SEQ ID NO: 37), the animal cell expression vector pcDNA3.1(+)-Nectin-2ED-Fc (V60A) having a cDNA sequence encoding the protein V60A with mutation of Val to Ala at position 60 in Nectin-2ED-Fc (SEQ ID NO: 37), the animal cell expression vector pcDNA3.1(+)-Nectin-2ED-Fc (Y64A) having a cDNA sequence encoding the protein Y64A with mutation of Tyr to Ala at position 64 in Nectin-2ED-Fc (SEQ ID NO: 37), the animal cell expression vector pcDNA3.1 (+)-Nectin-2ED-Fc (Q71A) having a cDNA sequence encoding the protein Q71A with mutation of Gln to Ala at position 71 in Nectin-2ED-Fc (SEQ ID NO: 37), the animal cell expression vector pcDNA3.1(+)-Nectin-2ED-Fc (A75G) having a cDNA sequence encoding the protein A75G with mutation of Ala to Gly at position 75 in Nectin-2ED-Fc (SEQ ID NO: 37), the animal cell expression vector pcDNA3.1(+)-Nectin-2ED-Fc (P76G) having a cDNA sequence encoding the protein P76G with mutation of Pro to Gly at position 76 in Nectin-2ED-Fc (SEQ ID NO: 37), the animal cell expression vector pcDNA3.1(+)-Nectin-2ED-Fc (A77G) having a cDNA sequence encoding the protein A77G with mutation of Ala to Gly at position 77 in Nectin-2ED-Fc (SEQ ID NO: 37), the animal cell expression vector pcDNA3.1(+)-Nectin-2ED-Fc (N78A) having a cDNA sequence encoding the protein N78A with mutation of Asn to Ala at position 78 in Nectin- 2ED-Fc (SEQ ID NO: 37), the animal cell expression vector pcDNA3.1(+)-Nectin-2ED-Fc (H79A) having a cDNA sequence encoding the protein H79A with mutation of His to Ala at position 79 in Nectin-2ED-Fc (SEQ ID NO: 37), the animal cell expression vector pcDNA3.1(+)-Nectin-2ED-Fc (Q80A) having a cDNA sequence encoding the protein Q80A with mutation of Gln to Ala at position 80 in Nectin-2ED-Fc (SEQ ID NO: 37), the animal cell expression vector pcDNA3.1 (+)-Nectin-2ED-Fc (N81A) having a cDNA sequence encoding the protein N81A with mutation of Asn to Ala at position 81 in Nectin-2ED-Fc (SEQ ID NO: 37), the animal cell expression vector pcDNA3.1(+)-Nectin-2ED-Fc (K88A) having a cDNA sequence encoding the protein K88A with mutation of Lys to Ala at position 88 in Nectin-2ED-Fc (SEQ ID NO: 37), the animal cell expression vector pcDNA3.1(+)-Nectin-2ED-Fc (S95A) having a cDNA sequence encoding the protein S95A with mutation of Ser to Ala at position 95 in Nectin-2ED-Fc (SEQ ID NO: 37), the animal cell expression vector pcDNA3.1(+)-Nectin-2ED-Fc (K109A) having a cDNA sequence encoding the protein K109A with mutation of Lys to Ala at position 109 in Nectin-2ED-Fc (SEQ ID NO: 37), the animal cell expression vector pcDNA3.1(+)-Nectin-2ED-Fc (E117A) having a cDNA sequence encoding the protein E117A with mutation of Glu to Ala at position 117 in Nectin-2ED-Fc (SEQ ID NO: 37), the animal cell expression vector pcDNA3.1(+)-Nectin-2ED-Fc (D122A) having a cDNA sequence encoding the protein D122A with mutation of Asp to Ala at position 122 in Nectin-2ED-Fc (SEQ ID NO: 37), the animal cell expression vector pcDNA3.1(+)-Nectin-2ED-Fc (H128A) having a cDNA sequence encoding the protein H128A with mutation of His to Ala at position 128 in Nectin-2ED-Fc (SEQ ID NO: 37), the animal cell expression vector pcDNA3.1(+)-Nectin-2ED-Fc (N137A) having a cDNA sequence encoding the protein N137A with mutation of Asn to Ala at position 137 in Nectin-2ED-Fc (SEQ ID NO: 37), the animal cell expression vector pcDNA3.1(+)-Nectin-2ED-Fc (F145A) having a cDNA sequence encoding the protein F145A with mutation ofPhe to Ala at position 145 in Nectin-2ED-Fc (SEQ ID NO: 37), the animal cell expression vector pcDNA3.1(+)-Nectin-2ED-Fc (K147A) having a cDNA sequence encoding the protein K147A with mutation of Lys to Ala at position 147 in Nectin-2ED-Fc (SEQ ID NO: 37), the animal cell expression vector pcDNA3.1(+)-Nectin-2ED-Fc (V150A) having a cDNA sequence encoding the protein V150A with mutation of Val to Ala at position 150 in Nectin-2ED-Fc (SEQ ID NO: 37), the animal cell expression vector pcDNA3.1(+)-Nectin-2ED-Fc (M153A) having a cDNA sequence encoding the protein M153A with mutation of Met to Ala at position 153 in Nectin-2ED-Fc (SEQ ID NO: 37), the animal cell expression vector pcDNA3.1(+)-Nectin-2ED-Fc (T154A) having a cDNA sequence encoding the protein T154A with mutation of Thr to Ala at position 154 in Nectin-2ED-Fc (SEQ ID NO: 37).

## REFERENCE EXAMPLE 35

### Construction of animal cell expression vector for human Nectin-2ED-Fc protein with a single amino acid substitution in the Ig2 domain

Using the animal cell expression vector pcDNA3.1(+)-Nectin-2ED-hFc prepared in REFERENCE EXAMPLE 15 as a template, the DNA sequence encoding 13 amino acid residues in the Ig2 domain of nectin-2 were mutated to replace each of the amino acid residues with alanine residue or glycine residue, by using Quick Change XL Site-Directed Mutagenesis Kit (Stratagene). In the reaction, the reaction solution composed of 3.5 μL of the expression vector described above(20 ng/ μL), 17.5 μL of 10x Buffer, 3.5 μL of dNTP mix, 10.5 μL of Quick Solution and 3.5 μL of PfuTurbo DNA Polymerase (2.5U/μL) to make the total volume 150 μL by adding distilled water thereto. A 9 μL aliquot of the solution was separately dispensed into 13 tubes for PCR, to which each combination of 0.5 μL of the respective primers (3.7 μM) was added: primer Q165A (SEQ ID NO: 150) and primer Q165A R (SEQ ID NO: 151), primer K170A (SEQ ID NO: 152) and primer K170AR (SEQ ID NO: 153), primer F173A (SEQ ID NO: 154) and primer F173AR (SEQ ID NO: 155), primer P177G (SEQ ID NO: 156) and primer P177G R (SEQ ID NO: 157), primer I184A (SEQ ID NO: 158) and primer I184A R (SEQ ID NO: 159), primer K186A (SEQ ID NO: 160) and primer K186AR (SEQ ID NO: 161), primer L197A (SEQ ID NO: 162) and primer L197AR (SEQ ID NO: 163), primer W202A (SEQ ID NO: 164) and primer W202A R (SEQ ID NO: 165), primer E206A (SEQ ID NO: 166) and primer E206A R (SEQ ID NO: 167), primer T212A (SEQ ID NO: 168) and primer T212A R (SEQ ID NO: 169), primer T235A(SEQ ID NO: 170) and primer T23 5A R (SEQ ID NO: 171), primer K239A (SEQ ID NO: 172) and primer K239A R (SEQ ID NO: 173), or, primer A249G (SEQ ID NO: 174) and primer A249G R (SEQ ID NO: 175). PCR was carried out by reacting at 95°C for 1 minute and then repeating 18 times the cycle set to include 95°C for 50 seconds, 60°C for 50 seconds and 68°C for 7 minutes and 40 seconds, followed by reaction at 68°C for 7 minutes. After the reaction, 1 μL each of restriction enzyme DpnI (2U/μL) was added to 13 tubes of the PCR solution, followed by reacting them at 37°C for an hour. These reaction mixtures, 2 μL, were transfected to 20 μL of Escherichia coli XL10-Gold ultracompetent cells and incubated for selection in ampicillin-containing LB agar medium. As a result of sequencing of the individual gene clones recovered from the grown colony of Escherichia coli, the following animal cell expression vectors were obtained, respectively: the animal cell expression vector pcDNA3. 1 (+)-Nectin-2ED-Fc (Q165A) having a cDNA sequence encoding the protein Q165A with mutation of Gln to Ala at position 165 in Nectin-2ED-Fc (SEQ ID NO: 37), the animal cell expression vector pcDNA3.1(+)-Nectin-2ED-Fc (K170A) having a cDNA sequence encoding the protein K170A with mutation of Lys to Ala at position 170 in Nectin-2ED-Fc (SEQ ID NO: 37), the animal cell expression vector pcDNA3.1(+)-Nectin-2ED-Fc (F 173 A) having a cDNA sequence encoding the protein F 173 A with mutation of Phe to Ala at position 173 in Nectin-2ED-Fc (SEQ ID NO: 37), the animal cell expression vector pcDNA3.1(+)-Nectin-2ED-Fc (P177G) having a cDNA sequence encoding the protein P177G with mutation of Pro to Gly at position 177 in Nectin-2ED-Fc (SEQ ID NO: 37), the animal cell expression vector pcDNA3.1 (+)-Nectin-2ED-Fc (I184A) having a cDNA sequence encoding the protein I184A with mutation of Ile to Ala at position 184 in Nectin-2ED-Fc (SEQ ID NO: 37), the animal cell expression vector pcDNA3.1(+)-Nectin-2ED-Fc (K186A) having a cDNA sequence encoding the protein K186A with mutation of Lys to Ala at position 186 in Nectin-2ED-Fc (SEQ ID NO: 37), the animal cell expression vector pcDNA3.1(+)-Nectin-2ED-Fc (L197A) having a cDNA sequence encoding the protein L197A with mutation of Leu to Ala at position 197 in Nectin-2ED-Fc (SEQ ID NO: 37), the animal cell expression vector pcDNA3. 1(+)-Nectin-2ED-Fc (W202A) having a cDNA sequence encoding the protein W202A with mutation of Trp to Ala at position 202 in Nectin-2ED-Fc (SEQ ID NO: 37), the animal cell expression vector pcDNA3. 1(+)-Nectin-2ED-Fc (E206A) having a cDNA sequence encoding the protein E206A with mutation of Glu to Ala at position 206 in Nectin-2ED-Fc (SEQ ID NO: 37), the animal cell expression vector pcDNA3.1(+)-Nectin-2ED-Fc (T212A) having a cDNA sequence encoding the protein T212A with mutation of Thr to Ala at position 212 in Nectin-2ED-Fc (SEQ ID NO: 37), the animal cell expression vector pcDNA3.1(+)-Nectin-2ED-Fc (T235A) having a cDNA sequence encoding the protein T235A with mutation of Thr to Ala at position 235 in Nectin-2ED-Fc (SEQ ID NO: 37), the animal cell expression vector pcDNA3.1(+)-Nectin-2ED-Fc (K239A) having a cDNA sequence encoding the protein K239A with mutation of Lys to Ala at position 239 in Nectin-2ED-Fc (SEQ ID NO: 37), the animal cell expression vector pcDNA3.1(+)-Nectin-2ED-Fc (A249G) having a cDNA sequence encoding the protein A249G with mutation of Ala to Gly at position 249 in Nectin-2ED-Fc (SEQ ID NO: 37).

EXAMPLE 1

Generation of anti-nectin-2 human monoclonal antibodies

Each five KM mice (10 weeks old, 12 weeks old, male, Kirin Brewery) were immunized with an emulsion prepared by mixing the Nectin-2ED-Fc protein (1.6 mg/mL PBS solution) prepared in REFERENCE EXAMPLE 16 or the Nectin-2ED-FLAG protein (2 mg/mL PBS solution) prepared in REFERENCE EXAMPLE 13 with Freund's complete adjuvant (Difco) in equal volumes, subcutaneously and intracutaneously at 50 μg each/animal, respectively. For the second and subsequent immunization, the emulsions prepared by mixing these recombinant nectin-2 extracellular domain proteins and Freund's incomplete adjuvant (Difco) in equal volumes were likewise given every 2 weeks as additional immunization.

Also, the FM3A cell line (#60-6) stably expressing nectin-2δ, which was established in REFERENCE EXAMPLE 19, and the NS0 cell line (#2-75) stably expressing nectin-2δ, which was established in REFERENCE EXAMPLE 18, were cutured in a flask, respectively. The cells were recovered by centrifugation (1,200 rpm x 5 minutes), resuspended in RPMI1640 medium (Invitrogen), and then recovered by centrifugation (1,200 rpm x 5 minutes), which procedure was repeated 3 times to remove the serum components. The respective cells recovered were resuspended in RPMI1640 medium at 5 x $10^7$ cells/mL, and a solution of mitomycin C (Wako Pure Chemical) in RPMI1640 medium in a final concentration of 20 μg/mL was added to the cells, followed by incubation at 37°C for 30 minutes. Both cell lines treated with mitomycin C were likewise washed 3 times with 10 mL of PBS and then resuspended in PBS at 2 x $10^7$ cells/mL. The suspension was intraperitoneally injected to each five KM mice (10-12 weeks old, male) repeatedly once every week.

Also, each five KM mice (12 weeks old, male) were immunized with an emulsion prepared by mixing the Nectin-2ED-Fc protein or the Nectin-2ED-FLAG protein with Freund's complete adjuvant (Difco) in equal volumes subcutaneously and intracutaneously at 50 μg each/animal, respectively. One week after the first immunization, mitomycin C-treated nectin-2δ expressing FM3A cell line (#60-6) was intraperitoneally given at 1 x $10^7$ cells each/500 μL for booster. For the third immunization, an emulsion prepared by mixing Nectin-2ED-Fc or Nectin-2ED-FLAG and Freund's incomplete adjuvant in equal volumes was subcutaneously and intracutaneously administered at 50 μg/animal, respectively, and at the same time, mitomycin C-treated nectin-2δ expressing FM3A cell line (#60-6) was intraperitoneally injected at 1 x $10^7$ cells each/500 μL, by the same procedure as described above. For the fourth and subsequent booster, the mitomycin C-treated nectin-2δ expressing FM3A cell line (#60-6) only was intraperitoneally injected at 1 x $10^7$ cells each/500 μL, by the procedure described above.

Prior to the first immunization and one week after the third immunization, blood was collected from the ocular fundus of all mice under ethereal anesthesia to prepare antisera, and the antibody titer in the sera was determined by the flow cystometry described below. That is, cell suspensions (PBS) of the CHO cell line (#43-2) stably expressing nectin-2δ, which was established in REFERENCE EXAMPLE 20, and mock-CHO cell line were separately dispensed in polypropylene tubes at 5 x $10^5$ cells/tube, respectively, and then PBS was removed by centrifugation (1,200 rpm x 5 minutes). These cell debris were resuspended in 50 μL each of the mouse antisera diluted to 100-fold with PBS containing 1% BSA and 10% FBS, and reacted on ice in the dark for 30 minutes. After 200 μL of PBS was added to the cell debris and the mixture was centrifuged (1,200 rpm x 5 minutes), the supernatant was removed by aspiration. The cell debris were resuspended in 50 μL of a solution of anti-human IgG (H+L) Alexa 488 (Invitrogen) diluted to 100-fold with PBS containing 1% BSA and 10% FBS, and reacted on ice in the dark for 30 minutes. After the cell suspension was likewise washed 3 times with PBS, the cell debris was resuspended in 200 μL of PBS Fluorescence intensities of the respective cells were analyzed with the flow cytometer MPL500 (BECKMAN COULTER) to prepare a graph having an abscissa representing the fluorescence intensity and an ordinate representing the cell count, whereby antibody titers of antisera were compared.

In the KM mice in which a sufficient increase in the serum antibody titer was confirmed, the mice immunized with Nectin-2ED-Fc or Nectin-2ED-FLAG were injected via tail vein with these protein antigens at a dose of 10 μg each/ animal, and the mice immunized with the cell line stably expressing nectin-2δ were intraperitoneally injected with the same cell line at 1 x $10^7$ cells each for final booster. Three days after the final booster, the mice were bled to death and spleen was withdrawn. The mouse spleen cells obtained were mixed in 5 : 1 with mouse myeloma cells (P3X63Ag8U. 1 (P3U1)), which had previously been adapted to a medium in which 1 vial of 8-azaguanine (Sigma) per 500 mL of 10% FBS-supplemented Daigo T medium (a medium mixture of F-12 Nutrient Mixture (HAM) (Invitrogen) and Iscove's Modified Dulbecco's Medium (Invitrogen) in equal volumes, supplemented with MEM Non-Essential Amino Acid Solution (Invitrogen), Sodium Pyruvate (Invitrogen) and L-Glutamine (Invitrogen)) thereby to cause fusion using polyethylene glycol (PEG) 1,500 (Roche Diagnostics). Cell fusion manipulations were performed according to the manual attached. The cells after fusion were resuspended in Daigo T medium supplemented with 10% FBS and 10% BM Condimed H1 (Roche Diagnostics), seeded on a 96-well culture plate at 5 x $10^4$ spleen cells/100 μL/well and incubated at 37°C for a day in a 5% carbon dioxide gas flow. Subsequently, Daigo T medium (HAT selection medium) supplemented with 0.1 mM hypoxanthine, 0.4 μM aminoputerine, 0.016 mM thymidine (HAT), 10% BM Condimed H1 and 10% FBS was added thereto at 100 μL/well, followed by a further incubation at 37°C in a 5% carbon dioxide gas flow with replacing twice 3/4 of the culture supernatant with a fresh HAT selection medium every 3 days.

The culture supernatant in which growth of the colony was observed during days 7 to 14 of the incubation was applied to Cell ELISA using the CHO cell line (#43-2) stably expressing nectin-2δ or the mock-CHO cell line, whereby an anti-nectin-2 human monoclonal antibody-producing hybridoma was screened. In other words, the CHO cell line (#43-2) stably expressing nectin-2δ and the mock-CHO cell line were incubated in a 96-well tissue culture plate charged with GS-selection DMEM medium supplemented with 10% dialyzed FBS and GS supplement. After the culture supernatant of the plate where each cell line became confluent was removed by aspiration, 200 μL/well of PBS(+) supplemented with 2% FBS was added thereto and incubated on ice in the dark for an hour. After the supernatant of each well was removed by aspiration, 50 μL each/well of the hybridoma culture supernatant was added and reacted on ice in the dark for 2 hours. After this plate was washed once with PBS(+) chilled at 4°C, anti-human IgG (H+L) chain specific (GOAT) peroxidase conjugate (CALBIOCHEM) diluted to 3,000-fold with PBS(+) supplemented with 2% FBS was added by 100 μL each/well and reacted on ice in the dark for 2 hours. After the plate was washed 3 times with PBS(+) chilled at 4°C, a 3,3',5,5'-tetramethylbenzidine (TMB) solution (SureBlue Microwell TMB peroxidase substrate; Kirkegaard & Perry Laboratories) was added by 100 μL each/well and maintained at room temperature for 5 minutes to cause color formation. By adding 2N sulfuric acid (Wako Pure Chemical) by 100 μL each/well, the enzyme reaction was terminated. Absorbance (450 nm) of each well was measured using a plate reader (Multiskan BICHROMATIC; Thermo Electron Co.), and those showing absorbance of 0.5 or more in the nectin-2 expression CHO cell line plate and showing absorbance of less than 0.3 in the mock-CHO cell line plate were judged to be positive wells. IgG antibody-producing hybridomas, whose antigenic specificity and affinity are expected to be particularly high, were selected from them. These hybridomas were resuspended in Daigo T medium supplemented with 10% FBS and 10% BM Condimed H1 and plated on a 96-well tissue culture plate at 0.5 cell/well. The culture supernatants of the hybridomas, which were confirmed to be monoclones by microscopic observation, were again screened by the Cell ELISA described above to establish anti-nectin-2 human monoclonal antibody-producing hybridoma clones. The thus obtained 256 anti-nectin-2 human monoclonal antibody-producing hybridomas were incubated in flasks, respectively, charged with 100 mL of Daigo T medium supplemented with 10% FBS Ultra low IgG (Invitrogen), and the culture supernatants were centrifuged (1,200 rpm x 5 minutes) to give the supernatants containing monoclonal antibodies. After 200 μL of Protein A Sepharose FF (Amersham Biosciences, which name was changed to GE Healthcare Bio-sciences) equilibrated with PBS was added to these culture supernatants, the antibodies were adsorbed thereto while gently shaking overnight at 4°C. This protein A carrier was recovered by centrifugal operation and washed with PBS. Then, the IgG fraction was eluted with 1.2 mL of 0.1 M glycine-HCl (pH 3.0) containing 0.3 M NaCl. After this eluate was immediately neutralized with 1 M Tris-HCl (pH 8.0), the buffer was replaced with PBS by ultraconcentration using ultrafiltration membrane (Vivaspin 6: molecular weight cut off = 10,000, Sartorius), which was used in the following *in vitro* characterizations as the anti-nectin-2 human monoclonal antibody preparation.

EXAMPLE 2

Binding activities of anti-nectin-2 human monoclonal antibodies

As the binding affinities of the anti-nectin-2 human monoclonal antibodies, the $EC_{50}$ value of each antibody was determined by applying serial dilutions of the anti-nectin-2 human monoclonal antibodies prepared in EXAMPLE 1 with PBS(+) supplemented with 2% FBS to Cell ELISA using the nectin-2 stably expressed CHO cell line shown in EXAMPLE 1 and preparing a concentration-dependent curve, which were relatively assessed. The antigenic specificity of each monoclonal antibody was confirmed by comparing the binding property to the nectin-2 stable expression CHO cell line plate and the binding property to the mock-CHO cell line plate. The $EC_{50}$ of respective anti-nectin-2 human monoclonal antibodies are collectively shown in TABLE 3.

EXAMPLE 3

Subclass of anti-nectin-2 human monoclonal antibodies

The subclass of the anti-nectin-2 human monoclonal antibodies obtained in EXAMPLE 1 were identified by ELISA shown below. Six antibodies (Anti-human $IgG_1$, Fc Fragment (Mouse), purified; CALBIOCHEM, Anti-human $IgG_2$, Fc Fragment (Mouse), purified; CALBIOCHEM, Mouse Anti-Human $IgG_3$ ; Zymed, Ms x Hu $IgG_4$ Fc; CHEMICON, Monoclonal Mouse Anti-Human-IgM; Zymed, Goat anti-Human Kappa Light Chain Antibody b+f affinity purified; Bethyl) capable of specifically recognizing the H chain of human $IgG_1$, $IgG_2$, $IgG_3$, $IgG_4$ and IgM and human $\kappa$ chain were diluted to a concentration of 2 $\mu$g/mL, respectively, in 50 mM sodium carbonate-sodium bicarbonate buffer (pH 9.6). Each dilution was added to a 96-well half well immunoplate (Costar) at 50 $\mu$L each/well, followed by reacting at room temperature for 5 hours. After the reaction solution was removed from each well, 100 $\mu$L each /well of 25% Block Ace-containing distilled water (DAINIPPON PHARMACEUTICALS) was added for a blocking at 4°C overnight.

The thus prepared plate for ELISA was washed twice with PBS containing 0.05% Tween 20. Thereafter, each anti-nectin-2 human monoclonal antibody purified and obtained in EXAMPLE 1 was diluted to 1 $\mu$g/mL in 10% Block Ace-containing distilled water and 50 $\mu$L/well of the resulting dilution was added to the plate, followed by reacting at room temperature for 2 hours. After this plate was washed 4 times with PBS containing 0.05% Tween 20, Anti-IgG+IgA+IgM (H+L), Human, Goat, Horseradish Peroxidase (Zymed) diluted to 5,000-fold in 10% Block Ace-containing distilled water was added by 50 $\mu$L each/well, which was then reacted at room temperature for 2 hours. The plate was further washed 6 times with PBS containing 0.05% Tween 20. After TMB solution (SureBlue Microwell TMB peroxidase substrate) was added by 50 $\mu$L each/well, the plate was maintained at room temperature for 2 minutes to cause color formation. Then, 2N sulfuric acid (Wako Pure Chemical) was added by 50 $\mu$L each/well to terminate the enzyme reaction. Absorbance (450 nm) of each well was measured using a plate reader (Multiskan BICHROMATIC) and the subclass of each antibody was identified from the antigen specificity of the antibody immobilized to the well showing a significantly higher absorbance than the others. The results are shown in TABLE 3A.

EXAMPLE 4

Grouping of anti-nectin-2 human monoclonal antibodies based on epitopes

The anti-nectin-2 human monoclonal antibodies obtained in EXAMPLE 1 were grouped according to the difference in their recognition epitopes. Its process is shown below. To perform the competitive inhibition reaction between the antibodies, 187 anti-nectin-2 human monoclonal antibodies in the antibodies obtained in EXAMPLE 1 were biotinylated. That is, 10 $\mu$g of the anti-nectin-2 human monoclonal antibody was added to 50 $\mu$L ofWS Buffer attached to Biotin Labeling Kit-NH2 (Dojindo) and the mixture was ultraconcentrated almost to dryness using Microcon YM50 (MILLIPORE). To the liquid residue, 50 $\mu$L of Reaction Buffer attached to Biotin Labeling Kit-NH2 and a solution of 4 $\mu$L of NH2 Reactive Biotin in 50 $\mu$L of DMSO were added sequentially in this order. The mixture was reacted at 37°C for 10 minutes. The reaction mixture was again ultraconcentrated for buffer replacement with WS Buffer to give biotinylated anti-nectin-2 human monoclonal antibodies. These antibodies were used for the assay described below. To FMAT plate (384 well plate Black/Clear Bottom with Lid; Applied Biosystems), 5 $\mu$L of a 2% FBS-supplemented PBS solution (25 $\mu$g/mL) of the anti-nectin-2 human monoclonal antibody obtained in EXAMPLE 1, 15 $\mu$L of a 2% FBS-supplemented PBS suspension (2 x $10^5$ cells/mL) of the CHO cell line stably expression nectin-2$\delta$ and 5 $\mu$L of Streptavidin-Alexa Fluor 647 conjugate (Invitrogen) were added and mixed with each other, followed by reacting them at room temperature for 10 minutes. After 5 $\mu$L of a 2% FBS-supplemented PBS solution (0.5 $\mu$g/mL) of the biotinylated anti-nectin-2 human monoclonal antibody prepared by the procedure described above was added to each well, the plate was incubated at room temperature for 60 minutes. For control runs, wells in which 2% FBS-supplemented PBS was added in place of the solution unlabeled anti-nectin-2 monoclonal antibody were provided. This competitive inhibition reaction were examined with the all combinations of the antibodies provided for the biotinylation. This plate was set on the Applied Biosystems 8200 Cellular Detection System (Applied Biosystems) to measure the fluorescence intensity of each well. The competitive inhibition rate in the combination of each anti-nectin-2 human monoclonal antibody was calculated according to the formula shown below.

$$\text{Competitive inhibition rate} = (1\text{-}A/B) \times 100$$

A: Total FL1 value of well in which unlabeled antibody is added

B: Total FL1 value of well in which unlabeled antibody is not added

The inhibition rate obtained by this formula for all the combination of respective antibodies were analyzed using multivariable analysis software SpotFire DecisionSite for Lead Discovery (Spotfire). Based on the tree diagram thus obtained, the anti-nectin-2 human monoclonal antibodies were grouped by epitopes. As a result, the antibodies were classified into those belonging to seven big groups I through VII and the antibodies unaffiliated with any groups. Epitope groups of each nectin-2 human monoclonal antibody are collectively shown in TABLE 4.

EXAMPLE 5

Nectin-2-nectin-3 trans-binding inhibitory activity of anti-nectin-2 human monoclonal antibodies

It is known that nectin-2 heterophilically trans-interacts with nectin-3 Nectin-2-nectin-3 trans-binding inhibitory activity of the respective anti-nectin-2 human monoclonal antibodies obtained in EXAMPLE 1 was quantitatively assessed by the Biacore assay (Biacore 2000; Biacore, which name was changed to GE Healthcare) described below. Sensor chip CM5 (Biacore, which name was changed to GE Healthcare) was mounted on Biacore 2000 and the Nectin-3ED-Fc protein-immobilized chips were prepared by the following procedure. That is, N-ethyl-N'-(3-dimethylaminopropyl) carbodiimide hydrochloride (EDC) and N-hydroxysuccinimide (NHS), attached to Amine Coupling Kit (Biacore, which name was changed to GE Healthcare), were dissolved in distilled water, and the resulting solutions were mixed by 100 μL each in equal volumes. Using HBS-EP buffer (Biacore, which name was changed to GE Healthcare) as a running buffer, the solution mixture was passed through the sensor chip at a flow rate of 10 μL/min. for 7 minutes. Thereafter, Nectin-3ED-mFc (1 mg/mL PBS solution) prepared in REFERENCE EXAMPLE 22 was diluted to 160 μg/mL in 10 mM acetate buffer (pH 5.0) (Biacore, which name was changed to GE Healthcare) and the dilution was passed through the sensor chip at a flow rate of 10 μL/min for 7 minutes to immobilize the protein on the chip. Subsequently, the ethanolamine solution attached to the same kit was passed through the sensor chip at a flow rate of 10 μL/min. for 7 minutes to block the remaining active NHS groups. Furthermore, 10 mM NaOH was passed through to wash the sensor chip at a flow rate of 10 μL/min. for 1 minute. An equivolume mixture of the Nectin-2ED-hFc protein solution (80 μg/mL HBS-EP buffer) and the anti-nectin-2 human monoclonal antibody solution or control human antibody solution (Human IgG Whole Molecule Chrom Pure; Jackson ImmunoResearch Laboratories) (60 μg/mL HBS-EP buffer) was passed through the thus prepared Nectin-3ED-mFc protein-immobilized sensor chip at a flow rate of 20 μL/min. for 2 minutes. Changes in response were recorded and the nectin 2-nectin-3 trans-binding inhibition rate was calculated according to the formula described below.

$$\text{Nectin 2-nectin 3 trans-binding inhibition rate (\%)} = (A-B) \times 100/A$$

A: Response when control antibody was used

B: Response when the anti-nectin-2 human monoclonal antibody was used

The nectin 2-nectin 3 trans-binding inhibitory activities of the respective anti-nectin-2 human monoclonal antibodies are collectively shown in TABLE 5.

EXAMPLE 6

Cell growth inhibitory activity of anti-nectin-2 human monoclonal antibodies against OV-90 human ovarian cancer cell line

The *in vitro* growth inhibitory activity of respective anti-nectin-2 human monoclonal antibodies obtained in EXAMPLE 1 against human OV-90 ovarian cancer cell line was assayed by the method described below. For culture of the OV-90 cell line, an equivolume medium mixture of MCDB 105 (Sigma) and Medium 199 (Sigma), supplemented with 15% FBS (JRH) was used. The cells were seeded in a 10 cm tissue culture Petri dish (Becton Dickinson) at a cell density of 4.5 x $10^5$/dish every other day and incubated for subculture at 37°C in a 5% carbon dioxide gas flow. The OV-90 cell line was detached from the Petri dish by treating with 400 U of Collagenase N-2 (Nitta Gelatin) at 37°C for 2 minutes, followed by further treatment with 2 mL of Cell Dissociation Buffer (Invitrogen) at 37°C for 15 minutes. The cell suspension thus obtained was centrifuged (1000 rpm, 3 minutes) and the recovered cells were resuspended in an equivolume medium mixture of MCDB105 and Medium 199, supplemented with 1% FBS at a density of 3 x $10^4$ cells/mL.

The solutions of the anti-nectin-2 human monoclonal antibodies obtained in EXAMPLE 1, the anti-nectin-2 polyclonal antibody (N2-No. 1) prepared in REFERENCE EXAMPLE 14 and control human antibody (Human IgG Whole molecule Chrom Pure; Jackson ImmunoResearch Laboratories) in PBS, which were prepared to be 300 μg/mL, or PBS was added to a 96-well culture plate (Becton Dickinson) by 10 μL each/well, and the OV-90 cell suspension described above was added by 100 μL each/well. In addition, for measurement of the background level for calculation of the cell growth inhibition rate described below, wells containing 100 μL of the same medium and 10 μL of PBS were prepared. After incubating the plate at 37°C for 6 days in a 5% carbon dioxide gas flow, a WST-8 solution (Cell Counting Kit-8; Dojindo) as a cell growth assay reagent was added by 10 μL each/well. After the plate was incubated at 37°C for an hour in a 5% carbon dioxide gas flow, absorbance (450 nm) of each well resulting from the formazan produced was measured with a plate reader (Multiskan BICHROMATIC) and the OV-90 cell line growth inhibition rate was calculated according to the formula below.

$$\text{Cell growth inhibition rate} = [(A-B)-(C-B)] \times 100/(A-B)$$

A: Absorbance of the well to which control human antibody was added
B: Absorbance of the well to which PBS is added (OV90 cell line is not added)
C: Absorbance of the well to which the anti-nectin-2 human monoclonal antibody or the anti-nectin-2 rabbit polyclonal antibody

In the anti-nectin-2 human monoclonal antibodies obtained, some antibodies showed a strong cell growth inhibitory activity or a weak cell growth inhibitory activity against the OV-90 cell line. Moreover, the anti-nectin-2 rabbit polyclonal antibody (N2-No. 1) exhibited about 10% of growth inhibitory activity against the OV-90 cell line at the final concentration of 30 μg/mL. The *in vitro* OV-90 cell line growth inhibition rates of respective anti-nectin-2 human monoclonal antibodies (final concentration of 30 μg/mL) are collectively shown in TABLE 6.

The 30 antibodies which showed a relatively strong *in vitro* OV-90 cell line growth inhibitory activity in the primary screening described above were again prepared by incubation of the respective hybridomas, followed by purifications with Protein A column chromatography and gel filtration HPLC. Using the resulting highly purified antibody samples, the concentration-dependent (100, 30, 10, 3, 1, 0.3 and 0.03 μg/mL) cell growth inhibitory activity against the OV-90 cell line was assayed by the procedure described above. As a result, eight anti-nectin-2 human monoclonal antibodies (Nec1-803-2, Nec1-520-1, Nec1-530-1, Nec1-845-2, Nec1-834-1, Nec1-244-3, Nec1-303-2 and Nec1-903-1) were selected as exhibiting the strong OV-90 cell line growth inhibitory activity concentration-dependently with good reproductivity. These antibodies exhibited the same activity even when the OV-90 cell line was engrafted on a 96-well cell culture plate followed by addition of the antibody solution thereto. The OV-90 cell line growth inhibitory activity of the eight anti-nectin-2 human monoclonal antibodies described above is collectively shown in TABLE 7.

TABLE 3

| name | EC50 nM | name | EC50 nM | name | EC50 nM |
|---|---|---|---|---|---|
| Nec1-102-1 | 0.44 | Nec1-555-5 | 1.48 | Nec1-1142-1 | 4.45 |
| Nec1-103-1 | 0.13 | Ned-568-1 | 0.62 | Nec1-1150-2 | 0.69 |
| Nec1-105-1 | 1.78 | Nec1-608-1 | 0.60 | Nec1-1163-2 | 0.98 |
| Nec1-108-2 | 2.48 | Nec1-610-2 | 0.29 | Nec1-1202-5 | 0.71 |

(continued)

| name | EC50 nM | name | EC50 nM | name | EC50 nM |
|---|---|---|---|---|---|
| Nec1-111-3 | 0.51 | Nec1-614-5 | 0.95 | Nec1-1203-1 | 0.49 |
| Nec1-119-1 | 0.17 | Nec1-631-10 | 0.91 | Nec1-1204-4 | 10.77 |
| Nec1-124-2 | 0.90 | Nec1-704-1 | 9.91 | Nec1-1209-8 | 0.96 |
| Nec1-133-2 | 0.75 | Nec1-716-6 | 0.66 | Nec1-1212-7 | 0.63 |
| Nec1-141-3 | 1.38 | Nec1-718-2 | 2.15 | Nec1-1214-5 | 0.82 |
| Nec1-144-1 | 10.95 | Nec1-726-1 | 2.07 | Nec1-1216-1 | 1.02 |
| Nec1-145-2 | 0.18 | Nec1-730-4 | 0.54 | Nec1-1218-3 | 7.22 |
| Nec1-202-1 | 0.37 | Nec1-738-2 | 0.39 | Nec1-1232-2 | 0.43 |
| Nec1-205-1 | 0.48 | Nec1-740-2 | 0.81 | Nec1-1234-1 | 0.95 |
| Nec1-206-2 | 0.92 | Nec1-749-1 | 3.62 | Nec1-1236-1 | 1.84 |
| Noc1-208-1 | 0.51 | Nec1-755-5 | 0.40 | Nec1-1239-2 | 1.33 |
| Nec1-209-2 | 0.26 | Nec1-758-8 | 11.85 | Nec1-1302-2 | 0.53 |
| Nec1-213-2 | 9.06 | Nec1-759-9 | 0.33 | Nec1-1314-3 | 85.64 |
| Nec1-215-3 | 1.19 | Nec1-765-1 | 2.68 | Nec2-1409-12 | 5.06 |
| Nec1-217-2 | 5.40 | Nec1-769-2 | 0.16 | Nec2-1411-1 | 17.31 |
| Nec1-226-3 | 0.29 | Nec1-773-7 | 0.36 | Nec2-1422-1 | 47.54 |
| Nec1-231-1 | 3.92 | Nec1-803-2 | 0.23 | Nec2-1613-3 | 1.69 |
| Nec1-233-1 | 2.35 | Nec1-812-4 | 1.86 | Nec2-1625-4 | 0.35 |
| Nec1-235-1 | 2.07 | Nec1-815-1 | 0.46 | Nec2-1633-4 | 0.63 |
| Nec1-244-3 | 6.49 | Nec1-818-4 | 2.18 | Nec3-1829-2 | 8.01 |
| Nec1-259-1 | 0.13 | Nec1-819-2 | 0.68 | Nec3-1907-1 | 0.26 |
| Nec1-301-2 | 1.82 | Nec1-821-3 | 1.74 | Nec3-1908-4 | 1.11 |
| Nec1-302-1 | 2.49 | Nec1-831-4 | 4.26 | Nec3-1927-3 | 0.96 |
| Nec1-303-2 | 26.77 | Nec1-834-1 | 5.74 | Nec3-1932-1 | 0.71 |
| Nec1-304-2 | 0.16 | Nec1-835-1 | 3.38 | Nec3-2006-1 | 1.57 |
| Nec1-308-2 | 0.36 | Nec1-842-2 | 0.80 | Nec3-2025-3 | 0.41 |
| Nec1-311-2 | 1.35 | Nec1-843-1 | 0.46 | Nec3-2036-6 | 0.45 |
| Nec1-313-1 | 0.47 | Nec1-845-2 | 0.53 | Nec3-2109-2 | 1.08 |
| Nec1-316-1 | 0.80 | Nec1-847-2 | 0.50 | Nec3-2123-1 | 1.60 |
| Nec1-319-2 | 0.17 | Nec1-868-7 | 0.59 | Nec3-2134-1 | 0.72 |
| Nec1-320-1 | 1.46 | Nec1-869-6 | 0.59 | Nec3-2213-1 | 1.21 |
| Nec1-322-5 | 0.17 | Nec1-878-1 | 1.58 | Nec5-326-1 | 0.54 |
| Nec1-326-7 | 2.33 | Nec1-888-11 | 4.42 | Nec5-532-1 | 4.61 |
| Nec1-332-1 | 2.07 | Nec1-903-1 | 0.21 | Nec5-617-7 | 1.80 |
| Nec1-333-1 | 1.78 | Nec1-907-1 | 0.22 | Nec5-1906-6 | 0.41 |
| Nec1-336-2 | 0.14 | Nec1-908-2 | 1.41 | Nec5-2309-1 | 0.37 |
| Nec1-338-1 | 0.81 | Nec1-909-1 | 1.25 | Nec6-151-4 | 0.82 |
| Nec1-341-10 | 7.95 | Nec1-914-1 | 0.19 | Nec6-505-2 | 0.47 |

(continued)

| name | EC50 nM | name | EC50 nM | name | EC50 nM |
|---|---|---|---|---|---|
| Nec1-349-1 | 0.29 | Nec1-917-2 | 0.18 | Nec6-940-7 | 3.10 |
| Nec1-372-2 | 0.19 | Nec1-918-2 | 22.64 | Nec6-947-4 | 1.31 |
| Nec1-410-3 | 0.67 | Nec1-919-1 | 0.37 | Nec8-3330-1 | 0.61 |
| Nec1-411-1 | 0.66 | Nec1-920-1 | 0.79 | Nec8-3350-1 | 0.33 |
| Nec1-416-1 | 1.00 | Nec1-927-14 | 0.53 | Nec8-3410-1 | 0.34 |
| Nec1-427-2 | 0.35 | Nec1-928-1 | 0.23 | Nec8-3424-1 | 1.05 |
| Nec1-428-1 | 0.58 | Nec1-929-2 | 0.71 | Nec8-3517-11 | 0.25 |
| Nec1-445-4 | 1.41 | Nec1-930-1 | 4.05 | Nec8-3523-2 | 0.51 |
| Nec1-458-6 | 3.39 | Nec1-938-1 | 3.73 | Nec8-3524-14 | 0.30 |
| Nec1-460-1 | 4.42 | Nec1-938-2 | 2.09 | Nec8-3669-4 | 0.50 |
| Nec1-464-1 | 3.92 | Nec1-940-1 | 2.21 | Nec8-3704-7 | 0.88 |
| Nec1-470-2 | 1.75 | Nec1-948-3 | 1.31 | Nec8-3717-4 | 0.96 |
| Nec1-501-1 | 0.33 | Nec1-964-1 | 0.36 | Nec8-3723-3 | 0.25 |
| Nec1-503-8 | 0.42 | Nec1-1004-2 | 0.69 | Nec8-3734-1 | 0.38 |
| Nec1-505-3 | 117.40 | Nec1-1005-2 | 2.32 | Nec8-3906-2 | 1.32 |
| Nec1-506-1 | 0.73 | Nec1-1008-1 | 10.97 | Nec8-3814-17 | 0.19 |
| Nec1-507-1 | 0.27 | Nec1-1012-1 | 0.12 | Nec8-3823-5 | 0.40 |
| Nec1-508-2 | 0.56 | Nec1-1020-1 | 0.60 | Nec8-3833-6 | 0.87 |
| Nec1-520-1 | 0.39 | Nec1-1021-1 | 0.27 | Nec8-3941-4 | 0.45 |
| Nec1-521-3 | 10.60 | Nec1-1036-5 | 1.59 | Nec8-4024-5 | 0.47 |
| Nec1-522-2 | 1.00 | Nec1-1039-3 | 0.72 | Nec8-4111-2 | 0.20 |
| Nec1-526-1 | 0.27 | Nec1-1044-4 | 2.05 | Nec8-4116-8 | 0.36 |
| Nec1-528-2 | 0.62 | Nec1-1085-1 | 7.70 | Nec8-4144-2 | 0.28 |
| Nec1-530-1 | 0.79 | Nec1-1115-2 | 0.71 | Nec8-4188-1 | 0.98 |
| Nec1-538-3 | 1.10 | Nec1-1128-1 | 0.87 | Nec8-4244-8 | 0.48 |
| Nec1-546-5 | 1.58 | Nect-1132-2 | 1.58 | Nec8-4315-1 | 0.78 |
| Nec1-549-4 | 0.38 | Nec1-1138-1 | 4.07 | Nec8-4324-5 | 0.29 |
| Nec1-554-1 | 1.11 | Nec1-1139-1 | 0.58 | | |

TABLE 3A

| Name | subtype H chain/L chain | name | subtype H chain/L chain | name | subtype H chain/L chain |
|---|---|---|---|---|---|
| Nec1-102-1 | G1/k | Nec1-555-5 | G4/k | Nec1-1138-1 | G1/k |
| Nec1-103-1 | G1/k | Nec1-568-1 | G1/k | Nec1-1139-1 | G4/k |
| Nec1-105-1 | G2/k | Nec1-608-1 | G1/k | Nec1-1142-1 | G4/k |
| Nec1-108-2 | G1/k | Nec1-610-2 | G1/k | Nec1-1150-2 | G1/k |
| Nec1-111-3 | G1/k | Nec1-614-5 | G1/k | Nec1-1163-2 | G1/k |
| Nec1-119-1 | G1/k | Nec1-631-10 | G2/k | Nec1-1202-5 | G1/k |

(continued)

| Name | subtype H chain/L chain | name | subtype H chain/L chain | name | subtype H chain/L chain |
|---|---|---|---|---|---|
| Nec1-124-2 | G1/k | Nec1-704-1 | G1/k | Nec1-1203-1 | G1/k |
| Nec1-133-2 | G4/k | Nec1-716-6 | G1/k | Nec1-1204-4 | G1/k |
| Nec1-141-3 | G1/k | Nec1-718-2 | G1/k | Nec1-1209-8 | G1/k |
| Nec1-144-1 | G1/k | Nec1-726-1 | G1/k | Nec1-1212-7 | G4/k |
| Nec1-145-2 | G1/k | Nec1-730-4 | G1/k | Nec1-1214-5 | G1/k |
| Nec1-202-1 | G1/k | Nec1-738-2 | G1/k | Nec1-1216-1 | G1/k |
| Nec1-205-1 | G1/k | Nec1-740-2 | G1/k | Nec1-1218-7 | G2/k |
| Nec1-206-2 | G1/k | Nec1-755-5 | G1/k | Nec1-1232-2 | G1/k |
| Nec1-208-1 | G1/k | Nec1-758-8 | G1/k | Nec1-1234-1 | G1/k |
| Nec1-209-2 | G1/k | Nec1-759-9 | G2/k | Nec1-1236-1 | G1/k |
| Nec1-213-2 | G1/k | Nec1-765-1 | G2/k | Nec1-1239-2 | G1/k |
| Nec1-215-3 | G1/k | Nec1-769-2 | G1/k | Nec1-1302-2 | G1/k |
| Nec1-217-2 | G1/k | Nec1-773-7 | G4/k | Nec1-1305-1 | G1/k |
| Nec1-231-1 | G1/k | Nec1-803-3 | G1/k | Nec1-1314-3 | G2/k |
| Nec1-235-1 | G4/k | Nec1-812-4 | G1/k | Nec2-1409-12 | G1/k |
| Nec1-244-3 | G1/k | Nec1-815-1 | G1/k | Nec2-1411-1 | G1/k |
| Nec1-259-1 | G1/k | Nec1-818-4 | G2/k | Nec2-1422-1 | G4/k |
| Nec1-301-2 | G1/k | Nec1-819-2 | G1/k | Nec2-1613-3 | G2/k |
| Nec1-302-1 | G1/k | Nec1-821-3 | G1/k | Nec2-1625-4 | G4/k |
| Nec1-303-2 | G1/k | Nec1-831-4 | G1/k | Nec2-1633-4 | G1/k |
| Nec1-304-2 | G1/k | Nec1-834-1 | G1/k | Nec3-1829-2 | G4/k |
| Nec1-308-2 | G1/k | Nec1-835-1 | G1/k | Nec3-1907-1 | G4/k |
| Nec1-313-1 | G1/k | Nec1-842-2 | G1/k | Nec3-1908-4 | G1/k |
| Nec1-316-1 | G1/k | Nec1-843-1 | G1/k | Nec3-1927-3 | G4/k |
| Nec1-319-2 | G1/k | Nec1-845-2 | G1/k | Nec3-1932-1 | G4/k |
| Nec1-320-1<br>Nec1-322-5 | G1/k<br>G1/k | Nec1-847-2<br>Nec1-868-7 | G1/k<br>G4/k | Nec3-2006-1<br>Nec3-2025-3 | G1/k<br>G1/k |
| Nec1-326-7 | G1/k | Nec1-869-6 | G1/k | Nec3-2036-6 | G1/k |
| Nec1-332-1 | G1/k | Nec1-878-1 | G1/k | Nec3-2109-2 | G2/k |
| Nec1-333-1 | G1/k | Nec1-888-11 | G1/k | Nec3-2123-1 | G1/k |
| Nec1-336-2 | G4/k | Nec1-903-1 | G1/k | Nec3-2134-1 | G4/k |
| Nec1-338-1 | G2/k | Nec1-907-1 | G1/k | Nec3-2213-1 | G1/k |
| Nec1-341-10 | G1/k | Nec1-908-2 | G1/k | Nec5-323-2 | G4/k |
| Nec1-349-1 | G1/k | Nec1-909-1 | G1/k | Nec5-326-1 | G2/k |
| Nec1-411-1 | G4/k | Nec1-914-1 | G1/k | Nec5-532-1 | G1/k |
| Nec1-416-1 | G2/k | Nec1-917-2 | G1/k | Nec5-617-7 | G4/k |
| Nec1-427-2 | G4/k | Nec1-918-2 | G1/k | Nec5-2309-7 | G2/k |

(continued)

| Name | subtype H chain/L chain | name | subtype H chain/L chain | name | subtype H chain/L chain |
|---|---|---|---|---|---|
| Nec1-428-1 | G1/k | Nec1-919-1 | G1/k | Nec6-505-2 | G4/k |
| Nec1-445-4 | G1/k | Nec1-920-1 | G1/k | Nec6-940-7 | G4/k |
| Nec1-458-6 | G1/k | Nec1-927-14 | G4/k | Nec8-3350-1 | G4/k |
| Nec1-460-1 | G4/k | Nec1-930-1 | G1/k | Nec8-3410-1 | G1/k |
| Nec1-464-1 | G1/k | Nec1-938-2 | G1/k | Nec8-3517-11 | G1/k |
| Nec1-470-2 | G1/k | Nec1-940-2 | G1/k | Nec8-3523-3 | G1/k |
| Nec1-501-1 | G4/k | Nec1-948-3 | G1/k | Nec8-3524-14 | G1/k |
| Nec1-503-8 | G4/k | Nec1-964-1 | G1/k | Nec8-3669-4 | G1/k |
| Nec1-505-3 | G2/k | Nec1-1004-2 | G1/k | Nec8-3704-7 | G1/k |
| Nec1-506-1 | G1/k | Nec1-1005-2 | G4/k | Nec8-3717-4 | G4/k |
| Nec1-507-1 | G1/k | Nec1-1008-1 | G4/k | Nec8-3723-3 | G1/k |
| Nec1-508-2 | G4/k | Nec1-1012-1 | G2/k | Nec8-3734-1 | G2/k |
| Nec1-520-1 | G1/k | Nec1-1020-1 | G4/k | Nec8-3806-2 | G2/k |
| Nec1-522-2 | G2/k | Nec1-1021-2 | G1/k | Nec8-3814-17 | G1/k |
| Nec1-526-1 | G4/k | Nec1-1036-5 | G1/k | Nec8-3823-5 | G1/k |
| Nec1-528-2 | G1/k | Nec1-1039-3 | G1/k | Nec8-3833-6 | G4/k |
| Nec1-530-2 | G1/k | Nec1-1044-4 | G1/k | Nec8-4024-5 | G1/k |
| Nec1-538-3 | G2/k | Nec1-1085-1 | G1/k | Nec8-4116-8 | G1/k |
| Nec1-546-5 | G1/k | Nec1-1115-2 | G1/k | Nec8-4144-2 | G4/k |
| Nec1-549-4 | G4/k | Nec1-1132-2 | G1/k | Nec8-4188-1 | G4/k |
| Nec1-554-1 | G1/k | | | | |

TABLE 4

| name | epitope | name | epitope | name | epitope |
|---|---|---|---|---|---|
| Nec1-102-1 | VI | Nec1-555-5 | IV | Nec1-1142-1 | IV |
| Nec1-103-1 | VI | Nec1-568-1 | VII | Nec1-1150-2 | VI |
| Nec1-105-1 | VI | Nec1-608-1 | VII | Nec1-1163-2 | VI |
| Nec1-108-2 | VI | Nec1-610-2 | V | Nec1-1202-5 | V |
| Nec1-111-3 | VI | Nec1-614-5 | VII | Nec1-1203-1 | I |
| Nec1-119-1 | VII | Nec1-631-10 | V | Nec1-1204-4 | I |
| Nec1-124-2 | VII | Nec1-704-1 | VI | Nec1-1209-8 | I |
| Nec1-133-2 | V | Nec1-716-6 | V | Nec1-1212-7 | VII |
| Nec1-141-3 | VI | Nec1-718-2 | V | Nec1-1214-5 | VI |
| Nec1-144-1 | VI | Nec1-726-1 | IV, | Nec1-1216-1 | V |
| Nec1-145-2 | VII | Nec1-730-4 | VI | Nec1-1218-7 | IV |
| Nec1-202-1 | I | Nec1-738-2 | VI | Nec1-1232-2 | VII |
| Nec1-205-1 | V | Nec1-740-2 | V | Nec1-1234-1 | I |

(continued)

| name | epitope | name | epitope | name | epitope |
|---|---|---|---|---|---|
| Nec1-206-2 | VII | Nec1-749-1 | ND | Nec1-1236-1 | IV |
| Nec1-208-1 | V | Nec1-755-5 | VI | Nec1-1239-2 | IV |
| Nec1-209-2 | VI | Nec1-758-8 | n | Nec1-1302-2 | IV |
| Nec1-213-2 | III | Nec1-759-9 | VI | Nec1-1305-1 | V |
| Nec1-215-3 | IV | Nec1-765-1 | VI | Nec1-1314-3 | VI |
| Nec1-217-2 | III | Nec1-769-2 | V | Nec2-1409-12 | IV |
| Nec1-226-3 | V | Nec1-773-7 | VII | Nec2-1411-1 | ND |
| Noc1-231-1 | IV | Nec1-803-2 | VI | Nec2-1422-1 | ND |
| Nec1-233-1 | ND | Nec1-812-4 | IV | Nec2-1613-3 | IV |
| Nec1-235-1 | V | Nec1-815-1 | VII | Nec2-1625-4 | VI |
| Nec1-244-3 | VI | Nec1-818-4 | VI | Nec2-1633-4 | VII |
| Nec1-259-1 | VI | Nec1-819-2 | VII | Nec3-1829-2 | VI |
| Nec1-301-2 | V | Nec1-821-3 | I | Nec3-1907-1 | V |
| Nec1-302-1 | I | Nec1-831-4 | IV | Nec3-1908-4 | VI |
| Nec1-303-2 | IV | Nec1-834-1 | VI | Nec3-1927-3 | V |
| Nec1-304-2 | VII | Nec1-835-1 | IV | Nec3-1932-1 | V |
| Nec1-308-2 | VII | Nec1-842-2 | I | Nec3-2006-1 | VII |
| Nec1-311-2 | ND | Nec1-843-1 | VI | Nec3-2025-3 | V |
| Nec1-313-1 | VI | Nec1-845-2 | VI | Nec3-2036-6 | VII |
| Nec1-316-1 | VI | Nec1-847-2 | VI | Nec3-2109-2 | V |
| Nec1-319-2 | VI | Nec1-868-7 | VII | Nec3-2123-1 | V |
| Nec1-320-1 | I | Nec1-869-8 | VI | Nec3-2134-1 | VII |
| Nec1-322-5 | V | Nec1-878-1 | V | Nec3-2213-1 | V |
| Nec1-326-7 | IV | Nec1-888-11 | III | Nec5-328-1 | V |
| Nec1-332-1 | VI | Nec1-903-1 | VI | Nec5-532-1 | IV |
| Necl-333-1 | IV | Necl-907-1 | VII | Nec5-617-7 | VII |
| Nec1-336-2 | VII | Nec1-908-2 | VII | Nec5-1906-6 | I |
| Nec1-338-1 | VII | Nec1-909-1 | VI | Nec5-2309-7 | I |
| Nec1-341-10 | IV | Nec1-914-1 | VII | Nec6-151-4 | ND |
| Nec1-349-1 | VII | Nec1-917-2 | VII | Nec6-505-2 | VI |
| Nec1-372-2 | V | Nec1-918-2 | VI | Nec6-940-7 | IV |
| Nec1-410-3 | ND | Nec1-919-1 | V | Nec6-947-4 | ND |
| Nec1-411-1 | VII | Nec1-920-1 | V | Nec8-3734-1 | I |
| Nec1-416-1 | I | Nec1-927-14 | VII | Nec8-3330-1 | V |
| Nec1-427-2 | VII | Nec1-928-1 | V | Nec8-3350-1 | VII |
| Nec1-428-1 | V | Nec1-929-2 | V | Nec8-3410-1 | VII |
| Nec1-445-4 | IV | Nec1-930-1 | III | Nec8-3424-1 | V |
| Nec1-458-6 | IV | Nec1-938-1 | ND | Nec8-3517-11 | VII |

(continued)

| name | epitope | name | epitope | name | epitope |
|---|---|---|---|---|---|
| Nec1-460-1 | II | Nec1-938-2 | III | Nec8-3523-3 | V |
| Nec1-464-1 | II | Nec1-940-2 | IV | Nec8-3524-14 | VII |
| Nec1-470-2 | IV | Nec1-948-3 | VI | Nec8-3669-4 | VII |
| Nec1-501-1 | VII | Nec1-964-1 | I | Nec8-3704-7 | VII |
| Nec1-503-8 | VII | Nec1-1004-2 | V | Nec8-3717-4 | V |
| Nec1-505-3 | VI | Nec1-1005-2 | IV | Nec8-3723-3 | VII |
| Nec1-508-1 | V | Nec1-1008-1 | IV | Nec8-3806-2 | VII |
| Nec1-507-1 | V | Nec1-1012-1 | V | Nec8-3814-17 | VII |
| Nec1-508-2 | VI | Nec1-1020-1 | VII | Nec8-3823-5 | V |
| Nec1-520-1 | VI | Nec1-1021-2 | V | Nec8-3833-6 | VII |
| Nec1-521-3 | ND | Nec1-1036-5 | I | Nec8-3941-4 | VI |
| Nec1-522-2 | VI | Nec1-1039-3 | I | Nec8-4024-5 | VI |
| Nec1-526-1 | VII | Nec1-1044-4 | IV | Nec8-4111-2 | V |
| Nec1-528-2 | V | Nec1-1085-1 | II | Nec8-4116-8 | VII |
| Nec1-530-1 | VI | Nec1-1115-2 | I | Nec8-4144-2 | VII |
| Nec1-538-3 | VI | Nec1-1128-1 | ND | Nec8-4188-1 | VII |
| Nec1-546-5 | V | Nec1-1132-2 | V | Nec8-4244-8 | V |
| Nec1-549-4 | VII | Nec1-1138-1 | IV | Nec8-4315-1 | V |
| Nec1-554-1 | IV | Nec1-1139-1 | VII | Nec8-4324-5 | V |

TABLE 5

| name | trans binding inhibition (%) | name | trans binding inhibition (%) |
|---|---|---|---|
| Nec1-102-1 | 31 | Nec1-803-2 | 91 |
| Nec1-111-3 | 44 | Nec1-818-4 | 97 |
| Nec1-209-2 | 88 | Nec1-834-1 | 86 |
| Nec1-244-3 | 35 | Nec1-843-1 | 89 |
| Nec1-303-2 | 6 | Nec1-845-2 | 86 |
| Nec1-313-1 | 12 | Nec1-869-6 | 80 |
| Nec1-316-1 | 56 | Nec1-903-1 | 85 |
| Nec1-319-2 | 70 | Nec1-918-2 | 73 |
| Nec1-332-1 | 93 | Nec1-1115-2 | 13 |
| Nec1-505-3 | 8 | Nec1-1128-1 | 23 |
| Nec1-506-1 | 83 | Nec1-1150-2 | 82 |
| Nec1-508-2 | 92 | Nec1-1209-8 | 8 |
| Nec1-530-1 | 36 | Nec1-1214-5 | 6 |
| Nec1-704-1 | 23 | Nec3-2025-3 | 91 |
| Nec1-730-4 | 43 | Nec5-323-2 | 60 |
| Nec1-738-2 | 79 | Nec6-505-2 | 86 |

(continued)

| name | trans binding inhibition (%) | name | trans binding inhibition (%) |
|---|---|---|---|
| Nec1-755-5 | 84 | Nec8-4024-5 | 93 |
| Nec1-765-1 | 5 | | |

TABLE 6

| name | growth inhibition mean(%) (vs OV-90) | name | growth inhibition mean(%) (vs OV-90) |
|---|---|---|---|
| Nec1-102-1 | 6.7 | Nec1-834-1 | 14.5 |
| Nec1-141-3 | 11.2 | Nec1-843-1 | 9.5 |
| Nec1-144-1 | 9.9 | Nec1-845-2 | 12.3 |
| Nec1-209-2 | 9.7 | Nec1-878-1 | 17.7 |
| Nec1-244-3 | 16.7 | Nec1-888-11 | 8.3 |
| Nec1-259-1 | 12.4 | Nec1-903-1 | 16.9 |
| Nec1-303-2 | 18.4 | Nec1-908-2 | 14.7 |
| Nec1-311-2 | 12.3 | Nec1-909-1 | 16.9 |
| Nec1-313-1 | 7.1 | Nec1-918-2 | 15.0 |
| Nec1-316-1 | 13.5 | Nec1-938-2 | 9.4 |
| Nec1-319-2 | 12.9 | Nec1-1085-1 | 6.5 |
| Nec1-332-1 | 8.4 | Nec1-111-3 | 11.2 |
| Nec1-505-3 | 9.8 | Nec1-1150-2 | 13.0 |
| Nec1-508-2 | 6.2 | Nec1-1163-2 | 5.7 |
| Nec1-520-1 | 10.0 | Nec1-1204-4 | 11.6 |
| Nec1-530-1 | 16.9 | Nec1-1209-8 | 13.2 |
| Nec1-555-5 | 11.3 | Nec1-1214-5 | 7.3 |
| Nec1-631-10 | 15.0 | Nec1-1314-3 | 12.8 |
| Nec1-730-4 | 9.2 | Nec2-1625-4 | 7.2 |
| Nec1-738-2 | 21.2 | Nec5-323-2 | 10.2 |
| Nec1-749-1 | 16.5 | Nec6-505-2 | 6.6 |
| Nec1-755-5 | 12.7 | Nec8-3704-7 | 16.9 |
| Nec1-758-8 | 6.3 | Nec8-3941-4 | 10.5 |
| Nec1-765-1 | 7.6 | Nec8-4024-5 | 13.4 |
| Nec1-803-2 | 13.3 | Nec8-4116-8 | 14.1 |
| Nec1-818-4 | 5.3 | | |

TABLE 7

| name | Antibody conc. (μg/ml) | growth inhibition (%) | name | Antibody conc. (μg/ml) | growth inhibition (%) |
|---|---|---|---|---|---|
| Nec1-244-3 | 100ug/mL<br>30ug/ml<br>10ug/mL | 9.2<br>7.0<br>6.9 | Nec1-803-2 | 100ug/mL<br>30ug/ml<br>10ug/mL | 17.4<br>14.3<br>16.8 |

(continued)

| name | Antibody conc. (μg/ml) | growth inhibition (%) | name | Antibody conc. (μg/ml) | growth inhibition (%) |
|---|---|---|---|---|---|
| | 3ug/ml | 7.0 | | 3ug/ml | 13.5 |
| | 1 ug/mL | 4.9 | | 1 ug/mL | 15.5 |
| | 0.3ug/ml | 2.0 | | 0.3ug/ml | 15.1 |
| | 0.03ug/ml | -1.3 | | 0.03ug/ml | 3.8 |
| Nec1-303-2 | 100ug/mL | 6.2 | Nec1-834-1 | 100ug/mL | 11.8 |
| | 30ug/ml | 6.9 | | 30ug/ml | 12.9 |
| | 10ug/mL | 5.5 | | 10ug/mL | 8.3 |
| | 3ug/ml | 3.0 | | 3ug/ml | 9.6 |
| | 1 ug/mL | 5.1 | | 1ug/mL | 12.8 |
| | 0.3ug/ml | -2.9 | | 0.3ug/ml | 0.8 |
| | 0.03ug/ml | -7.4 | | 0.03ug/ml | 2.9 |
| Nec1-520-1 | 100ug/mL | 8.5 | Nec1-845-1 | 100ug/mL | 15.7 |
| | 30ug/ml | 11.3 | | 30ug/ml | 8.3 |
| | 10ug/mL | 13.7 | | 10ug/mL | 10.3 |
| | 3ug/ml | 9.3 | | 3ug/ml | 5.8 |
| | 1 ug/mL | 7.0 | | 1 ug/mL | 11.8 |
| | 0.3ug/ml | 1.2 | | 0.3ug/ml | 4.6 |
| | 0.03ug/ml | 2.0 | | 0.03ug/ml | -3.2 |
| Nec1-530-1 | 100ug/mL | 13.7 | Nec1-903-1 | 100ug/mL | 5.8 |
| | 30ug/ml | 9.6 | | 30ug/ml | 7.3 |
| | 10ug/mL | 8.2 | | 10ug/mL | 2.8 |
| | 3ug/ml | 10.3 | | 3ug/ml | 3.9 |
| | 1 ug/mL | 8.2 | | 1 ug/mL | 4.5 |
| | 0.3ug/ml | 0.5 | | 0.3ug/ml | -1.3 |
| | 0.03ug/ml | -4.6 | | 0.03ug/ml | -6.3 |

EXAMPLE 7

ADCC (Antibody-dependent cellular cytotoxicity) of anti-nectin-2 human monoclonal antibodies

In the anti-nectin-2 human monoclonal antibodies prepared in EXAMPLE 1, Nec1-803-2, Nec8-4116-8, Nec1-520-1, Nec1-530-1, Nec1-845-2, Nec8-3941-4, Nec1-834-1, Nec1-244-3, Nec1-918-2, Nec8-3806-1, Nec1-303-2 and Nec1-903-1 were applied to an ADCC measurement. The human ovarian cancer cell line OV-90 was used as target cells and commercially available frozen human peripheral blood mononuclear cells (Asahi Techno Glass Corp.) which had been cultured overnight in RPMI 1640 medium (Invitrogen) supplemented with 10 nM recombinant human IL-2 (DIACLONE Research Inc.), 55 μM 2-mercaptoethanol (Invitrogen) and 10% FBS (JRH) were used as effector cells, respectively.

The OV-90 cell line in the logarithmic growth phase was recovered by the procedure described in EXAMPLE 6. The one million cells were labeled with $^{51}Cr$ by adding 250 μCi of $Na_2{}^{51}CrO_4$ (Amersham Biosciences, which name was changed to GE Healthcare Bio-sciences), followed by incubation at 37°C for an hour. These cells were washed 4 times with 0.4% BSA (Invitrogen)-containing RPMI 1640 medium (hereinafter referred to as 0.4% BSA/RPMI medium) and then resuspended at 1 x $10^5$ cells/mL in 0.4% BSA/RPMI medium. Then, 100 μL (1 x $10^4$ cells) of this target cell suspension and 50 μL solution in which the anti-nectin-2 human monoclonal antibodies described above were diluted in 0.4% BSA/RPMI medium in final concentrations of 0.015 μg/mL, 0.15 μg/mL and 1.5 μg/mL were added to each well of a 96-well RMC plate (BIOBIK). The same volume of non-immune human IgG (final concentration of 1.5 μg/mL) or D-PBS (Invitrogen) was added as a negative control. After incubating these plates on ice for an hour, 5 x $10^5$ cells each/well of the effector cell suspension described above was added (effector cells : target cells = 50 : 1), which was reacted at 37°C for 4 hours in a 5% carbon dioxide gas flow. The cell suspension in each well was transferred to a 96-well multiscreen 45 μm (Millipore) and centrifuged to recover the culture supernatants. The radioactivity (sample release) leaked out of the cells in these culture supernatants was measured using γ counter (AccuFLEX γ7000; Aloka Co.). The maximum cytotoxic activity (maximum release) of ADCC was defined as the radioactivity detected in the culture supernatant when Triton-X 100 (Sigma) was added to be a final concentration of 1%, whereas the spontaneous release activity (spontaneous release) was defined as the radioactivity detected in the culture supernatant when 10% FBS-supplemented RPMI 1640 medium was added in place of the effector cells, respectively. The specific lysis (%) as an indicator of the ADCC intensity was calculated by ([sample release]-[spontaneous release])/([maximum release]-[spontaneous release]) x 100 (TABLE 8). ADCC (specific lysis (%)) was both 17% when non-immune human IgG (final concentration of 1.5 μg/mL) or D-PBS was added, whereas ADCC of the anti-nectin-2 human monoclonal antibodies Nec1-803-2, Nec8-4116-8, Nec1-520-1, Nec1-530-1, Nec1-845-2, Nec8-3941-4, Nec1-834-1 and Nec1-903-1, which belong to the subclass $IgG_1$, showed percentages as significantly high as 35%, 34%, 30%, 27%, 30%, 34%, 31% and 32%, respectively, at 1.5 μg/mL. In particular, the antibody Nec8-4116-8 demonstrated more potent ADCC than the other anti-nectin-2 human monoclonal antibodies, represented by the specific lysis of 33% even at the final antibody concentration of 0.015 μg/mL.

TABLE 8

| Antibody | Antibody conc. (μg/ml) | Specific lysis (%) |
|---|---|---|
| non-immune IgG | 1.5 | 17 |
| No Ab | - | 17 |
| Nec1-803-2 | 0.015<br>0.15<br>1.5 | 23<br>31<br>35 |
| Nec8-4116-8 | 0.015<br>0.15<br>1.5 | 33<br>33<br>34 |
| Nec1-520-1 | 0.015<br>0.15<br>1.5 | 21<br>27<br>30 |
| Nec1-530-1 | 0.015<br>0.15<br>1.5 | 19<br>25<br>27 |
| Nec1-845-2 | 0.015<br>0.15<br>1.5 | 22<br>28<br>30 |
| Nec8-3941-4 | 0.015<br>0.15<br>1.5 | 23<br>32<br>34 |
| Nec1-834-1 | 0.015<br>0.15<br>1.5 | 19<br>26<br>31 |
| Nec1-244-3 | 0.015<br>0.15<br>1.5 | 16<br>22<br>24 |
| Nec1-918-2 | 0.015<br>0.15<br>1.5 | 21<br>22<br>25 |
| Nec8-3806-1 | 0.015<br>0.15<br>1.5 | 16<br>20<br>22 |
| Nec1-303-2 | 0.015<br>0.15 | 21<br>26 |

(continued)

| Antibody | Antibody conc. (μg/ml) | Specific lysis (%) |
|---|---|---|
| | 1.5 | 24 |
| Nec1-903-1 | 0.015<br>0.15<br>1.5 | 24<br>33<br>32 |
| Spontaneous release | - | 0 |
| Maximum release | - | 100 |

EXAMPLE 8

Additional preparation of anti-nectin-2 human monoclonal antibodies

In addition to the antibodies prepared in EXAMPLE 1, KM mice were immunized by the following procedure to prepare anti-nectin-2 human monoclonal antibodies additionally. In other words, an emulsion prepared by mixing the nectin-2ED-Fc protein (1.6 mg/mL PBS solution) prepared in REFERENCE EXAMPLE 16 with an equivalent volume of Freund's complete adjuvant (Difco) was administered subcutaneously and intracutaneously to five KM mice (10 weeks old, 12 weeks old, male; Kirin Brewery Co., Ltd.) at a dose of 50 μg/mouse. Two weeks after, the protein emulsion prepared using Freund's incomplete adjuvant (Difco) was administered subcutaneously and intracutaneously to the mice at a dose of 25 μg/mouse, followed by further immunization of the same protein emultion at a dose of 10 μg/mouse after interval of two weeks.

Prior to the first immunization and one week after the third immunization, blood was collected from the ocular fundus of all the mice under ethereal anesthesia to prepare antisera, and the antibody titer in the sera was determined in a manner similar to the procedure described in EXAMPLE 1. Nectin-2ED-Fc was administered in a dose of 10 μg each/mouse by tail vein injection to the KM mice of which a sufficient increase in the serum antibody titer was confirmed, as a final booster. Anti-nectin-2 human monoclonal antibody-producing hybridomas were additionally obtained from the mice, and the antibody was prepared from the culture supernatant thereof by the same procedure as described in EXAMPLE 1.

EXAMPLE 9

Cross-reactivity of anti-nectin-2 human monoclonal antibodies to the nectin-1, nectin-3, nectin-4 and Necl-5 proteins

In order to confirm the specificity of the anti-nectin-2 human monoclonal antibody prepared in EXAMPLE 1 to human nectin-2, the cross-reactivity to the human nectin family proteins (Nectin-1, Nectin-3 and Nectin-4) and human Nec1-5 was examined by flow cystometry (hereinafter referred to as FCM) using cells transiently expressing these proteins. Specifically, CHO-K1 cell line was incubated in a T75 flask charged with a medium mixture of DMEM and F-12 in equivalent volumes (Invitrogen) supplmented with 10% FBS (JRH) at 37°C for 1 to 2 days in a 5% carbon dioxide gas flow. When the cells became about 90% confluent, pEE12.4-Nectin-2δ prepared in REFERENCE EXAMPLE 18, pCMV-Tag4-Nectin-1, pCMV-Tag4-Nectin-3, pCMV-Tag4-Nectin-4 and pCMV-Tag4-Necl-5 prepared in REFERENCE EXAMPLE 29 and pcDNA3.1(+) (Invitrogen) as a negative control plasmid were transfected using Lipofectamine 2000 (Invitrogen), according to the protocol attached. Four hours after the transfection, the medium for the CHO-K1 cells was exchanged with a fresh aliquot of the medium described above, followed by incubation for further 2 days. These cells were washed twice with D-PBS, detached by Cell Dissociation Buffer, enzyme-free, PBS-based (Invitrogen) and resuspended in D-PBS (-) containing 1%FBS and 0.1% sodium azide (hereinafter referred to as FCM buffer) at a density of 5 x $10^6$ cells/mL. These cell suspensions were added to a 96-well V-bottom plate by 30 μL each, and 20 μL each of anti-human nectin-2 human monoclonal antibody (Nec1-803-2, Nec1-520-1, Nec1-530-1, Nec1-834-1, Nec1-244-3, Nec1-303-2, Nec1-903-1 or Nec8-4116-8) and positive control antibodies [anti-human nectin-1 mouse monoclonal antibody (ZYMED), anti-nectin-2 rabbit polyclonal antibody N2-No. 2 prepared in REFERENCE EXAMPLE 14, anti-human nectin-3 rabbit polyclonal antibody prepared in REFERENCE EXAMPLE 27, anti-human nectin-4 goat polyclonal antibody (R & D) or anti-human Nec1-5 mouse monoclonal antibody (LAB VISION)] diluted in FCM buffer to 15 μg/mL (final concentration: 6 μg/mL) were added thereto, followed by reacting them on ice for an hour. After 200 μL of FCM buffer was added to each well and washed once by centrifugal operation, 50 μL/well of Alexa488-labeled secondary antibody diluted in FCM buffer to 10 μg/mL was added for suspension, which was then reacted on ice for an hour. The secondary antibody used was Alexa Fluor488 goat anti-human IgG (H+L) when human antibody was used as the primary antibody, Alexa Fluor488 goat anti-mouse IgG (H+L) when mouse antibody was used as the primary antibody, Alexa Fluor488 goat anti-rabbit IgG (H+L) when rabbit antibody was used as the primary antibody, and Alexa Fluor488 donkey anti-goat IgG (H+L) (Invitrogen) when goat antibody was used as the primary antibody, respectively. After these cells were further washed twice with FCM buffer and then resuspended in 250 μL of FCM buffer, fluorescence intensity of the stained cells was measured by the flow cytometer MPL500 (BECKMAN COULTER). A ratio of the median value of fluorescence intensities of negative control plasmid pcDNA3.1(+)-transfected CHO-K1 cells to the median value of fluorescence intensities of each gene-transfected CHO-K1 cells was calculated for each antibody. The results are shown in TABLE 9. When this ratio is 1, it is meant that the antibody does not bind at all to the protein, whereas the larger the ratio, the stronger the antibody binds to the protein. These results reveal that the eight anti-human nectin-2 human monoclonal antibodies provided for this experiment are human nectin-2 specific antibodies which don't cross-react to the other human nectin family proteins (nectin-1, nectin-3 and nectin-4) or human Necl-5.

TABLE 9

| | Nectin-1 | Nectin-2 | Nectin-3 | Nectin-4 | Necl-5 |
|---|---|---|---|---|---|
| Positive control antibody | 79.9 | 151.6 | 10.7 | 3.7 | 496.8 |
| Nec1-803-2 | 1.2 | 75.0 | 1.2 | 1.1 | 1.1 |
| Nec1-520-1 | 1.1 | 62.6 | 1.1 | 1.2 | 1.0 |
| Nec1-530-1 | 1.0 | 66.7 | 1.2 | 1.1 | 1.0 |
| Nec1-834-1 | 1.2 | 63.1 | 1.2 | 1.1 | 1.0 |
| Nec1-244-3 | 1.2 | 57.5 | 1.2 | 1.2 | 1.1 |
| Nec1-303-2 | 1.0 | 11.1 | 1.0 | 0.9 | 0.9 |
| Nec1-903-1 | 1.2 | 71.9 | 1.2 | 1.1 | 1.2 |
| Nec8-4116-8 | 1.3 | 71.7 | 1.1 | 1.1 | 1.0 |

EXAMPLE 10

Nectin-2-nectin-2 trans-binding inhibitory activity of anti-nectin-2 human monoclonal antibodies

The nectin-2-nectin-2 trans-binding inhibitory activities of the anti-nectin-2 human monoclonal antibodies obtained in EXAMPLES 1 and 8 were quantitatively assessed by the following method utilizing time-resolved fluorescence spectroscopy. First, 1 mg of the nectin-2ED-Fc protein prepared in REFERENCE EXAMPLE 16 was concentrated and replaced with 50 mM sodium carbonate buffer (pH 9.6) by means of ultrafiltration to prepare 4 mg/mL of the solution. After adding Eu-labeling Reagent attached to DELFIA Eu-Labeling Kit (Perkin Elmer) to this nectin-2ED-Fc solution, the mixture was reacted at 4°C overnight. The unreacted $Eu^{3+}$ was removed from the reaction mixture by the aforesaid ultrafiltration and at the same time, the buffer was replaced with PBS to prepare Eu-labeled nectin-2ED-Fc. The number of $Eu^{3+}$ introduced in this case was 5.23 molecules per molecule of nectin-2-ED-Fc. Next, nectin-2-ED-Fc was diluted in 50 mM sodium carbonate buffer (pH 9.6) to a concentration of 5 μg/mL, and the dilution was added to a Wallac Delfia plate (Perkin Elmer) by 100 μL each/well, which was then reacted at room temperature for 5 hours. Thereafter, 200 μL each of PBS containing 2% BSA was added to each well for blocking overnight at 4°C. After washing the plate twice with PBS containing 0.05% Tween 20 (PBS-T), anti-nectin-2 human monoclonal antibody or Control hIgG (Human IgG Whole molecule Chrom Pure; Jackson ImmunoResearch Laboratories) diluted in PBS containing 0.2% BSA to 600 μg/mL was mixed with an equivalent volume ofEu-labeled nectin-2ED-Fc diluted in PBS containing 0.2% BSA to 6.4 μg/mL, which was added by 100 μL each/well, followed by reacting at room temperature for 1.5 hours. After this plate was washed 6 times with PBS-T, 200 μL each/well of Enhancement Solution (Perkin Elmer) was added, followed by stirring at room temperature for a minute with a plate mixer. The fluorescence of each well thus reacted was measured at 615 nm (excitation light: 340 nm, delayed time: 400 μsecs.) using ARVO 1420 Multilabel Counter (Perkin Elmer), and the nectin-2-nectin-2 trans-binding inhibition rate was calculated by the following formula.

$$\text{Nectin-2-nectin-2 trans-binding inhibition rate (\%)} = (A - B) \times 100/A$$

A: Count when control hIgG is used

B: Count when anti-nectin-2 human monoclonal antibody is used

The nectin-2-nectin-2 trans-binding inhibitory activities of the respective anti-nectin-2 human monoclonal antibodies are collectively shown in TABLE 10.

TABLE 10

| Name | Nectin-2-Nectin-2 trans-binding inhibition (%) |
|---|---|
| Nec1-244-3 | 28 |
| Nec1-259-1 | 24 |
| Nec1-303-2 | 19 |
| Nec1-316-1 | 30 |
| Nec1-631-10 | 78 |
| Nec1-738-3 | 38 |
| Nec1-740-1 | 71 |
| Nec1-834-1 | 40 |
| Nec1-878-1 | 69 |
| Nec1-918-2 | 34 |
| Nec1-1115-2 | 7 |
| Nec1-1150-2 | 48 |
| Nec1-1209-8 | 28 |
| Nec8-3717-4 | 70 |
| Nec8-3823-4 | 79 |
| Nec8-4024-5 | 30 |
| Nec8-4111-2 | 76 |
| Control hIgG | 0 |

EXAMPLE 11

ADCC (Antibody-dependent cellular cytotoxicity) of anti-nectin-2 human monoclonal antibodies

In the anti-nectin-2 human monoclonal antibodies prepared in EXAMPLE 1, Nec1-964-1 of Group I, Nec1-303-1, Nec1-554-1 and Nec1-1302 of Group IV, Nec1-769-2 and Nec1-1305-1 of Group V, Nec1-141-3, Nec1-209-2, Nec1-909-1, Nec1-847-2 and Nec1-803-2 of Group VI, Nec8-4116-8 of Group VII were applied to an ADCC measurement. The human ovarian cancer cell line OV-90 was used as target cells and commercially available frozen human peripheral blood mononuclear cells (Asahi Techno Glass Corp.) which had been cultured overnight in RPMI 1640 medium (Invitrogen) supplemented with 0.1 nM recombinant human IL-2 (DIACLONE Research Inc.), 55 μM 2-mercaptoethanol (Invitrogen) and 10% FBS (JRH) was used as effector cells, respectively.

The OV-90 cell line in the logarithmic growth phase was recovered by the procedure described in EXAMPLE 6. The one million cells were labeled with $^{51}$Cr by adding 250 μCi of $Na_2{}^{51}CrO_4$ (GE Healthcare Bio-sciences), followed by incubation at 37°C for an hour. These cells were washed 4 times with 0.4% BSA (Invitrogen)-containing RPMI 1640 medium (hereinafter referred to as 0.4% BSA/RPMI medium) and resuspended at 1 x $10^5$ cells/mL in 0.4% BSA/RPMI medium. Then, 100 μL (1 x $10^4$ cells) of this target cell suspension and 50 μL solution in which the anti-nectin-2 human monoclonal antibodies described above were diluted in 0.4% BSA/RPMI medium in final concentrations of 0.0015 μg/mL, 0.015 μg/mL, 0.15 μg/mL and 1.5 μg/mL were added to each well of a 96-well RMC plate (BIOBIK). The same volume of non-immune human IgG (final concentration of 1.5 μg/mL) or D-PBS (Invitrogen) was added as a negative control. After incubating these plates on ice for an hour, 5 x $10^5$ cells each/well of the effector cell suspension described above was added (effector cells : target cells = 50 : 1), which was reacted at 37°C for 4 hours in a 5% carbon dioxide gas flow. The cell suspension in each well was transferred to a 96-well multiscreen 45 μm (Millipore) and centrifuged to recover the culture supernatants. The radioactivity (sample release) leaked out of the cells in these culture supernatants was measured using γ counter (AccuFLEX γ7000; Aloka Co.). The maximum cytotoxic activity (maximum release) of ADCC was defined as the radioactivity detected in the culture supernatant when Triton-X 100 (Sigma) was added to be a final concentration of 1%, whereas the spontaneous release activity (spontaneous release) was defined as the radioactivity detected in the culture supernatant when 10% FBS-supplemented RPMI 1640 medium was added in place of the effector cells, respectively. The specific lysis (%) as an indicator of the ADCC intensity was calculated by ([Sample release]-[Spontaneous release])/([Maximum release]-[Spontaneous release]) x 100 (TABLES 11 and 12).

ADCC (specific lysis (%)) was 4% or 3%, respectively, when non-immune human IgG (final concentration of 1.5 μg/mL) or D-PBS was added, whereas ADCC of the anti-nectin-2 human monoclonal antibodies: Nec1-964-1 of Group I, Nec1-554-1, Nec1-1302-2 and Nec1-769-2 of Group IV, Nec1-803-2 of Group VI, and Nec8-4116-8 of Group VII, showed the rates as significantly high as 12%, 19%, 15%, 12%, 15% and 17%, respectively, at 1.5 μg/mL. In particular, the antibody Nec1-554-1 of Group IV and Nec8-4116-8 of Group VII exhibited more potent ADCC than the other anti-nectin-2 human monoclonal antibodies, represented by the specific lysis was 15% and 12%, respectively, at the final antibody concentration of 0.15 μg/mL..

TABLE 11

| Antibody | Antibody (μg/ml) | Specific lysis (%) |
|---|---|---|
| Non-immune IgG | 1.5 | 4 |
| D-PBS | - | 3 |
| Nec1-964-1 | 0.0015<br>0.015<br>0.15<br>1.5 | -3<br>2<br>8<br>12 |
| Nec1-303-1 | 0.0015<br>0.015<br>0.15<br>1.5 | -1<br>1<br>0<br>3 |
| Nec1-554-1 | 0.0015<br>0.015<br>0.15<br>1.5 | 0<br>7<br>15<br>19 |
| Nec1-1302-2 | 0.0015<br>0.015<br>0.15<br>1.5 | 3<br>3<br>7<br>15 |
| Nec1-769-2 | 0.0015<br>0.015<br>0.15<br>1.5 | 4<br>5<br>10<br>12 |
| Nec1-1305-1 | 0.0015<br>0.015<br>0.15 | 3<br>4<br>4 |

TABLE 12

| Antibody | Antibody (μg/ml) | Specific lysis (%) |
|---|---|---|
| Nec1-1305-1 | 1.5 | 5 |
| Nec1-141-3 | 0.0015<br>0.015<br>0.15<br>1.5 | -3<br>-2<br>-3<br>-2 |

(continued)

| Antibody | Antibody (μg/ml) | Specific lysis (%) |
|---|---|---|
| Nec1-209-2 | 0.0015 0.015<br>0.15<br>1.5 | -3 -1<br>2<br>4 |
| Nec1-909-1 | 0.0015<br>0.015<br>0.15<br>1.5 | -1<br>-1<br>0<br>3 |
| Nec1-847-2 | 0.0015<br>0.015<br>0.15<br>1.5 | 1<br>1<br>8<br>11 |
| Nec1-803-2 | 0.0015<br>0.015<br>0.15<br>1.5 | 0<br>3<br>8<br>15 |
| Nec8-4116-8 | 0.0015<br>0.015<br>0.15<br>1.5 | 2<br>1<br>12<br>17 |

EXAMPLE 12

*In vivo* anti-tumor activity of anti-nectin-2 human monoclonal antibodies

In the anti-nectin-2 human monoclonal antibodies obtained in EXAMPLE 1, the anti-tumor activities of Nec1-803-2, Nec1-964-1, Nec1-303-2, Nec1-554-1SP3, Nec1-1302-2, Nec1-769-2, Nec1-1305-1, Nec1-141-3, Nec1-209-2, Nec1-909-1 and Nec1-847-2 were evaluated in nude mice models subcutaneously-transplanted with the OV-90 human ovarian cancer cell line. The anti-nectin-2 human monoclonal antibody samples used were those prepared by the procedure described in REFERENCE EXAMPLE 28. The OV-90 cell line was seeded on a 150 $cm^2$ tissue culture flask (Corning) using an equivolume medium mixture of MCDB105 (Sigma) and Medium 199 (Sigma), supplemented with 15% FBS (JRH), and incubated at 37°C in a 5% carbon dioxide gas flow. The OV-90 cell line suspension harvested at the logarithmic growth phase by detachment treatment with trypsin-EDTA, was washed 3 times with Hank's balanced salt solution (HBSS) (Invitrogen) using centrifugal operation (1,000 rpm, 3 minutes). The cells thus obtained were resuspended in HBSS at a density of 8 x $10^7$ cells/mL.

After nude mice (BALB/cAJc1-nu/nu) (5 weeks old, female), purchased from Nippon Crea, were tamed for a week, the OV-90 cell suspension described above was inoculated subcutaneously into the ventral area at a dose of 100 μL each/animal. Ten days after the cell inoculation, the long diameter and short diameter of the OV-90 tumor mass were measured with calipers and the tumor volume was calculated according to the formula described below.

$$\text{Tumor volume (mm}^3\text{)} = \text{long diameter x (short diameter)}^2/2$$

From the nude mice transplanted with the OV-90 cell line, the mice in which engrafted tumor mass was observed were selected, and the weight of each animal was measured. The mice were grouped such that a mean tumor mass volume of each group was equivalent (about 50 $mm^3$). On Days 10, 13, 17, 20, 24, 27 and 31 after the cell inoculation, the anti-nectin-2 human monoclonal antibody solution diluted in PBS to 0.15 mg/mL or PBS was intravenously administered at 10 mL each/kg through the tail vein and at the same time, the tumor volume was measured by the procedure described above. The growth inhibitory activity of the anti-nectin-2 human monoclonal antibody was assessed by calculating T/C (Treatment/Control) value based on the tumor volume 4 weeks after commencement of drug administration, according to the formula below. For the significance test between the administration groups, the parametric Dunnett multiple comparison test (SAS preclinical package Version 5.0) was used.

T/C (%) = [(Increased tumor volume in the antibody group from commencement of drug administration)/( Increased tumor volume in the PBS group from commencement of drug administration)] x 100

In the anti-nectin-2 human monoclonal antibodies described above, some antibodies strongly suppressed the growth of OV-90 cell line tumor mass and some antibodies weakly suppressed the growth. The T/C of the respective anti-nectin-2 human monoclonal antibodies and the significance test values (P values) against the PBS group are collectively shown in TABLE 13.

TABLE 13

| | T/C (%) | *P* value |
|---|---|---|
| Nec1-803-2 | 47 | 0.0113 |
| Nec1-964-1 | 62 | |
| Nec1-303-2 | 102 | |
| Nec1-554-1SP3 | 34 | 0.0028 |
| Nec1-1302-2 | 63 | |
| Nec1-769-2 | 83 | |
| Nec1-1305-1 | 104 | |
| Nec1-141-3 | 72 | |
| Nec1-209-2 | 94 | |
| Nec1-909-1 | 74 | |
| Nec1-847-2 | 81 | |

EXAMPLE 13

Analysis of binding domains of anti-nectin-2 human monoclonal antibodies

As one of epitope search methods for the anti-nectin-2 human monoclonal antibodies, the reactivities with the Ig1 domain (47th-142nd of the amino acid sequence represented by SEQ ID NO: 1 or SEQ ID NO: 3)-deficient protein and of Ig2 domain (175th-240th of the amino acid sequence represented by SEQ ID NO: 1 or SEQ ID NO: 3)-deficient protein of nectin-2 were examined by FCM using the CHO-K1 cells wherein these proteins were transiently expressed. Specifically, suspensions of CHO-K1 cells, in which pEE12.4-Nectin-2δ prepared in REFERENCE EXAMPLE 18 and pcDNA3. 1(+)-Nectin-2ΔIg1 and pcDNA3.1(+)-Nectin-2ΔIg2 prepared in REFERENCE EXAMPLE 30, or pcDNA3.1(+) as a negative control were transiently expressed, were prepared by the same procedure as in EXAMPLE 9. These cell suspensions were added to each well of a 96-well V-bottom plate by 30 μL each, and several anti-nectin-2 human monoclonal antibodies prepared in EXAMPLE 1 or the anti-nectin-2 rabbit polyclonal antibody N2-No. 2 prepared in REFERENCE EXAMPLE 14, which was diluted in FCM buffer to 15 μg/mL, was added thereto by 20 μL (final concentration: 6 μg/mL) each, followed by reaction on ice for an hour. After 200 μL of FCM buffer was added to each well and washed once by centrifugal operation, 50 μL each ofAlexa488-labeled secondary antibody diluted in FCM buffer to 10 μg/mL was added to and mixed therewith, and the mixture was then reacted on ice for an hour. The Alexa488-labeled secondary antibody used was Alexa Fluor488 goat anti-human IgG (H+L) for the human antibodies and Alexa Fluor488 goat anti-rabbit IgG (H+L) (Invitrogen) for the rabbit antibody. The cells in each well were washed twice with FCM buffer and then resuspended in 250 μL of FCM buffer. Using the flow cytometer MPL500 (BECKMAN COULTER), the fluorescence intensity of stained cells was measured to determine the ratio of the median value of fluorescence intensities of the respective antibodies in the primary antibody group to the median value of fluorescence intensities in the negative control group. The ratio was expressed in terms of the reactivity with each of the gene-transfected CHO-K1 cells and shown in TABLES 14 and 15. In the tables, the reactivities with the pcDNA3.1(+)-Nectin-2ΔIg1-transfected cells and pcDNA3.1(+)-Nectin-2ΔIg2-transfected cells are expressed as delta-Ig1 and delta-Ig2, respectively. When the ratio to the negative control is 2 or more, it is defined that there is a reactivity, whereas when the ratio to delta-Ig1 was 2 or more and the ratio to delta-Ig2 is 2 or less, the antibody was judged to recognize the Ig2 domain of nectin-2; and when the ratio to delta-Ig1 was 2 or less and the ratio to delta-Ig2 is 2 or more, the antibody was judged to recognize the Ig1 domain of nectin-2. The binding domain (epitope domain) of an antibody showing 2 or less in the two ratios described above was listed as "unknown."

The results suggested that the antibodies belonging to epitope groups IV would recognize the Ig2 domain of nectin-2. The results also suggested that the antibodies belonging to epitope groups V and VI would recognize the Ig1 domain of nectin-2.

TABLE 14

| Name | delta-Ig1 | delta-Ig2 | Epitope domain |
|---|---|---|---|
| None | 1.0 | 1.1 | |
| Polyclonal Ab | 4.5 | 8.9 | |
| Nec1-102-1 | 1.0 | 4.6 | Ig1 |
| Nec1-141-3 | 1.0 | 2.6 | Ig1 |
| Nec1-202-1 | 1.0 | 1.4 | unknown |
| Nec1-244-3 | 1.0 | 2.9 | Ig1 |
| Nec1-308-2 | 1.1 | 1.1 | unknown |
| Nec1-313-1 | 1.0 | 1.0 | unknown |
| Nec1-333-1 | 4.2 | 1.1 | Ig1 |
| Nec1-410-3 | 2.9 | 1.3 | Ig2 |
| Nec1-460-1 | 0.8 | 2.8 | Ig1 |
| Nec1-464-1 | 1.0 | 3.5 | Ig1 |
| Nec1-503-3 | 1.0 | 1.0 | unknown |
| Nec1-738-2 | 1.0 | 5.1 | Ig1 |
| Nec1-755-5 | 1.0 | 4.8 | Ig1 |
| Nec1-803-2 | 1.0 | 5.3 | Ig1 |
| Nec1-888-11 | 1.0 | 1.0 | unknown |
| Nec1-909-1 | 1.0 | 3.5 | Ig1 |
| Nec1-919-1 | 1.0 | 6.9 | Ig1 |
| Nec1-930-1 | 1.0 | 1.1 | unknown |
| Nec1-964-1 | 1.1 | 1.1 | unknown |
| Nec1-1044-4 | 2.2 | 0.8 | Ig2 |
| Nec1-1302-2 | 4.1 | 1.1 | Ig2 |
| Nec6-151-4 | 1.0 | 1.0 | unknown |
| Nec8-3410-1 | 1.0 | 1.3 | unknown |
| Nec8-3823-5 | 0.9 | 3.0 | Ig1 |
| Nec8-4116-8 | 1.0 | 1.0 | unknown |

TABLE 15

| Name | delta-Ig1 | delta-Ig2 | Epitope domain |
|---|---|---|---|
| Polyclonal Ab | 7.8 | 10.6 | |
| None | 1.0 | 1.0 | |
| Nec1-554-1 SP3 | 4.3 | 1.0 | Ig2 |
| Nec1-1236-1 SP3 | 4.5 | 1.1 | Ig2 |
| Nec1-215-8 SP3 | 4.4 | 1.1 | Ig2 |
| Nec1-333-3 SP3 | 4.2 | 1.1 | Ig2 |
| Nec1-1044-4 | 2.9 | 1.1 | Ig2 |
| Nec1-554-1 | 3.6 | 1.0 | Ig2 |
| Nec1-215-8 | 4.3 | 1.1 | Ig2 |
| Nec1-1236-1 | 4.4 | 1.2 | Ig2 |
| Nec1-1613-3 | 5.1 | 1.1 | Ig2 |
| Nec1-333-3 | 3.6 | 1.1 | Ig2 |
| Nec1-326-7 | 1.1 | 1.3 | Unknown |
| Nec1-1138-1 | 4.0 | 1.0 | Ig2 |
| Nec1-1218-7 | 2.5 | 1.1 | Ig2 |
| Nec1-803-2 | 0.9 | 6.2 | Ig1 |
| Nec1-244-3 | 1.0 | 4.8 | Ig1 |
| Nec8-4116-8 | 0.9 | 1.0 | Unknown |
| Nec1-103-1 | 1.0 | 1.3 | Unknown |
| Nec1-1302-2 | 3.5 | 1.2 | Ig2 |
| Nec1-1005-2 | 2.0 | 0.8 | Ig2 |

EXAMPLE 14

Cross-reactivity of anti-nectin-2 monoclonal antibodies with cynomolgus monkey nectin-2

Cross-reactivities of the anti-nectin-2 human monoclonal antibodies prepared in EXAMPLE 1 with the cynomolgus monkey nectin-2 were examined by FCM using the CHO-K1 cells wherein the cynomolgus monkey nectin-2 was transiently expressed. By the same procedure as in EXAMPLE 9, the suspensions of CHO-K1 cells, in which pEE12.4-Nectin-2δ prepared in REFERENCE EXAMPLE 18 and pcDNA3. 1(+)-maNectin-2 prepared in REFERENCE EXAMPLE 32, or pcDNA3.1(+) as a negative control were transiently expressed, were prepared. These cell suspensions were added to each well of a 96-well V-bottom plate by 30 μL each, and several anti-human nectin-2 human monoclonal antibodies prepared in EXAMPLE 1 or the anti-nectin-2 rabbit polyclonal antibody N2-No. 2 prepared in REFERENCE EXAMPLE 14 as a positive control antibody, diluted in FCM buffer to 15 μg/mL was added by 20 μL each (final concentration: 6 μg/mL), followed by reaction on ice for an hour. Then, after 200 μL of FCM buffer was added to each well and washed once by centrifugal operation, 50 μL each of Alexa488-labeled secondary antibody diluted in FCM buffer to 10 μg/mL was added thereto for suspension, and the mixture was then reacted on ice for an hour. As secondary antibodies, Alexa Fluor488 goat anti-human IgG (H+L) was used for the human antibodies and Alexa Fluor488 goat anti-rabbit IgG (H+L) (Invitrogen) was used for the rabbit antibody, respectively. Next, the cells were washed twice with FCM buffer and then resuspended in 250 μL of FCM buffer. Using the flow cytometer MPL500 (BECKMAN COULTER), the fluorescence intensity of stained cells was measured to confirm the binding reactivities of respective antibodies. For the respective antibodies, the ratio of the median value of fluorescence intensities in the primary antibody group to the median value of fluorescence intensities in the negative control group is shown in TABLE 16. The results revealed that in the anti-human nectin-2 human monoclonal antibodies prepared in EXAMPLE 1 there were the antibodies which were cross-reactive to and not to cynomolgus monkey nectin-2. Nec1-554-1 belonging to Epitope Group IVb, Nec1-319-2 and Nec1-843-1 belonging to Epitope Group VI and Nec8-4116-8 belonging to Epitope Group VII showed the cross-reactivity to the cynomolgus monkey nectin-2. To the contrary, Nec1-111-3, Nec1-144-1, Nec1-209-2, Nec1-244-3, Nec1-316-1, Nec1-332-1, Nec1-520-1, Nec1-530-1, Nec1-704-1, Nec1-730-4, Nec1-803-2, Nec1-834-1, Nec1-843-1, Nec1-845-2, Nec1-903-1, Nec1-909-1, Nec1-918-2, Nec1-1214-5 and Nec8-4024-5 belonging to Epitope Group VI showed no cross-reactivity to the cynomolgus monkey nectin-2.

TABLE 16

| | Human Nectin-2 | Cynomolgus monkey Nectin-2 |
|---|---|---|
| No primary antibody | 0.8 | 0.9 |
| Positive control antibody | 18.7 | 15.8 |
| Nec1-111-3 | 29.9 | 1.3 |
| Nec1-144-1 | 37.9 | 1.2 |
| Nec1-209-2 | 45.8 | 1.6 |
| Nec1-244-3 | 24.6 | 1.1 |
| Nec1-316-1 | 38.4 | 1.1 |
| Nec1-319-2 | 47.8 | 4.2 |
| Nec1-332-1 | 51.7 | 1.3 |
| Nec1-520-1 | 25.4 | 1.0 |
| Nec1-530-1 | 40.5 | 1.0 |
| Nec1-554-1 | 43.0 | 14.8 |
| Nec1-704-1 | 22.2 | 1.3 |
| Nec1-730-4 | 36.3 | 1.4 |
| Nec1-803-2 | 48.6 | 1.1 |
| Nec1-834-1 | 49.5 | 1.1 |
| Nec1-843-1 | 47.6 | 3.1 |
| Nec1-845-2 | 42.9 | 1.7 |
| Nec1-903-1 | 43.9 | 1.1 |
| Nec1-909-1 | 43.7 | 1.7 |
| Nec1-918-2 | 28.3 | 1.3 |
| Nec1-1214-5 | 45.4 | 1.8 |
| Nec8-4024-5 | 59.2 | 2.0 |
| Nec8-4116-8 | 44.3 | 30.9 |

EXAMPLE 15

Cross-reactivity of anti-nectin-2 human monoclonal antibody to nectin-2 mutants introduced cynomolgus monkey-like mutation

Cross-reactivities of the anti-nectin-2 human monoclonal antibodies prepared in EXAMPLE 1 to human nectin-2 mutants introduced cynomolgus monkey-like mutation were examined by FCM using the CHO-K1 cells wherein the human nectin-2 mutants introduced cynomolgus monkey-like mutation were transiently expressed. By the same procedure as in EXAMPLE 9, the suspensions of CHO-K1 cells, in which pEE12.4-Nectin-2δ prepared in REFERENCE EXAMPLE 18 and the animal cell expression vector for human nectin-2 mutants introduced cynomolgus monkey-like mutation; pcDNA3.1(+)-Nectin-2 (AN77-78PD), pcDNA3.1(+)-Nectin-2 (G113R) or pcDNA3.1(+)-Nectin-2 (H128R) prepared in REFERENCE EXAMPLE 33, and pcDNA3.1(+) as a negative control were transiently expressed, were prepared. These cell suspensions were added to each well of a 96-well V-bottom plate by 30 μL each, and several anti-human nectin-2 human monoclonal antibodies prepared in EXAMPLE 1 diluted in FCM buffer to 15 μg/mL was added by 20 μL each (final concentration: 6 μg/mL), followed by reaction on ice for an hour. Then, after 200 μL of FCM buffer was added to each well and washed once by centrifugal operation, 50 μL of Alexa Fluor488 goat anti-human IgG (H+L) diluted in FCM buffer to 10 μg/mL was added thereto for suspension, and the mixture was then reacted on ice for an hour. Next, the cells were washed twice with FCM buffer and resuspended in 250 μL of FCM buffer. Using flow cytometer MPL500 (BECKMAN COULTER), the fluorescence intensities of stained cells were measured, respectively. The ratio of the median value of fluorescence intensities in the primary antibody group to the median value of fluorescence intensities of the respective antibodies in the negative control group is shown in TABLE 17. The results revealed that Nec1-111-3, Nec1-209-2, Nec1-244-3, Nec1-316-1, Nec1-332-1, Nec1-520-1, Nec1-530-1, Nec1-704-1, Nec1-730-4, Nec1-803-2, Nec1-834-1, Nec1-843-1, Nec1-845-2, Nec1-903-1, Nec1-909-1, Nec1-918-2, Nec1-1214-5 and Nec8-4024-5 belonging to Group VI bound equally to G113R and H128R, but did not bind to AN77-78PD. The results suggest the possibility that these human monoclonal antibodies would recognize the region containing $Ala^{77}$ or $Asn^{78}$ of nectin-2. On the other hand, Nec1-554-1 belonging to Group IVb, Nec1-144-1, Nec1-319-2 and Nec1-843-1 belonging to Group VI, and Nec8-4116-8 belonging to Group VII bound to AN77-78PD, G113R and H128R with equal affinity to nectin-2.

TABLE 17

| | Nectin-2 | AN77-78PD | G113R | H128R |
|---|---|---|---|---|
| No primary antibody | 0.8 | 0.9 | 0.8 | 0.8 |
| Positive control antibody | 18.7 | 20.3 | 17.9 | 19.8 |
| Necl-111-3 | 29.9 | 1.4 | 28.5 | 38.6 |
| Nec1-144-1 | 37.9 | 19.3 | 30.9 | 27.2 |
| Nec1-209-2 | 45.8 | 1.9 | 41.9 | 56.8 |
| Nec1-244-3 | 24.6 | 1.1 | 23.3 | 29.6 |
| Nec1-316-1 | 38.4 | 1.2 | 37.8 | 50.0 |
| Nec1-319-2 | 47.8 | 6.9 | 43.8 | 61.1 |
| Nec1-332-1 | 51.7 | 1.6 | 43.7 | 60.6 |
| Nec1-520-1 | 25.4 | 1.0 | 22.5 | 31.6 |
| Nec1-530-1 | 40.5 | 0.9 | 36.7 | 51.5 |
| Nec1-554-1 | 43.0 | 44.2 | 38.7 | 51.5 |
| Nec1-704-1 | 22.2 | 1.4 | 19.3 | 25.3 |
| Nec1-730-4 | 36.3 | 1.0 | 32.4 | 42.2 |
| Nec1-803-2 | 48.6 | 1.1 | 41.9 | 54.9 |
| Nec1-834-1 | 49.5 | 1.2 | 45.9 | 55.8 |
| Nec1-843-1 | 47.6 | 4.1 | 43.1 | 52.4 |
| Nec1-845-2 | 42.9 | 2.3 | 40.1 | 51.9 |
| Nec1-903-1 | 43.9 | 0.9 | 38.9 | 55.8 |
| Nec1-909-1 | 43.7 | 1.4 | 32.5 | 45.5 |
| Nec1-918-2 | 28.3 | 1.4 | 26.0 | 34.3 |
| Nec1-1214-5 | 45.4 | 1.7 | 42.4 | 53.7 |
| Nec8-4024-5 | 59.2 | 2.2 | 50.7 | 71.1 |
| Nec8-4116-8 | 44.3 | 40.9 | 37.5 | 50.5 |

EXAMPLE 16

Epitope analysis of the anti-nectin-2 human monoclonal antibodies belonging to Epitope Groups V and VI

In EXAMPLE 13, it was suggested that the antibodies belonging to Epitope Groups V and VI would recognize the Ig1 domain of nectin-2. In order to identify the epitopes of these anti-nectin-2 human monoclonal antibodies in more detail, recombinant proteins were prepared by a single amino acid substitution in the Ig1 domain of Nectin-2ED-Fc, and the binding activities of anti-nectin-2 human monoclonal antibodies against these mutants were examined. The pcDNA3.1 (+)-Nectin-2ED-hFc prepared in REFERENCE EXAMPLE 15 and the animal cell expression vectors for single amino acid substituted mutants of Nectin-2ED-Fc which were prepared in REFERENCE EXAMPLE 34: pcDNA3.1(+)-Nectin-2ED-Fc (Q37A), pcDNA3.1(+)-Nectin-2ED-Fc (P40G), pcDNA3.1(+)-Nectin-2ED-Fc (Q45A), pcDNA3.1(+)-Nectin-2ED-Fc (H55A), pcDNA3.1(+)-Nectin-2ED-Fc (V60A), pcDNA3.1(+)-Nectin-2ED-Fc (Y64A), pcDNA3.1(+)-Nectin-2ED-Fc (Q71A), pcDNA3.1(+)-Nectin-2ED-Fc (A75G), pcDNA3.1(+)-Nectin-2ED-Fc (P76G), pcDNA3.1(+)-Nectin-2ED-Fc (A77G), pcDNA3.1(+)-Nectin-2ED-Fc (N78A), pcDNA3.1(+)-Nectin-2ED-Fc (H79A), pcDNA3.1(+)-Nectin-2ED-Fc (Q80A), pcDNA3.1(+)-Nectin-2ED-Fc (N81A), pcDNA3.1(+)-Nectin-2ED-Fc (K88A), pcDNA3.1(+)-Nectin-2ED-Fc (S95A), pcDNA3.1(+)-Nectin-2ED-Fc (K 109A), pcDNA3.1(+)-Nectin-2ED-Fc (E 117A), pcDNA3.1(+)-Nectin-2ED-Fc (D122A), pcDNA3.1(+)-Nectin-2ED-Fc (H128A), pcDNA3.1(+)-Nectin-2ED-Fc (N137A), pcDNA3.1(+)-Nectin-2ED-Fc (F145A), pcDNA3.1(+)-Nectin-2ED-Fc (K 147A), pcDNA3.1(+)-Nectin-2ED-Fc (V150A), pcDNA3.1(+)-Nectin-2ED-Fc (M153A) or pcDNA3.1(+)-Nectin-2ED-Fc (T154A), were transfected to the 293F cell line by using 293 Fectin (Invitrogen). These cells were rotation cultured at 37°C for 3 days in an 8% carbon dioxide gas flow to secret the Nectin-2ED-Fc protein encoded by the plasmid described above, and its single amino acid mutant proteins [Q37A, P40G, Q45A, H55A, V60A, Y64A, Q71A, A75G, P76G, A77G, N78A, H79A, Q80A, N81A, K88A, S95A, K109A, E117A, D122A, H128A, N137A, F145A, K147A, V150A, M153A, T154A] into the culture supernatant. The culture supernatant was prepared from each cell suspension by centrifugal operation and filter filtration and provided for the subsequent ELISA.

The anti-nectin-2 human monoclonal antibody Nec8-4116-8 prepared in EXAMPLE 1 was diluted in 50 mM sodium carbonate-sodium bicarbonate buffer (pH 9.6) to a concentration of 5 μg/mL. The dilution was added to a 96-well half well immunoplate (Costar) by 50 μL each/well, followed by reacting at room temperature for 5 hours. After the reaction solution was removed from each well, 100 μL each /well of PBS containing 2% BSA was added for blocking at 4°C overnight. The plate for ELISA thus prepared was washed twice with PBS containing 0.05% Tween 20, a 5-fold dilution in PBS containing 0.2 % BSA of the culture supernatant of 293F cell line, in which the Nectin-2ED-Fc protein (wild type) and the single amino acid substitution protein of Nectin-2ED-Fc were transiently expressed, was added by 50 μL each/well, followed by reaction at room temperature for 2 hours. Also, the medium for 293F cell line was added to the same plate as a negative control. After washing this plate 6 times with PBS containing 0.05% Tween 20, the biotinylated anti-nectin-2 human monoclonal antibody prepared in EXAMPLE 4 diluted in PBS containing 0.2% BSA to concentrations of 5 ng/mL to 20 ng/mL and the resulting dilution was added by 50 μL each/well, followed by reaction at room temperature for 2 hours. After washing this plate 6 times with PBS containing 0.05% Tween 20, avidin-HRP (Vector) diluted in PBS containing 0.2% BSA to 3,000-fold was added by 50 μL each/well, followed by reaction at room temperature for 2 hours. After washing this plate 6 times with PBS containing 0.05% Tween 20, TMB solution (SureBlue Microwell TMB peroxidase substrate) was added by 50 μL each/well, which was maintained at room temperature for 1 minute for color formation. Then, 2N sulfuric acid (Wako Pure Chemical) was added by 50 μL each/well to terminate the enzyme reaction. Absorbance (450 nm) of each well was measured using a plate reader (Multiskan BICHROMATIC). The reactivity of each biotinylated anti-nectin-2 human monoclonal antibody against the individual single amino acid substitution product of Nectin-2ED-Fc was calculated according to the following formula.

Reactivity (% wild type) = [absorbance (single amino acid substitution product) - absorbance (negative control)]/[absorbance (wild type) - absorbance (negative control)] x 100

In TABLE 18, the antibodies which are classified into Epitope Group VI in EXAMPLE 4 included those having decreased binding affinity to A75G, P76G and N78A and those having decreased binding affinity to A75G, P76G, N78A and N137A, suggesting that the former antibody group would recognize the epitopes containing $Ala^{75}$, $Pro^{76}$ and $Asn^{78}$ and the latter antibody group would recognize the epitopes containing $Ala^{75}$, $Pro^{76}$, $Asn^{78}$ and $Asn^{137}$. Also the antibodies classified into Epitope Group V in EXAMPLE 4 demonstrated reduction in reactivity with F145A, suggesting that these antibodies would recognize the epitopes containing $Phe^{145}$.

TABLE 18

(See attached papers)

EXAMPLE 17

Epitope analysis of anti-nectin-2 human monoclonal antibodies belonging to Epitope Groups I and VII

In order to identify the epitopes for anti-nectin-2 human monoclonal antibodies prepared in EXAMPLE 1 in more detail, recombinant proteins were prepared by a single amino acid substitution in the Ig2 domain of Nectin-2ED-Fc, and the binding activities of these proteins were examined. The pcDNA3. 1(+)-Nectin-2ED-hFc prepared in REFERENCE EXAMPLE 15 and the animal cell expression vectors for single amino acid substituted mutants of Nectin-2ED-Fc which were prepared in REFERENCE EXAMPLE 35: pcDNA3.1(+)-Nectin-2ED-Fc (Q165A), pcDNA3.1(+)-Nectin-2ED-Fc (K170A), pcDNA3.1(+)-Nectin-2ED-Fc (F173A), pcDNA3.1(+)-Nectin-2ED-Fc (P177G), pcDNA3.1(+)-Nectin-2ED-Fc (I184A), pcDNA3.1(+)-Nectin-2ED-Fc (K186A), pcDNA3.1(+)-Nectin-2ED-Fc (L197A), pcDNA3.1(+)-Nectin-2ED-Fc (W202A), pcDNA3.1(+)-Nectin-2ED-Fc (E206A), pcDNA3. 1(+)-Nectin-2ED-Fc (T212A), pcDNA3.1(+)-Nectin-2ED-Fc (T235A), pcDNA3.1(+)-Nectin-2ED-Fc (K239A) and pcDNA3.1(+)-Nectin-2ED-Fc (A249G), were transfected to the 293F cell line by using 293 Fectin (Invitrogen). These cells were rotation cultured at 37°C for 3 days in an 8% carbon dioxide gas flow to secret the Nectin-2ED-Fc protein encoded by the plasmid described above, and its single amino acid mutant proteins [Q165A, K170A, F173A, P177G, I184A, K186A, L197A, W202A, E206A, T212A, T235A, K239A, A249G] into the culture supernatant. The culture supernatant was prepared from each cell suspension by centrifugal operation and filter filtration and provided for the subsequent ELISA.

The anti-nectin-2 human monoclonal antibody Nec1-803-2 prepared in EXAMPLE 1 was diluted in 50 mM sodium carbonate-sodium bicarbonate buffer (pH 9.6) to a concentration of 5 μg/mL. The dilution was added to a 96-well half well immunoplate (Costar) by 50 μL each/well, followed by reacting at room temperature for 5 hours. After the reaction solution was removed from each well, 100 μL each /well of PBS containing 2% BSAwas added to for blocking at 4°C overnight. The plate for ELISA thus prepared was washed twice with PBS containing 0.05% Tween 20, a 25-fold or 125-fold dilution in PBS containing 0.2% BSA of the culture supernatant of 293F cell line, in which the Nectin-2ED-Fc protein (wild type) and the single amino acid substitution protein of Nectin-2ED-Fc were transiently expressed, was added by 50 μL each/well, followed by reaction at room temperature for 2 hours. Also, the medium for 293F cell line was added to the same plate as a negative control. After washing this plate 6 times with PBS containing 0.05% Tween 20, the biotinylated anti-nectin-2 antibody prepared in EXAMPLE 4 diluted in PBS containing 0.2% BSA to concentrations of 1 ng/mL to 20 ng/mL was added to the plate by 50 μL each/well, followed by reaction at room temperature for 2 hours. After washing this plate 6 times with PBS containing 0.05% Tween 20, avidin-HRP (Vector) diluted in PBS containing 0.2% BSA to 3,000-fold was added to the plate by 50 μL each/well, followed by reaction at room temperature for 2 hours. After washing this plate 6 times with PBS containing 0.05% Tween 20, TMB solution (SureBlue Microwell TMB peroxidase substrate; Kirkegaard & Perry Laboratories, Inc.) was added by 50 μL each/well, which was maintained at room temperature for 1 minute for color formation. Then, 2N sulfuric acid (Wako Pure Chemical) was added by 50 μL each/well to terminate the enzyme reaction. Absorbance (450 nm) of each well was measured using a plate reader (SPECTRAmax 340PC; Molecular Devices, Inc.). The reactivity of each biotinylated anti-nectin-2 human monoclonal antibody against the product by a single amino acid substitution of Nectin-2ED-Fc was calculated according to the formula below. The results are summarized in TABLE 19.

Reactivity (% wild type) = [absorbance (product by single amino acid substitution)-absorbance (negative control)]/[ absorbance (wild type)- absorbance (negative control)] x 100

The binding activity of Nec1-964-1 belonging to Epitope Group I to the single amino acid substitution products of Nectin-2ED-hFc markedly decreased with I184A, K186A and T212A, suggesting that this antibody would recognize the epitopes containing $Ile^{184}$, $Lys^{186}$ and $Thr^{212}$. On the other hand, the binding activity of Nec8-4116-8, Nec9-1004-1, Nec9-1236-1 and Nec9-1637-1 in the antibody group belonging to Epitope Group VII to the single amino acid substitution products of Nectin-2ED-hFc markedly decreased with F173A, suggesting that these antibodies would recognize the epitopes containing $Phe^{173}$.

TABLE 19

| Name of clone | Nec1-964-1 |
|---|---|
| Nectin 2-ED-Fc | 100 |
| Q165A | 89 |
| K170A | 95 |
| F173A | 104 |
| P177G | 104 |
| I184A | 33 |
| K186A | 16 |
| L197A | 98 |
| W202A | 102 |
| E206A | 100 |
| T212A | 21 |
| T235A | 104 |
| K239A | 104 |

EXAMPLE 18

Subgrouping of epitope groups

The anti-nectin-2 human monoclonal antibodies prepared in EXAMPLES 1 and 8 were roughly classified into 7 epitope groups as shown in EXAMPLE 4. Subsequent studies revealed that these groups were further segmented. For example, when the results of competitive inhibition tests performed in EXAMPLE 4 were examined more closely, a group of antibodies belonging to Epitope Group IV were found to show the competitive inhibition with the antibodies of Epitope Group VII, too. These antibodies are considered to be somewhat different in epitope from the antibodies belonging to Epitope Group IV ,which showed no competitive inhibition with the antibodies of Epitope Group VII, which were subgrouped into Epitope Group IVb for convenience, and the antibody group belonging to Epitope Group IV but showing no competitive inhibition with Epitope Group VII were subgrouped into Epitope Group IVa (TABLE 20). In addition, a group of antibodies belonging to Epitope Group VI showed competitive inhibition with the antibodies belonging to Epitope Group I. These antibodies are considered to recognize somewhat different epitopes from the antibodies ofEpitope Group VI but showing no competitive inhibition with Epitope Group I, and for convenience, these antibodies were subgrouped into Epitope Group VIa. Furthermore, the antibody group belonging to Epitope Group VI but showing no competitive inhibition with Epitope Group I were divided into the antibody group which demonstrated decreased binding affinity to A75G, P76G and N78A and the antibody group which demostrated decreased binding affinity to A75G, P76G, N78A and N137A, from the results in EXAMPLE 16. Therefore, for convenience, the antibody group which demonstrated decreased binding affinity to A75C, P76G and N78A was subgrouped into Epitope Group VIb, and the antibody group which demonstrated decreased binding affinity to A75G , P76G, N78A and N137A was subgrouped into Epitope Group VIc (TABLE 20). In EXAMPLE 16, the antibody Nec1-141-3 belonging to Epitope Group VIa showed substantially the same binding affinity to any amino acid substitution mutants as to the wild type, suggesting that they are different in epitope from the antibodies of Epitope Group VIb or VIc. Further in EXAMPLE 17, the antibody group showing decreased binding affinity to F173A was found in the antibodies belonging to Epitope Group VII. Thus, for convenience, the antibody group belonging to Epitope Group VII which demonstrated decreased binding affinity to F173A was subgrouped into Epitope Group VIIa, and a group of the other antibodies belonging to Epitope Group VII were subgrouped into Epitope Group VIIb (TABLE 20).

TABLE 20

| Name | Epitope group | Name | Epitope group |
|---|---|---|---|
| Nec1-102-1 | VIa | Nec1-903-1 | VIb |

(continued)

| Name | Epitope group | Name | Epitope group |
|---|---|---|---|
| Nec1-105-1 | VIa | Nec1-948-3 | VIa |
| Nec1-111-3 | IVa | Nec1-1005-2 | IVa |
| Nec1-141-3 | VIa | Nec1-1008-1 | IVa |
| Nec1-209-3 | VIc | Nec1-1044-4 | IVb |
| Nec1-215-3 | IVb | Nec1-1138-1 | IVb |
| Nec1-231-1 | IVa | Nec1-1142-1 | IVa |
| Nec1-244-3 | VIc | Nec1-1163-2 | VIa |
| Nec1-303-2 | IVa | Nec1-1218-7 | IVb |
| Nec1-313-1 | VIa | Nec1-1236-1 | IVb |
| Nec1-326-7 | IVb | Nec1-1239-2 | IVa |
| Nec1-333-1 | IVb | Nec1-1302-2 | IVa |
| Nec1-341-10 | IVa | Nec2-1409-12 | IVa |
| Nec1-445-4 | IVa | Nec2-1613-3 | IVb |
| Nec1-458-6 | IVa | Nec2-1625-4 | VIa |
| Nec1-470-2 | IVa | Nec3-1908-4 | VIa |
| Nec1-520-1 | VIb | Nec5-532-1 | IVa |
| Nec1-522-2 | VIa | Nec6-940-7 | IVa |
| Nec1-530-1 | VIb | Nec8-4116-8 | VIIa |
| Nec1-538-3 | VIa | Nec9-143-3 | VIIb |
| Nec1-554-1 | IVb | Nec9-136-1 | VIIb |
| Nec1-555-5 | IVa | Nec9-1004-1 | VIIa |
| Nec1-726-1 | IVa | Nec9-1018-1 | VIIb |
| Nec1-759-9 | VIa | Nec9-1236-1 | VIIa |
| Nec1-765-1 | VIa | Nec9-1502-2 | VIc |
| Nec1-803-2 | VIb | Nec9-1637-1 | VIIa |
| Nec1-812-4 | IVa | Nec10-1666-1 | VIb |
| Nec1-831-4 | IVa | Nec10-1707-3 | VIb |
| Nec1-834-1 | VIc | Nec10-1861-1 | VIc |
| Nec1-835-1 | IVa | Nec10-2005-1 | VIb |
| Nec1-845-2 | VIb | Nec10-2439-2 | VIc |

EXAMPLE 19

Base sequences and amino acid sequences of variable regions in anti-nectin-2 human monoclonal antibodies

The base sequences and amino acid sequences of the variable regions in anti-nectin-2 human monoclonal antibodies Nec1-244-3, Nec1-530-1, Nec1-554-1, Nec1-803-2, Nec1-834-1, Nec1-845-2, Nec1-903-1 and Nec8-4116-8 prepared in EXAMPLE 1 were determined by the following procedures. Specifically, cDNA was prepared by using SuperScriptIII Cells Direct cDNA Synthesis System (Invitrogen). The anti-nectin-2 human monoclonal antibody-producing hybridoma established in EXAMPLE 1 (3 to 10 x $10^5$ cells) was suspended in 80 μL of PBS, and 1 μL of the suspension was added on ice to a PCR tube charged with 1 μL of RNaseOUT and 10 μL of Resuspension Buffer, which was heated at 75°C for 10 minutes. Next, 1.6 μL of 10 x DNaseI Buffer and 5 μL of DNaseI were added thereto on ice and the mixture was allowed to stand at room temperature for 5 minutes. Then, the tube was put back on ice and 1.2 μL of 25 mM EDTA was added thereto, followed by heating at 70°C for 5 minutes. Subsequently, 2 μL of Oligo(dT)$_{20}$ and 1 μL of 10 mM dNTP Mix were added thereto. After heating at 70°C for 5 minutes, the tube was allowed to stand on ice for 2 minutes, and 6 μL of 5 x RT Buffer, 1 μL of RNase OUT, 1 μL of SuperScriptIII RT and 1 μL of 0.1 mM DTT were added thereto. The resulting mixture was reacted at 50°C for 50 minutes and further at 85°C for 5 minutes to synthesize cDNA.

Using the above cDNA synthesized from each hybridoma as a template, PCR was carried out to amplify the H chain genes using primer 176 (SEQ ID NO: 176) and primer 177 (SEQ ID NO: 177) and the L chain genes using primer 178 (SEQ ID NO: 178) and primer 179 (SEQ ID NO: 179), in the case of Nec1-244-3, Nec1-530-1, Nec1-803-2, Nec1-834-1, Nec1-845-2 and Nec1-903-1; the H chain gene using primer 177 and primer 178 and the L chain gene using primer 180 (SEQ ID NO: 180) and primer 179, in the case of Nec8-4116-8; and the H chain gene using primer 181 (SEQ ID NO: 181) and primer 177 and the L chain gene using primer 182 (SEQ ID NO: 182) and primer 179, in the case of Necl-554-1; respectively. In this reaction, the reaction solution was composed of 4 μL of the cDNA described above, 1 U of KOD-Plus- (TOYOBO), 0.3 μM of each primer, 200 μM dNTPs, 1 mM $MgSO_4$ and 10 x PCR buffer (TOYOBO), which was made the total 50 μL. PCR was carried out by reacting at 94°C for 2 minutes and then repeating 30 times the cycle set to include 94°C for 15 seconds, 60°C for 30 seconds and 68°C for 1 minute. The H chain genes were again subjected to the same PCR as described above, using 1 μL of the reaction solution after completion of abovementioned PCR as a template. The PCR product was purified using PCR Purification Kit (QIAGEN) and eluted with 75 μL of EB buffer.

The PCR reaction for DNA base sequencing was carried out by using a solution of 20 μL volume composed of 10 μL of the purified PCR production solution described above, 8 μL of ABI PRISM BigDye Terminator v3.1 Cycle Sequencing Kit Cycle Sequencing Mix (Applied Biosystems) and 3.2 pmol of primer 183 (SEQ ID NO: 183). PCR was carried out by reacting at 96°C for 2 minutes and then repeating 25 times the cycle set to include 96°C for 10 seconds, 50°C for 5 seconds and 60°C for 4 minutes. The reaction solution was purified by replacing with 30 μL of distilled water using Sephadex G-75 Superfine (GE healthcare Bio-sciences). This reaction product was applied to DNA sequence analyzer ABI PRISM 3100 (Applied Biosystems) and the DNA sequence was determined based on the manual attached to the analyzer. The amino acid sequence of each antibody was deduced from the DNA sequence in a conventional manner.

The results revealed that the H chain variable region of Nec1-244-3 consisted of the base sequence (SEQ ID NO: 191) encoding the amino acid sequence represented by SEQ ID NO: 187, the L chain variable region of Nec1-244-3 consisted of the base sequence (SEQ ID NO: 199) encoding the amino acid sequence represented by SEQ ID NO: 195, the H chain variable region of Nec1-530-1 consisted of the base sequence (SEQ ID NO: 207) encoding the amino acid sequence represented by SEQ ID NO: 203, the L chain variable region of Necl-530-1 consisted of the base sequence (SEQ ID NO: 215) encoding the amino acid sequence represented by SEQ ID NO: 211, the H chain variable region of Nec1-554-1 consisted of the base sequence (SEQ ID NO: 223) encoding the amino acid sequence represented by SEQ ID NO: 219, the L chain variable region of Nec1-554-1 consisted of the base sequence (SEQ ID NO: 231) encoding the amino acid sequence represented by SEQ ID NO: 227, the H chain variable region of Nec1-803-2 consisted of the base sequence (SEQ ID NO: 239) encoding the amino acid sequence represented by SEQ ID NO: 235, the L chain variable region of Nec1-803-2 consisted of the base sequence (SEQ ID NO: 247) encoding the amino acid sequence represented by SEQ ID NO: 243, the H chain variable region of Necl-834-1 consisted of the base sequence (SEQ ID NO: 255) encoding the amino acid sequence represented by SEQ ID NO: 251, the L chain variable region of Necl-834-1 consisted of the base sequence (SEQ ID NO: 263) encoding the amino acid sequence represented by SEQ ID NO: 259, the H chain variable region of Nec1-845-2 consisted of the base sequence (SEQ ID NO: 271) encoding the amino acid sequence represented by SEQ ID NO: 267, the L chain variable region of Nec1-845-2 consisted of the base sequence (SEQ ID NO: 279) encoding the amino acid sequence represented by SEQ ID NO: 275, the H chain variable region of Nec1-903-1 consisted of the base sequence (SEQ ID NO: 287) encoding the amino acid sequence represented by SEQ ID NO: 283, the L chain variable region of Nec1-903-1 consisted of the base sequence (SEQ ID NO: 295) encoding the amino acid sequence represented by SEQ ID NO: 291, the H chain variable region of Nec8-4116-8 consisted of the base sequence (SEQ ID NO: 303) encoding the amino acid sequence represented by SEQ ID NO: 299, and the L chain variable region of Nec8-4116-8 consisted of the base sequence (SEQ ID NO: 311) encoding the amino acid sequence represented by SEQ ID NO: 307.

The combinations of CDR1 to 3 (amino acid sequences) of the heavy chains of the antibodies obtained here are listed in TABLE 21. The combinations of CDR1 to 3 (amino acid sequences) of their light chains are listed in TABLE 22. The CDR1 to 3 (base sequences) of the heavy chains of the antibodies obtained here are base sequences encoding the amino acid sequences described in TALBE 21 and their combinations are listed in TABLE 23. The CDR1 to 3 (base sequences) of the light chains of the antibodies obtained here are base sequences encoding the amino acid sequences described in TALBE 22 and their combinations are listed in TABLE 24.

In addition, the amino acid sequences and base sequences of the variable regions of the antibodies obtained here are listed in TABLE 25.

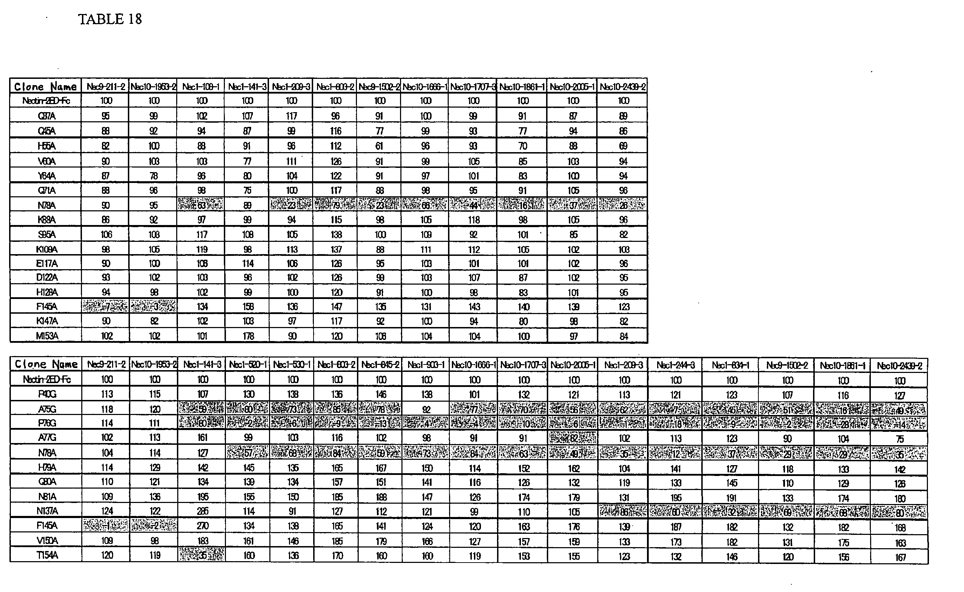

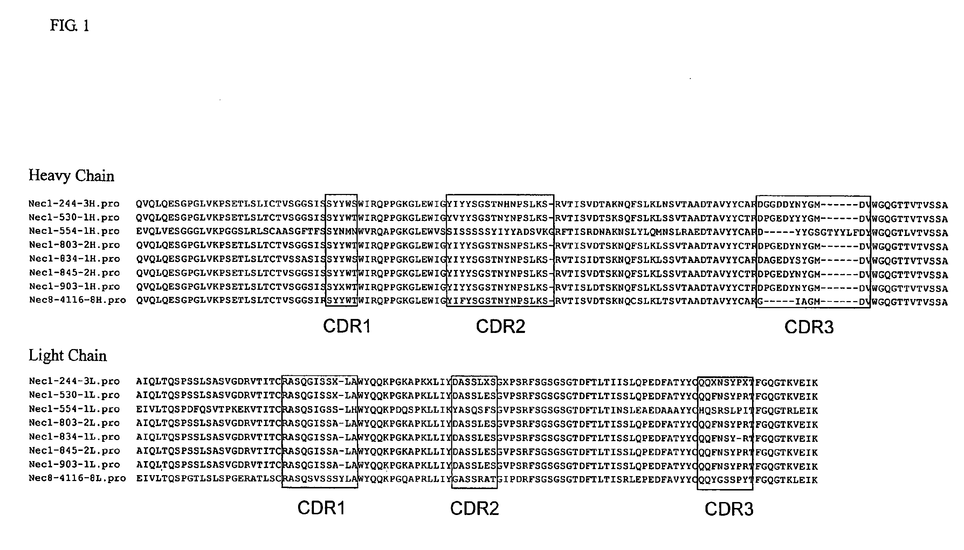

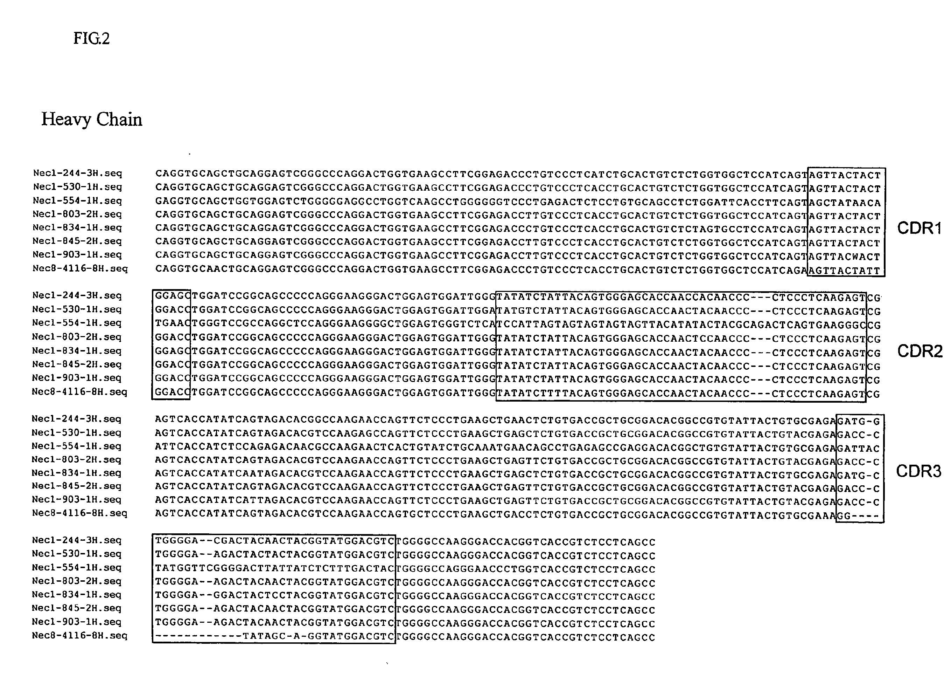

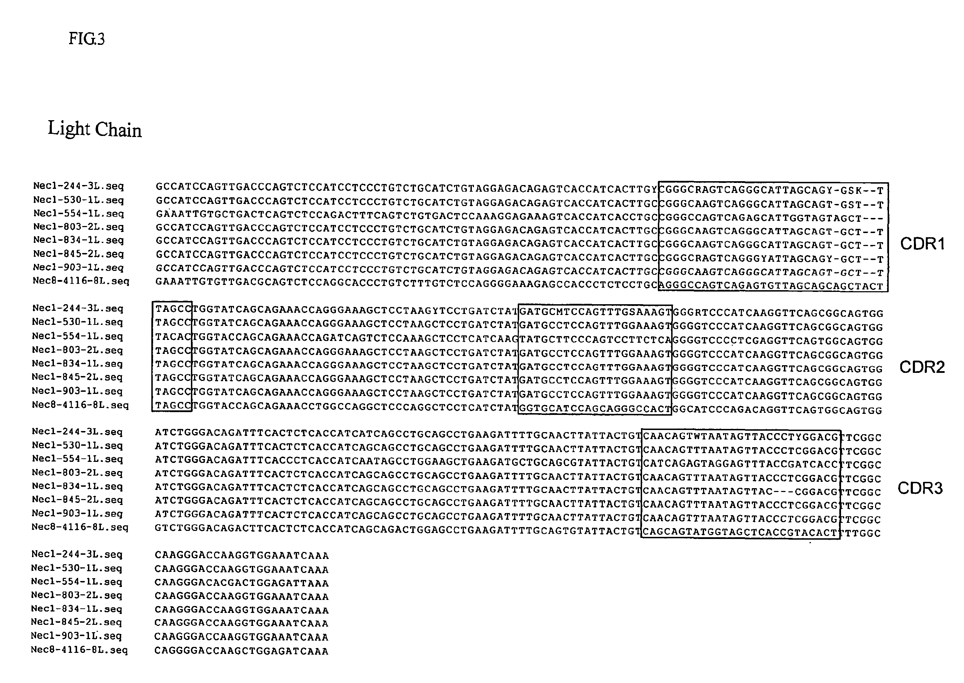

For reference, the amino acid sequences of the H chain and L chain variable regions of these antibodies are shown in FIG 1 and the respective base sequences are shown in FIGS. 2 and 3.

TABLE 21 CDR Sequence (amino acid sequence) of Heavy Chain of Anti-Nectin-2 Antibody

| Clone No. | Heavy Chain | | |
|---|---|---|---|
| | CDR1 | CDR2 | CDR3 |
| Nec1-244-3 | SYYWS (184) | YIYYSGSTNHNPSLKS (185) | DGGDDYNYGMDV (186) |
| Nec1-530-1 | SYYWT (200) | YVYYSGSTNYNPSLKS (201) | DPGEDYYYGMDV (202) |
| Nec1-554-1 | SYNMN 216) | SISSSSSYIYYADSVKG (217) | DYYGSGTYYLFDY (218) |
| Nec1-803-2 | SYYWT (232) | YIYYSGSTNSNPSLKS (233) | DPGEDYNYGMDV (234) |
| Nec1-834-1 | SYYWS (248) | YIYYSGSTNYNPSLKS (249) | DAGEDYSYGMDV (250) |
| Nec1-845-2 | SYYWT (264) | YIYYSGSTNYNPSLKS (265) | DPGEDYNYGMDV (266) |
| Nec1-903-1 | SYXWT (280) | YIYYSGSTNYNPSLKS (281) | DPGEDYNYGMDV (282) |
| Nec8-4116-8 | SYYWT (296) | YIFYSGSTNYNPSLKS (297) | GIAGMDV (298) |

Numerals within parentheses following the sequences denote Sequencing Numbers.

TABLE 22 CDR Sequence (amino acid sequence) of Light Chain of Anti-Nectin-2 Antibody

| Clone No. | Light Chain | | |
|---|---|---|---|
| | CDR1 | CDR2 | CDR3 |
| Nec1-244-3 | RASQGISSXLA (192) | DASSLXS (193) | QQXNSYPXT (194) |
| Nec1-530-1 | RASQGISSXLA (208) | DASSLES (209) | QQFNSYPRT (210) |
| Nec1-554-1 | RASQSIGSSLH (224) | YASQSFS (225) | HQSRSLPIT (226) |
| Nec1-803-2 | RASQGISSALA (240) | DASSLES (241) | QQFNSYPRT (242) |
| Nec1-834-1 | RASQGISSALA (256) | DASSLES (257) | QQFNSYRT (258) |
| Nec1-845-2 | RASQGISSALA (272) | DASSLES (273) | QQFNSYPRT (274) |
| Nec1-903-1 | RASQGISSALA (288) | DASSLES (289) | QQFNSYPRT (290) |
| Nec8-4116-8 | RASQSIGSSLH (304) | YASQSFS (305) | HQSRSLPIT (306) |

Numerals within parentheses following the sequences denote Sequencing Numbers.

TABLE 23 CDR Sequence (base sequence) of Heavy Chain of Anti-Nectin-2 Antibody

| Clone No. | Heavy Chain | | |
|---|---|---|---|
| | CDR1 | CDR2 | CDR3 |
| Nec1-244-3 | AGTTACTACTGGAGC (188) | TATATCTATTACAGTGG GAGCACCAACCACAAC CCCTCCCTCAAGAGT (189) | GATGGTGGGGACGAC TACAACTACGGTATG GACGTC (190) |
| Nec1-530-1 | AGTTACTACTGGACC (204) | TATGTCTATTACAGTGG GAGCACCAACTACAACC CCTCCCTCAAGAGT (205) | GACCCTGGGGAAGAC TACTACTACGGTATG GACGTC (206) |

(continued)

| Clone No. | Heavy Chain | | |
|---|---|---|---|
| | CDR1 | CDR2 | CDR3 |
| Nec1-554-1 | AGCTATAACATGAAC (220) | TCCATTAGTAGTAGTAG TAGTTACATATACTACG CAGACTCAGTGAAGGGC (221) | GATTACTATGGTTCG GGGACTTATTATCTC TTTGACTAC (222) |
| Nec1-803-2 | AGTTACTACTGGACC (236) | TATATCTATTACAGTGG GAGCACCAACTCCAAC CCCTCCCTCAAGAGT (237) | GACCCTGGGGAAGAC TACAACTACGGTATG GACGTC (238) |
| Nec1-834-1 | AGTTACTACTGGAGC (252) | TATATCTATTACAGTGG GAGCACCAACTACAACC CCTCCCTCAAGAGT (253) | GATGCTGGGGAGGAC TACTCCTACGGTATG GACGTC (254) |
| Nec1-845-2 | AGTTACTACTGGACC (268) | TATATCTATTACAGTGG GAGCACCAACTACAACC CCTCCCTCAAGAGT (269) | GACCCTGGGGAAGAC TACAACTACGGTATG GACGTC (270) |
| Nec1-903-1 | AGTTACWACTGGACC (284) | TATATCTATTACAGTGG GAGCACCAACTACAACC CCTCCCTCAAGAGT (285) | GACCCTGGGGAAGAC TACAACTACGGTATG GACGTC (286) |
| Nec8-4116-8 | AGTTACTATTGGACC (300) | TATATCTTTTACAGTGG GAGCACCAACTACAACC CCTCCCTCAAGAGT (301) | GGTATAGCAGGTATG GACGTC (302) |

Numerals within parentheses following the sequences denote Sequencing Numbers.

TABLE 24 CDR Sequence (base sequence) of Light Chain of Anti-Nectin-2 Antibody

| Clone No. | Light Chain | | |
|---|---|---|---|
| | CDR1 | CDR2 | CDR3 |
| Nec1-244-3 | CGGGCRAGTCAGGGCAT TAGCAGYGSKTTAGCC (196) | GATGCMTCCAGTTTG SAAAGT (197) | CAACAGTWTAATAGTTACCCTYGGACG (198) |
| Nec1-530-1 | CGGGCAAGTCAGGGCAT TAGCAGTGSTTTAGCC (212) | GATGCCTCCAGTTTG GAAAGT (213) | CAACAGTTTAATAGTT ACCCTCGGACG (214) |
| Nec1-554- 1 | CGGGCCAGTCAGAGCATTGGTAGTAGCTTACAC (228) | TATGCTTCCCAGTCCTTCTCA (229) | CATCAGAGTAGGAGT TTACCGATCACC (230) |
| Nec1-803-2 | CGGGCAAGTCAGGGCAT TAGCAGTGCTTTAGCC (244) | GATGCCTCCAGTTTG GAAAG (245) | CAACAGTTTAATAGTT ACCCTCGGACG (246) |
| Nec1-834-1 | CGGGCAAGTCAGGGCAT TAGCAGTGCTTTAGCC (260) | GATGCCTCCAGTTTG GAAAGT (261) | CAACAGTTTAATAGTT ACCGGACG (262) |
| Nec1-845-2 | CGGGCRAGTCAGGGYAT TAGCAGYGCTTTAGCC (276) | GATGCCTCCAGTTTG GAAAGT (277) | CAACAGTTTAATAGTT ACCCTCGGACG (278) |
| Nec1-903-1 | CGGGCAAGTCAGGGCAT TAGCAGTGCTTTAGCC (292) | GATGCCTCCAGTTTG GAAAGT (293) | CAACAGTTTAATAGTT ACCCTCGGACG (294) |
| Nec8-4116-8 | AGGGCCAGTCAGAGTGT TAGCAGCAGCTACTTAG CC (308) | GGTGCATCCAGCAGG GCCACT (309) | CAGCAGTATGGTAGC TCACCGTACACT (310) |

Numerals within parentheses following the sequences denote Sequencing Numbers.

TABLE 25 Base Sequence and Amino Acid Sequence of Anti-Nectin-2 Antibody Variable Regions

| Clone No. | Base Sequence | | Amino Acid Sequence | |
|---|---|---|---|---|
| | Heavy Chain | Light Chain | Heavy Chain | Light Chain |
| Nec1-244-3 | SEQ ID NO: 191 | SEQ ID NO: 199 | SEQ ID NO: 187 | SEQ ID NO: 195 |
| Nec1-530-1 | SEQ ID NO: 207 | SEQ ID NO: 215 | SEQ ID NO: 203 | SEQ ID NO: 211 |
| Nec1-554-1 | SEQ ID NO: 223 | SEQ ID NO: 231 | SEQ ID NO: 219 | SEQ ID NO: 227 |
| Nec1-803-2 | SEQ ID NO: 239 | SEQ ID NO: 247 | SEQ ID NO: 235 | SEQ ID NO: 243 |
| Nec1-834-1 | SEQ ID NO: 255 | SEQIDNO:263 | SEQ ID NO : 25 | SEQIDNO:259 |
| Nec1-845-2 | SEQ ID NO: 271 | SEQ ID NO: 279 | SEQ ID NO: 267 | SEQ ID NO: 275 |
| Nec1-903-1 | SEQ ID NO: 287 | SEQ ID NO: 295 | SEQ ID NO: 283 | SEQ ID NO: 291 |
| Nec8-4116-8 | SEQ ID NO: 303 | SEQ ID NO: 311 | SEQ ID NO: 299 | SEQIDNO:307 |

The human monoclonal antibody against the protein comprising the same or substantially the same amino acid sequence as the amino acid sequence represented by SEQ ID NO: 1 or SEQ ID NO: 3, its partial peptide, or a salt thereof, can be safely used, for example, as an agent for preventing/treating cancer (e.g., colorectal cancer, breast cancer, lung cancer, prostate cancer, esophageal cancer, gastric cancer, liver cancer, biliary tract cancer, spleen cancer, renal cancer, bladder cancer, uterine cancer, ovarian cancer, testicular cancer, thyroid cancer, pancreatic cancer, brain tumor, blood tumor, etc.) (preferably an agent for preventing/treating breast cancer, lung cancer, colorectal cancer, prostate cancer, ovarian cancer, pancreatic cancer, etc.), an apoptosis inducer of cancer cells, a growth inhibitor of cancer cells, an inducer of cell cycle change in cancer cells, an effector cell-dependent cytotoxic agent against cancer cells, and the like.

REFERENCE EXAMPLE 36

Generation of anti-nectin-2 human monoclonal antibodies

(i) A recombinant nectin-2ED-hFc fusion protein (1.6 mg/mL PBS solution) prepared in REFERENCE EXAMPLE 16 in which an animal cell expression vector encoding the fusion protein of nectin-2δ extracellular domain ($Met^1$-$Gly^{361}$) and human $IgG_1$ antibody Fc region ($Pro^{217}$-$Lys^{447}$) were transiently expressed in human 293F cell line (Invitrogen); or (ii) a recombinant nectin-2ED-FLAG fusion protein (2 mg/mL PBS solution) prepared inREFERENCE EXAMPLE 13 in which an animal cell expression vector encoding the fusion protein of nectin-2δ extracellular domain ($Met^1$-$Gly^{361}$) and FLAG tag (Asp-Tyr-Lys-Asp-Asp-Asp-Asp-Lys) were transiently expressed in human 293F cell line (Invitrogen), was mixed with Freund's complete adjuvant (Difco) in equal volumes to prepare emulsions, respectively.

These emulsions were injected to each five KM mice (10 weeks old, 12 weeks old, male; Kirin Brewery) subcutaneously and intracutaneously for primary immunization, respectively, in 10 to 50 μg each/animal. For the second and subsequent immunization, an emulsion prepared by mixing the recombinant nectin-2ED-hFc protein or the recombinant necin-2ED-FLAG protein with Freund's incomplete adjuvant (Difco) in equal volumes was likewise injected every 2 weeks for booster. In addition, recombinant FM3A cell line (#60-6) (cf. PCT/JP2006/320429) stably expressing nectin-2δ or recombinant NS0 cell line (#2-75) stably expressing nectin-2δ was cultured in a flask. These cells were collected and washed to remove the serum components, followed by treating with mitomycin C (Wako Pure Chemical) at 37°C for 30 minutes. The two cell lines treated with mitomycin C were washed 3 times with 10 mL of PBS and then resuspended in PBS at 2 x $10^7$ cells/mL. The suspension was intraperitoneally injected to each five KM mice (Kirin Brewery, 10-12 weeks old, male) at 1 x $10^7$ cells/500 μL repeatedly once every week.

Also, each five KM mice (12 weeks old, male) were immunized for primary immunization, with an emulsion prepared by mixing the aforesaid recombinant Nectin-2ED-hFc protein or the aforesaid recombinant Nectin-2ED-FLAG protein with Freund's complete adjuvant in equal volumes subcutaneously and intracutaneously at 10 to 50 μg each/animal, respectively. One week after the primary immunization, the mitomycin C-treated recombinant FM3A cell line expressing nectin-2δ was intraperitoneally injected at 1 x $10^7$ cells each/500 μL for booster. For the third immunization, an emulsion prepared by mixing the recombinant nectin-2ED-hFc or the recombinant nectin-2ED-FLAG with Freund's incomplete adjuvant in equal volumes was subcutaneously and intracutaneously immunized, respectively, and at the same time, mitomycin C-treated recombinant FM3A cell line expressing nectin-2δ (#60-6) was intraperitoneally injected

at 1 x $10^7$ cells each/500 μL once every week. For the fourth and subsequent immunization, the mitomycin C-treated recombinant FM3A cell line (#60-6) expressing nectin-2δ only was intraperitoneally injected at 1 x $10^7$ cells each/500 μL by the same procedure as described above.

Prior to the immunization and after the immunization, blood was collected from the ocular fundus of all mice under ethereal anesthesia to prepare antisera, and the antibody titer in the sera was determined by the flow cytometry method described below. That is, cell suspensions (PBS) of the recombinant CHO cell line (#43-2) stably expressing nectin-2δ (cf. PCT/JP2006/320429) and mock-CHO cell line were separately dispensed in polypropylene tubes at 5 x $10^5$ cells/tube, respectively, and then PBS was removed by centrifugation (1,200 rpm x 5 minutes). These cell debris were resuspended in 50 μL each of the aforesaid mouse antisera diluted to 100-fold with PBS containing 1% BSA and 10% FBS, and reacted on ice in the dark for 30 minutes. After 200 μL of PBS was added to the cell debris and the mixture was centrifuged (1,200 rpm x 5 minutes), the supernatant was removed by aspiration. The cell debris were resuspended in 50 μL of a solution of anti-human IgG (H+L) Alexa 488 (Invitrogen) diluted to 100-fold with PBS containing 1% BSA and 10% FBS, and reacted on ice in the dark for 30 minutes. After the cell suspension was likewise washed 3 times with PBS, the cell debris was resuspended in 200 μL of PBS. Fluorescence intensities of the respective cells were analyzed with the flow cytometer MPL500 (BECKMAN COULTER) to prepare a graph having an abscissa representing the fluorescence intensity and an ordinate representing the cell count, whereby antibody titers of antisera were compared.

In the KM mice in which a sufficient increase in the serum antibody titer was confirmed by the procedure described above, the mice which had been immunized with the recombinant nectin-2ED-hFc or the recombinant nectin-2ED-FLAG were injected via tail vein with these protein antigens, and the mice which had been immunized with the recombinant cell line stably expressing nectin-2δ were intraperitoneally injected with the same cell line . Three days after the final immunization, the mice were bled to death and spleen was withdrawn. The mouse spleen cells obtained were mixed in 5 : 1 with mouse myeloma cells P3X63Ag8U.1 (P3U1) (ATCC), which had previously been adapted to a medium in which 1 vial of 8-azaguanine (HybriMax, Sigma-Aldrich) per 500 mL of 10% FBS-supplemented Daigo T medium (a medium mixture of F-12 Nutrient Mixture (HAM) (Invitrogen) and Iscove's Modified Dulbecco's Medium (Invitrogen) in equal volumes, supplemented with MEM Non-Essential Amino Acid Solution (Invitrogen), sodium pyruvate (Invitrogen) and L-glutamine (Invitrogen)) thereby to cause fusion using polyethylene glycol (PEG) 1,500 (Roche Diagnostics). Cell fusion manipulations were performed according to the manual attached to the reagent. The cells after fusion were resuspended in Daigo T medium supplemented with 10% FBS and 10% BM Condimed H1 (Roche Diagnostics), seeded in a 96-well culture plate at 5 x $10^4$ spleen cells/100 μL/well and incubated at 37°C for a day in a 5% carbon dioxide gas flow. Subsequently, Daigo T medium (HAT selection medium) supplemented with 0.1 mM hypoxanthine, 0.4 μM aminopterin, 0.016 mM thymidine (HAT), 10% BM Condimed H1 and 10% FBS was added thereto at 100 μL/well, followed by a further incubation at 37°C in a 5% carbon dioxide gas flow with replacing twice 3/4 of the culture supernatant with a fresh HAT selection medium every 3 days.

The culture supernatant in which growth of the colony was observed during days 7 to 14 of the incubation was screened by Cell ELISA using the recombinant CHO cell line stably expressing nectin-2δ (#43-2) or the mock-CHO cell line to establish the anti-nectin-2 human monoclonal antibody-producing hybridoma. In other words, the recombinant CHO cell line stably expressing nectin-2δ (#43-2) and the mock-CHO cell line were incubated in a 96-well tissue culture plate charged with GS-selection DMEM medium containing 10% dialyzed FBS and GS supplements. After the culture supernatant of the plate where each cell line became confluent was removed by aspiration, 200 μL each/well of PBS (+) containing 2% FBS was added thereto and incubated on ice in the dark for an hour. After the supernatant of each well was removed by aspiration, 50 μL each/well of the hybridoma culture supernatant was added and reacted on ice in the dark for 2 hours. After this plate was washed once with PBS(+) chilled at 4°C, 100 μL each/well of anti-human IgG (H+L) chain specific (GOAT) peroxidase conjugate (CALBIOCHEM) diluted to 3,000-fold with PBS(+) containing 2% FBS was added and reacted on ice in the dark for 2 hours. After the plate was washed 3 times with PBS(+) chilled at 4°C, 100 μL each/well of 3,3',5,5'-tetramethylbenzidine (TMB) solution (SureBlue Microwell TMB peroxidase substrate; Kirkegaard & Perry Laboratories) was added and maintained at room temperature for 5 minutes to cause color formation. By adding 100 μL each/well of 2N sulfuric acid (Wako Pure Chemical) , the enzyme reaction was terminated. Absorbance (450 nm) of each well was measured using a plate reader (Multiskan BICHROMATIC; Thermo Electron Co.), and those showing absorbance of 0.5 or more in the plate in which the nectin-2 expression recombinant CHO cells expressing nectin-2 were seeded and showing absorbance of less than 0.3 in the plate in which the mock-CHO cell line were seeded were determined to be positive wells. IgG antibody-producing The hybridomas producing IgG antibody with particularly high antigen specificity and affinity were selected, and resuspended in Daigo T medium containing 10% FBS and 10% BM Condimed H1. The cells were seeded in a 96-well tissue culture plate at 0.5 cell/well,and the culture supernatants of the hybridomas, which were confirmed to be monoclones by microscopic observation, were again screened by the Cell ELISA described above to establish 258 hybridoma clones producing anti-nectin-2 human monoclonal antibody.

The thus obtained each anti-nectin-2 human monoclonal antibody-producing hybridoma was cultured in flask charged with 100 mL ofDaigo T medium containing 10% FBS Ultra low IgG (Invitrogen), and the culture supernatant was centrifuged (1,200 rpm x 5 minutes) to give the supernatant. After 200 μL of Protein A Sepharose FF (GE Healthcare Bio-sciences) equilibrated with PBS was added to these culture supernatant, the antibody was adsorbed thereto while gently shaking overnight at 4°C. This protein A carrier was recovered and washed with PBS by centrifugal operation. Then, the IgG fraction was eluted out with 1.2 mL of 0.1 M glycine-HCl (pH 3.0) containing 0.3 M NaCl. Immediately, this eluate was neutralized with 1 M Tris-HCl (pH 8.0), and the buffer was replaced with PBS by ultraconcentration using an ultrafiltration membrane (Vivaspin 6: molecular weight cut off, 10,000; Sartorius), which was used in the following *in vitro* characterizations as the anti-nectin-2 human monoclonal antibody preparation.

The hybridoma clone Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682), Nec8-4116-8 (FERM BP-10685), Nec1-1044-4 Nec8-3517-11 or Nec8-3704-7, which produce the antibody used in the present invention, was obtained from the antibody-producing hybridoma clones.

REFERENCE EXAMPLE 37

Selection of antibodies binding to nectin-2 competitively with the antibody used in the present invention

The monoclonal antibodies binding to nectin-2 competitively with the antibody used in the present invention, which was prepared in REFERENCE EXAMPLE 36, were selected by the procedures described below.

First, to perform the competitive binding inhibition reaction between the anti-nectin-2 human monoclonal antibodies against nectin-2, the anti-nectin-2 human monoclonal antibodies obtained in REFERENCE EXAMPLE 36 were biotinylated. That is, 10 μg of the anti-nectin-2 human monoclonal antibody was added to 50 μL of WS Buffer attached to Biotin Labeling Kit-NH2 (Dojindo) and the mixture was concentrated almost to dryness by ultrafiltration using Microcon YM50 (MILLIPORE). To the liquid residue, 50 μL of Reaction Buffer attached to Biotin Labeling Kit-NH2 and a solution of 4 μL of NH2 Reactive Biotin dissolved in 50 μL of DMSO were added sequentially in this order, followed by reaction at 37°C for 10 minutes. The buffer of the reaction mixture was again replaced with WS Buffer by ultrafiltration to prepare biotinylated anti-nectin-2 human monoclonal antibodies. These antibodies were used for the assay described below.

To FMAT plate (384 well plate Black/Clear Bottom with Lid; Applied Biosystems), 5 μL of the antibody used in the present invention (unlabeled anti-nectin-2 monoclonal antibody), which was prepared in REFERENCE EXAMPLE 36, diluted in PBS containing 2% FBS at 25 μg/mL, 15 μL of a cell suspension (2 x $10^5$ cells/mL) of the CHO cell line stably expression nectin-2δ (#43-2) in PBS containing 2% FBS, and 5 μL of Streptavidin-Alexa Fluor 647 conjugate (Invitrogen) were added and mixed with each other, followed by reacting them at room temperature for 10 minutes. After adding 5 μL of the biotinylated anti-nectin-2 human monoclonal antibody prepared by the procedure described above, diluted in PBS containing 2% FBS at 0.5 μg/mL to each well, the plate was incubated at room temperature for 60 minutes. For control runs, wells in which PBS containing 2% FBS was added in place of the solution of unlabeled anti-nectin-2 monoclonal antibody were provided. This competitive inhibition reaction against the binding to nectin-2 was examined with the all combinations of the antibodies provided for the biotinylation and the antibodies used in the present invention. The plate was set on the Applied Biosystems 8200 Cellular Detection System (Applied Biosystems) to measure the fluorescence intensity (total FL1 value) of each well. The competitive inhibition rate in the combination of each anti-nectin-2 human monoclonal antibody was calculated according to the formula shown below.

$$\text{Competitive binding inhibition rate} = (1\text{-A/B}) \times 100$$

A: Total FL1 value of the well in which the unlabeled antibody is added to an antibody tested
B: Total FL1 value of the well in which the unlabeled antibody is not added to an antibody tested

By this procedure, for example, Nec1-1044-4 and Nec1-1302-2 were selectively obtained from the anti-nectin-2 human monoclonal antibodies prepared in, e.g., REFERENCE EXAMPLE 36 as the antibodies which bind to nectin-2 competitively with Nec1-554-1 for binding. Also, e.g., Nec8-3704-7 and Nec8-3517-11 were selectively obtained as the antibodies which bind to nectin-2 competitively with Nec8-4116-8.

TABLE 26 shows the rate of inhibiting the binding between biotinylated Nec8-4116-8 and nectin-2 by the anti-nectin-2 human monoclonal antibodies. TABLE 27 shows the competitive binding inhibition rate by the anti-nectin-2 human monoclonal antibodies against the binding to biotinylated Nec1-554- and nectin-2.

The antibodies Nec1-1044-4 and Nec1-1302-2, and Nec8-3 704-7 and Nec8-3517-11, which bind to nectin-2 competitively with the thus selected antibodies used in the present invention, have been deposited, respectively.

TABLE 26

| Biotinylated Antibody | Unlabeled Antibody | Inhibition Rate (%) |
|---|---|---|
| Nec8-4116-8 | Nec8-3517-11 | 91 |
| Nec8-4116-8 | Nec8-3704-7 | 98 |
| Nec8-4116-8 | Nec8-4116-8 | 98 |
| Nec8-4116-8 | Nec1-244-3 | 0 |
| Nec8-4116-8 | Nec1-845-2 | 22 |
| Nec8-4116-8 | Nec1-520-1 | 48 |
| Nec8-4116-8 | Nec1-769-2 | 0 |

TABLE 27

| Biotinylated Antibody | Unlabeled Antibody | Iinhibition Rate (%) |
|---|---|---|
| Nec1-554-1 | Nec1-554-1 | 87 |
| Nec1-554-1 | Nec1-1044-4 | 92 |
| Nec1-554-1 | Nec1-1302-2 | 82 |
| Nec1-554-1 | Nec1-244-3 | 0 |
| Nec1-554-1 | Nec1-845-2 | 0 |
| Nec1-554-1 | Nec1-520-1 | 41 |
| Nec1-554-1 | Nec1-769-2 | 0 |

REFERENCE EXAMPLE 38

Large scale preparation of recombinant anti-nectin-2 human monoclonal antibody (Nec8-4116-8)

Recombinant anti-nectin-2 human monoclonal antibody (Nec8-4116-8) was prepared by stably expressing a gene for the antibody in CHOK1SV cell line (Lonza Biologics). The antibody preparation which had been purified from the culture supernatant of anti-nectin-2 human monoclonal antibody-producing hybridoma Nec8-4116-8 using a Protein A column, was applied to SDS-polyacrylamide gel electrophoresis under reducing conditions to separate the H and L chains. The H and L chain proteins were transferred onto PVDF membrane. Since the H chain was often pyroglutamated at the N-terminus, the removal of the pyroglutamate was performed using Pfu Pyroglutamate Aminopeptidase (Takara Bio). The N-terminal amino acid sequences of the proteins transferred onto PVDF membrane were sequenced using a protein sequencer (ABI). The thus obtained N-terminal amino acid sequences (H chain (SEQ ID NO: 312) and L chain (SEQ ID NO: 313)) were subjected to homology search using the V BASE database (http://vbase.mrc-cpe.cam.ac.uk) thereby to select the germline (VH4, VKIIIA27) coincident with the amino acid sequences described above and the base sequences (H chain (SEQ ID NO: 314) and L chain (SEQ ID NO: 315)) encoding the N-terminus of the putative signal sequences therefrom. Restriction enzyme Hind III site and Kozak sequence were added to these signal sequences to synthesize oligo DNAs as forward primers (H chain (SEQ ID NO: 176) and L chain (SEQ ID NO: 180)). On the other hand, oligo DNAs with restriction enzyme EcoRI site added downstream the termination codons of genes for antibody H and L chains were synthesized as reverse primers, respectively (H chain (SEQ ID NO: 316) and L chain (SEQ ID NO: 317)). Using a cDNA prepared from hybridoma cell (Nec8-4116-8) using SuperScriptIII Cells Direct cDNA Synthesis System (Invitrogen) as a template, PCR was performed using the primers described above to obtain the full length genes for the H and L chains of Nec8-4116-8 antibody, respectively.

The thus obtained H chain gene and the L chain gene were digested with restriction enzymes HindIII and EcoRI, respectively. The purified DNA fragments were inserted into the animal cell expression vector pEE6.4 (Lonza Biologics) and the animal cell expression vector pEE12.4 (Lonza Biologics) containing glutamine synthetase (GS) gene at the Hind III - EcoR I site, respectively to construct the H chain expression vector and the L chain expression vector. Also, the H chain gene-inserted fragment and the L chain gene-inserted fragment, which were excised from the respective vectors by digestion with restriction enzymes Not I/Sal I, were isolated and then ligated each other to construct the double gene vector bearing the GS gene, H chain gene and L chain gene altogether. This vector was transfected into CHOK1SV cells according to the Lonza Biologics' manual and the transfectant was selection-cultured in a 96-well tissue culture plate containing Methionine Sulphoximine (MSX). Human antibody concentrations in the culture supernatants from the wells where a single colony was grown was quantified by ELISA to select 22 recombinant cell lines stably expressing Nec8-4116-8. These cell lines were expanded, and then adapted to a suspension culture using serum-free medium. Thus, the CHOK1SV cell line (Clone 25-78) stably expressing Nec8-4116-8 which shows excellent cell growth and antibody productivity was established.

The CHOK1SV cell line described above was expanded in a ProCHO5 medium (Cambrex) containing 25 μM methionine sulphoximine (MP Biomedicals) and GS supplement (50 x, SAFC), and cultured at 37°C in a 5 % carbon dioxide gas flow, followed by a stirring culture for 32 days using a 2 L jar fermentor (37°C, dissolved oxygen concentration of 2 mg/L, pH of 6.7-7.0, rotation speed of 80 rpm, aerated at 100-300mL/min). During this period, glucose solution, L-glutamine solution, GS supplement (50 x, SAFC), amino acids (50 x, SAFC), trace elements (10,000 x, SAFC), vitamins (100 x, SAFC) and soy hydrolysate (50 x, SAFC) were added based on the component analysis of the medium. The main culture was terminated at the point of time when cell viability was decreased to about 10%.

Next, the culture supernatant was centrifuged (7,460 x g, 20 minutes) to harvest the supernatant. The supernatant was concentrated through an ultrafiltration membrane (Hydrosart membrane, molecular weight cut-off of 10,000; Sartorius AG) for buffer exchange to 20 mM phosphate buffer (pH 7.0) containing 0.15 M NaCl. The concentrate was centrifuged (14,300 x g, 20 minutes) to obtain the supernatant, which was further microfiltrated (Stericup HV, 0.45 μm; Millipore) to obtain the concentrate. The concentrate was adsorbed to a Protein A Sepharose column (22 mm ID x 79 mm, GE Healthcare Biosciences) equilibrated with 20 mM phosphate buffer (pH 7.0) containing 0.15 M NaCl. After washing the column with 20 column volume of the same buffer, the antibody fraction adsorbed to the column was eluted out with 0.1 M sodium citrate buffer (pH 3.0). Immediately thereafter, the fraction was neutralized by adding 1/10 volume of 1 M Tris-HCl buffer (pH 9.0) and concentrated using an ultrafiltration membrane (AmiconUltra, molecular weight cut-off, 30,000; Millipore). The concentrate was applied onto the Superdex 200 column (26 mm ID x 60 cm, GE Healthcare Biosciences) equilibrated with PBS and eluted with the same buffer to give the antibody monomer fraction. The antibody fraction was passed through an ActiClean EtOX column (25 mm ID x 59 mm, Sterogene Bioseparations), which had been equilibrated with PBS, to remove endotoxin, followed by a concentration using an ultrafiltration membrane (AmiconUltra, molecular weight cut-off, 10,000; Millipore). The concentrate was filtrated aseptically (Millex GV, 0.22 μm; Millipore) to give the purified recombinant Nec8-4116-8 preparation. The purified antibody thus obtained demonstrated the purity of 95% or higher on SDS-PAGE and gel filtration HPLC using Superdex 200 column. The endotoxin content in the antibody preparation was found to be 0.1 EU/mg antibody or less by the analyses using both Endospecy ES-24S Set (Seikagaku Corp.) and Toxicolor DIA Set (Seikagaku Corp.).

REFERENCE EXAMPLE 39

Large scale preparation of anti-nectin-2 human monoclonal antibody (Nec1-554-1)

The anti-nectin-2 human monoclonal antibody Nec1-554-1 was prepared from the said antibody-producing hybridoma cell line (Nec1-554-1) by the following procedure. After expanding the culture of the hybridoma cell line Nec1-554-1 at 37°C in a 5% carbon dioxide gas flow in IH medium (Iscove's Modified Dulbecco's Medium : Ham's F-12 = 1:1, 0.1 mM MEM non-essential amino acid solution, 1 mM sodium pyruvate solution, 2 mM L-glutamine solution; Invitrogen) containing 10% FBS (Ultra-Low IgG, Invitrogen), the cells were further expanded with a primary adaptation medium (IH medium: CD Hybridoma Medium with 8 mM L-glutamine solution = 1:1, Invitrogen), followed by incubation for one day. The cells were further expanded with the medium for main culture (IH medium: CD Hybridoma Medium with 8 mM L-glutamine solution = 1:3, Invitrogen), followed by incubation for 5 to 7 days (37°C, dissolved oxygen concentration of 2 mg/L, pH 7.0, rotation speed of 40 rpm). During this period, glucose solution and L-glutamine solution were added based on the component analysis of the medium. The main culture was terminated at the point of time when cell viability was decreased to about 50%.

Next, the culture supernatant was harvested by centrifugation (7,460 x g, 20 minutes) and concentrated through an ultrafiltration membrane (Hydrosart membrane, molecular weight cut-off, 10,000; Sartorius AG) for buffer exchange to 20 mM phosphate buffer (pH 7.0) containing 0.15 M NaCl, followed by a centrifugation (14,300 x g, 20 minutes) to obtain the supernatant. The supernatant was further microfiltrated (Stericup HV, 0.45 μm; Millipore) to obtain the concentrate. The concentrate was adsorbed to a Protein A Sepharose column (22 mm ID x 79 mm, GE Healthcare Biosciences) equilibrated with 20 mM phosphate buffer (pH 7.0) containing 0.15 M NaCl. After washing the column with 20 column volume of the same buffer, the antibody fraction adsorbed to the column was eluted out with 0.1 M sodium citrate buffer (pH 3.0). Immediately thereafter, the fraction was neutralized by adding 1/10 volume of 1 M Tris-HCl buffer (pH 9.0).

The purified antibody fraction thus obtained was found to be exceptionally a mixture of the active and inactive antibodies. Therefore, the antibody fraction was diluted to 3-fold with 20 mM sodium acetate buffer (pH 5.0) and further adsorbed to an SP-5PW cation exchange column (21.5 mm ID x 150 mm, Toso) equilibrated with 20 mM sodium acetate buffer (pH 5.0) containing 100 mM NaCl. After washing the column with about 2 column volume of the same buffer, the antibodies were separated to 3 eluted fractions with a linear gradient of the NaCl concentration from 100 mM to 300 mM over 70 minutes. The binding activities of these eluted fractions to nectin-2 were determined by ELISA using the immobilized recombinant nectin-2-ED-Fc protein, and the antibody fraction (SP3) demonstrating the highest specific activity was collected as an anti-nectin-2 Ab fraction. The thus obtained antibody eluate was concentrated using an ultrafiltration membrane (AmiconUltra, molecular weight cut-off, 30,000; Millipore), and was applied onto a Superdex 200 column (26 mm ID x 60 cm, GE Healthcare Biosciences) equilibrated with PBS and eluted with the same buffer to give the antibody monomer fraction. The fraction was passed through the ActiClean EtOX column (25 mm ID x 59 mm, Sterogene Bioseparations) equilibrated with PBS to remove endotoxin, followed by concentration using an ultrafiltration membrane (AmiconUltra, molecular weight cut-off, 10,000; Millipore), and the concentrate was further filtrated aseptically (Millex GV, 0.22 μm; Millipore) to give the purified antibody. The purified antibody was named Nec1-554-1 SP3.

The purified antibody thus obtained demonstrated the purity of 95% or higher on SDS-PAGE and gel filtration HPLC using Superdex 200 column. The endotoxin content in the antibody preparation was found to be 0.1 EU/mg antibody or less by the analyses using both Endospecy ES-24S Set (Seikagaku Corp.) and Toxicolor DIA Set (Seikagaku Corp.).

EXAMPLE 20

ADCC of the anti-nectin-2 human monoclonal antibodies against breast cancer cell line

ADCC of the anti-nectin-2 human monoclonal antibodies Nec1-554-1, Nec1-1044-4, Nec1-1302-2, Nec8-4116-8, Nec8-3517-11 and Nec8-3704-7 prepared in REFERENCE EXAMPLES 36 and 37 was measured. Human breast cancer cell line MDA-MB-231 (ATCC) cultured according to the procedure described in EXAMPLE 22 was used as target cells, and commercially available frozen human peripheral blood mononuclear cells (ALLCELLS) which had been cultured overnight in RPMI 1640 medium (Invitrogen) supplemented with 0.1 nM recombinant human IL-2 (DIACLONE Research), 55 μM 2-mercaptoethanol (Invitrogen) and 10% fetal bovine serum (FBS) (JRH) (hereinafter referred to as 10% FBS/RPMI 1640 medium) were used as effector cells. As Nec1-1044-4, Nec1-1302-2, Nec8-4116-8, Nec8-3517-11 and Nec8-3704-7, the antibody preparations described in REFERENCE EXAMPLE 36 were used and the antibody preparation (Nec1-554-1 SP3) described in REFERENCE EXAMPLE 39 were used as Nec1-554-1, respectively.

Human breast cancer cell line MDA-MB-231 in the logarithmic growth phase was collected, and 250 μCi of $Na_2{}^{51}CrO_4$ (GE Healthcare Bio-sciences) was added to 1.0 x $10^6$ cells, followed by incubation at 37°C for an hour in order to label the cells with $^{51}Cr$. The cell line was washed 4 times with 10% FBS/RPMI 1640 medium and resuspended at 1 x $10^5$ cells/mL in 10% FBS/RPMI 1640 medium. ADCC assay was then performed by adding 50 μL (5 x $10^3$ cells) of the cell suspension thus obtained and 25 μL of a solution in which the antibodies described above were diluted in 10% FBS/RPMI 1640 at final concentrations of 0.0015 μg/mL, 0.015 μg/mL, 0.15 μg/mL and 1.5 μg/mL, to each well of a 96-well RMC plate (BIOBIK). The same volume of non-immune human IgG (final concentration of 0.0015 μg/mL, 0.015 μg/mL, 0.15 μg/mL and 1.5 μg/mL) or D-PB S (Invitrogen) was added as a negative control for detecting non-specific activity when no antibody was added (No Ab). After incubating these plates on ice for an hour, 2.5 x $10^5$ cells each/well of the effector cell suspension described above was added (effector cells : target cells = 50 : 1) and reacted at 37°C for 4 hours in a 5% carbon dioxide gas flow. The radioactivity (sample release) leaked from the cells in each well to the culture supernatant was measured by a scintillation counter (TopCount NXT). That is, the cell suspension after the reaction was transferred to a Multi-screen 0.45 μm (Millipore) and centrifuged to collect the culture supernatants. After 50 μL of the each culture supernatant was mixed with 150 μL/well of MicroScinti-40 in a 96-well plate (Costar) at room temperature for 30 minutes, the fluorescence of each well was measured by a scintillation counter (TABLE 33). The maximum cytotoxicity (maximum release) of ADCC was defined as the radioactivity detected when Triton-X 100 (Sigma) was added at a final concentration of 1%, whereas the spontaneous release activity (spontaneous release) was defined as the radioactivity detected when 10% FBS/RPMI 1640 medium was added in place of the effector cells. The specific lysis (%) as an indicator of the ADCC intensity was calculated by ([sample release]-[spontaneous release])/( [maximum release]-[spontaneous release]) x 100.

TABLE 33

| Antibody | Antibody (μg/ml) | Specific lysis (%) |
|---|---|---|
| Non-immune IgG | 0.0015 | 7 |

(continued)

| Antibody | Antibody (μg/ml) | Specific lysis (%) |
|---|---|---|
| | 0.015<br>0.15<br>1.5 | 9<br>8<br>7 |
| No Ab | - | 6 |
| Nec1-554-1 | 0.0015<br>0.015<br>0.15<br>1.5 | 8<br>11<br>16<br>16 |
| Nec1-1044-4 | 0.0015<br>0.015<br>0.15<br>1.5 | 10<br>9<br>9<br>14 |
| Nec1-1302-2 | 0.0015<br>0.015<br>0.15<br>1.5 | 8<br>8<br>11<br>14 |
| Nec8-4116-8 | 0.0015<br>0.015<br>0.15<br>1.5 | 8<br>11<br>15<br>16 |
| Nec8-3517-11 | 0.0015<br>0.015<br>0.15<br>1.5 | 10<br>12<br>15<br>17 |
| Nec8-3704-7 | 0.0015<br>0.015<br>0.15<br>1.5 | 9<br>12<br>16<br>16 |

EXAMPLE 21

ADCC of anti-nectin-2 human monoclonal antibodies against human prostate cancer cell line, human lung cancer cell line and human blood cancer cell line

ADCC of the anti-nectin-2 human monoclonal antibodies Nec1-554-1 and Nec8-4116-8 prepared in REFERENCE EXAMPLE 36 were measured. Human lung cancer cell line NCI-H1299, human prostate cancer cell line Du145 and human blood cancer cell line U937 were used as target cells, and commercially available frozen human peripheral blood mononuclear cells (ALLCELLS) which had been cultured overnight in RPMI 1640 medium (Invitrogen) supplemented with 0.1 nM recombinant human IL-2 (DIACLONE Research), 55 μM 2-mercaptoethanol (Invitrogen) and 10% fetal bovine serum (FBS) (JRH) (hereinafter referred to as 10% FBS/RPMI 1640 medium) were used as effector cells. Human lung cancer cell line NCI-H1299, human prostate cancer cell line Du145 and human blood cancer cell line U937 were purchased from ATCC. These cell lines were incubated according to a modification of the method recommended by ATCC. The antibody preparation described in REFERENCE EXAMPLE 36 and the antibody preparation (Nec1-554-1 SP3) described in REFERENCE EXAMPLE 39 were used as Nec8-4116-8 and Nec1-554-1, respectively.

The abovementioned cancer cells in the logarithmic growth phase were collected, and 250 μCi of $Na_2{}^{51}CrO_4$ (GE Healthcare Bio-sciences) was added to 1.0 x $10^6$ cells, followed by incubation at 37°C for an hour in order to label the cells with $^{51}Cr$. These cell lines were washed 4 times with 10% FBS/RPMI 1640 medium and resuspended at 1 x $10^5$ cells/mL in 10% FBS/RPMI 1640 medium. ADCC assay was then performed by adding 50 μL (5 x $10^3$ cells) of the

cell suspension thus obtained and 25 μL of a solution in which the antibodies described above were diluted in 10% FBS/RPMI 1640 medium at final concentrations of 0.0015 μg/mL, 0.015 μg/mL, 0.15 μg/mL and 1.5 μg/mL, to each well of a 96-well RMC plate (BIOBIK). The same volume of non-immune human IgG (final concentration of 0.0015 μg/mL, 0.015 μg/mL, 0.15 μg/mL and 1.5 μg/mL) or D-PBS (Invitrogen) was added as a negative control for detecting non-specific activity when no antibody was added (No Ab). After incubating these plates on ice for an hour, 2.5 x $10^5$ cells each/well of the effector cell suspension described above was added (effector cells : target cells = 50 : 1), which was reacted at 37°C for 4 hours in a 5% carbon dioxide gas flow. The radioactivity (sample release) leaked from the cells in each cell was measured using a scintillation counter (TopCount NXT) by the same procedure as described in EXAMPLE 20 (TABLE 34). The maximum cytotoxicity (maximum release) of ADCC was defined as the radioactivity detected when Triton-X 100 (Sigma) was added at a final concentration of 1%, whereas the spontaneous release activity (spontaneous release) was defined as the radioactivity detected when 10% FBS/RPMI 1640 medium was added in place of the effector cells. The specific lysis (%) as an indicator of the ADCC intensity was calculated by ([Sample release]-[Spontaneous release])/([Maximum release]-[Spontaneous release]) x 100.

TABLE 34

| Antibody | Antibody (μg/ml) | Specific lysis for NCI-H1299 (%) | Specific lysis for Du145 (%) | Specific lysis for U937 (%) |
|---|---|---|---|---|
| Non-immune IgG | 0.0015 | 18 | 9 | 26 |
| | 0.015 | 23 | 9 | 27 |
| | 0.15 | 22 | 10 | 27 |
| | 1.5 | 20 | 9 | 27 |
| No Ab | - | 16 | 7 | 21 |
| Nec1-554-1 | 0.0015 | 19 | 10 | 28 |
| | 0.015 | 24 | 13 | 27 |
| | 0.15 | 28 | 14 | 36 |
| | 1.5 | 26 | 16 | 34 |
| Nec8-4116-8 | 0.0015 | 21 | 9 | 28 |
| | 0.015 | 23 | 12 | 28 |
| | 0.15 | 28 | 15 | 36 |
| | 1.5 | 29 | 15 | 32 |

EXAMPLE 22

*In vivo* anti-tumor activity of anti-nectin-2 human monoclonal antibodies (SCID mouse models for treating lung metastasis with MDA-MB-231 human breast cancer cell line)

The anti-tumor activities of the anti-nectin-2 human monoclonal antibodies (Nec8-4116-8 and Nec1-554-1) prepared in REFERENCE EXAMPLE 36 were evaluated in SCID mouse models for treating lung metastasis with MDA-MB-231 human breast cancer cell line (purchased from ATCC). The Nec8-4116-8 and Nec1-554-1 antibody preparations used were those prepared by the procedures described in REFERENCE EXAMPLES 38 and 39, respectively. The MDA-MB-231 cell line was seeded on a 10 cm tissue culture dish (Corning) using Leibovitz's L-15 (Invitrogen) containing 10% FBS (Thermo Electron), and incubated at 37°C without carbon dioxide gas. The MDA-MB-231 cell suspension harvested at the logarithmic growth phase by detachment with trypsin-EDTA treatment was washed 3 times with Hank's balanced salt solution (HBSS) (Invitrogen) by centrifugal operation (1,000 rpm, 3 minutes). The cells thus obtained were resuspended in HBSS at a density of 5 x $10^6$ cells/mL.

SCID mice (C.B-17/Icr-scid/scidJcl) (5 weeks old, female), purchased from CLEA Japan, Inc., were inoculated via tail vein with the MDA-MB-231 cell suspension described above at 200 μL each/animal. On day 14 after the cell inoculation, each animal was weighed and then divided by 5 mice per group to make their mean weight equivalent in each group (about 20 g). In the test for evaluating the anti-tumor activity of Nec1-554-1, the Nec1-554-1 SP3 preparation diluted in PBS to 0.3 mg/mL or 0.03 mg/mL or PBS was injected via tail vein at 10 mL each/kg on days 14, 21, 28, 35, 42 and 49 after the cell inoculation. On the other hand, in the test for evaluating the anti-tumor activity of Nec8-4116-8, the Nec8-4116-8 preparation diluted in PBS to 0.3 mg/mL or 0.03 mg/mL or PBS was injected via tail vein at 10 mL each/kg on days 14, 21, 28, 35, 42, 49 and 56 after the cell inoculation.

The anti-tumor activities of Nec1-554-1 and Nec8-4116-8 were evaluated by counting the number of cancer colonies formed in the lungs at the diaphragmatic site. Significance of differences between the test groups were evaluated by the parametric Dunnett multiple comparison test (SAS Preclinical Package Version 5.0).

Both Nec1-554-1 and Nec8-4116-8 inhibited growth of the MDA-MB-231 cells at the lung significantly ($p<0.0001$) at any dose level. The number of cancer colonies (mean ± standard deviation) in the Nec1-554-1 group and the PBS group as well as the significance test values (*P* values) are shown in TABLE 35, and the number of cancer colonies (mean ± standard deviation) in the Nec8-4116-8 group and the PBS group as well as the significance test values (*P* values) are shown in TABLE 36.

TABLE 35

| | Number of cancer colonies | *P* value |
|---|---|---|
| PBS | 234±33 | - |
| Nec1-554-1 3 mg/kg | 31+24 | <0.0001 |
| Nec1-554-1 0.3 mg/kg | 20±6 | <0.0001 |

TABLE 36

| | Mean number of cancer colonies | *P* value |
|---|---|---|
| PBS | 152±46 | - |
| Nec8-4116-8 3 mg/kg | 18±10 | <0.0001 |
| Nec8-4116-8 0.3 mg/kg | 41±23 | <0.0001 |

EXAMPLE 23

*In vivo* anti-tumor activity of anti-nectin-2 human monoclonal antibodies (SCID mouse models for treating subcutaneously transplanted MDA-MB-231 human breast cancer cell line)

The anti-tumor activities of Nec8-4116-8 and Nec1-554-1 prepared in REFERENCE EXAMPLE 36 were evaluated in SCID mouse models for treating subcutaneously transplanted MDA-MB-231 human breast cancer cell line (purchased from ATCC). The Nec8-4116-8 and Nec1-554-1 antibody preparations used were those prepared by the procedures described in REFERENCE EXAMPLES 38 and 39, respectively. The MDA-MB-231 cell line was seeded on a 10 cm tissue culture dish (Becton Dickinson) using Leibovitz's L-15 (Invitrogen) containing 10% FBS (Hyclone) and 1/40 volume of 7.5% sodium bicarbonate (Invitrogen), followed by incubation at 37°C in a 5% carbon dioxide gas flow. The MDA-MB-231 cell suspension harvested at the logarithmic growth phase by detachment with trypsin-EDTA treatment was washed 3 times with Hank's balanced salt solution (HBSS) (Invitrogen) by centrifugal operation (1,000 rpm, 3 minutes). The cells thus obtained were resuspended in an equivolume mixture of HBSS and Matrigel (BD Biosciences) to obtain the cell suspension at a density of $3 \times 10^7$ cells/mL.

After SCID mice (C.B-17/Icr-scid/scidJcl) (5 weeks old, female), purchased from CLEA Japan, Inc., were tamed for a week, the MDA-MB-231 cell suspension described above was inoculated subcutaneously into the ventral area at a dose of 100 μL each/animal. Ten days after the cell inoculation, the long diameter and short diameter of the MDA-MB-231 tumor mass were measured with calipers and the tumor volume was calculated according to the formula described below.

$$\text{Tumor volume (mm}^3\text{)} = \text{long diameter x (short diameter)}^2/2$$

Each SCID mouse inoculated with the MDA-MB-231 cells was weighed and then divided into groups to make the mean volume of tumor mass equivalent (about 170 $mm^3$) in each group. On days 28, 31, 35, 38 and 42 after the cell inoculation, the Nec8-4116-8 or Nec1-554-1 SP3 antibody solution diluted in PBS to 3 mg/mL or PBS was intravenously administered via tail vein at 10 mL each/kg and at the same time, the tumor volume was measured by the procedure described above. The growth inhibitory activities of Nec8-4116-8 and Nec1-554-1 were evaluated by calculating the T/C (Treatment/Control) value, based on the tumor volume 3 weeks after commencement of drug administration, according to the formula below. Significance differences between the administration groups were evaluated by the parametric Dunnett multiple comparison test (SAS Preclinical Package Version 5.0).

$$T/C\ (\%) = [(\text{Increased tumor volume in the antibody group from commencement of drug administration})/(\text{Increased tumor volume in the PBS group from commencement of drug administration})] \times 100$$

FIG 4 shows changes in the mean tumor volume with passage of time after subcutaneous transplantation of the cancer cell lines.

The T/C values in the Nec8-4116-8 group and the Nec1-554-1 group were 39.2% and 38.7%, respectively, indicating that the antibodies both significantly inhibited the growth of MDA-MB-231 cell line-induced tumor ($p<0.0001$) (FIG. 4)

EXAMPLE 24

*In vivo* anti-tumor activity of anti-nectin-2 human monoclonal antibody (Nude mouse models for treating liver metastasis with OV-90 human ovarian cancer cell line)

The anti-tumor activities of the anti-nectin-2 human monoclonal antibody (Nec8-4116-8) prepared in REFERENCE EXAMPLE 36 were evaluated in nude mouse models for treating liver metastasis with the OV-90 human ovarian cancer cell line (purchased from ATCC). The Nec8-4116-8 antibody preparation used was the one prepared by the procedures described in REFERENCE EXAMPLE 38. The OV-90 cell line was seeded in a 150 $cm^2$ culture flask (Corning) charged with a medium, which was prepared by dissolving MCDB 105 Medium (Sigma) in 1L of distilled water, adjusting the pH to 7.5, aseptically filtrating, mixing 500 mL of this MCDB 105 Medium with 500 mL of Medium 199 (Sigma) and adding 150 mL of FBS (Thermo Electron) thereto, followed by incubation at 37°C in a 5% carbon dioxide gas flow. The OV-90 cell suspension harvested at the logarithmic growth phase by detachment with trypsin-EDTA treatment was washed 3 times with Hank's balanced salt solution (HBSS) (Invitrogen) by centrifugal operation (1,000 rpm, 3 minutes). The cells thus obtained were resuspended in HBSS at $1 \times 10^7$ cells/mL.

Nude mice (BALB/cAJcl-nu/nu) (5 weeks old, female), purchased from CLEA Japan, Inc., underwent laparotomy under ethereal anesthesia to expose the spleen. The OV-90 cell suspension described above was inoculated into the spleen at 100 μL each/animal. After the cell inoculation, the animal was intravenously injected via tail vein with the 3 mg/mL dilution of Nec8-4116-8 in PBS to reach 30 mg/kg every week on and after day 14 when engrafting into the liver was observed (on days 14, 21, 28, 35 and 42). For the control group, physiological saline was administered. One week after the final administration, 1 mL of 2.5% Evans Blue was administered via tail vein, the liver was taken out and immersed in 10% neutral buffered formalin solution for fixation. Then, the growth of the cancer cells metastasized in the liver was visually observed.

As a result, tumor growth in the liver was noted in 6 out of 6 cases in the control group, whereas in the Nec8-4116-8 group, tumor growth in the liver was noted only one out of 6 cases, indicating that Nec8-4116-8 markedly suppressed growth of the cancer cells engrafted into the liver.

## Claims

1. A monoclonal antibody against a protein comprising the same or substantially the same amino acid sequence as the amino acid sequence represented by SEQ ID NO: 1 or SEQ ID NO: 3, its partial peptide, or a salt thereof.

2. The antibody according to claim 1, which is a monoclonal antibody against a protein comprising the amino acid sequence represented by SEQ ID NO: 3, its partial peptide, or a salt thereof.

3. The antibody according to claim 1, which is a human monoclonal antibody.

4. The antibody according to claim 1, which is a chimeric monoclonal antibody.

5. The antibody according to claim 1, which is a humanized monoclonal antibody.

6. The antibody according to claim 1, which is a monoclonal antibody belonging to human $IgG_1$ subclass.

7. The antibody according to claim 1, which has a cancer cell growth inhibitory activity.

8. The antibody according to claim 1, which has antibody-dependent cellular cytotoxicity (ADCC).

9. The antibody according to claim 1, which has a nectin-2/nectin-3 or nectin-2/nectin-2 trans-binding inhibitory activity.

10. The antibody according to claim 1, which is a monoclonal antibody capable of recognizing the epitope present in the 1st-350th amino acid sequence in the amino acid sequence represented by SEQ ID NO: 1 or SEQ ID NO: 3.

11. The antibody according to claim 1, which is a monoclonal antibody capable of recognizing the epitope present in the 47th-142nd or 175th-240th amino acid sequence in the amino acid sequence represented by SEQ ID NO: 1 or SEQ ID NO: 3.

12. The antibody according to claim 1, which is a monoclonal antibody capable of recognizing the amino acid sequence containing at least one amino acid residue of the 75th, 76th, 77th, 78th, 95th, 137th, 145th, 173rd, 184th, 186th and 212th amino acid residues in the amino acid sequence represented by SEQ ID NO: 1 or SEQ ID NO: 3.

13. The antibody according to claim 1, wherein the antibody bind competitively with a monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1(FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685), to the protein comprising the same or substantially the same amino acid sequence as the amino acid sequence represented by SEQ ID NO: 1 or SEQ ID NO: 3, its partial peptide, or a salt thereof.

14. The antibody according to claim 1, which is capable of recognizing the same or substantially the same amino acid sequence as the amino acid sequence recognized by a monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427) or Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685).

15. A hybridoma cell, which is capable of producing the antibody according to claim 1.

16. The hybridoma cell according to claim 15, which is represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685).

17. A monoclonal antibody produced by the hybridoma cell according to claim 16.

**18.** The antibody according to claim 1, which is a recombinant monoclonal antibody.

**19.** The antibody according to claim 1, wherein the amino acid sequences of a first complementarity determining region (CDR1), a second complementarity determining region (CDR2) and a third complementarity determining region (CDR3) in a heavy chain variable region of said antibody comprise the same or substantially the same amino acid sequence as the amino acid sequence represented by (i) the sequence identification number selected from the group consisting of SEQ ID NOS: 184, 200, 216, 232, 248, 264, 280 and 296, (ii) the sequence identification number selected from the group consisting of SEQ ID NOS: 185, 201, 217, 233, 249, 265, 281 and 297, and (iii) the sequence identification number selected from the group consisting of SEQ ID NOS:186, 202, 218, 234, 250, 266, 282 and 298, respectively.

**20.** The antibody according to claim 1, wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) the sequence identification number selected from the group consisting of SEQ ID NOS: 192, 208, 224, 240, 256, 272, 288 and 304, (v) the sequence identification number selected from the group consisting of SEQ ID NOS: 193, 209, 225, 241, 257, 273, 289 and 305, and (vi) the sequence identification number selected from the group consisting of SEQ ID NOS: 194, 210, 226, 242, 258, 274, 290 and 306, respectively.

**21.** A diagnostic agent, which comprises a monoclonal antibody against a protein comprising the same or substantially the same amino acid sequence as the amino acid sequence represented by SEQ ID NO: 1 or SEQ ID NO: 3, its partial peptide, or a salt thereof.

**22.** The diagnostic agent according to claim 21, which is a diagnostic agent for cancer.

**23.** A medicament, which comprises a monoclonal antibody against a protein comprising the same or substantially the same amino acid sequence as the amino acid sequence represented by SEQ ID NO: 1 or SEQ ID NO: 3, its partial peptide, or a salt thereof.

**24.** The medicament according to claim 23, which is an agent for preventing/treating cancer.

**25.** The medicament according to claim 23, which is an apoptosis inducer of cancer cells.

**26.** The medicament according to claim 23, which is a growth inhibitor of cancer cells.

**27.** The medicament according to claim 23, which is a cytotoxic agent against cancer cells wherein a host defense mechanism mediated by the Fc region of an antibody is utilized.

**28.** A method for preventing/treating cancer, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof.

**29.** A method for inducing apoptosis of cancer cells, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof.

**30.** A method for inhibiting growth of cancer cells, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof

**31.** A method for killing cancer cells through a host defense mechanism mediated by the Fc region of an antibody, which comprises administering to a mammal an effective dose of a monoclonal antibody against a protein comprising the same or substantially the same amino acid sequence as the amino acid sequence represented by SEQ ID NO: 1 or SEQ ID NO: 3, its partial peptide, or a salt thereof

**32.** Use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of an agent for preventing/treating cancer.

**33.** Use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of an apoptosis inducer of cancer cells.

**34.** Use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of a growth inhibitor of cancer cells.

**35.** Use of a monoclonal antibody against a protein comprising the same or substantially the same amino acid sequence as the amino acid sequence represented by SEQ ID NO: 1 or SEQ ID NO: 3, its partial peptide, or a salt thereof, in the manufacture of a cytotoxic agent against cancer cells through a host defense mechanism mediated by the Fc region of an antibody.

**36.** An agent for preventing or treating breast cancer which comprises a monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685).

**37.** An agent for preventing or treating breast cancer which comprises a monoclonal antibody binding to nectin-2 competitively with a monoclonal antibody produced by the hybridoma cell represented by Nec1-803-2 (FERM BP-10417), Nec1-244-3 (FERM BP-10423), Nec1-530-1 (FERM BP-10424), Nec1-903-1 (FERM BP-10425), Nec1-520-1 (FERM BP-10426), Nec1-845-2 (FERM BP-10427), Nec1-834-1 (FERM BP-10428), Nec1-964-1 (FERM BP-10683), Nec1-1302-2 (FERM BP-10684), Nec1-554-1 (FERM BP-10681), Nec1-769-2 (FERM BP-10682) or Nec8-4116-8 (FERM BP-10685).

**38.** The agent for preventing or treating breast cancer according to claim 37, wherein the monoclonal antibody binding to nectin-2 competitively with a monoclonal antibody produced by the hybridoma cell represented by Nec1-554-1 (FERM BP-10681) is Nec1-1044-4 (FERM BP-10805) or Nec1-1302-2 (FERM BP-10684).

**39.** The agent for preventing or treating breast cancer according to claim 37, wherein the monoclonal antibody binding to nectin-2 competitively with a monoclonal antibody produced by the hybridoma cell represented by Nec8-4116-8 (FERM BP-10685) is Nec8-3704-7 (FERM BP-10807) or Nec8-3517-11 (FERM BP-10806).

**40.** The agent for preventing or treating breast cancer according to any one of claims 36 to 39, wherein a host defense mechanism mediated by the Fc region of the antibody is utilized.

**41.** An agent for preventing or treating breast cancer, which comprises an antibody wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) a sequence identification number selected from the group consisting of SEQ ID NOS: 184, 200, 216, 232, 248, 264, 280 and 296, (ii) a sequence identification number selected from the group consisting of SEQ ID NOS: 185, 201, 217, 233, 249, 265, 281 and 297, and (iii) a sequence identification number selected from the group consisting of SEQ ID NOS: 186, 202, 218, 234, 250, 266, 282 and 298, respectively.

**42.** An agent for preventing or treating breast cancer, which comprises an antibody wherein the amino acid sequences of the first complementarity determining region (CDR1), the second complementarity determining region (CDR2) and the third complementarity determining region (CDR3) in a light chain variable region of said antibody, which are amino acid sequence as the amino acid sequence represented by (iv) a sequence identification number selected from the group consisting of SEQ ID NOS: 192, 208, 224, 240, 256, 272, 288 and 304, (v) a sequence identification number selected from the group consisting of SEQ ID NOS: 193, 209, 225, 241, 257, 273, 289 and 305, and (vi) a sequence identification number selected from the group consisting of SEQ ID NOS: 194, 210, 226, 242, 258, 274, 290 and 306, respectively.

**43.** An agent for preventing or treating breast cancer, which comprises an antibody wherein the amino acid sequences of the first complementarity determining region (CDR1), the second complementarity determining region (CDR2) and the third complementarity determining region (CDR3) in a heavy chain variable region of said antibody comprise the same or substantially the same amino acid sequence as the amino acid sequence represented by (i) a sequence identification number selected from the group consisting of SEQ ID NOS: 184, 200, 216, 232, 248, 264, 280 and 296, (ii) a sequence identification number selected from the group consisting of SEQ ID NOS: 185, 201, 217, 233, 249, 265, 281 and 297, and (iii) a sequence identification number selected from the group consisting of SEQ ID NOS: 186, 202, 218, 234, 250, 266, 282 and 298, respectively; wherein the amino acid sequences of the first complementarity determining region (CDR1), the second complementarity determining region (CDR2) and the third complementarity determining region (CDR3) in a light chain variable region of said antibody comprise the same or substantially the same amino acid sequence as the amino acid sequence represented by (iv) a sequence identification number selected from the group consisting of SEQ ID NOS: 192, 208, 224, 240, 256, 272, 288 and 304, (v) a sequence identification number selected from the group consisting of SEQ ID NOS: 193, 209, 225, 241, 257, 273, 289 and 305, and (vi) a sequence identification number selected from the group consisting of SEQ ID NOS: 194, 210, 226, 242, 258, 274, 290 and 306, respectively; and, a constant region of said antibody.

**44.** A hybridoma cell represented by Nec1-1044-4 (FERM BP-10805), Nec8-3517-11 (FERM BP-10806) or Nec8-3 704-7 (FERM BP-10807).

**45.** A monoclonal antibody produced by the hybridoma cell according to claim 44.

**46.** A monoclonal antibody binding competitively with a monoclonal antibody produced from the hybridoma cell represented by Nec1-1044-4 (FERM BP-10805), Nec8-3517-11 (FERM BP-10806) or Nec8-3 704-7 (FERM BP-10807).

**47.** An agent for preventing or treating breast cancer which comprises the monoclonal antibody according to claim 45 or 46.

TABLE 18

| Clone Name | Nec9-211-2 | Nec10-1953-2 | Nec1-109-1 | Nec1-141-3 | Nec1-209-3 | Nec1-803-2 | Nec9-1502-2 | Nec10-1666-1 | Nec10-1707-3 | Nec10-1861-1 | Nec10-2005-1 | Nec10-2439-2 |
|---|---|---|---|---|---|---|---|---|---|---|---|---|
| Nectin-2ED-Fc | 100 | 100 | 100 | 100 | 100 | 100 | 100 | 100 | 100 | 100 | 100 | 100 |
| Q37A | 95 | 99 | 102 | 107 | 117 | 96 | 91 | 100 | 99 | 91 | 87 | 89 |
| Q45A | 88 | 92 | 94 | 87 | 99 | 116 | 77 | 99 | 93 | 77 | 94 | 86 |
| H55A | 82 | 100 | 88 | 91 | 96 | 112 | 61 | 96 | 93 | 70 | 88 | 69 |
| V60A | 90 | 103 | 103 | 77 | 111 | 126 | 91 | 99 | 105 | 85 | 103 | 94 |
| Y64A | 87 | 78 | 96 | 80 | 104 | 122 | 91 | 97 | 101 | 83 | 100 | 94 |
| Q71A | 88 | 96 | 98 | 75 | 100 | 117 | 88 | 98 | 95 | 91 | 105 | 96 |
| N78A | 90 | 95 | 63 | 89 | 23 | 79 | 23 | 66 | 44 | 16 | 37 | 26 |
| K88A | 86 | 92 | 97 | 99 | 94 | 115 | 98 | 105 | 118 | 98 | 105 | 96 |
| S95A | 106 | 103 | 117 | 108 | 105 | 138 | 100 | 109 | 92 | 101 | 85 | 82 |
| K109A | 98 | 105 | 119 | 98 | 113 | 137 | 88 | 111 | 112 | 105 | 102 | 103 |
| E117A | 90 | 100 | 106 | 114 | 106 | 126 | 95 | 103 | 101 | 101 | 102 | 96 |
| D122A | 93 | 102 | 103 | 96 | 102 | 126 | 99 | 103 | 107 | 87 | 102 | 95 |
| H128A | 94 | 98 | 102 | 99 | 100 | 120 | 91 | 100 | 98 | 83 | 101 | 95 |
| F145A | -7 | -3 | 134 | 158 | 136 | 147 | 135 | 131 | 143 | 140 | 139 | 123 |
| K147A | 90 | 82 | 102 | 103 | 97 | 117 | 92 | 100 | 94 | 80 | 98 | 82 |
| M153A | 102 | 102 | 101 | 178 | 90 | 120 | 106 | 104 | 104 | 100 | 97 | 84 |

| Clone Name | Nec9-211-2 | Nec10-1953-2 | Nec1-141-3 | Nec1-520-1 | Nec1-530-1 | Nec1-803-2 | Nec1-845-2 | Nec1-903-1 | Nec10-1666-1 | Nec10-1707-3 | Nec10-2005-1 | Nec1-209-3 | Nec1-244-3 | Nec1-834-1 | Nec9-1502-2 | Nec10-1861-1 | Nec10-2439-2 |
|---|---|---|---|---|---|---|---|---|---|---|---|---|---|---|---|---|---|
| Nectin-2ED-Fc | 100 | 100 | 100 | 100 | 100 | 100 | 100 | 100 | 100 | 100 | 100 | 100 | 100 | 100 | 100 | 100 | 100 |
| P40G | 113 | 115 | 107 | 130 | 138 | 136 | 146 | 138 | 101 | 132 | 121 | 113 | 121 | 123 | 107 | 116 | 127 |
| A75G | 118 | 120 | 59 | 80 | 73 | 85 | 78 | 92 | 77 | 70 | 56 | 62 | 7 | 45 | 51 | 16 | 49 |
| P76G | 114 | 111 | 80 | -2 | -6 | -9 | -13 | 4 | -4 | -10 | -6 | -1 | -18 | -9 | -2 | -28 | -14 |
| A77G | 102 | 113 | 161 | 99 | 103 | 116 | 102 | 98 | 91 | 91 | 82 | 102 | 113 | 123 | 90 | 104 | 75 |
| N78A | 104 | 114 | 127 | 57 | 68 | 84 | 59 | 73 | 84 | 63 | 49 | 35 | 12 | 37 | 29 | 29 | 35 |
| H79A | 114 | 129 | 142 | 145 | 135 | 165 | 167 | 150 | 114 | 152 | 162 | 104 | 141 | 127 | 118 | 133 | 142 |
| Q80A | 110 | 121 | 134 | 139 | 134 | 157 | 151 | 141 | 116 | 126 | 132 | 119 | 133 | 145 | 110 | 129 | 126 |
| N81A | 109 | 136 | 195 | 155 | 150 | 185 | 188 | 147 | 126 | 174 | 179 | 131 | 195 | 191 | 133 | 174 | 180 |
| N137A | 124 | 122 | 285 | 114 | 91 | 127 | 112 | 121 | 99 | 110 | 105 | 86 | 60 | 32 | 69 | 68 | 80 |
| F145A | -1 | -2 | 270 | 134 | 138 | 165 | 141 | 124 | 120 | 163 | 176 | 139 | 187 | 182 | 132 | 182 | 168 |
| V150A | 109 | 98 | 183 | 161 | 146 | 185 | 179 | 166 | 127 | 157 | 159 | 133 | 173 | 182 | 131 | 175 | 163 |
| T154A | 120 | 119 | 35 | 160 | 136 | 170 | 160 | 160 | 119 | 153 | 155 | 123 | 132 | 146 | 120 | 156 | 167 |

FIG. 1

Heavy Chain

```
Nec1-244-3H.pro   QVQLQESGPGLVKPSETLSLICTVSGGSISSYYWSWIRQPPGKGLEWIGYIYYSGSTNHNPSLKS-RVTISVDTAKNQFSLKLNSVTAADTAVYYCARDGGDDYNYGM------DVWGQGTTVTVSSA
Nec1-530-1H.pro   QVQLQESGPGLVKPSETLSLTCTVSGGSISSYYWTWIRQPPGKGLEWIGYVYYSGSTNYNPSLKS-RVTISVDTSKSQFSLKLSSVTAADTAVYYCTRDPGEDYYYGM------DVWGQGTTVTVSSA
Nec1-554-1H.pro   EVQLVESGGGLVKPGGSLRLSCAASGFTFSSYNMNWVRQAPGKGLEWVSSISSSSSYIYYADSVKGRFTISRDNAKNSLYLQMNSLRAEDTAVYYCARD-----YYGSGTYYLFDYWGQGTLVTVSSA
Nec1-803-2H.pro   QVQLQESGPGLVKPSETLSLTCTVSGGSISSYYWTWIRQPPGKGLEWIGYIYYSGSTNSNPSLKS-RVTISVDTSKNQFSLKLSSVTAADTAVYYCTRDPGEDYNYGM------DVWGQGTTVTVSSA
Nec1-834-1H.pro   QVQLQESGPGLVKPSETLSLTCTVSSASISSYYWSWIRQPPGKGLEWIGYIYYSGSTNYNPSLKS-RVTISIDTSKNQFSLKLSSVTAADTAVYYCARDAGEDYSYGM------DVWGQGTTVTVSSA
Nec1-845-2H.pro   QVQLQESGPGLVKPSETLSLTCTVSGGSISSYYWTWIRQPPGKGLEWIGYIYYSGSTNYNPSLKS-RVTISVDTSKNQFSLKLSSVTAADTAVYYCTRDPGEDYNYGM------DVWGQGTTVTVSSA
Nec1-903-1H.pro   QVQLQESGPGLVKPSETLSLTCTVSGGSISSYXWTWIRQPPGKGLEWIGYIYYSGSTNYNPSLKS-RVTISLDTSKNQFSLKLSSVTAADTAVYYCTRDPGEDYNYGM------DVWGQGTTVTVSSA
Nec8-4116-8H.pro  QVQLQESGPGLVKPSETLSLTCTVSGGSIRSYYWTWIRQPPGKGLEWIGYIFYSGSTNYNPSLKS-RVTISVDTSKNQCSLKLTSVTAADTAVYYCAKG-----IAGM------DVWGQGTTVTVSSA
```

CDR1 CDR2 CDR3

Light Chain

```
Nec1-244-3L.pro   AIQLTQSPSSLSASVGDRVTITCRASQGISSX-LAWYQQKPGKAPKXLIYDASSLXSGXPSRFSGSGSGTDFTLTIISLQPEDFATYYCQQXNSYPXTFGQGTKVEIK
Nec1-530-1L.pro   AIQLTQSPSSLSASVGDRVTITCRASQGISSX-LAWYQQKPGKAPKLLIYDASSLESGVPSRFSGSGSGTDFTLTISSLQPEDFATYYCQQFNSYPRTFGQGTKVEIK
Nec1-554-1L.pro   EIVLTQSPDFQSVTPKEKVTITCRASQSIGSS-LHWYQQKPDQSPKLLIKYASQSFSGVPSRFSGSGSGTDFTLTINSLEAEDAAAYYCHQSRSLPITFGQGTRLEIK
Nec1-803-2L.pro   AIQLTQSPSSLSASVGDRVTITCRASQGISSA-LAWYQQKPGKAPKLLIYDASSLESGVPSRFSGSGSGTDFTLTISSLQPEDFATYYCQQFNSYPRTFGQGTKVEIK
Nec1-834-1L.pro   AIQLTQSPSSLSASVGDRVTITCRASQGISSA-LAWYQQKPGKAPKLLIYDASSLESGVPSRFSGSGSGTDFTLTISSLQPEDFATYYCQQFNSY-RTFGQGTKVEIK
Nec1-845-2L.pro   AIQLTQSPSSLSASVGDRVTITCRASQGISSA-LAWYQQKPGKAPKLLIYDASSLESGVPSRFSGSGSGTDFTLTISSLQPEDFATYYCQQFNSYPRTFGQGTKVEIK
Nec1-903-1L.pro   AIQLTQSPSSLSASVGDRVTITCRASQGISSA-LAWYQQKPGKAPKLLIYDASSLESGVPSRFSGSGSGTDFTLTISSLQPEDFATYYCQQFNSYPRTFGQGTKVEIK
Nec8-4116-8L.pro  EIVLTQSPGTLSLSPGERATLSCRASQSVSSSYLAWYQQKPGQAPRLLIYGASSRATGIPDRFSGSGSGTDFTLTISRLEPEDFAVYYCQQYGSSPYTFGQGTKLEIK
```

CDR1 CDR2 CDR3

FIG.2

## Heavy Chain

```
Necl-244-3H.seq    CAGGTGCAGCTGCAGGAGTCGGGCCCAGGACTGGTGAAGCCTTCGGAGACCCTGTCCCTCATCTGCACTGTCTCTGGTGGCTCCATCAGTAGTTACTACT
Necl-530-1H.seq    CAGGTGCAGCTGCAGGAGTCGGGCCCAGGACTGGTGAAGCCTTCGGAGACCCTGTCCCTCACCTGCACTGTCTCTGGTGGCTCCATCAGTAGTTACTACT
Necl-554-1H.seq    GAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAACA
Necl-803-2H.seq    CAGGTGCAGCTGCAGGAGTCGGGCCCAGGACTGGTGAAGCCTTCGGAGACCTTGTCCCTCACCTGCACTGTCTCTGGTGGCTCCATCAGTAGTTACTACT
Necl-834-1H.seq    CAGGTGCAGCTGCAGGAGTCGGGCCCAGGACTGGTGAAGCCTTCGGAGACCCTGTCCCTCACCTGCACTGTCTCTAGTGCCTCCATCAGTAGTTACTACT
Necl-845-2H.seq    CAGGTGCAGCTGCAGGAGTCGGGCCCAGGACTGGTGAAGCCTTCGGAGACCTTGTCCCTCACCTGCACTGTCTCTGGTGGCTCCATCAGTAGTTACTACT
Necl-903-1H.seq    CAGGTGCAGCTGCAGGAGTCGGGCCCAGGACTGGTGAAGCCTTCGGAGACCTTGTCCCTCACCTGCACTGTCTCTGGTGGCTCCATCAGTAGTTACWACT
Nec8-4116-8H.seq   CAGGTGCAACTGCAGGAGTCGGGCCCAGGACTGGTGAAGCCTTCGGAGACCCTGTCCCTCACCTGCACTGTCTCTGGTGGCTCCATCAGAAGTTACTATT

Necl-244-3H.seq    GGAGCTGGATCCGGCAGCCCCCAGGGAAGGGACTGGAGTGGATTGGGTATATCTATTACAGTGGGAGCACCAACCACAACCC---CTCCCTCAAGAGTCG
Necl-530-1H.seq    GGACCTGGATCCGGCAGCCCCCAGGGAAGGGACTGGAGTGGATTGGATATGTCTATTACAGTGGGAGCACCAACTACAACCC---CTCCCTCAAGAGTCG
Necl-554-1H.seq    TGAACTGGGTCCGCCAGGCTCCAGGGAAGGGGCTGGAGTGGGTCTCATCCATTAGTAGTAGTAGTAGTTACATATACTACGCAGACTCAGTGAAGGGCCG
Necl-803-2H.seq    GGACCTGGATCCGGCAGCCCCCAGGGAAGGGACTGGAGTGGATTGGGTATATCTATTACAGTGGGAGCACCAACTCCAACCC---CTCCCTCAAGAGTCG
Necl-834-1H.seq    GGAGCTGGATCCGGCAGCCCCCAGGGAAGGGACTGGAGTGGATTGGGTATATCTATTACAGTGGGAGCACCAACTACAACCC---CTCCCTCAAGAGTCG
Necl-845-2H.seq    GGACCTGGATCCGGCAGCCCCCAGGGAAGGGACTGGAGTGGATTGGGTATATCTATTACAGTGGGAGCACCAACTACAACCC---CTCCCTCAAGAGTCG
Necl-903-1H.seq    GGACCTGGATCCGGCAGCCCCCAGGGAAGGGACTGGAGTGGATTGGGTATATCTATTACAGTGGGAGCACCAACTACAACCC---CTCCCTCAAGAGTCG
Nec8-4116-8H.seq   GGACCTGGATCCGGCAGCCCCCAGGGAAGGGACTGGAGTGGATTGGGTATATCTTTTACAGTGGGAGCACCAACTACAACCC---CTCCCTCAAGAGTCG

Necl-244-3H.seq    AGTCACCATATCAGTAGACACGGCCAAGAACCAGTTCTCCCTGAAGCTGAACTCTGTGACCGCTGCGGACACGGCCGTGTATTACTGTGCGAGAGATG-G
Necl-530-1H.seq    AGTCACCATATCAGTAGACACGTCCAAGAGCCAGTTCTCCCTGAAGCTGAGCTCTGTGACCGCTGCGGACACGGCCGTGTATTACTGTACGAGAGACC-C
Necl-554-1H.seq    ATTCACCATCTCCAGAGACAACGCCAAGAACTCACTGTATCTGCAAATGAACAGCCTGAGAGCCGAGGACACGGCTGTGTATTACTGTGCGAGAGATTAC
Necl-803-2H.seq    AGTCACCATATCAGTAGACACGTCCAAGAACCAGTTCTCCCTGAAGCTGAGTTCTGTGACCGCTGCGGACACGGCCGTGTATTACTGTACGAGAGACC-C
Necl-834-1H.seq    AGTCACCATATCAATAGACACGTCCAAGAACCAGTTCTCCCTGAAGCTGAGCTCTGTGACCGCTGCGGACACGGCCGTGTATTACTGTGCGAGAGATG-C
Necl-845-2H.seq    AGTCACCATATCAGTAGACACGTCCAAGAACCAGTTCTCCCTGAAGCTGAGTTCTGTGACCGCTGCGGACACGGCCGTGTATTACTGTACGAGAGACC-C
Necl-903-1H.seq    AGTCACCATATCATTAGACACGTCCAAGAACCAGTTCTCCCTGAAGCTGAGTTCTGTGACCGCTGCGGACACGGCCGTGTATTACTGTACGAGAGACC-C
Nec8-4116-8H.seq   AGTCACCATATCAGTAGACACGTCCAAGAACCAGTGCTCCCTGAAGCTGACCTCTGTGACCGCTGCGGACACGGCCGTGTATTACTGTGCGAAAGG----

Necl-244-3H.seq    TGGGGA--CGACTACAACTACGGTATGGACGTCTGGGGCCAAGGGACCACGGTCACCGTCTCCTCAGCC
Necl-530-1H.seq    TGGGGA--AGACTACTACTACGGTATGGACGTCTGGGGCCAAGGGACCACGGTCACCGTCTCCTCAGCC
Necl-554-1H.seq    TATGGTTCGGGGACTTATTATCTCTTTGACTACTGGGGCCAGGGAACCCTGGTCACCGTCTCCTCAGCC
Necl-803-2H.seq    TGGGGA--AGACTACAACTACGGTATGGACGTCTGGGGCCAAGGGACCACGGTCACCGTCTCCTCAGCC
Necl-834-1H.seq    TGGGGA--GGACTACTCCTACGGTATGGACGTCTGGGGCCAAGGGACCACGGTCACCGTCTCCTCAGCC
Necl-845-2H.seq    TGGGGA--AGACTACAACTACGGTATGGACGTCTGGGGCCAAGGGACCACGGTCACCGTCTCCTCAGCC
Necl-903-1H.seq    TGGGGA--AGACTACAACTACGGTATGGACGTCTGGGGCCAAGGGACCACGGTCACCGTCTCCTCAGCC
Nec8-4116-8H.seq   -----------TATAGC-A-GGTATGGACGTCTGGGGCCAAGGGACCACGGTCACCGTCTCCTCAGCC
```

CDR1

CDR2

CDR3

FIG.3

## Light Chain

```
Necl-244-3L.seq    GCCATCCAGTTGACCCAGTCTCCATCCTCCCTGTCTGCATCTGTAGGAGACAGAGTCACCATCACTTGYCGGGCRAGTCAGGGCATTAGCAGY-GSK--T
Necl-530-1L.seq    GCCATCCAGTTGACCCAGTCTCCATCCTCCCTGTCTGCATCTGTAGGAGACAGAGTCACCATCACTTGCCGGGCAAGTCAGGGCATTAGCAGT-GST--T
Necl-554-1L.seq    GAAATTGTGCTGACTCAGTCTCCAGACTTTCAGTCTGTGACTCCAAAGGAGAAAGTCACCATCACCTGCCGGGCCAGTCAGAGCATTGGTAGTAGCT---
Necl-803-2L.seq    GCCATCCAGTTGACCCAGTCTCCATCCTCCCTGTCTGCATCTGTAGGAGACAGAGTCACCATCACTTGCCGGGCAAGTCAGGGCATTAGCAGT-GCT--T
Necl-834-1L.seq    GCCATCCAGTTGACCCAGTCTCCATCCTCCCTGTCTGCATCTGTAGGAGACAGAGTCACCATCACTTGCCGGGCAAGTCAGGGCATTAGCAGT-GCT--T   CDR1
Necl-845-2L.seq    GCCATCCAGTTGACCCAGTCTCCATCCTCCCTGTCTGCATCTGTAGGAGACAGAGTCACCATCACTTGCCGGGCRAGTCAGGGYATTAGCAGY-GCT--T
Necl-903-1L.seq    GCCATCCAGTTGACCCAGTCTCCATCCTCCCTGTCTGCATCTGTAGGAGACAGAGTCACCATCACTTGCCGGGCAAGTCAGGGCATTAGCAGT-GCT--T
Nec8-4116-8L.seq   GAAATTGTGTTGACGCAGTCTCCAGGCACCCTGTCTTTGTCTCCAGGGGAAAGAGCCACCCTCTCCTGCAGGGCCAGTCAGAGTGTTAGCAGCAGCTACT

Necl-244-3L.seq    TAGCCTGGTATCAGCAGAAACCAGGGAAAGCTCCTAAGYTCCTGATCTATGATGCMTCCAGTTTGSAAAGTGGGRTCCCATCAAGGTTCAGCGGCAGTGG
Necl-530-1L.seq    TAGCCTGGTATCAGCAGAAACCAGGGAAAGCTCCTAAGCTCCTGATCTATGATGCCTCCAGTTTGGAAAGTGGGGTCCCATCAAGGTTCAGCGGCAGTGG
Necl-554-1L.seq    TACACTGGTACCAGCAGAAACCAGATCAGTCTCCAAAGCTCCTCATCAAGTATGCTTCCCAGTCCTTCTCAGGGGTCCCCTCGAGGTTCAGTGGCAGTGG
Necl-803-2L.seq    TAGCCTGGTATCAGCAGAAACCAGGGAAAGCTCCTAAGCTCCTGATCTATGATGCCTCCAGTTTGGAAAGTGGGGTCCCATCAAGGTTCAGCGGCAGTGG
Necl-834-1L.seq    TAGCCTGGTATCAGCAGAAACCAGGGAAAGCTCCTAAGCTCCTGATCTATGATGCCTCCAGTTTGGAAAGTGGGGTCCCATCAAGGTTCAGCGGCAGTGG   CDR2
Necl-845-2L.seq    TAGCCTGGTATCAGCAGAAACCAGGGAAAGCTCCTAAGCTCCTGATCTATGATGCCTCCAGTTTGGAAAGTGGGGTCCCATCAAGGTTCAGCGGCAGTGG
Necl-903-1L.seq    TAGCCTGGTATCAGCAGAAACCAGGGAAAGCTCCTAAGCTCCTGATCTATGATGCCTCCAGTTTGGAAAGTGGGGTCCCATCAAGGTTCAGCGGCAGTGG
Nec8-4116-8L.seq   TAGCCTGGTACCAGCAGAAACCTGGCCAGGCTCCCAGGCTCCTCATCTATGGTGCATCCAGCAGGGCCACTGGCATCCCAGACAGGTTCAGTGGCAGTGG

Necl-244-3L.seq    ATCTGGGACAGATTTCACTCTCACCATCATCAGCCTGCAGCCTGAAGATTTTGCAACTTATTACTGTCAACAGTWTAATAGTTACCCTYGGACGTTCGGC
Necl-530-1L.seq    ATCTGGGACAGATTTCACTCTCACCATCAGCAGCCTGCAGCCTGAAGATTTTGCAACTTATTACTGTCAACAGTTTAATAGTTACCCTCGGACGTTCGGC
Necl-554-1L.seq    ATCTGGGACAGATTTCACCCTCACCATCAATAGCCTGGAAGCTGAAGATGCTGCAGCGTATTACTGTCATCAGAGTAGGAGTTTACCGATCACCTTCGGC
Necl-803-2L.seq    ATCTGGGACAGATTTCACTCTCACCATCAGCAGCCTGCAGCCTGAAGATTTTGCAACTTATTACTGTCAACAGTTTAATAGTTACCCTCGGACGTTCGGC
Necl-834-1L.seq    ATCTGGGACAGATTTCACTCTCACCATCAGCAGCCTGCAGCCTGAAGATTTTGCAACTTATTACTGTCAACAGTTTAATAGTTAC---CGGACGTTCGGC   CDR3
Necl-845-2L.seq    ATCTGGGACAGATTTCACTCTCACCATCAGCAGCCTGCAGCCTGAAGATTTTGCAACTTATTACTGTCAACAGTTTAATAGTTACCCTCGGACGTTCGGC
Necl-903-1L.seq    ATCTGGGACAGATTTCACTCTCACCATCAGCAGCCTGCAGCCTGAAGATTTTGCAACTTATTACTGTCAACAGTTTAATAGTTACCCTCGGACGTTCGGC
Nec8-4116-8L.seq   GTCTGGGACAGACTTCACTCTCACCATCAGCAGACTGGAGCCTGAAGATTTTGCAGTGTATTACTGTCAGCAGTATGGTAGCTCACCGTACACTTTGGC

Necl-244-3L.seq    CAAGGGACCAAGGTGGAAATCAAA
Necl-530-1L.seq    CAAGGGACCAAGGTGGAAATCAAA
Necl-554-1L.seq    CAAGGGACACGACTGGAGATTAAA
Necl-803-2L.seq    CAAGGGACCAAGGTGGAAATCAAA
Necl-834-1L.seq    CAAGGGACCAAGGTGGAAATCAAA
Necl-845-2L.seq    CAAGGGACCAAGGTGGAAATCAAA
Necl-903-1L.seq    CAAGGGACCAAGGTGGAAATCAAA
Nec8-4116-8L.seq   CAGGGGACCAAGCTGGAGATCAAA
```

FIG4

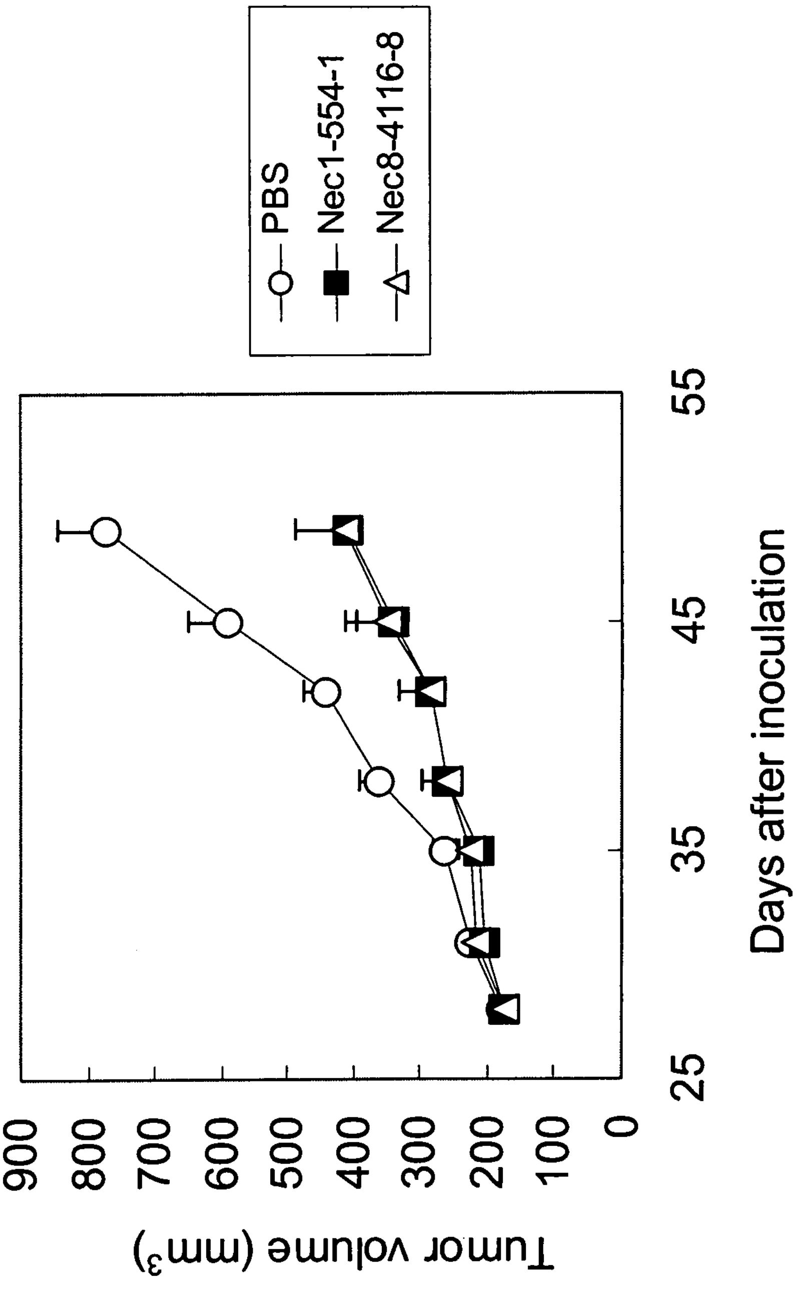
Tumor volume (mm3)
0
100
200
300
400
500
600
700
800
900
Days after inoculation
25
35
45
55
PBS
Nec1-554-1
Nec8-4116-8

**INTERNATIONAL SEARCH REPORT**

International application No.
PCT/JP2007/069908

A. CLASSIFICATION OF SUBJECT MATTER
*C07K16/32*(2006.01)i, *A61K39/395*(2006.01)i, *A61P35/00*(2006.01)i, *C07K16/18*(2006.01)i, *C12N5/10*(2006.01)i, *C12N15/02*(2006.01)i, *C12P21/08*(2006.01)i

According to International Patent Classification (IPC) or to both national classification and IPC

B. FIELDS SEARCHED

Minimum documentation searched (classification system followed by classification symbols)
C07K16/32, A61K39/395, A61P35/00, C07K16/18, C12N5/10, C12N15/02, C12P21/08

Documentation searched other than minimum documentation to the extent that such documents are included in the fields searched

Electronic data base consulted during the international search (name of data base and, where practicable, search terms used)
BIOSIS/MEDLINE/WPIDS(STN), JMEDPlus(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X/Y | WO 2005/097204 A1 (Takeda Chemical Industries, Ltd.), 20 October, 2005 (20.10.05), Full text & EP 1733743 A1 & CA 2561947 A1 | 1,2,7,10-12, 15,21-26, 32-34/3-6,8, 9,13,14, 16-20,27, 35-47 |
| Y | EBERLE F. ET AL, The human PRR2 gene, related to the human poliovirus receptor gene (PVR), is the true homolog of the murine MPH gene., Gene, 1995, Vol.159, No.2, p.267-272, full text | 1-27,32-47 |
| Y | PENDE D. ET AL, PVR (CD155) and Nectin-2 (CD112) as ligands of the human DNAM-1 (CD226) activating receptor: involvement in tumor cell lysis., Mol. Immunol., 2005, Vol.42, No.4, p.463-469, full text | 1-27,32-47 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 October, 2007 (23.10.07) | 06 November, 2007 (06.11.07) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/069908

C (Continuation). DOCUMENTS CONSIDERED TO BE RELEVANT

| Category* | Citation of document, with indication, where appropriate, of the relevant passages | Relevant to claim No. |
|---|---|---|
| Y | TAHARA-HANAOKA S. ET AL, Functional characterization of DNAM-1 (CD226) interaction with its ligands PVR (CD155) and nectin-2 (PRR-2/CD112)., Int. Immunol., 2004, Vol.16, No.4, p.533-538, full text | 1-27,32-47 |

Form PCT/ISA/210 (continuation of second sheet) (April 2007)

**INTERNATIONAL SEARCH REPORT**

International application No.
PCT/JP2007/069908

**Box No. II Observations where certain claims were found unsearchable (Continuation of item 2 of first sheet)**

This international search report has not been established in respect of certain claims under Article 17(2)(a) for the following reasons:

1. [X] Claims Nos.: 28-31
because they relate to subject matter not required to be searched by this Authority, namely:
The inventions as set forth in the above claims involve methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0299040 A
- WO 0228902 A
- WO 9963063 A
- WO 0200677 A
- WO 03088808 A
- WO 04030615 A
- WO 2005097204 A
- EP 0125023 A
- EP 0546073 A
- WO 02098217 A
- WO 03020743 A
- JP 2006320429 W
- WO 0029442 A

### Non-patent literature cited in the description


- *J. Biol. Chem.,* 2004, vol. 279 (17), 18015, 18025
- *Protein, Nucleic Acid and Enzyme,* 2003, vol. 48 (2), 105, 112
- *Curr. Biol.,* 2002, vol. 12, 1145, 1150
- *J. Cell Biol.,* 2002, vol. 156, 555, 565
- *J. Biol. Chem.,* 2002, vol. 277 (30), 27006, 27013
- *J. Cell Sci.,* 2003, vol. 116 (1), 17, 27
- *J. Exp. Med.,* 2003, vol. 198 (4), 557, 567
- *Blood,* 1998, vol. 92 (12), 4602, 4611
- *J. Virol.,* 2000, vol. 74 (3), 1267, 1274
- *Intl. Immunol.,* 2004, vol. 16 (4), 533, 538
- *Mol. Immunol.,* 2005, vol. 42, 463, 469
- *JEM,* 2003, vol. 198 (4), 557, 567
- *Virol.,* 1998, vol. 246, 179, 189
- *J. Virol.,* 2001, vol. 75 (2), 11185, 11195
- *FEBS,* 2005, vol. 579, 2243-2249
- *JBC,* 2001, vol. 276, 48350, 48355
- *Oncogene,* 1999, vol. 18, 1609, 1617
- *JCB,* 1999, vol. 145, 539, 549
- *Exp. Cell Res.,* 1997, vol. 235, 374, 384
- **J. Sambrook et al.** Molecular Cloning. Cold Spring Harbor Lab. Press, 1989
- *Proc. Natl. Acad. Sci. U.S.A.,* 1968, vol. 60, 160
- **Nucleic Acids Research.** *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **Vaughn, J. L. et al.** *In Vivo,* 1977, vol. 13, 213-217
- **Maeda et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Enzymology,* 1991, vol. 194, 182-187
- *Proc. Natl. Acad. Sci. U.S.A.,* 1978, vol. 75, 1929
- *Bio/Technology,* 1988, vol. 6, 47-55
- New Cell Engineering Experimental Protocol. Shujunsha, 1995, 263-267
- *Virology,* 1973, vol. 52, 456
- **Miller.** Journal of Experiments in Molecular Genetics. Cold Spring Harbor Laboratory, 1972, 431-433
- **Bostian, K. L. et al.** *Proc. Natl. Acad. Sci. U.S.A.,* 1980, vol. 77, 4505
- **Bitter, G A. et al.** *Proc. Natl. Acad. Sci. U.S.A.,* 1984, vol. 81, 5330
- *Nature,* 1962, vol. 195, 788
- *Science,* 1952, vol. 122, 501
- *Virology,* vol. 8, 396
- *The Journal of the American Medical Association,* 1967, vol. 199, 519
- *Proceeding of the Society for the Biological Medicine,* 1950, vol. 73, 1
- **M. Bodanszky ; M.A. Ondetti.** Peptide Synthesis. Interscience Publishers, 1966
- **Schroeder ; Luebke.** The Peptide. Academic Press, 1965
- **Nobuo Izumiya et al.** Peptide Gosei-no-Kiso to Jikken (Basics and experiments of peptide synthesis). Maruzen Co, 1975
- **Haruaki Yajima ; Shunpei Sakakibara.** Seikagaku Jikken Koza (Biochemical Experiment) 1, Tanpakushitsu no Kagaku (Chemistry of Proteins). 1977, vol. IV, 205

- Zoku Iyakuhin no Kaihatsu (A sequel to Development of Pharmaceuticals). Hirokawa Shoten, vol. 14
- *Nature,* vol. 356, 152-154
- **Koehler ; Milstein.** *Nature,* 1975, vol. 256, 495
- *Nature Biotechnology,* 2005, vol. 23, 1105
- *Curr. Opin. Biotechnol.,* 1996, vol. 7, 536
- *Nature Rev. Genet.,* 2003, vol. 4, 794
- *Appl. Environ. Microbiol.,* 2004, vol. 70, 2567
- *Protein, Nucleic Acid and Enzyme,* 1992, vol. 37, 2977-2984
- Radioimmunoassay. 1974
- Sequel to the Radioimmunoassay. 1979
- Enzyme immunoassay. 1978
- Enzyme immunoassay. 1982
- Enzyme immunoassay. 1987
- Immunochemical Techniques. *Methods in ENZYMOLOGY,* vol. 70
- Immunochemical Techniques. ¨METHODS IN ENZYMOLOGY. vol. 73
- Immunochemical Techniques. ¨METHODS IN ENZYMOLOGY. vol. 74
- Immunochemical Techniques. ¨METHODS IN ENZYMOLOGY. vol. 84
- Immunochemical Techniques. ¨METHODS IN ENZYMOLOGY. vol. 92
- Immunochemical Techniques. ¨METHODS IN ENZYMOLOGY. Academic Press Publishing, vol. 121